(19) Europäisches Patentamt
European Patent Office
Office européen des brevets

(11) EP 4 428 134 A1

(12) EUROPEAN PATENT APPLICATION

(43) Date of publication:
**11.09.2024 Bulletin 2024/37**

(21) Application number: **23161182.3**

(22) Date of filing: **10.03.2023**

(51) International Patent Classification (IPC):
***C07D 487/04*** (2006.01) ***A61P 35/00*** (2006.01)
***A61P 35/02*** (2006.01) ***A61K 31/498*** (2006.01)

(52) Cooperative Patent Classification (CPC):
**C07D 487/04; A61P 35/00; A61P 35/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dark Blue Therapeutics Ltd**
**Oxford, Oxfordshire OX4 4GB (GB)**

(72) Inventor: **The designation of the inventor has not yet been filed**

(74) Representative: **J A Kemp LLP**
**80 Turnmill Street**
**London EC1M 5QU (GB)**

(54) **PROTAC DEGRADERS OF MLLT1 AND/OR MLLT3**

(57) The invention relates to a compound which is a Proteolysis Targeting Chimera (PROTAC) or a pharmaceutically acceptable salt thereof, wherein the PROTAC has the structure:

M - LINK - U

wherein U is an E3 ubiquitin ligase binding moiety, LINK is a moiety that covalently links M and U, and M is an MLLT1 and/or MLLT3 binder of formula (I):

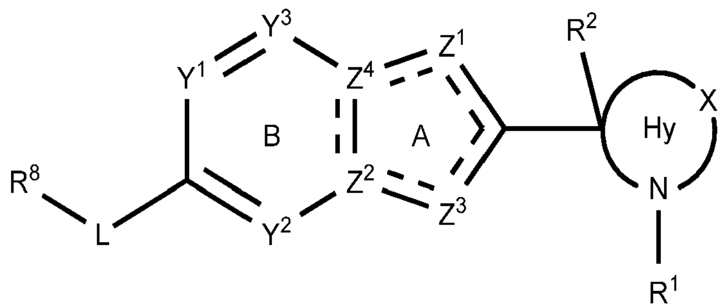

(I)

wherein:
wherein $Z^1$, $Z^2$, $Z^3$, $Z^4$, $Y^1$, $Y^2$, $Y^3$, $R^1$, $R^2$, $R^8$, X, L and Hy are as defined herein, and
either $R^8$ is a bond to LINK, or M is bonded to LINK via a C or N atom within group $R^8$ or ring Hy such that a hydrogen atom on the C or N atom within group $R^8$ or ring Hy is replaced with a bond to LINK. The compounds are useful in the treatment of cancer.

EP 4 428 134 A1

## Description

### Field of the Invention

The present invention relates to compounds that find use in the treatment of cancer by inducing selective degradation of MLLT1 and/or MLLT3. The invention also provides such compounds *per se,* pharmaceutical compositions comprising such compounds, and methods of treating cancer by administering such compounds.

### Background

The processes controlling gene transcription are highly regulated during development and normal homeostasis. Dysregulation of gene transcription is a common driver of cancer with somatic defects in proteins that control gene transcription a frequent occurrence (Cell, 2013, 153, 17; Cell, 2017, 168, 629). Transcriptional elongation is a central step in gene transcription, carried out by the RNA polymerase II (PolII), which itself is kept under tight control by regulatory protein complexes. During development PolII is recruited to sites of the genome proximal to the transcription start site for target genes and is kept in a paused state. Such target genes are often immediate response genes such as heat-shock genes and key developmental genes. Productive elongation is initiated by the coordinated interplay of regulatory protein complexes including the super elongation complex (SEC). The SEC includes multiple proteins with diverse functions including protein phosphorylation, histone reader, histone modification and regulatory activities. Critical to the recruitment of the SEC to target genes are the histone reader proteins MLLT1 (mixed lineage leukaemia translocated to 1, also known as eleven-nineteen leukaemia, ENL) and MLLT3 (mixed lineage leukaemia translocated to 3, also known as AF-9). MLLT1 and 3 contain essential YEATS domains that bind acetylated histones. Mutations in the YEATS domains reduce loading of PolII on to SEC target genes and suppression of gene transcription. (Cell Mol Life Sci 2018, 75, 3931; Nat Rev Mol Cell Biol 2012, 13, 543).

Gain-of-function mutations in the MLLT1 Yeats domain have been causally associated with Wilm's tumor (also known as nephroblastoma) a kidney cancer most commonly observed in children (Nature 2020, 577, 121). Additionally, it has been shown that for certain acute leukaemias MLLT1 represents a critical dependency (Nature 2017, 543, 270). Such leukaemias include mixed-lineage leukaemia (MLL) rearranged leukaemia. MLL rearrangements arise from in frame fusions of the MLL gene with more than 80 different partner genes, many of which are involved in the regulation of transcription elongation including components of the SEC (Front. Pediatr. 5:4. doi: 10.3389/fped.2017.00004). MLL rearrangements are observed in approximately 10% of all acute leukaemias including a high frequency in infantile ALL where it accounts for 70-80% of all cases (Front. Pediatr. 5:4. doi: 10.3389/fped.2017.00004). Notably it is reported that patients with MLL rearranged leukaemias have an especially poor prognosis (New Engl J Med 2016, 374, 2209). Target genes for MLL-SEC complexes include potent oncogenes such as BCL-2, Myc and CDK6 along with many other genes implicated in maintaining cancer cell self-renewal, growth and survival, such as the HOX family genes and MEIS1 (Front. Pediatr. 5:4. doi: 10.3389/fped.2017.00004). Consistent with a role in the regulation of multiple oncogenes, it has been demonstrated that the SEC can play a critical role in the transcriptional addiction of both hematopoietic and solid cancers. For example, data supports potential in non-MLL rearranged leukaemia (Cancer Disc 2022, 12, 2684) and it is reported that with some breast cancer cells, growth and survival is dependent on a SEC mediated transcription of the Myc oncogene (Cell Rep. 2021 Feb 16;34(7):108749. doi: 10.1016/j.celrep.2021.108749. PMID: 33596420; PMCID: PMC8006859).

Taken together the evidence supports MLLT1 as an attractive therapeutic target across acute leukaemias and solid cancers.

Proteolysis targeting chimeras (PROTACs) have been proposed as a small molecule-based platform technology capable of inducing proteolysis of a target protein in the body. The PROTAC is a bifunctional compound in which a molecule that binds to a disease-related target protein and an E3 ubiquitin ligase binding moiety are linked by a chemical linker. Theoretically, the PROTAC compound is capable of inducing degradation of the target protein by placing the disease-related target protein near the E3 ubiquitin ligase.

Drug discovery efforts have resulted in agents that either bind the YEATS domain to block the MLLT1 and/or MLLT3 protein to histone interaction or that result in degradation of the MLLT1 and/or MLLT3 protein (using a PROTAC approach). In all cases however, the reported activity has been weak (ACS Cent Sci 2021, 7, 815; Cancer Disc 2022, 12, 2684; Angew. Chem. Int. Ed. 2018, 57, 16302). Accordingly, there remains a high need to identify potent modulators of MLLT cell function.

### Summary of the Invention

The inventors have discovered a series of compounds that induce selective degradation of MLLT1 and/or MLLT3. Accordingly, the invention provides a compound which is a Proteolysis Targeting Chimera (PROTAC) or a

pharmaceutically acceptable salt thereof, wherein the PROTAC has the structure:

M-LINK-U

wherein U is an E3 ubiquitin ligase binding moiety, LINK is a moiety that covalently links M and U, and M is an MLLT1 and/or MLLT3 binder of formula (I):

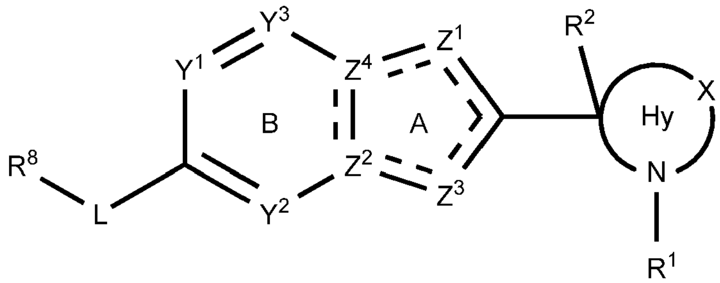

(I)

wherein:

one of $Z^2$ and $Z^4$ is N and the other is C, $Z^1$ is N or -C($R^4$)-, $Z^3$ is -C($R^4$)-, $Y^1$ is N or - C($R^7$)-, $Y^2$ is N or -C($R^6$)-, and $Y^3$ is N or -C($R^5$)-;
Hy is a 4- to 7-membered heterocyclyl ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;
L is -C(O)N(H)-, wherein either (i) the C atom of L is bonded to $R^8$, and the N atom of L is bonded to ring B; or (ii) the C atom of L is bonded to ring B, and the N atom of L is bonded to $R^8$;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$, $R^6$, and $R^7$ are independently selected from H, halo, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
$R^8$ is (i) a bond to LINK or (ii) a group selected from a 6- to 10-membered aryl ring, a 5- to 10-membered heteroaryl ring, a 5- to 10-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
each $R^{11}$ is independently selected from H, $C_{1-4}$ alkyl, and $C_{1-4}$ cycloalkyl;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a preferred embodiment, M is of formula (II):

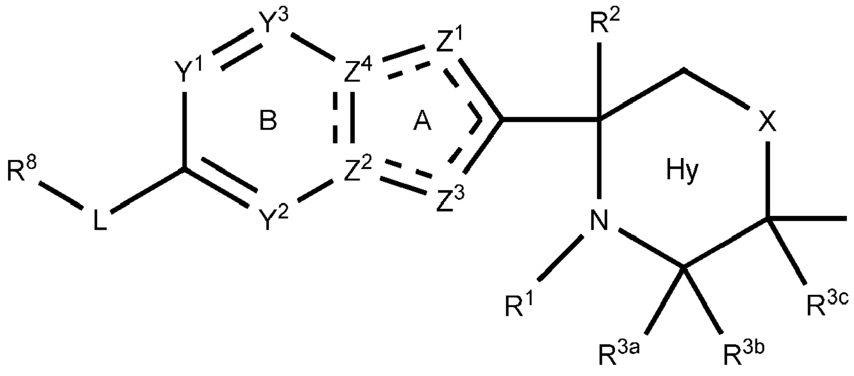

(II)

wherein:

$Z^1$, $Z^2$, $Z^3$, $Z^4$, $Y^1$, $Y^2$, $Y^3$, $R^1$, $R^2$, $R^8$, X and L are as defined above;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, with the proviso that when X is $NR^{11}$, O, or S, then neither $R^{3c}$ nor $R^{3d}$ are halo, or $R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a more preferred embodiment, M is of formula (III):

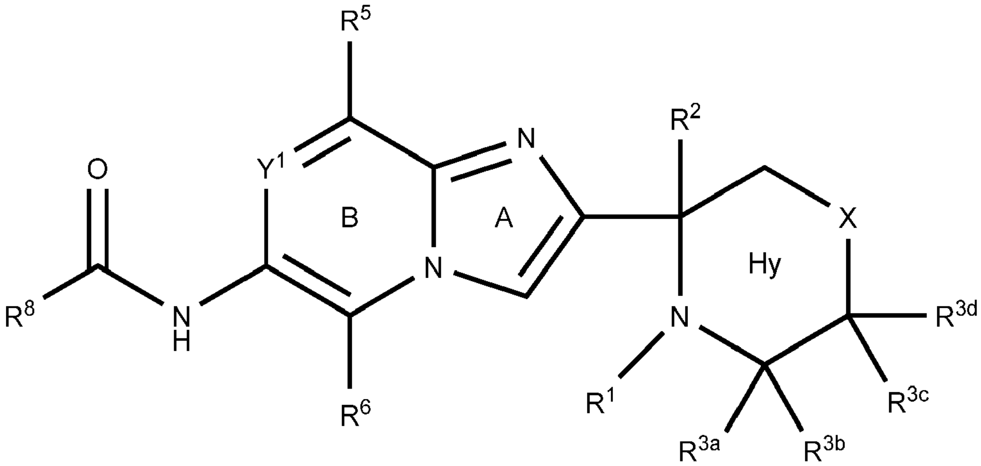

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or $R^{3a}$ and $R^{3c}$ are H, and $R^{3b}$ and $R^{3d}$ form, together with the C atoms to which they are attached, a $C_{5-6}$ cycloalkyl ring;

wherein at least two of $R^{3a}$, $R^{3b}$, $R^{3c}$ and $R^{3d}$ are H;
X is a bond, -N(Me)-, O or $-CH_2-$;
$Y^1$ is N or $-C(R^7)-$;
$R^5$, $R^6$ and $R^7$ are independently selected from H, halo, $C_{1-4}$ alkoxy, and $C_{1-4}$ alkyl;
$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy; and $R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In some embodiments, M is of formula (IIIa):

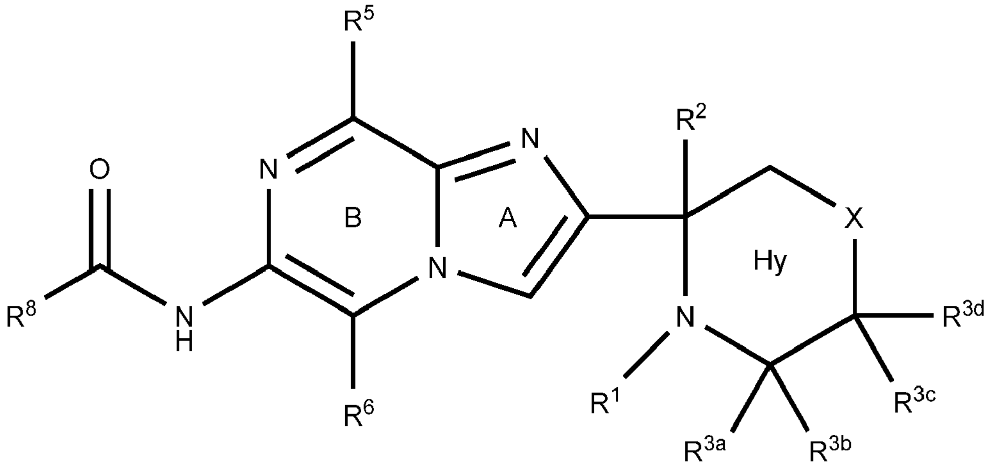

(IIIa)

wherein $R^1$, $R^2$, $R^{3a}$, $R^{3b}$, $R^{3c}$, $R^{3d}$, $R^5$, $R^6$, $R^8$ and X are as defined in formula (III).

In other embodiments, M is of formula (IIIb):

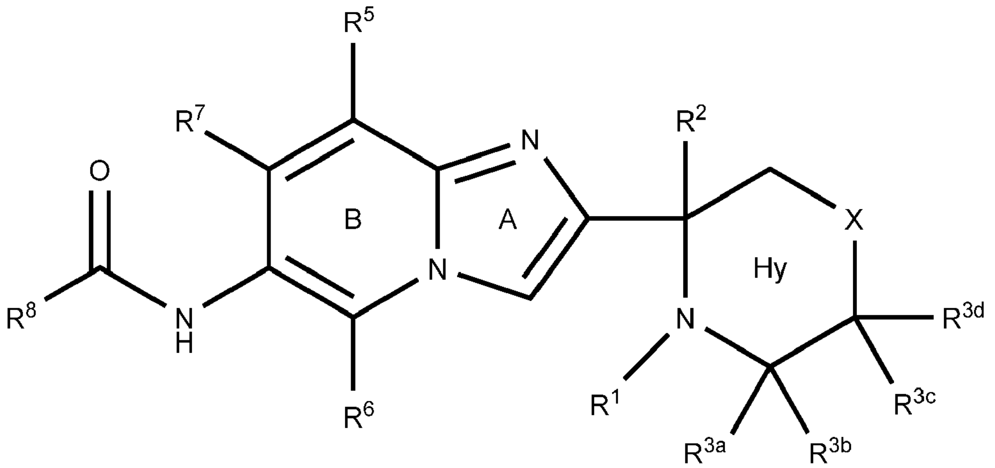

(IIIb)

wherein $R^1$, $R^2$, $R^{3a}$, $R^{3b}$, $R^{3c}$, $R^{3d}$, $R^5$, $R^6$, $R^7$, $R^8$ and X are as defined in formula (III).

In a particularly preferred embodiment, M is of formula (IV):

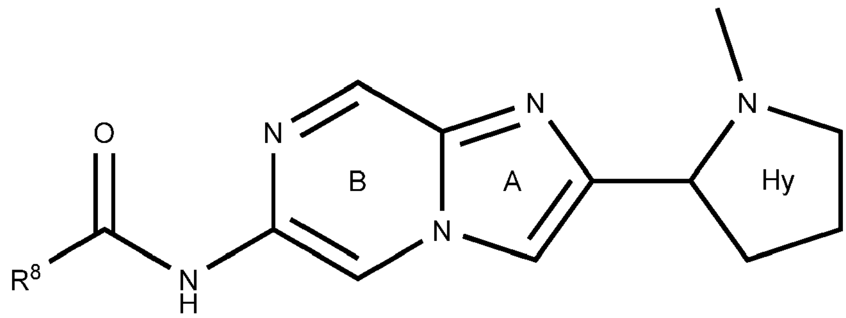

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;

wherein M is bonded to LINK via a C or N atom within group $R^8$ such that a hydrogen atom on the C or N atom within group $R^8$ is replaced with LINK.

In the compounds of the invention, moiety M possesses one N atom in a bridgehead position of the bicyclic heteroaryl ring made up of rings A and B.

The present invention also provides a compound as described herein for use in a method of treating cancer in a subject in need thereof. Also provided is a method for treating cancer in a subject, which method comprises administering to said subject an effective amount of a compound as described herein. Further provided is the use of a compound as described herein in the manufacture of a medicament for use in treating cancer in a subject.

## Brief Description of the Drawings

**Figure 1:** Western blot showing degradation of MLLT1 by compound of Example 95, following procedure described in Example 100.

## Detailed Description of the Invention

*Definitions*

As used herein, a $C_{1-4}$ alkyl group is a linear or branched alkyl group containing from 1 to 4 carbon atoms. A $C_{1-4}$ alkyl group is often a $C_{1-3}$ alkyl group. Examples of $C_{1-4}$ alkyl groups include methyl, ethyl, n-propyl, iso-propyl, n-butyl, sec-butyl, and tert-butyl. A $C_{1-3}$ alkyl group is typically a $C_{1-2}$ alkyl group. A $C_{1-2}$ alkyl group is methyl or ethyl, typically methyl. For the avoidance of doubt, where multiple alkyl groups are present, the alkyl groups may be the same or different.

As used herein, a $C_{1-4}$ alkoxy group is typically a said $C_{1-4}$ alkyl group which is joined to the rest of the molecule via an oxygen atom. Typically, a $C_{1-4}$ alkoxy group is a $C_{1-3}$ alkoxy group. Examples of $C_{1-4}$ alkoxy groups include methoxy, ethoxy, propoxy and butoxy. Typically, a $C_{1-3}$ alkoxy group is a $C_{1-2}$ alkoxy group such as a methoxy or ethoxy group. For the avoidance of doubt, where two alkoxy groups are present, the alkoxy groups may be the same or different.

As used herein, a $C_{1-30}$ alkylene group is a linear or branched divalent alkyl group that contains from 1 to 30 carbon atoms. A $C_{1-30}$ alkylene group is sometimes a $C_{1-20}$ alkylene group, and often a $C_{1-10}$ alkylene group. $C_{1-30}$ alkylene groups, $C_{1-20}$ alkylene groups and $C_{1-10}$ alkylene groups are preferably linear.

A $C_{1-30}$ alkylene group is sometimes a $C_{1-6}$ alkylene group, typically a $C_{1-4}$ alkylene group or a $C_{1-3}$ alkylene group. Examples of $C_{1-4}$ alkylene groups include methylene, ethylene, n-propylene, iso-propylene, n-butylene, sec-butylene, and tert-butylene. A $C_{1-3}$ alkylene group is typically a $C_{1-2}$ alkylene group. A $C_{1-2}$ alkylene group is methylene or ethylene, typically methylene. For the avoidance of doubt, where multiple alkylene groups are present, the alkylene groups may be the same or different.

As used herein, a $C_{2-6}$ alkenylene group is a linear or branched divalent alkenyl group containing from 2 to 6 carbon atoms and having one or more, e.g. one or two, typically one double bonds. Typically a $C_{2-6}$ alkenylene group is a $C_{2-4}$ alkenylene group. Examples of $C_{2-4}$ alkenylene groups include divalent ethenylene, propenylene and butenylene. For the avoidance of doubt, where multiple alkenylene groups are present, the alkenylene groups may be the same or different.

An alkyl, alkoxy, alkylene or alkenylene group as used herein may be unsubstituted or substituted. Unless otherwise stated, substituted alkyl, alkoxy, alkylene or alkenylene groups typically carry one or more, e.g. one, two or

three e.g. one, or two, e.g. one substituent selected from halo, OH, and unsubstituted $C_{1-4}$ alkoxy. Preferred substituents are halo and $C_{1-4}$ alkoxy unless otherwise stated. The substituents on a substituted alkyl, alkoxy, alkylene or alkenylene group are typically themselves unsubstituted. Where more than one substituent is present, these may be the same or different.

As used herein, a halo typically refers to chlorine, fluorine, bromine or iodine, preferably chlorine, bromine or fluorine, more preferably chorine or fluorine, most preferably fluorine unless otherwise stated.

A $C_{3-8}$ cycloalkyl ring, is a cyclic hydrocarbon containing from 3 to 8 carbon atoms. A cycloalkyl ring may be saturated or partially unsaturated, but is typically saturated. A $C_{3-8}$ cycloalkyl ring is typically a $C_{3-6}$ cycloalkyl ring. A $C_{5-6}$ partially unsaturated cycloalkyl ring is a cyclic hydrocarbon containing from 5 to 6 carbon atoms and containing 1 or 2, e.g. 1 double bond. $C_{3-6}$ cycloalkyl and $C_{5-6}$ cycloalkyl rings may also be referred to herein as 3- to 6-membered cycloalkyl rings and 5- to 6-membered cycloalkyl rings respectively.

A $C_{3-6}$ cycloalkyl ring may be a saturated $C_{3-6}$ cycloalkyl ring. A $C_{3-6}$ cycloalkyl ring may be a $C_{5-6}$ cycloalkyl ring, in particular a saturated $C_{5-6}$ cycloalkyl ring. Examples of $C_{3-6}$ cycloalkyl rings are cyclopropyl, cyclobutyl cyclopentyl and cyclohexyl groups.

A $C_{3-8}$ cycloalkyl ring may be a $C_{7-8}$ cycloalkyl ring, in particular a saturated $C_{7-8}$ cycloalkyl ring. Examples of $C_{7-8}$ cycloalkyl rings are cycloheptanly, cyclooctanyl, bicyclo[2.2.1]heptanyl and bicyclo[2.2.2]octanyl groups.

A 5- to 10-membered heterocyclyl ring is a cyclic group containing from 5 to 10 atoms selected from C, O, N and S in the ring, including at least one heteroatom, and typically one or two heteroatoms unless otherwise stated. The heteroatom or heteroatoms are typically selected from O, N, and S, most typically from S and N, especially N. For example, where the heterocyclyl ring is denoted a nitrogen-containing heterocyclyl group, it contains one nitrogen atom and optionally a further heteroatom selected from O, N and S. A heterocyclyl ring may be saturated or partially unsaturated. A 5- to 10-membered partially unsaturated heterocyclyl ring is a cyclic group containing from 5 to 10 atoms selected from C, O, N and S in the ring and containing 1 or 2, e.g. 1 double bond.

A 5- to 10- membered heterocyclyl ring is typically a 4- to 7- membered heterocyclyl ring or a 5- to 6-membered ring. A 4- to 7- membered heterocyclyl ring is typically a monocyclic ring and may be a monocyclic 5- or 6- membered heterocyclyl ring. Alternatively, a 5- to 10- membered heterocyclyl ring may be a 9- or 10- membered fused bicyclic heterocyclyl ring (i.e. a fused heterobicyclic group). In some compounds described herein, a 5- to 10- membered heterocyclyl group is a 4- to 7- membered nitrogen-containing heterocyclyl ring which is unsubstituted or is substituted as described herein. Preferred 4- to 7- membered nitrogen-containing heterocyclyl rings include morpholine, pyrrolidine, piperidine, and piperazine.

Examples of 5- and 6- membered saturated heterocyclyl rings include piperazine, piperidine, morpholine, diazinane and pyrrolidine. Diazinane is typically 1,4-diazinane.

Examples of 9- and 10- membered bicyclic heterocyclyl rings include azaspiro[4.4]nonane and octahydro-1H-indole. Preferably, the bicyclic heterocyclyl ring comprises 1, 2 or 3, preferably 1 or 2 nitrogen atoms.

For the avoidance of doubt, references to a heterocyclyl ring also include fused polycyclic ring systems, including for instance fused bicyclic systems in which a heterocyclyl ring is fused to an aryl group. When the heterocyclyl ring is such a fused heterocyclyl group, preferred examples are fused ring systems wherein a 5- to 6-membered heterocyclyl group is fused to a phenyl group.

As used herein, a 6- to 10-membered aryl ring is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 10- membered heteroaryl ring is a substituted or unsubstituted monocyclic or fused polycyclic aromatic group containing from 5 to 10 atoms in the ring portion, including at least one heteroatom, for example 1, 2 or 3 heteroatoms, typically selected from O, S and N. A heteroaryl ring is typically a 5- or 6-membered heteroaryl ring or a 9- or 10- membered heteroaryl ring. Preferably, the heteroaryl ring comprises 1, 2 or 3, preferably 1 or 2 nitrogen atoms.

Examples of 5- and 6- membered heteroaryl rings include thiazole, pyrazole, pyrimidine, triazole, 1,2,4-oxadiazole and pyrazine. Examples of 8-, 9- and 10-membered heteroaryl rings include indazole, thieno[2,3-c]pyrazole, imidazo[4,5-b]pyridine, pyrazolo[3,4-b]pyridine, furo[2,3-c]pyridine, indole, benzoxazole, benzothiazole, [1,2,4]triazolo[4,3-a]pyridine, thieno[2,3-d]pyrimidine, 1,2,3-benzotriazole, imidazo[1,5-a]pyridine, imidazo[1,2-a]pyrazine, oxazolo[5,4-b]pyridine, quinoline, naphthyridine, isoquinoline, quinazoline, and quinoxaline.

For the avoidance of doubt, references to a heteroaryl ring also include fused polycyclic ring systems, including for instance fused bicyclic systems in which a heteroaryl ring is fused to an aryl group. When the heteroaryl ring is such a fused heteroaryl group, preferred examples are fused ring systems wherein a 5- to 6-membered heteroaryl group is fused to a phenyl group. Indazole is preferred.

As used herein, a fused bicyclic group is a group comprising two cyclic moieties sharing a common bond between two atoms.

A cycloalkyl, heterocyclyl, aryl or heteroaryl ring may be unsubstituted or substituted as described herein unless

otherwise stated. For example, a cycloalkyl, heterocyclyl, aryl or heteroaryl ring may be unsubstituted or substituted with 1, 2 or 3, typically 1 or 2 such as e.g. 1 substituent. Suitable substituents include halo, $C_{1-4}$ alkoxy, $R^{10}$, -($C_{1-4}$ alkylene)-$R^{10}$, =O, -CN, and $C_{1-4}$ alkyl, wherein $R^{10}$ is as defined herein). The substituents on a substituted cycloalkyl, heterocyclyl, aryl or heteroaryl ring are typically themselves unsubstituted, unless otherwise stated.

The compounds described herein comprise at least one heterocyclyl ring comprising at least one nitrogen atom. Said nitrogen atom(s) are independently selected from secondary, tertiary and quaternary nitrogen atom(s). A quaternary nitrogen atom is present when the compound comprises a quaternised derivative of one or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

The compounds described herein comprise a heterocyclyl ring identified as ring Hy, which is a 4- to 7-membered heterocyclyl ring. Ring Hy contains X and at least one N atom in the ring portion, wherein X is a bond, -N($R^{11}$)-, O, S, -S(O)$_2$-, -S(O)(N$R^{11}$)-, or - C($R^{11}$)$_2$-, and $R^{11}$ is as defined herein. Ring Hy is linked to ring A (as identified in formula (I) above) via a C atom within ring Hy, said C atom also being linked to $R^2$ which is as defined herein. A N atom within ring Hy is substituted by $R^1$ which is as defined herein.

The rest of ring Hy is unsubstituted or substituted by one or two $R^3$ (leaving aside the potential substitution of ring Hy by LINK). Each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring, as defined herein. In option (ii), the skilled person would understand that the C atom to which the two $R^3$ are attached is a spiro atom (i.e. the common atom that connects the two rings of a spiro compound).

As used herein, the terms "monovalent" or "monovalent moiety" are used to describe a chemical group obtainable by removing a hydrogen atom from the corresponding compound. As used herein, the terms "divalent" or "divalent moiety" are used to describe a chemical group obtainable by removing a hydrogen atom from the corresponding monovalent moiety. Thus, as used herein, the terms "divalent" and "divalent moiety" are used to describe a chemical group obtainable by removing two hydrogen atoms from the corresponding compound.

In the compounds of the invention, the stereochemistry is not limited. In particular, where moiety M of formula (I) contains one or more chiral centre, the moiety M may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound which is enantiomerically or diasteriomerically pure. Thus, the compound is preferably substantially optically pure.

The moiety M of formula (I) typically contains at least one chiral centre at the carbon atom of ring Hy which is linked to $R^2$ and to ring A. Moiety M may be provided in the form of the R-enantiomer at said carbon atom, in the form of the S-enantiomer at said carbon atom, or in the form of a mixture of the two enantiomers. A pure enantiomeric form of either the R- or the S-enantiomer may be preferred. In some embodiments, the R-enantiomer at said carbon atom is preferred.

As discussed further herein, where the compounds of the invention contain a chiral centre, and in particular where moiety M contains a chiral centre, the individual stereoisomers may be obtained by chiral synthesis, or by separation of stereoisomers. Stereoisomers may be separated, for example, using chiral chromatography. The individual stereoisomers may be identified using the IUPAC labels R and S as appropriate. Alternatively, the individual stereoisomers may be identified by the order in which they elute, for example the first, second, third or fourth stereisomer respectively, as obtained by separation using chiral chromatography. Where two or more stereocentres are present in a compound or moiety, a combination of IUPAC nomenclature for stereocentres where absolute stereochemistry has been defined, and rate of elution, may be used.

For example, where two or more individual stereoisomers are unidentified by IUPAC nomenclature, these stereoisomers may be identified as the fast- and slow-eluting isomers, as obtained by separation using chiral chromatography. Therefore, the stereoisomers at a chiral carbon atom of ring Hy which is linked to $R^2$ and to ring A may be defined as the fast-eluting and slow-eluting stereoisomers, as obtained by separation using chiral chromatography. Chiral chromatography is typically reverse phase HPLC.

The compounds of the present invention may exist in unsolvated as well as solvated forms with pharmaceutically acceptable solvents such as water, ethanol, and the like, and it is intended that the invention embrace both solvated and unsolvated forms.

A compound of the present invention can be converted into a pharmaceutically acceptable salt thereof, and a salt can be converted into the free compound, by conventional methods. For instance, a compound of the present invention can be contacted with a pharmaceutically acceptable acid to form a pharmaceutically acceptable salt. A pharmaceutically acceptable salt is a salt with a pharmaceutically acceptable acid or base.

Pharmaceutically acceptable acids include both inorganic acids such as hydrochloric, sulphuric, phosphoric,

diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p*-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts.

*Compounds of the Invention*

The compounds of the invention comprise a binder of MLLT1 and/or MLLT3 (M) that is covalently attached via a linker moiety (LINK) to an E3 ubiquitin ligase binding moiety (U). The inventors have surpisingly discovered that such compounds may have substantially enhanced efficacy compared with a corresponding MLLT1 and/or MLLT3 inhibitor that is not attached to an E3 ubiquitin ligase binding moiety. For example, the antiproliferative effects of the compounds may be substantially enhanced by covalent attachment to the E3 ubiquitin ligase binding moiety.

Thus, the invention provides a compound which is a Proteolysis Targeting Chimera (PROTAC) or a pharmaceutically acceptable salt thereof, wherein the PROTAC has the structure:

M - LINK - U

wherein U is an E3 ubiquitin ligase binding moiety, LINK is a moiety that covalently links M and U, and M is an MLLT1 and/or MLLT3 binder of formula (I) as defined herein.

In the compounds of the present invention, the MLLT1 and/or MLLT3 binder is covalently attached to the E3 ubiquitin ligase binding moiety via the moiety labelled LINK. LINK is either a single bond or a chemical group that covalently attaches the MLLT1 and/or MLLT3 binder to the E3 ubiquitin ligase binding moiety.

The MLLT1 and/or MLLT3 binder is attached to LINK via (i) a single bond at $R^8$, or (ii) a C or N atom within group $R^8$ or ring Hy (as described herein) such that a hydrogen atom on the C or N atom within group $R^8$ or ring Hy is replaced with a bond to LINK. In case (ii), a hydrogen atom on the C or N atom within group $R^8$ or ring Hy is replaced by either a direct single bond to the E3 ubiquitin ligase binding moiety or by a chemical group that covalently attaches the MLLT1 and/or MLLT3 binder to the E3 ubiquitin ligase binding moiety.

The description herein discusses the substitution of group $R^8$ and ring Hy by various chemical groups aside from LINK. For completeness, the skilled person would readily appreciate that where group $R^8$ and/or ring Hy are defined as being unsubstituted, this is intended as a reference to the absence of substituents other than a bond to LINK. Therefore, such a definition retains the option that group $R^8$ or ring Hy is attached to LINK via a C or N atom within group $R^8$ or ring Hy such that a hydrogen atom on the C or N atom within group $R^8$ or ring Hy is replaced with a bond to LINK. Furthermore, the skilled person would readily appreciate that where group $R^8$ and/or ring Hy are defined as being substituted by one or more chemical groups (suitable such chemical groups are defined herein), this is intended as a reference to substituents other than a bond to LINK. Therefore, such a definition includes group $R^8$ or ring Hy being attached to LINK via a C or N atom within group $R^8$ or ring Hy such that a hydrogen atom on the C or N atom within group $R^8$ or ring Hy is replaced with a bond to LINK. Therefore, where group $R^8$ and/or ring Hy are defined as being unsubstituted or substituted by one or more chemical groups (suitable such chemical groups are defined herein), then one of group $R^8$ or ring Hy may also be attached to LINK via a C or N atom within group $R^8$ or ring Hy such that a hydrogen atom on the C or N atom within group $R^8$ or ring Hy is replaced with a bond to LINK. A bond to LINK may optionally be positioned on a carbon atom which carries a further substituent (where chemically possible). Typically, a carbon or nitrogen atom which is bonded to LINK does not carry a further substituent.

For the avoidance of doubt, where group $R^8$ and/or ring Hy are defined as being substituted by a hydrogen atom (e.g. where $R^1$, $R^2$, $R^{11}$, $R^{3a}$, $R^{3b}$, $R^{3c}$ and $R^{3d}$ are defined as being a hydrogen atom), then a reference herein to a hydrogen atom within group $R^8$ or ring Hy being replaced with a bond to LINK, includes the option that said hydrogen atom (e.g. a hydrogen atom at $R^1$, $R^2$, $R^{11}$, $R^{3a}$, $R^{3b}$, $R^{3c}$ and $R^{3d}$) is replaced with a bond to LINK. Therefore, where $R^1$, $R^2$, $R^{11}$, $R^{3a}$, $R^{3b}$, $R^{3c}$ and $R^{3d}$ are defined as being a hydrogen atom, then said hydrogen atom may be replaced with a bond to LINK.

For the avoidance of doubt, where ring Hy or group $R^8$ is substituted, a C or N atom which is bonded to LINK may be within the aryl, heteroaryl, heterocyclyl, or cycloalkyl ring of ring Hy or group $R^8$, or it may be a C or N atom of a substituent on ring Hy or group $R^8$. Preferably, a C or N atom which is bonded to LINK is a C or N atom on the heterocyclyl ring of ring Hy (i.e. directly to the ring and not via a substituent), a C or N atom of the aryl, heteroaryl, heterocyclyl, or cycloalkyl ring of ring Hy or group $R^8$, or the heteroaryl, cycloalkyl, heterocyclyl or a phenyl ring or $R^{10}$ in the case that a group $R^{10}$ is present as, or as part of, a substituent on $R^8$.

As the skilled person would readily appreciate, methods for preparation of compounds (in which two discrete chemical entities contribute discrete biological functions to the overall compound) are very well known in the art. A hugely diverse range of techniques for covalently attaching the respective chemical entities, via a vast number of chemical

linker moieties, is well established. Such is the ubiquity of these methodologies, that standard text books devoted entirely to this topic have long been available. One such textbook is "Bioconjugate Techniques" (Greg T. Hermanson, Academic Press Inc., 1996), the content of which is herein incorporated by reference in its entirety.

*MLLT1 and/or MLLT3 binders*

In the compounds of the invention, the MLLT1 and/or MLLT3 binder M is a monovalent moiety of formula (I) as defined herein.

In the description that follows, the skilled person will readily understand that M is a monovalent moiety that is bonded to LINK via (i) a single bond at $R^8$, or (ii) a C or N atom within group $R^8$ or ring Hy (as described herein) such that a hydrogen atom on the C or N atom within group $R^8$ or ring Hy is replaced with a bond to LINK. Thus, in the description that follows, any of the hydrogen atoms on any of the C or N atoms within group $R^8$ or ring Hy may be removed to form the point of attachment of the monovalent moiety M to LINK.

Typically, in formula (I), ring Hy is a 5- to 6- membered heterocyclyl ring containing X and at least one N atom, for instance one or two N atoms. X is typically a bond, $-N(R^{11})-$, O or $-C(R^{11})_2-$. Preferably, X is a bond, -N(Me)-, O or $-CH_2-$, most preferably X is a bond. In particular, when ring Hy is a 4- or 5-membered heterocyclyl ring, X is most preferably a bond. For example, ring Hy may be selected from a pyrrolidinyl, piperidinyl, morpholinyl or diazinanyl ring. Preferably, ring Hy is a 5-membered heterocyclyl ring containing X and at least one N atom, for instance one N atom. For example, ring Hy may be a pyrrolidinyl ring.

Ring Hy is linked to ring A via a C atom within ring Hy, said C atom also being linked to $R^2$. A N atom within ring Hy is substituted by $R^1$. Typically, the N atom of ring Hy which is substituted by $R^1$ is adjacent to the C atom of ring Hy that is bonded to ring A.

A C or N atom within ring Hy may be bonded to LINK by a hydrogen atom on that C or N atom being replaced by a bond to LINK. Typically, where ring Hy is bonded to LINK, this is via a C atom on Hy. Preferably, the bond to LINK is via (i) a single bond at $R^8$, or (ii) replacement of a hydrogen atom on a C or N within group $R^8$, i.e. the bond to LINK is preferably not via ring Hy.

Typically, in formula (I), $R^1$ is H, $C_{1-4}$ cycloalkyl, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy, or $R^1$ may be H or $C_{1-2}$ alkyl which is unsubstituted or substituted with one $C_{1-2}$ alkoxy. Preferably, $R^1$ is H, methyl, ethyl or methoxyethyl. Most preferably, $R^1$ is methyl.

In formula (I), $R^2$ is H or methyl. Preferably, $R^2$ is H.

Typically, in formula (I), each $R^{11}$ is independently selected from H and methyl. When X is $-N(R^{11})-$ or $-S(O)(NR^{11})-$, $R^{11}$ is preferably methyl. When X is $-C(R^{11})_2-$, optionally one $R^{11}$ is replaced with a single bond to LINK and the other $R^{11}$ is H. When X is $-C(R^{11})_2-$, each $R^{11}$ is preferably H.

Aside from groups $R^1$ and $R^2$, ring Hy may further be unsubstituted or substituted by one or two $R^3$. Typically, each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring. More typically, each $R^3$ is independently selected from methyl, ethyl, t-butyl methoxy and fluoro, or (i) two $R^3$ linked to adjacent C atoms in ring Hy form, together with the C atoms to which they are attached, a cyclohexyl ring, or (ii) two $R^3$ linked to the same C atom in ring Hy form, together with the C atom to which they are attached, a cyclopentyl ring. Preferably, ring Hy is substituted by no $R^3$ groups.

Typically, therefore, in formula (I), ring Hy is a 5- to 6- membered heterocyclyl ring containing X and at least one N atom, for example a pyrrolidinyl, piperidinyl, morpholinyl or diazinanyl ring; the N atom of ring Hy which is substituted by $R^1$ is adjacent to the C atom of ring Hy that is bonded to ring A; $R^1$ is H or $C_{1-2}$ alkyl which is unsubstituted or substituted with one $C_{1-2}$ alkoxy or one, two or three halo, preferably with one $C_{1-2}$ alkoxy; $R^2$ is H or methyl; X is a bond, $-N(R^{11})-$, O or $-C(R^{11})_2-$; $R^{11}$ is H or methyl; ring Hy is further unsubstituted or substituted by one or two $R^3$; and each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring.

Preferably, in formula (I), ring Hy is a pyrrolidinyl ring; the N atom of ring Hy which is substituted by $R^1$ is adjacent to the C atom of ring Hy that is bonded to ring A; $R^1$ is H, methyl, ethyl or methoxyethyl, more preferably methyl; $R^2$ is H; and ring Hy is substituted by no $R^3$ groups, i.e. ring Hy is not further substituted and carries substituent $R^1$ only.

Typically, in formula (I), no more than two of $Y^1$, $Y^2$ and $Y^3$ are N. Preferably, no more than one of $Y^1$, $Y^2$ and $Y^3$ are N. Most preferably, $Y^1$ is N, $Y^2$ is $-C(R^6)-$, and $Y^3$ is $-C(R^5)-$.

Typically, $Z^2$ is N, $Z^4$ is C, $Z^1$ is N, $Z^3$ is $-C(R^4)-$, $Y^1$ is N or $-C(R^7)-$, $Y^2$ is $-C(R^6)-$, and $Y^3$ is $-C(R^5)-$. Thus, the bicyclic structure formed by rings A and B has the structure:

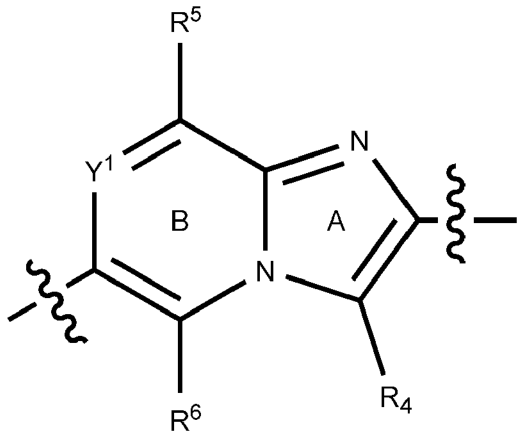

In one preferred embodiment, $Z^2$ is N, $Z^4$ is C, $Z^1$ is N, $Z^3$ is -C($R^4$)-, $Y^1$ is N, $Y^2$ is - C($R^6$)-, and $Y^3$ is -C($R^5$)-, such that the bicyclic structure formed by rings A and B has the structure:

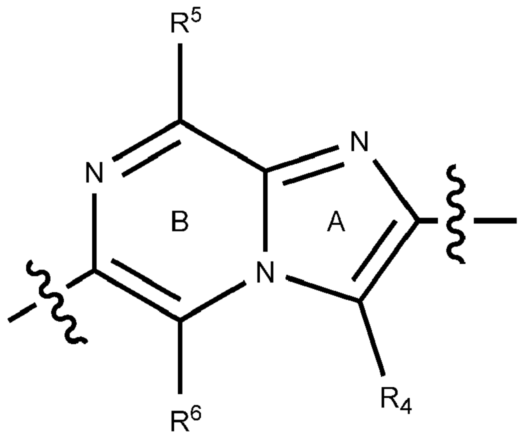

In another preferred embodiment, $Z^2$ is N, $Z^4$ is C, $Z^1$ is N, $Z^3$ is -C($R^4$)-, $Y^1$ is -C($R^7$)-, $Y^2$ is -C($R^6$)-, and $Y^3$ is -C($R^5$)- , such that the bicyclic structure formed by rings A and B has the structure:

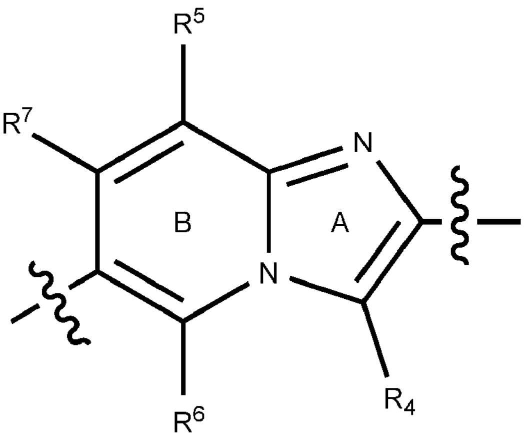

Typically, in formula (I), $R^4$, $R^5$, $R^6$, and $R^7$ are independently selected from H, halo, $C_{1-4}$ alkoxy, and $C_{1-4}$ alkyl which is itself unsubstituted or substituted by one, two or three halo. Typically, $R^4$, $R^5$, $R^6$, and $R^7$ are independently selected from H, halo (e.g. fluoro), methoxy, and methyl. Preferably, either $R^4$, $R^5$, $R^6$, and $R^7$ are H, or one of $R^4$, $R^5$, $R^6$, and $R^7$ is selected from halo (e.g. fluoro), methoxy, and methyl, and the rest are H. More preferably, $R^4$, $R^5$, $R^6$, and $R^7$ are H.

In one preferred embodiment, $R^5$, $R^6$, and $R^7$ are as defined herein and $R^4$ is selected from H and halo. More preferably, $R^5$, $R^6$, and $R^7$ are as defined herein and $R^4$ is selected from H and fluoro. Most preferably, $R^5$, $R^6$, and $R^7$

are as defined herein $R^4$ is H.

In one preferred embodiment, $R^5$ is selected from H, cyclopropyl, $C_{1-4}$ alkoxy, and $C_{1-4}$ alkyl, for example from H, $C_{1-4}$ alkoxy, and $C_{1-4}$ alkyl. More preferably, $R^5$ is selected from H, cyclopropyl, methoxy and methyl, for example H, methoxy and methyl.

In one preferred embodiment, $R^6$ is H.

In one preferred embodiment, $R^7$ is selected from H and halo. More preferably, $R^7$ is selected from H and fluoro.

Typically, therefore, in formula (I):

- $R^4$ is selected from H and halo, and $R^5$, $R^6$, and $R^7$ when present are H;
- $R^5$ is selected from H, cyclopropyl, $C_{1-4}$ alkoxy, and $C_{1-4}$ alkyl, typically from H, $C_{1-4}$ alkoxy, and $C_{1-4}$ alkyl, and $R^4$, $R^6$, and $R^7$ when present are H;
- $R^7$ is selected from H and halo, and $R^4$, $R^5$, and $R^6$ when present are H; or
- $R^4$, $R^5$, $R^6$, and $R^7$ are H.

Preferably, in formula (I):

- $R^4$ is selected from H and fluoro, and $R^5$, $R^6$, and $R^7$ when present are H;
- $R^5$ is selected from H, cyclopropyl, methoxy and methyl, typically from H, methoxy and methyl, and $R^4$, $R^6$, and $R^7$ when present are H;
- $R^7$ is selected from H and fluoro, and $R^4$, $R^5$, and $R^6$ when present are H; or
- $R^4$, $R^5$, $R^6$, and $R^7$ are H.

In formula (I), L is -C(O)N(H)-, wherein either (i) the C atom of L is bonded to $R^8$, and the N atom of L is bonded to ring B; or (ii) the C atom of L is bonded to ring B, and the N atom of L is bonded to $R^8$. Typically, in formula (I), the C atom of L is bonded to $R^8$, and the N atom of L is bonded to ring B.

In one embodiment, $R^8$ is a bond to LINK.

In another embodiment, $R^8$ is a group selected from a 6- to 10-membered aryl ring, a 5-to 10-membered heteroaryl ring, a 5- to 10-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$. $R^9$ and $R^{10}$ are as defined herein.

Typically, in formula (I), $R^8$ is a group selected from a 6- to 10-membered aryl ring, a 5-to 10-membered heteroaryl ring, and a 5- to 6-membered heterocyclyl ring. The aryl, heteroaryl and heterocyclyl rings are as defined herein. For instance, $R^8$ may be selected from thiazolyl, pyrazolyl, pyrimidinyl, triazolyl, 1,2,4-oxadiazolyl, pyrazinyl, indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is a 5- to 10-membered heteroaryl ring preferably containing one, two or three N atoms. Most preferably, $R^8$ is an indazolyl group.

In some preferred embodiments, $R^8$ is a group selected from the following structures. For the avoidance of doubt, the following structures may be unsubstituted or substituted by one, two or three substituents as defined herein. [see below]

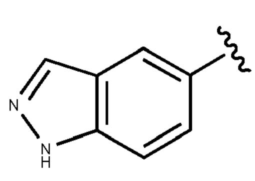
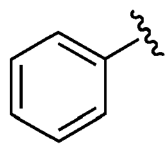
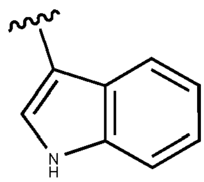
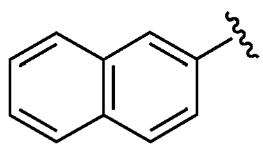

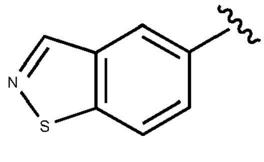
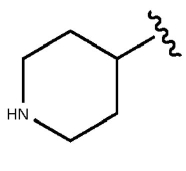
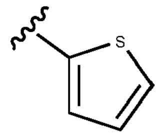
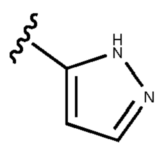

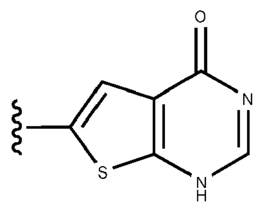 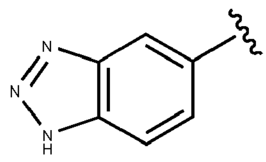 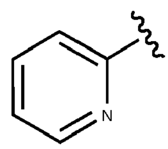 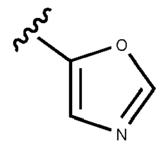

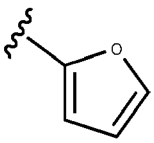 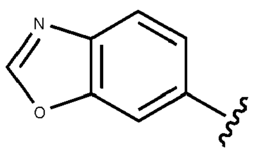 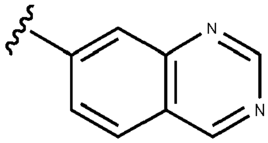 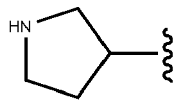

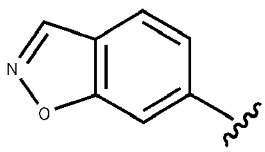 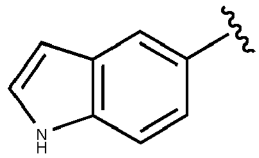 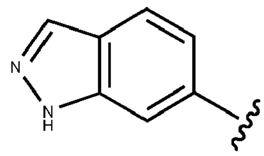 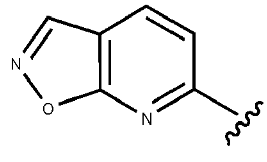

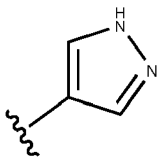 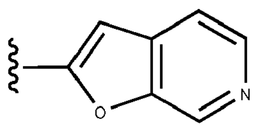 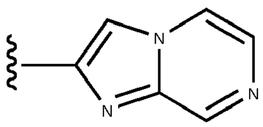 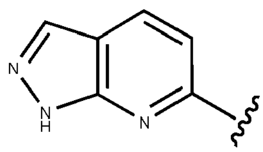

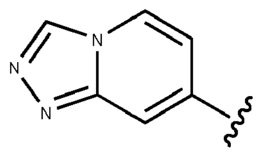 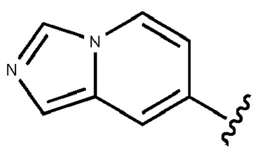 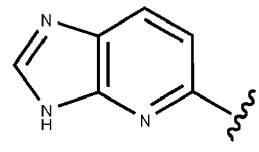 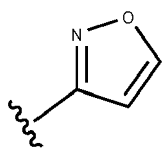

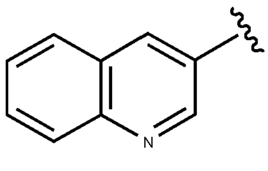 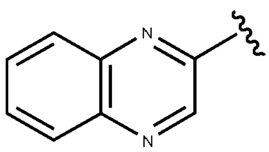 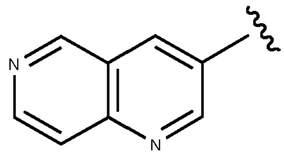 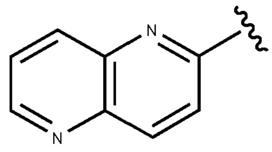

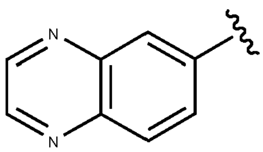 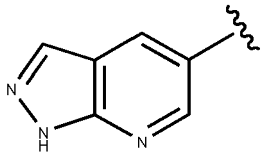 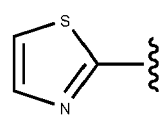 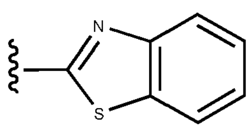

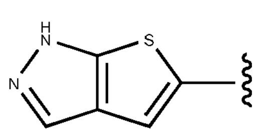 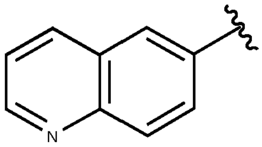 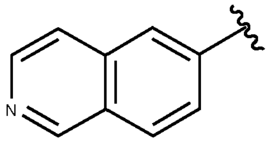

A C or N atom within group $R^8$ may be bonded to LINK by a hydrogen atom on that C or N atom being replaced by a bond to LINK. Typically, where group $R^8$ is bonded to LINK, this is via a C atom on $R^8$. Preferably, a C or N atom within group $R^8$ is bonded to LINK such that a hydrogen atom on that C or N atom is replaced by a bond to LINK. Preferably, the C or N atom that is bonded to LINK is a C or N atom of the aryl, heteroaryl, heterocyclyl or cycloalkyl ring of $R^8$ or a C or N atom of the heteroaryl, cycloalkyl, heterocyclyl or phenyl ring of $R^{10}$. More preferably, the C or N atom that is bonded to LINK is a C or N atom of the aryl, heteroaryl, heterocyclyl or cycloalkyl ring of $R^8$.

In formula (I), in addition to any bond to LINK (where present) $R^8$ is unsubstituted or substituted by one, two

or three substituents, for instance one or two substituents. Typically, each substituent is independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy. Preferably, each substituent is independently selected from fluoro, $R^{10}$ and methyl, Most preferably, each substituent is independently selected from fluoro and methyl. $R^{10}$ is as defined herein.

Typically, in formula (I), $R^{10}$ is a group selected from a 5- to 6-membered heteroaryl ring, a 3- to 6-membered cycloalkyl ring, a 4- to 5-membered heterocyclyl ring, and a phenyl ring. The cycloalkyl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl.

Typically, in formula (I), in addition to any bond to LINK (where present), $R^{10}$ is unsubstituted or substituted by one or two substituents independently selected from fluoro, methyl and methoxy. Preferably, $R^{10}$ is unsubstituted.

Typically, therefore, in formula (I), $R^8$ is (i) a bond to LINK or (ii) a group selected from a 6- to 10-membered aryl ring, a 5- to 10-membered heteroaryl ring, and a 5- to 6-membered heterocyclyl ring; the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; wherein $R^{10}$ is a group selected from a 5- to 6-membered heteroaryl ring, a 3- to 6-membered cycloalkyl ring, a 4- to 5-membered heterocyclyl ring, and a phenyl ring; and $R^{10}$ is unsubstituted or substituted by one or two substituents independently selected from fluoro, methyl and methoxy. Typically, where a C or N atom within group $R^8$ is bonded to LINK, the C or N atom that is bonded to LINK is a C or N atom of the aryl, heteroaryl, heterocyclyl or cycloalkyl ring of $R^8$ or a C or N atom of the heteroaryl, cycloalkyl, heterocyclyl or phenyl ring of $R^{10}$.

Preferably, in formula (I), $R^8$ is a 5- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

Preferably, a C or N atom within group $R^8$ is bonded to LINK, wherein the C or N atom that is bonded to LINK is a C or N atom of the aryl, heteroaryl, heterocyclyl or cycloalkyl ring of $R^8$.

In a typical embodiment of formula (I), the bicyclic structure formed by rings A and B has the structure:

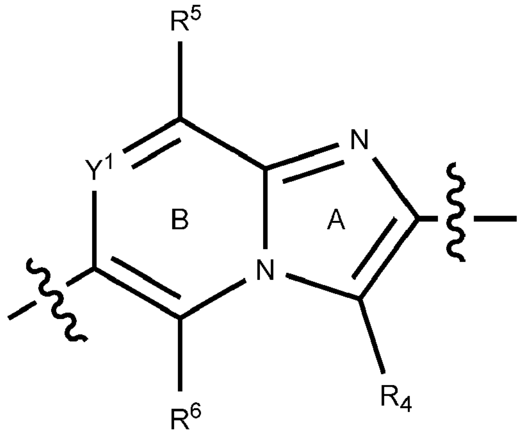

and:

$Y^1$ is N or $-C(R^7)-$;

ring Hy is a 5- to 6- membered heterocyclyl ring containing X and at least one N atom, for example a pyrrolidinyl, piperidinyl, morpholinyl or diazinanyl ring; wherein the N atom of ring Hy which is substituted by $R^1$ is adjacent to the C atom of ring Hy that is bonded to ring A;

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)-, O or $-CH_2-$;

ring Hy is further unsubstituted or substituted by one or two $R^3$; wherein each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring;
$R^4$, $R^5$, $R^6$, and $R^7$ are independently selected from H, halo (e.g. fluoro), methoxy, and methyl;
the C atom of L is bonded to $R^8$, and the N atom of L is bonded to ring B;
$R^8$ is (i) a bond to LINK or (ii) a group selected from a 6- to 10-membered aryl ring, a 5- to 10-membered heteroaryl ring, and a 5- to 6-membered heterocyclyl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from halo and $C_{1-2}$ alkoxy; and
$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a preferred embodiment of formula (I), the bicyclic structure formed by rings A and B has the structure:

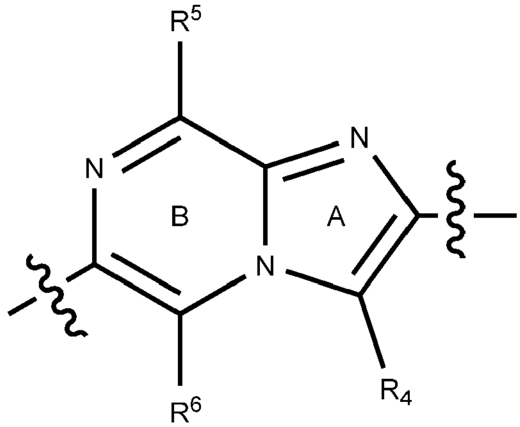

and:

ring Hy is a pyrrolidinyl ring; wherein the N atom of ring Hy which is substituted by $R^1$ is adjacent to the C atom of ring Hy that is bonded to ring A; $R^1$ is H, methyl, ethyl or
methoxyethyl, more preferably methyl; $R^2$ is H; and ring Hy is substituted by no $R^3$ groups;
either $R^4$, $R^5$, $R^6$, and $R^7$ are H, or one of $R^4$, $R^5$, $R^6$, and $R^7$ is selected from halo (e.g. fluoro), methoxy, and methyl, and the rest are H;
the C atom of L is bonded to $R^8$, and the N atom of L is bonded to ring B;
$R^8$ is (i) a bond to LINK or (ii) a 5- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted; wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a particular embodiment of the compounds of the invention, the MLLT1 and/or MLLT3 binder M is a monovalent moiety of formula (II):

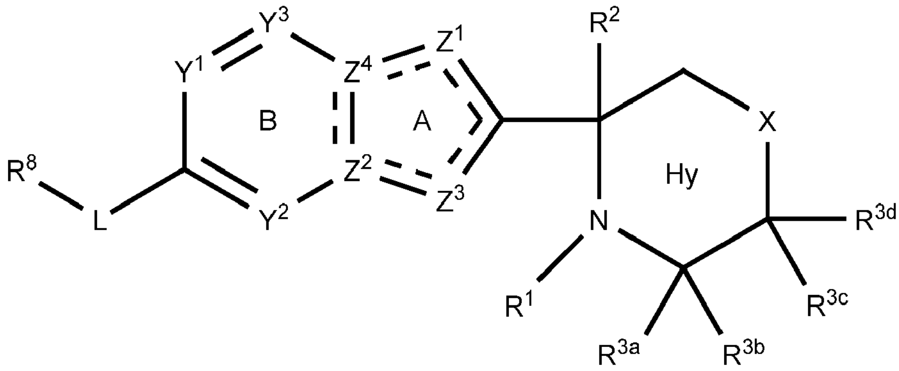

(II)

wherein:

$Z^1$, $Z^2$, $Z^3$, $Z^4$, $Y^1$, $Y^2$, $Y^3$, $R^1$, $R^2$, $R^8$, X and L are as defined above for formula (I);
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, with the proviso that when X is $NR^{11}$, O, or S, then neither $R^{3c}$ nor $R^{3d}$ are halo, or $R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
wherein either $R^8$ is (i) a bond to LINK, or $R^8$ is a group (ii) and M is bonded to LINK via a C or N atom within group $R^8$ or ring Hy (as described herein) such that a hydrogen atom on the C or N atom within group $R^8$ or ring Hy is replaced with a bond to LINK.

Typically, in formula (II): $R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$, O, or S, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Optionally, one of $R^{3a}$, $R^{3b}$, $R^{3c}$ and $R^{3d}$ is replaced with a single bond to LINK.

Preferably, in formula (II), $R^{3a}$, $R^{3b}$, $R^{3c}$ and $R^{3d}$ are all H.

In a typical embodiment of formula (II), the bicyclic structure formed by rings A and B has the structure:

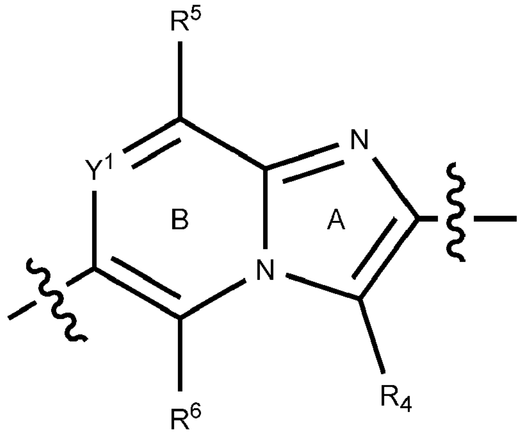

and:

$Y^1$ is N or $-C(R^7)-$;
$R^1$ is H or $C_{1-2}$ alkyl which is unsubstituted or substituted with one $C_{1-2}$ alkoxy or one, two or three halo, preferably

with one $C_{1-2}$ alkoxy;
$R^2$ is H or methyl;
X is a bond, -N(Me)-, O or $-CH_2-$;
$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$, O, or S, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;
$R^4$, $R^5$, $R^6$, and $R^7$ are independently selected from H, halo (e.g. fluoro), methoxy, and methyl;
the C atom of L is bonded to $R^8$, and the N atom of L is bonded to ring B;
$R^8$ is (i) a bond to LINK or (ii) a group selected from a 6- to 10-membered aryl ring, a 5- to 10-membered heteroaryl ring, and a 5- to 6-membered heterocyclyl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and $R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a preferred embodiment of formula (II), the bicyclic structure formed by rings A and B has the structure:

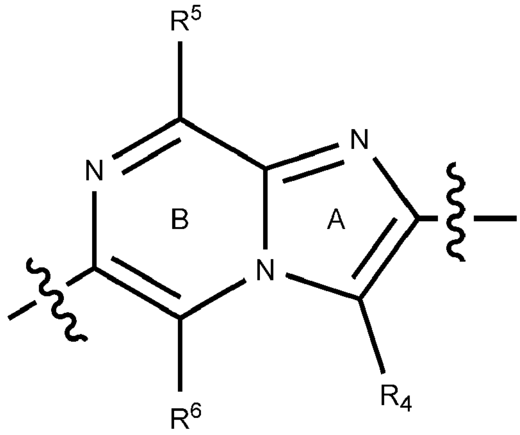

and:

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
either $R^4$, $R^5$, $R^6$, and $R^7$ are H, or one of $R^4$, $R^5$, $R^6$, and $R^7$ is selected from fluoro, methoxy, and methyl, and the rest are H;
the C atom of L is bonded to $R^8$, and the N atom of L is bonded to ring B;
$R^8$ is a 5- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted; and
wherein M is bonded to LINK via a C or N atom within group $R^8$ or ring Hy such that a hydrogen atom on the C or N atom within group $R^8$ or ring Hy is replaced with a bond to LINK.

In a further preferred embodiment of formula (II), the bicyclic structure formed by rings A and B has the structure:

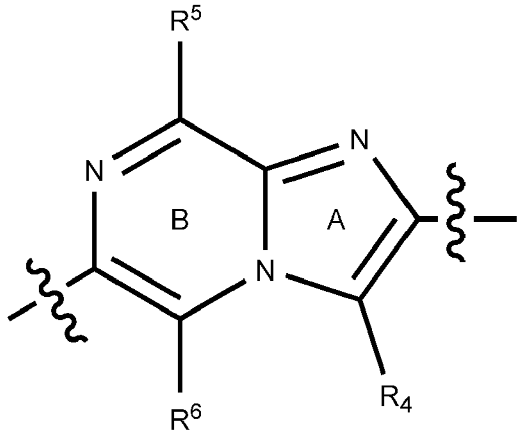

and:

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
either $R^4$, $R^5$, $R^6$, and $R^7$ are H, or one of $R^4$, $R^5$, $R^6$, and $R^7$ is selected from fluoro, methoxy, and methyl, and the rest are H;
the C atom of L is bonded to $R^8$, and the N atom of L is bonded to ring B; and $R^8$ is a bond to LINK.

In a particularly preferred embodiment of the compounds of the invention, the MLLT1 and/or MLLT3 binder M is a monovalent moiety of formula (III):

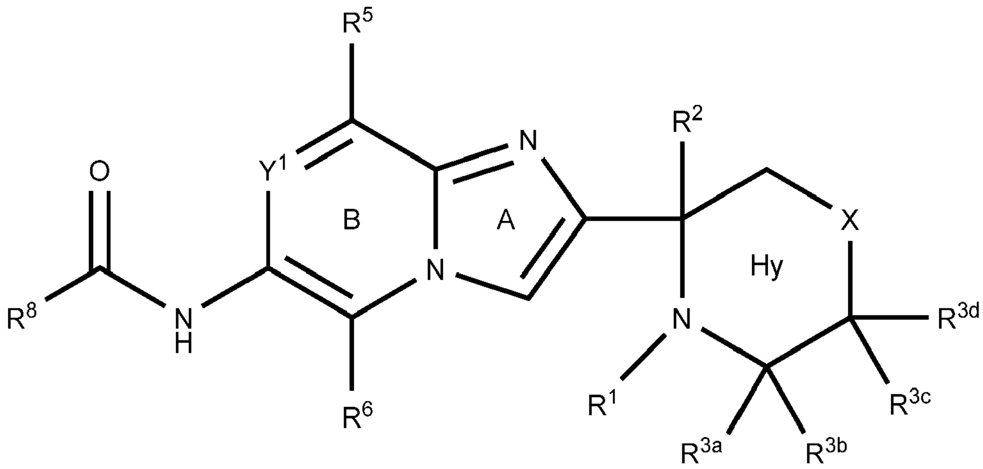

(III)

wherein $R^1$, $R^2$, $R^{3a}$, $R^{3b}$, $R^{3c}$, $R^{3d}$, X, $Y^1$, $R^5$, $R^6$ and $R^8$ are as defined herein.
Typically, in the compounds of formula (III):

$R^1$ is H or $C_{1-4}$ alkyl which is itself unsubstituted or substituted with $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or $R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
X is a bond, -N(Me)-, O or $-CH_2-$;

$Y^1$ is N or $-C(R^7)-$;

$R^5$, $R^6$ and $R^7$ are independently selected from H, halo $C_{1-4}$ alkoxy, and $C_{1-4}$ alkyl;

$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;

each $R^9$ is independently selected from halo and $C_{1-4}$ alkoxy; and $R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;

wherein either $R^8$ is (i) a bond to LINK, or $R^8$ is a group (ii) and M is bonded to LINK via a C or N atom within group $R^8$ or ring Hy (as described herein) such that a hydrogen atom on the C or N atom within group $R^8$ or ring Hy is replaced with a bond to LINK.

In some of the particularly preferred embodiments, the MLLT1 and/or MLLT3 binder M is a monovalent moiety of formula (IIIa):

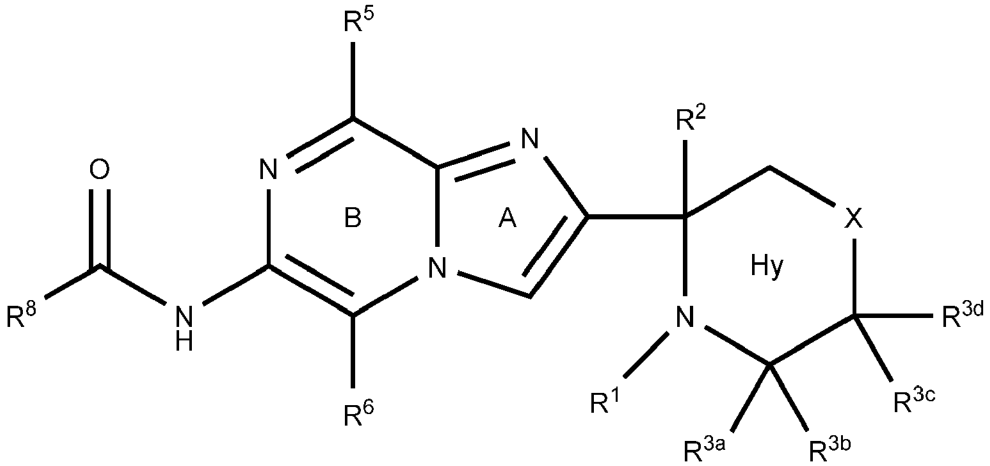

(IIIa)

wherein $R^1$, $R^2$, $R^{3a}$, $R^{3b}$, $R^{3c}$, $R^{3d}$, $R^5$, $R^6$, $R^8$ and X are as defined in formula (III).

In other of the particularly preferred embodiments, the MLLT1 and/or MLLT3 binder M is a monovalent moiety of formula (IIIb):

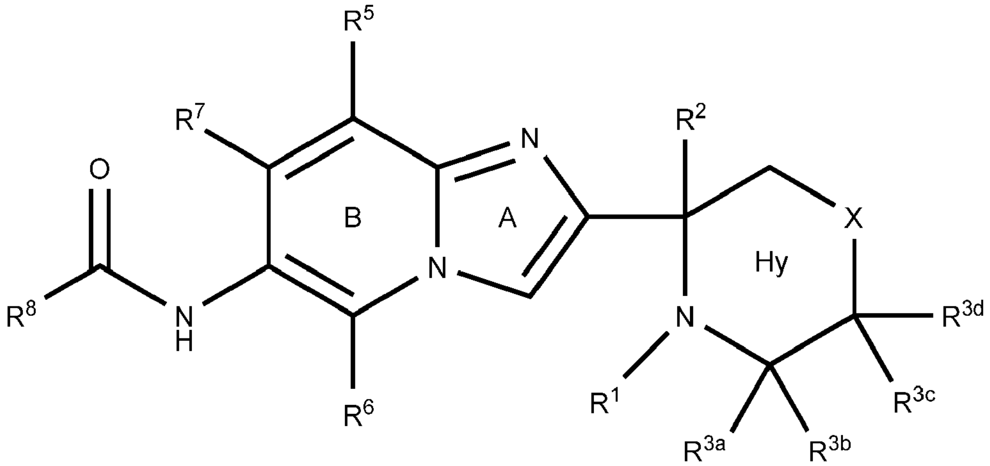

(IIIb)

wherein $R^1$, $R^2$, $R^{3a}$, $R^{3b}$, $R^{3c}$, $R^{3d}$, $R^5$, $R^6$, $R^7$, $R^8$ and X are as defined in formula (III).

Typically, in formula (III), formula (IIIa) and formula (IIIb), $R^1$ is H, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy. Preferably, $R^1$ is H, methyl, ethyl or methoxyethyl. Most preferably, $R^1$ is methyl.

In formula (III), formula (IIIa) and formula (IIIb), $R^2$ is H or methyl. Preferably, $R^2$ is H.

Typically, in formula (III), formula (IIIa) and formula (IIIb), X is a bond, $-N(R^{11})-$, O or $-C(R^{11})_2-$. Preferably, X is a bond, -N(Me)-, O or $-CH_2-$, most preferably X is a bond.

Typically, in formula (III), formula (IIIa) and formula (IIIb), each $R^{11}$ is independently selected from H and methyl. When X is $-N(R^{11})-$ or $-S(O)(NR^{11})-$, $R^{11}$ is preferably methyl. When X is $-C(R^{11})_2-$, one $R^{11}$ is optionally replaced with a single bond to LINK and the other $R^{11}$ is H. When X is $-C(R^{11})_2-$, each $R^{11}$ is preferably H.

Typically, in formula (III), formula (IIIa) and formula (IIIb), $R^{3a}$ and $R^{3b}$ are independently selected from H, $C_{1-4}$ alkyl, halo and $C_{1-4}$ alkoxy, in particular from H and methyl; $R^{3c}$ and $R^{3d}$ are independently selected from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$, O, or S, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Preferably, in formula (III), formula (IIIa) and formula (IIIb), $R^{3a}$, $R^{3b}$, $R^{3c}$ and $R^{3d}$ are all H.

Typically, in formula (III), formula (IIIa) and formula (IIIb), $R^5$ is selected from H, $C_{1-4}$ alkoxy, and $C_{1-4}$ alkyl. Preferably, $R^5$ is selected from H, methoxy and methyl.

Typically, in formula (III), formula (IIIa) and formula (IIIb), $R^6$ is H.

Typically, in formula (III) and formula (IIIb), $R^7$ is selected from H and halo. Preferably, $R^7$ is selected from H and fluoro.

Typically, therefore, in formula (III), formula (IIIa) and formula (IIIb):

- $R^5$ is selected from H, $C_{1-4}$ alkoxy, and $C_{1-4}$ alkyl, and $R^6$ and $R^7$ when present are H;
- $R^7$ when present is selected from H and halo, and $R^5$ and $R^6$ are H; or
- $R^5$, $R^6$, and $R^7$ when present are H.

Preferably, in in formula (III), formula (IIIa) and formula (IIIb):

- $R^5$ is selected from H, methoxy and methyl, and $R^6$ and $R^7$ when present are H;
- $R^7$ when present is selected from H and fluoro, and $R^5$ and $R^6$ are H; or
- $R^5$, $R^6$, and $R^7$ when present are H.

In one preferred embodiment of formula (III), formula (IIIa) and formula (IIIb), $R^8$ is a bond to LINK.

In another preferred embodiment of formula (III), formula (IIIa) and formula (IIIb), $R^8$ is a group selected from a 6- to 10-membered aryl ring, a 5- to 10-membered heteroaryl ring, and a 5- to 6-membered heterocyclyl ring. The aryl, heteroaryl and heterocyclyl rings are as defined herein. For instance, $R^8$ may be selected from thiazolyl, pyrazolyl, pyrimidinyl, triazolyl, 1,2,4-oxadiazolyl, pyrazinyl, indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is a 5- to 10-membered heteroaryl ring containing one, two or three N atoms. Most preferably, $R^8$ is an indazolyl group.

Typically, in formula (III), formula (IIIa) and formula (IIIb), $R^8$ is a group selected from the following structures. For the avoidance of doubt, the following structures may be unsubstituted or substituted by one, two or three substituents as defined herein.
[see below]

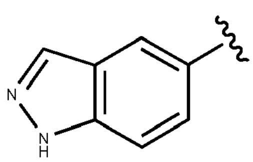 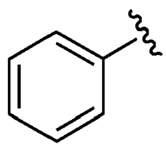 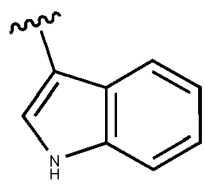 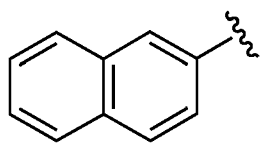

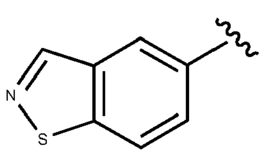 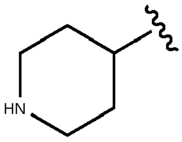 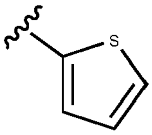 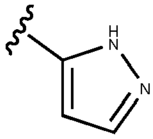

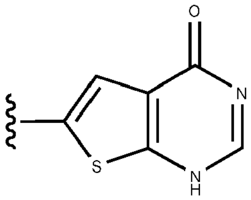 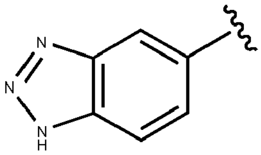 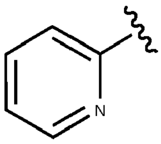 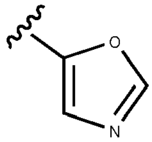

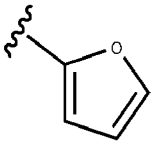 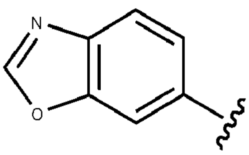 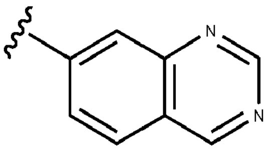 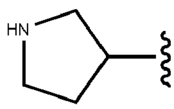

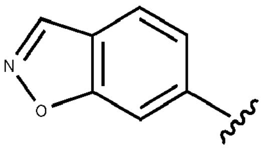 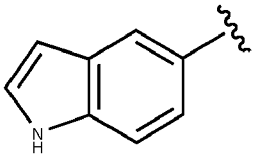 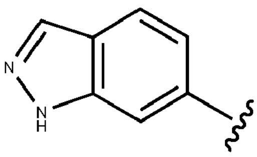 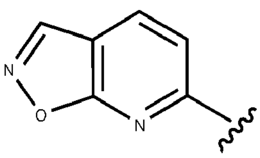

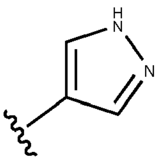 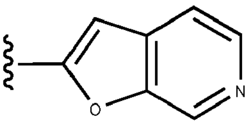 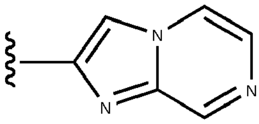 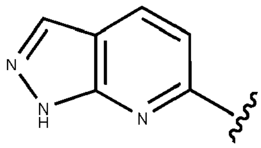

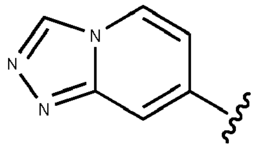 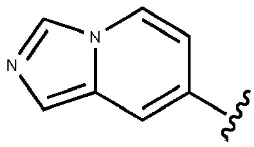 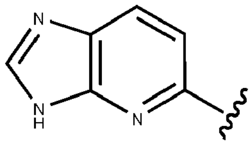 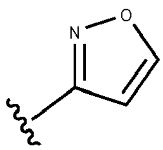

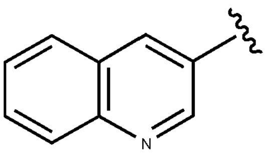 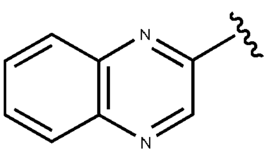 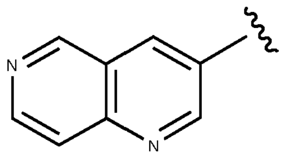 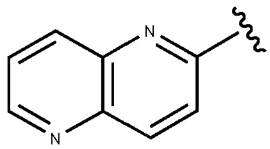

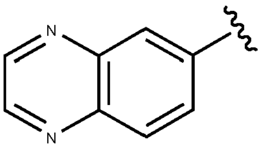 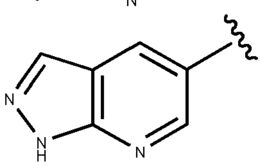 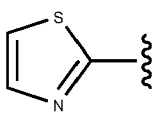 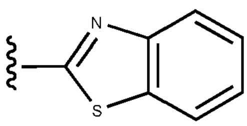

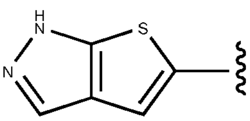 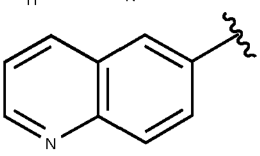 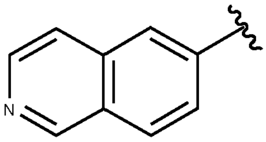

Preferably, $R^8$ is an indazolyl group.

Typically, in formula (III), formula (IIIa) and formula (IIIb), $R^8$ is unsubstituted or substituted by one, two or three substituents, for instance one or two substituents, independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy.

Preferably, each substituent is independently selected from fluoro, $R^{10}$ and methyl. Most preferably, each substituent is independently selected from fluoro and methyl. $R^{10}$ is as defined herein.

Typically, in formula (III), formula (IIIa) and formula (IIIb), $R^{10}$ is a group selected from a 5- to 6-membered heteroaryl ring, a 3- to 6-membered cycloalkyl ring, a 4- to 5-membered heterocyclyl ring, and a phenyl ring. The aryl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl.

Typically, in in formula (III), formula (IIIa) and formula (IIIb), $R^{10}$ is unsubstituted or substituted by one or two substituents independently selected from fluoro, methyl and methoxy. Preferably, $R^{10}$ is unsubstituted.

In typical embodiments of formula (III), formula (IIIa) and formula (IIIb):

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)- O or $-C(H)_2-$;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$, O, or S, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;;

one of the following options applies: (i) $R^5$ is selected from H, $C_{1-4}$ alkoxy, and $C_{1-4}$ alkyl, and $R^6$ and $R^7$ when present are H; or (ii) $R^7$ when present is selected from H and halo, and $R^5$ and $R^6$ are H; or (iii) $R^5$, $R^6$, and $R^7$ when present are H;

the group $R^8$, where present, is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and

$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy; and $R^8$ is (i) a bond to LINK or (ii) a group selected from the following structures:

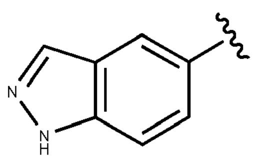
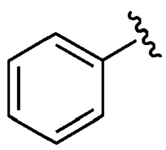
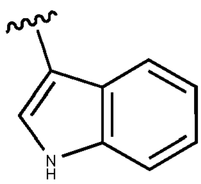
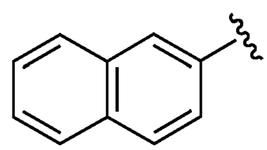
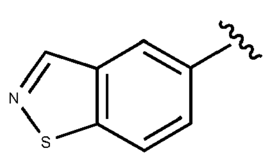
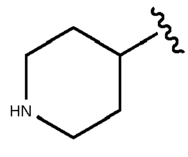
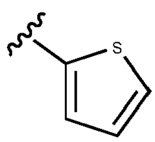
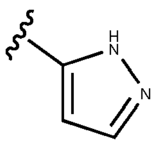
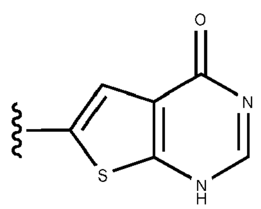
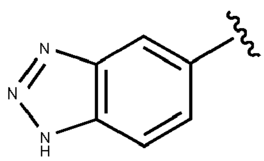
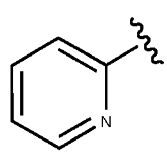
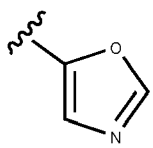

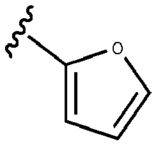 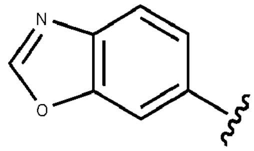 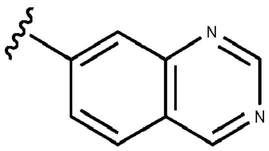 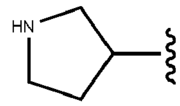

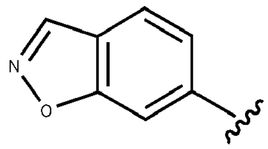 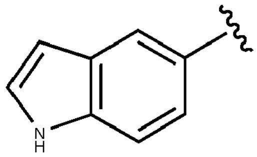 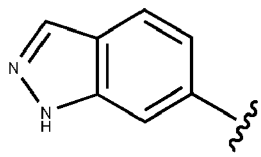 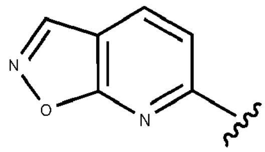

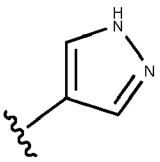 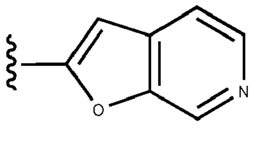 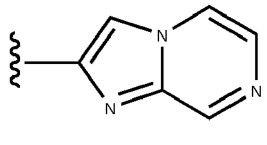 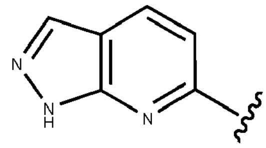

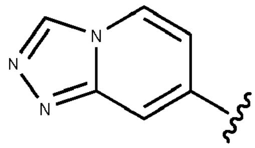 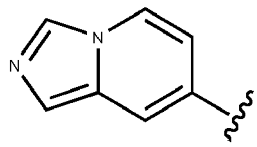 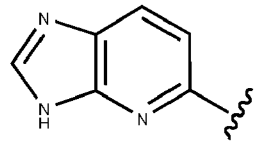 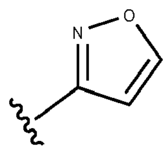

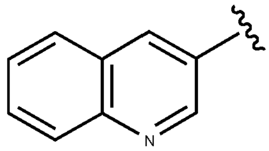 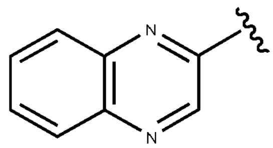 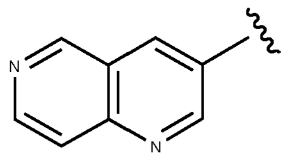 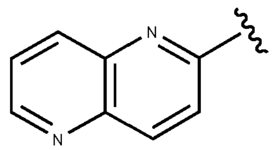

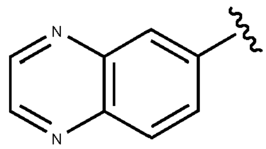 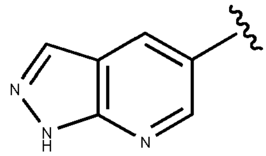 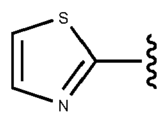 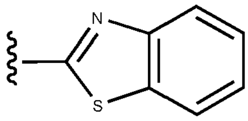

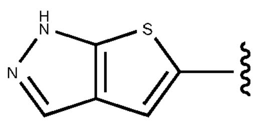 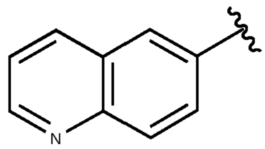 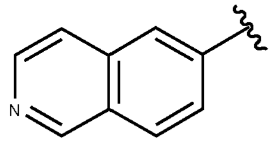

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In preferred embodiments of formula (III), formula (IIIa) and formula (IIIb),

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
$R^5$, $R^6$, and $R^7$ are H;
$R^8$ is (i) a bond to LINK or (ii) a 5- to 10-membered heteroaryl ring containing one, two or three N atoms, for example an indazolyl group; the group $R^8$ is unsubstituted or substituted by one or two substituents, each independently selected from fluoro, $R^{10}$ and methyl;
$R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

Particularly preferred MLLT1 and/or MLLT3 binders M are monovalent moieties of the compounds set out below, wherein in each case a hydrogen atom within group $R^8$ or ring Hy is removed and forms the position of bonding to LINK:

1-methyl-N-{8-methyl-2-[(2R)-1-methylpyrrolidin-2-yl]imidazo[1,2-a]pyrazin-6-yl}-1H-indazole-5-carboxamide;
1,3-dimethyl-N-{8-methyl-2-[(2R)-1-methylpyrrolidin-2-yl]imidazo[1,2-a]pyrazin-6-yl}-1H-indazole-6-carboxamide;
rel-7-fluoro-N-{2-[(2R)-1-methylpyrrolidin-2-yl]imidazo[1,2-a]pyridin-6-yl}quinoline-6-carboxamide;
N-{2-[(2R,4S)-4-fluoro-1-methylpyrrolidin-2-yl]imidazo[1,2-a]pyrazin-6-yl}-1-methyl-1H-indazole-5-carboxamide;
1,3-dimethyl-N-{2-[(2R)-pyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
N-{2-[4-ethyl-1-methylpyrrolidin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-7-fluoro-N-{2-[(2R)-1-methylpyrrolidin-2-yl]imidazo[1,2-a]pyridin-6-yl}isoquinoline-6-carboxamide;
rel-5-fluoro-1,3-dimethyl-N-{2-[(2R)-1-methylpyrrolidin-2-yl]imidazo[1,2-a]pyrazin-6-yl}-1H-indazole-6-carboxamide;
1-methyl-N-(2-{2-methyl-2-azaspiro[4.4]nonan-3-yl}imidazo[1,2-a]pyridin-6-yl)-1H-indazole-5-carboxamide;
rel-3-ethyl-1-methyl-N-{2-[(2R)-1-methylpyrrolidin-2-yl]imidazo[1,2-a]pyrazin-6-yl}-1H-indazole-6-carboxamide;
rel-3-cyclopropyl-1-methyl-N-{2-[(2R)-1-methylpyrrolidin-2-yl]imidazo[1,2-a]pyrazin-6-yl}-1H-indazole-6-carboxamide;
N-[2-(1,2-dimethylpyrrolidin-2-yl)imidazo[1,2-a]pyridin-6-yl]-1-methyl-1H-indazole-5-carboxamide;
rel-2-fluoro-4-(2-methyl-1,3-thiazol-5-yl)-N-{2-[(2R)-1-methylpyrrolidin-2-yl]imidazo[1,2-a]pyridin-6-yl}benzamide;
rel-1-methyl-N-{2-[(2R)-1-methylpiperidin-2-yl]imidazo[1,2-a]pyridin-6-yl}-1H-indazole-5-carboxamide;
rel-1-methyl-N-{2-[(2R)-1-methylpiperidin-2-yl]imidazo[1,2-a]pyridin-6-yl}-1H-indazole-5-carboxamide;
1-methyl-N-{2-[(2S)-1-methyl-octahydro-1H-indol-2-yl]imidazo[1,2-a]pyridin-6-yl}-1H-indazole-5-carboxamide;
1-methyl-N-{2-[(2R)-1-methyl-octahydro-1H-indol-2-yl]imidazo[1,2-a]pyridin-6-yl}-1H-indazole-5-carboxamide;
rel-2-fluoro-N-{2-[(2R)-1-methylpyrrolidin-2-yl]imidazo[1,2-a]pyridin-6-yl}-5-(pyrazin-2-yl)benzamide;
rel-1,3-dimethyl-N-{2-[(2R)-1-methylpyrrolidin-2-yl]imidazo[1,2-a]pyrazin-6-yl}-1H-indazole-6-carboxamide;
rel-1,3-dimethyl-N-{2-[(2R)-1-methylpyrrolidin-2-yl]imidazo[1,2-a]pyrazin-6-yl}-1H-indazole-6-carboxamide;
rel-1,3-dimethyl-N-{2-[(2R)-1-methylpyrrolidin-2-yl]imidazo[1,2-a]pyrazin-6-yl}-1H-indazole-6-carboxamide;
rel-1,3-dimethyl-N-{2-[(2R)-1-methylpyrrolidin-2-yl]imidazo[1,2-a]pyrazin-6-yl}-1H-indazole-6-carboxamide;
N-[2-(4-methoxy-1-methylpyrrolidin-2-yl)imidazo[1,2-a]pyridin-6-yl]-1-methyl-1H-indazole-5-carboxamide;
rel-2-fluoro-5-(2-methyl-1,3-thiazol-5-yl)-N-{ 2-[(2R)-1-methylpyrrolidin-2-yl]imidazo[1,2-a]pyridin-6-yl}benzamide;
N-[2-(1,4-dimethylpyrrolidin-2-yl)imidazo[1,2-a]pyridin-6-yl]-1-methyl-1H-indazole-5-carboxamide;
rel-2-fluoro-4-(4-methyl-1,3-thiazol-5-yl)-N-{ 2-[(2R)-1-methylpyrrolidin-2-yl]imidazo[1,2-a]pyridin-6-yl}benzamide;
N-[2-(1,5-dimethylpyrrolidin-2-yl)imidazo[1,2-a]pyridin-6-yl]-1-methyl-1H-indazole-5-carboxamide;
1-methyl-N-[2-(1-methylpyrrolidin-2-yl)imidazo[1,2-a]pyrazin-6-yl]-1H-indazole-5-carboxamide;
rel-2-fluoro-4-(1-methyl-1H-pyrazol-4-yl)-N-{2-[(2R)-1-methylpyrrolidin-2-yl]imidazo[1,2-a]pyridin-6-yl}benzamide;
rel-2-fluoro-N-{2-[(2R)-1-methylpyrrolidin-2-yl]imidazo[1,2-a]pyridin-6-yl}-4-(pyrimidin-2-yl)benzamide;
rel-2-fluoro-N-{2-[(2R)-1-methylpyrrolidin-2-yl]imidazo[1,2-a]pyridin-6-yl}-4-(pyrazin-2-yl)benzamide;
1,3-dimethyl-N-[2-(1-methylpyrrolidin-2-yl)imidazo[1,2-a]pyridin-6-yl]-1H-thieno[2,3-c]pyrazole-5-carboxamide;
6-methoxy-N-[2-(1-methylpyrrolidin-2-yl)imidazo[1,2-a]pyridin-6-yl]-1,3-benzothiazole-2-carboxamide;
4-(2-methyl-1,3-thiazol-4-yl)-N-[2-(1-methylpyrrolidin-2-yl)imidazo[1,2-a]pyridin-6-yl]benzamide;
4-chloro-1,3-dimethyl-N-[2-(1-methylpyrrolidin-2-yl)imidazo[1,2-a]pyridin-6-yl]-1H-pyrazolo[3,4-b]pyridine-5-carboxamide;
1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-6-(1H-pyrazol-1-yl)pyridine-3-carboxamide;
N-[2-(1-methylpyrrolidin-2-yl)imidazo[1,2-a]pyridin-6-yl]-4-(1H-1,2,4-triazol-1-yl)benzamide;
2,3-dimethyl-N-[2-(1-methylpyrrolidin-2-yl)imidazo[1,2-a]pyridin-6-yl]quinoxaline-6-carboxamide;
N-[2-(1-methylpyrrolidin-2-yl)imidazo[1,2-a]pyridin-6-yl]-1,5-naphthyridine-2-carboxamide;
7-chloro-N-[2-(1-methylpyrrolidin-2-yl)imidazo[1,2-a]pyridin-6-yl]quinoline-3-carboxamide;
N-[8-methoxy-2-(1-methylpyrrolidin-2-yl)imidazo[1,2-a]pyridin-6-yl]-1,3-dimethyl-1H-indazole-6-carboxamide;
N-{2-[1-(2-methoxyethyl)pyrrolidin-2-yl]imidazo[1,2-a]pyridin-6-yl}-1,3-dimethyl-1H-indazole-6-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;

rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;
5-fluoro-1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-[1,2,4]triazolo[4,3-a]pyridine-7-carboxamide;
4-chloro-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-3H-imidazo[4,5-b]pyridine-5-carboxamide;
N-[2-(1-methylpyrrolidin-2-yl)imidazo[1,2-a]pyridin-6-yl]imidazo[1,5-a]pyridine-7-carboxamide;
N-[2-(1-methylpyrrolidin-2-yl)imidazo[1,2-a]pyridin-6-yl]-4-(1,2,4-oxadiazol-3-yl)benzamide;
3-ethyl-N-[2-(1-methylpyrrolidin-2-yl)imidazo[1,2-a]pyridin-6-yl]-[1,2,4]triazolo[4,3-alpyridine-7-carboxamide;
1,3-dimethyl-N-[2-(1-methylpyrrolidin-2-yl)imidazo[1,2-a]pyridin-6-yl]-1H-pyrazolo[3,4-b]pyridine-6-carboxamide;
N-[2-(1-methylpyrrolidin-2-yl)imidazo[1,2-a]pyridin-6-yl]imidazo[1,2-a]pyrazine-2-carboxamide;
N-[2-(1-methylpyrrolidin-2-yl)imidazo[1,2-a]pyridin-6-yl]furo[2,3-c]pyridine-2-carboxamide;
1-methyl-N-[2-(4-methylmorpholin-3-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-[1,2]oxazolo[5,4-b] pyridine-6-carboxamide;
N-[7-fluoro-2-(1-methylpyrrolidin-2-yl)imidazo[1,2-a]pyridin-6-yl]-1,3-dimethyl-1H-indazole-6-carboxamide;
4-fluoro-N-[2-(1-methylpyrrolidin-2-yl)imidazo[1,2-a]pyridin-6-yl]-1H-indole-5-carboxamide;
3-(2-methoxyethyl)-1-methyl-N-[2-(1-methylpyrrolidin-2-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-1,2-benzoxazole-6-carboxamide;
N-[2-(1-methylpyrrolidin-2-yl)imidazo[1,2-a]pyridin-6-yl]quinazoline-7-carboxamide;
2-(4-methoxyphenyl)-N-[2-(1-methylpyrrolidin-2-yl)imidazo[1,2-a]pyridin-6-yl]-1,3-thiazole-4-carboxamide;
N-[2-(1-methylpyrrolidin-2-yl)imidazo[1,2-a]pyridin-6-yl]-1,3-benzoxazole-6-carboxamide;
N-[2-(1-methylpyrrolidin-2-yl)imidazo[1,2-a]pyridin-6-yl]-2-phenyl-1,3-oxazole-5-carboxamide;
1-cyclohexyl-N-[2-(1-methylpyrrolidin-2-yl)imidazo[1,2-a]pyridin-6-yl]-1H-1,2,3-benzotriazole-5-carboxamide;
5-methyl-N-[2-(1-methylpyrrolidin-2-yl)imidazo[1,2-a]pyridin-6-yl]-4-oxo-1H,4H-thieno[2,3-d]pyrimidine-6-carboxamide;
1-methyl-N-[2-(1-methylpyrrolidin-2-yl)imidazo[1,2-a]pyridin-6-yl]-3-(pyrrolidin-3-yl)-1H-indazole-5-carboxamide;
N-[2-(1-methylpyrrolidin-2-yl)imidazo[1,2-a]pyridin-6-yl]-3-(1H-pyrazol-1-yl)benzamide;
3-cyclopentyl-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-4-[(1H-pyrazol-1-yl)methyl] benzamide;
N-[2-(1-methylpyrrolidin-2-yl)imidazo[1,2-a]pyridin-6-yl]-1,3-benzothiazole-5-carboxamide;
N-[2-(1-methylpyrrolidin-2-yl)imidazo[1,2-a]pyridin-6-yl]-4-(1H-pyrazol-1-yl)benzamide;
6-methoxy-N-[2-(1-methylpyrrolidin-2-yl)imidazo[1,2-a]pyridin-6-yl]-1H-indole-3-carboxamide;
3-ethyl-1-methyl-N-[2-(1-methylpyrrolidin-2-yl)imidazo[1,2-a]pyridin-6-yl]-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
N-{2-[(rel-2R,4S)-4-methoxy-1-methylpyrrolidin-2-yl]imidazo[1,2-a]pyrazin-6-yl}-1-methylindazole-5-carboxamide;
N-{2-[(rel-2S,4S)-4-methoxy-1-methylpyrrolidin-2-yl]imidazo[1,2-a]pyrazin-6-yl}-1-methylindazole-5-carboxamide;
rac-N-[2-(4-tert-butyl-1-methylpyrrolidin-2-yl)imidazo[1,2-a]pyrazin-6-yl]-1,3-dimethylindazole-6-carboxamide;
N-[8-cyclopropyl-2-[(2R)-1-methylpyrrolidin-2-yl]imidazo[1,2-a]pyrazin-6-yl]-1-methyl-indazole-5-carboxamide;
N-[8-cyclopropyl-2-[(2R)-1-methylpyrrolidin-2-yl]imidazo[1,2-a]pyrazin-6-yl]-5-fluoro-1,3-dimethyl-indazole-6-carboxamide;
6-fluoro-1-methyl-N-[2-[(2R)-1-methylpyrrolidin-2-yl]imidazo[1,2-a]pyrazin-6-yl]indazole-5-carboxamide;
1-methyl-N-[2-[(6R)-5-methyl-5-azaspiro[2.4]heptan-6-yl]imidazo[1,2-a]pyrazin-6-yl]indazole-5-carboxamide;
N-{2-[(rel-2R)-1-ethylpyrrolidin-2-yl]imidazo[1,2-a]pyrazin-6-yl}-1,3-dimethylindazole-6-carboxamide;
N-{2-[(rel-2S)-1-ethylpyrrolidin-2-yl]imidazo[1,2-a]pyrazin-6-yl}-1,3-dimethylindazole-6-carboxamide;
N-(2-((2R, rel-4S)-1,4-dimethylpyrrolidin-2-yl)imidazo[1,2-a]pyrazin-6-yl)-1-methyl-1H-indazole-5-carboxamide;
N-(2-((2R,rel-4R)-1,4-dimethylpyrrolidin-2-yl)imidazo[1,2-a]pyrazin-6-yl)-1-methyl-1H-indazole-5-carboxamide;
N-{2-[(rel-2R,4R)-4-methoxy-1-methylpyrrolidin-2-yl]imidazo[1,2-a]pyrazin-6-yl}-1-methylindazole-5-carboxamide;
N-{2-[(rel-2S,4R)-4-methoxy-1-methylpyrrolidin-2-yl]imidazo[1,2-a]pyrazin-6-yl}-1-methylindazole-5-carboxamide;
and
N-(2-((2R,4R)-4-fluoro-1-methylpyrrolidin-2-yl)imidazo[1,2-a]pyrazin-6-yl)-1-methyl-1H-indazole-5-carboxamide.

A further preferred MLLT1 and/or MLLT3 binder M is a compound of formula (IVA):

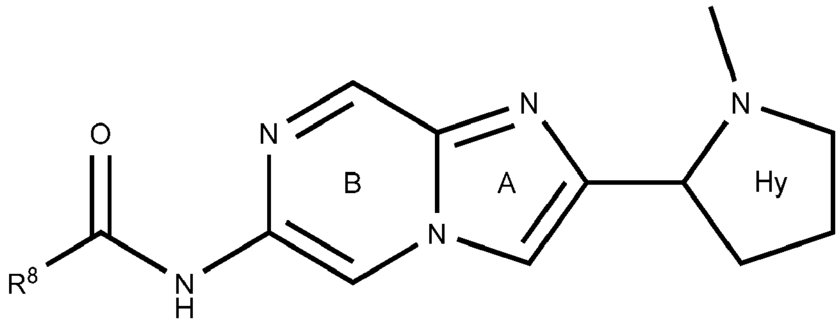

(IVA)

wherein $R^8$ is a bond to LINK.

In a most preferred embodiment of the compounds of the invention, the MLLT1 and/or MLLT3 binder M is a monovalent moiety of formula (IV):

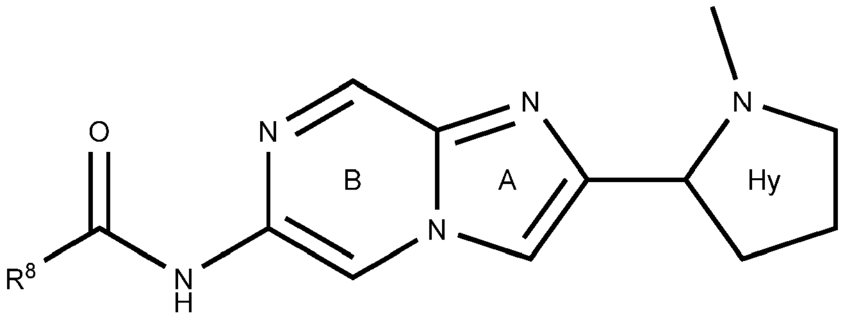

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;
wherein M is bonded to LINK via a C or N atom within group $R^8$ such that a hydrogen atom on the C or N atom within group $R^8$ is replaced with a bond to LINK.

Typically, in formula (IV), $R^8$ is a group selected from a 6- to 10-membered aryl ring and a 5- to 10-membered heteroaryl ring. The aryl and heteroaryl rings are as defined herein. For instance, $R^8$ may be selected from thiazolyl, pyrazolyl, pyrimidinyl, triazolyl, 1,2,4-oxadiazolyl, pyrazinyl, indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. More typically, in formula (IV), $R^8$ is a group selected from the following structures. For the avoidance of doubt, the following structures may be unsubstituted or substituted by one, two or three substituents as defined herein.
[see below]

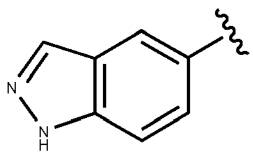
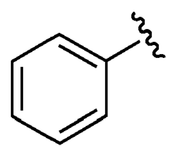
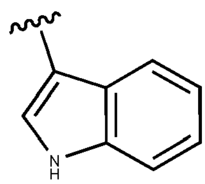
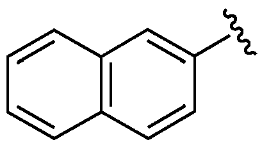

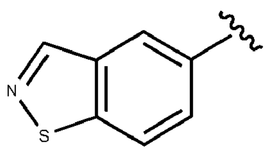
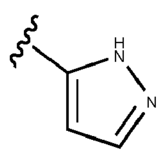
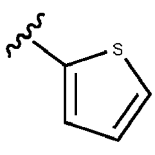

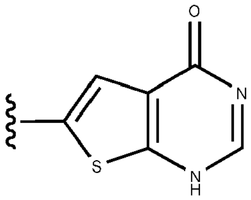 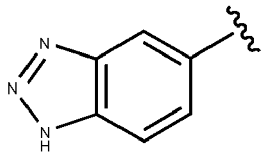 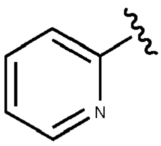 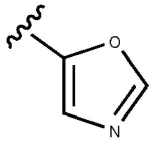

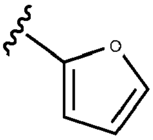 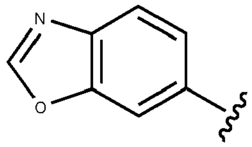 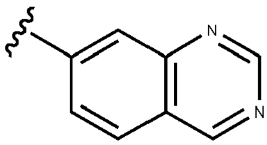

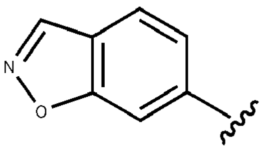 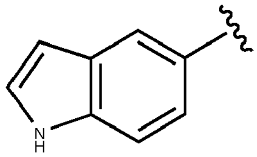 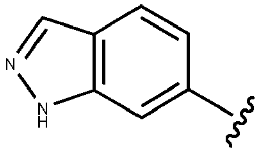 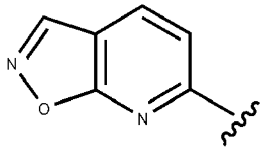

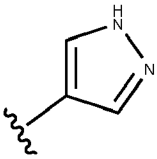 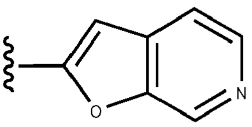 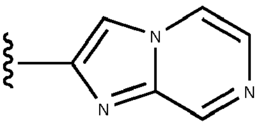 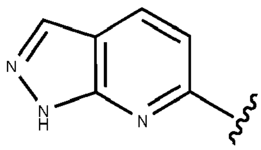

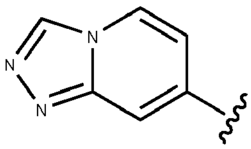 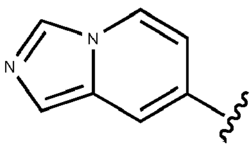 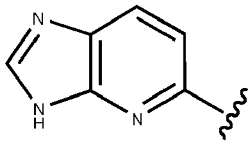 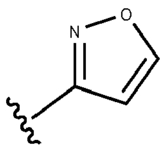

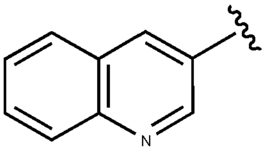 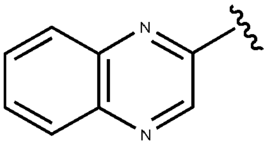 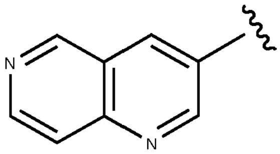 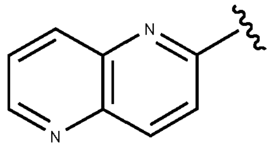

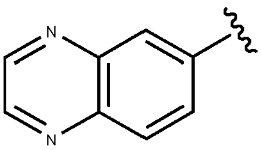 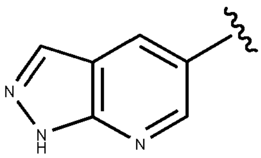 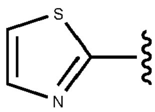 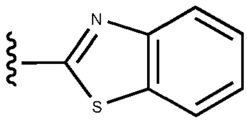

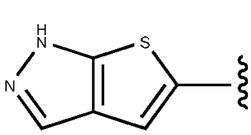 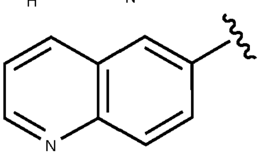 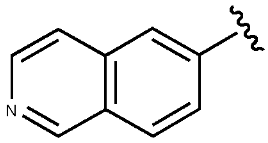

Preferably, $R^8$ is an indazolyl group or a phenyl group.

Typically, in formula (IV), $R^8$ is unsubstituted or substituted by one, two or three substituents, for instance one or two substituents, independently selected from halo and $C_{1-4}$ alkyl. Preferably, each substituent is independently selected from fluoro and methyl.

In a particularly preferred embodiment, in formula (IV), $R^8$ is selected from one of the following structures:

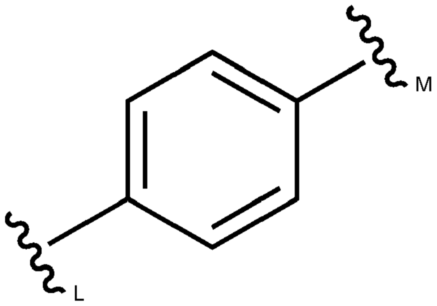

A

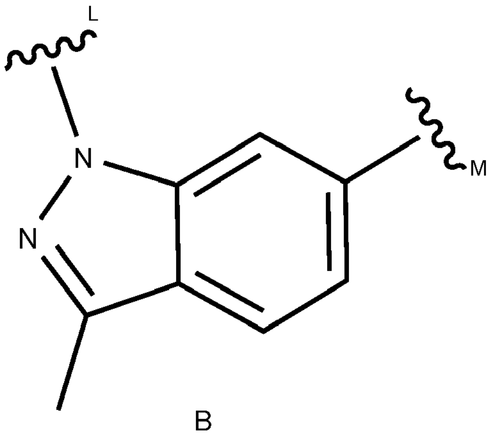

B

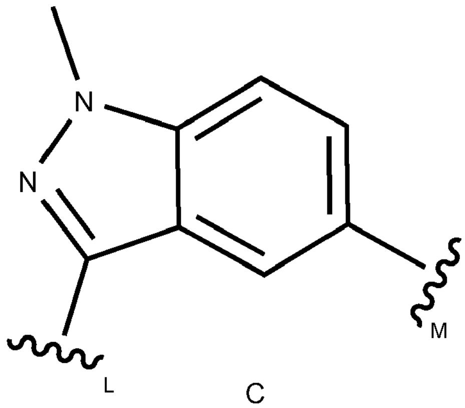

C

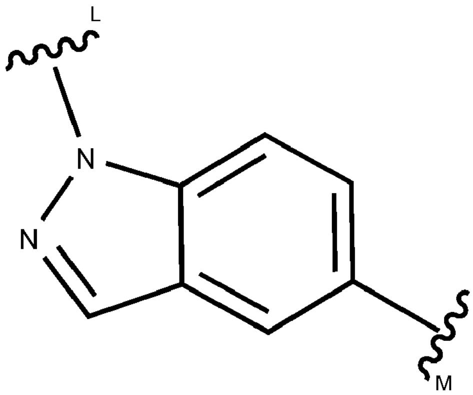

D

wherein:

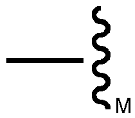

represents the point of attachment of $R^8$ to the rest of moiety M; and

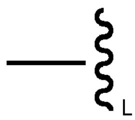

represents the point of attachment of $R^8$ to LINK.

For the avoidance of doubt, the group $R^8$ is further unsubstituted or substituted as described above. Preferably,

the group $R^8$ is further unsubstituted or substituted by one or two substituents independently selected from fluorine and methyl.

In one embodiment, the group $R^8$ is of structure A as defined above. In an alternative embodiment, the group $R^8$ is selected from structures B, C and D as defined above.

For the avoidance of doubt, $R^8$ may be selected from one of structures A, B, C and D in any of formulae (I), (II), (III), (IIIa) and (IIIb).

*E3 ubiquitin ligase binding moieties*

The presence of the E3 ubiquitin ligase binding moiety U in the compound of the invention means that the compound is a so-called "PROTAC".

The term PROTAC is an acronym for proteolysis targeting chimera. In general, PROTACs are, as is known in the art, heterobifunctional molecules that comprise two active moieties attached covalently by a linker group. In a PROTAC, the first active moiety (the MLLT1 and/or MLLT3 binder M in the compound of the present invention) binds to a target protein that is intended for degradation (target proteins for the compound of the present invention are MLLT1 and/or MLLT3). The second active moiety (U in the compound of the present invention) is capable of binding to an E3 ubiquitin ligase, thereby inducing selective intracellular proteolysis. Recruitment of the E3 ligase to the target protein results in ubiquitination and subsequent degradation of the target protein by the proteasome.

E3 ubiquitin ligase moities are known in the art. Substantially any such moiety can be used. The sole limitation on the moiety is that it be capable of binding to an E3 ubiquitin ligase. Those skilled in the art would appreciate that entirely routine laboratory methods can be used to determine whether a given substance binds to an E3 ubiquitin ligase (including but not limited to any of those disclosed specifically herein). Thus, those skilled in the art would have no difficulty in identifying E3 ubiquitin ligase moieties, nor in establishing whether any existing chemical moiety falls within the bounds of this definition. In certain embodiments, the moiety shows activity or binds to the E3 ubiquitin ligase with an $IC_{50}$ of less than about 200 mM. The $IC_{50}$ can be determined according to any method known in the art, e.g., a fluorescent polarization assay.

Merely by way of example of the extensive disclosure in the field concerning PROTACs, and hence E3 ubiquitin ligase binding moieties, reference can be made to Gu et al. (BioEssays 2018, 40, 1700247), Sun et al. (Signal Transduction and Targeted Therapy (2019) 4:64), WO 2020/041331, and WO 2019/140003, the contents of all of which are herein incorporated by reference in their entireties. Any of the numerous E3 ubiquitin ligase binding moieties disclosed in these documents can be used as an E3 ubiquitin ligase binding moiety in the compounds of the present invention. For the avoidance of doubt, Gu et al. refer to such moieties as ligands to recruit E3 ubiquitin ligase, Sun et al. refer to such moieties as E3 ubiquitin ligase (E3) recruiting ligands, in WO 2020/041331 such moieties are referred to as a "ULM" or (small molecule) E3 ubiquitin ligase binding moiety (that binds an E3 ubiquitin ligase) (and noting that the term "ULM" includes each of "ILM", "CLM", "VLM" and "MLM", any of which can be used in the present compounds), and in WO 2019/140003 such moieties (labelled "B" in WO 2019/140003's formula (I)) are referred to as a ubiquitin ligase ligand/binder.

Further examples of documents disclosing suitable moieties that can be used in the compounds of the present invention are WO2013/106643, US2016/0045607, WO2014187777, US20140356322 and US 9,249, 153, US2016/0058872, US2015/0291562 and Winter et al (Science, June 19, 2015, p. 1376), the contents of all of which are herein incorporated by reference in their entireties. Thalidomide, lenalidomide, pomalidomide and analogs thereof are still further examples of suitable moieties.

Examples of E3 ubiquitin ligases include von Hippel-Lindau (VHL) and cereblon (CRBN).

Preferably the E3 ubiquitin ligase binding moiety (U) is capable of binding to CRBN or VHL. Such moieties are referred to herein as CRBN or VHL E3 ubiquitin ligase binding moieties.

In one embodiment, U is a CRBN E3 ubiquitin ligase binding moiety. In another embodiment, U is a VHL E3 ubiquitin ligase binding moiety.

In some embodiments, the E3 ubiquitin ligase binding moiety (U) is:

(a) a CRBN E3 ubiquitin ligase binding moiety of formula (U1):

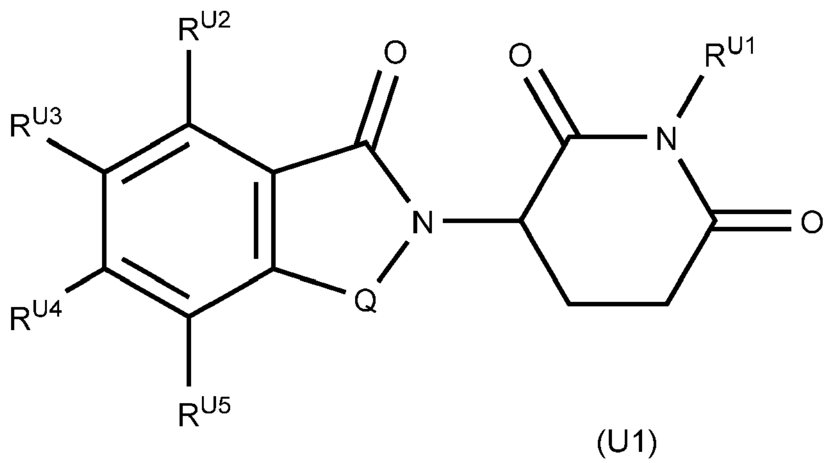

(U1)

wherein:

$R^{U1}$ is H;
Q is selected from $-CH(R^{U6})-$, $-N(R^{U6})-$, -O-, -C(O)-, $-NH-CH(R^{U6})-$, $-N=C(R^{U6})-$, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

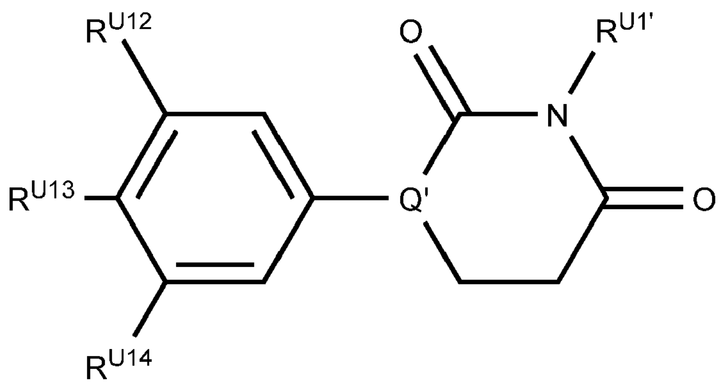

(U4)

wherein:

$R^{U1'}$ is H;
Q' is selected from CH and N;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

or (c) a VHL E3 ubiquitin ligase binding moiety of formula (U2):

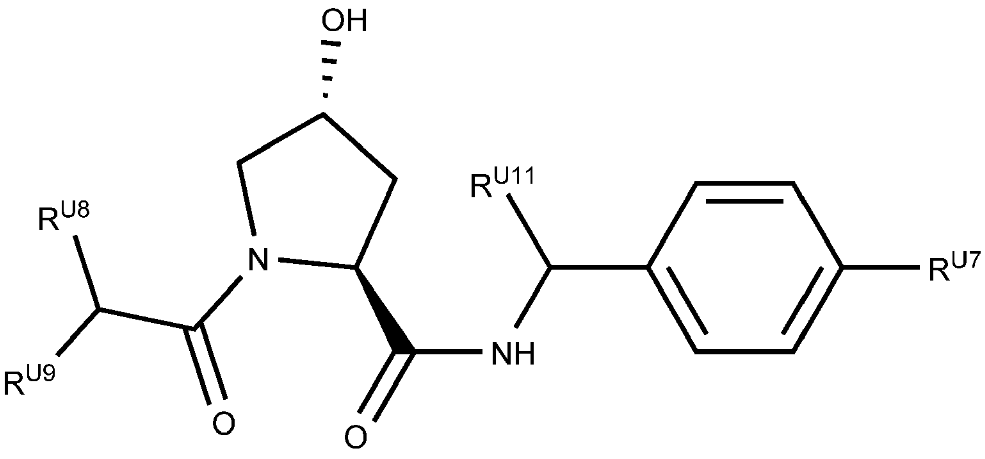

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

For the avoidance of doubt, in formula (U1), when Q is -NH-CH($R^{U6}$)- or -N=C($R^{U6}$)-, the N atom of Q is bonded to the phenyl ring in U, and the C atom of Q (not including any C atom that may be in $R^{U6}$) is bonded to the N atom in U that is adjacent to group Q.

Typically, in formula (U1), Q is selected from -CH($R^{U6}$)- and -C(O)-. Preferably, Q is selected from -$CH_2$- and -C(O)-.

Typically, in formula (U1), $R^{U6}$ is H or methyl. Preferably, $R^{U6}$ is H.

Typically, in formula (U1), $R^{U2}$ and $R^{U5}$ are each independently selected from H, fluoro, methyl, $NH_2$, $NO_2$, OH, COOH, CN, $CF_3$, and a single bond to LINK. Preferably, $R^{U2}$ and $R^{U5}$ are each independently selected from H and a single bond to LINK.

Typically, in formula (U1), $R^{U3}$ and $R^{U4}$ are each independently selected from H, fluoro, methyl, $NH_2$, $NO_2$, OH, COOH, CN, $CF_3$ and a single bond to LINK. Preferably, $R^{U3}$ and $R^{U4}$ are each independently selected from H and a single bond to LINK.

Typically, in formula (U1), two or three of $R^{U2}$, R $^{U3}$, $R^{U4}$ and $R^{U5}$ are H. Preferably, three of $R^{U2}$, $R^{u3}$, $R^{U4}$ and $R^{U5}$ are H. More preferably, $R^{U2}$ and $R^{U3}$ are H, one of $R^{U4}$ and $R^{U5}$ is a single bond to LINK, and the other of $R^{U4}$ and $R^{U5}$ is H.

Typically, therefore, in formula (U1):

Q is selected from -CH($R^{U6}$)- and -C(O)-;
$R^{U6}$ is H or methyl;
$R^{U2}$ and $R^{U5}$ are each independently selected from H, fluoro, methyl, $NH_2$, $NO_2$, OH, COOH, CN, $CF_3$, and a single bond to LINK;
$R^{U3}$ and $R^{U4}$ are each independently selected from H, fluoro, methyl, $NH_2$, $NO_2$, OH, COOH, CN, $CF_3$ and a single bond to LINK; and
two or three of $R^{U2}$, R $^{U3}$, $R^{U4}$ and $R^{U5}$ are H.

Preferably, in formula (U1):

Q is selected from -$CH_2$- and -C(O)-;
$R^{U6}$ is H;
one of $R^{U2}$, $R^{u3}$, $R^{U4}$ and $R^{U5}$ is a single bond to LINK, and the rest are H, for instance
wherein $R^{U2}$ and $R^{U3}$ are H, one of $R^{U4}$ and $R^{U5}$ is a single bond to LINK, and the other of $R^{U4}$ and $R^{U5}$ is H.

Typically, in formula (U4), Q' is selected from CH and N. Preferably, Q' is N.

Typically, in formula (U4), $R^{U12}$, $R^{U13}$ and $R^{U14}$ are each independently selected from H, fluoro, methyl, $NH_2$, $NO_2$, OH, COOH, CN, $CF_3$, and a single bond to LINK (wherein one and only one of $R^{U12}$, $R^{U13}$ and $R^{U14}$ is a single bond to LINK). Preferably, one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK.

Typically, therefore, in formula (U4):

Q' is selected from CH and N; and
$R^{U12}$, $R^{U13}$ and $R^{U14}$ are each independently selected from H, fluoro, methyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK.

Preferably, in formula (U4):

Q' is N;
one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK.

Typically, in formula (U2), $R^{U7}$ is a 5- to 6-membered heteroaryl ring. Preferably, $R^{U7}$ is a 5-membered heteroaryl ring containing one S atom and optionally one N atom.

Typically, in formula (U2), $R^{U7}$ is unsubstituted or substituted by $C_{1-4}$ alkyl. Preferably, $R^{U7}$ is unsubstituted or substituted by methyl.

Typically, in formula (U2), $R^{U11}$ is H or methyl. Preferably, $R^{U11}$ is H.

Typically, in formula (U2), $R^{U8}$ is $C_{1-4}$ alkyl or phenyl. Preferably, $R^{U8}$ is t-butyl or phenyl, more preferably t-butyl.

Typically, therefore, in formula (U2), $R^{U7}$ is a 5- to 6-membered heteroaryl ring, the group $R^{U7}$ being unsubstituted or substituted by $C_{1-4}$ alkyl, $R^{U11}$ is H or methyl, and $R^{U8}$ is $C_{1-4}$ alkyl or phenyl.

Preferably, in formula (U2), $R^{U7}$ is a 5-membered heteroaryl ring containing one S atom and optionally one N atom, the group $R^{U7}$ being unsubstituted or substituted by methyl, $R^{U11}$ is H, and $R^{U8}$ is t-butyl or phenyl, more preferably t-butyl.

In a preferred embodiment, the E3 ubiquitin ligase binding moiety (U) is a VHL E3 ubiquitin ligase binding moiety of formula (U3):

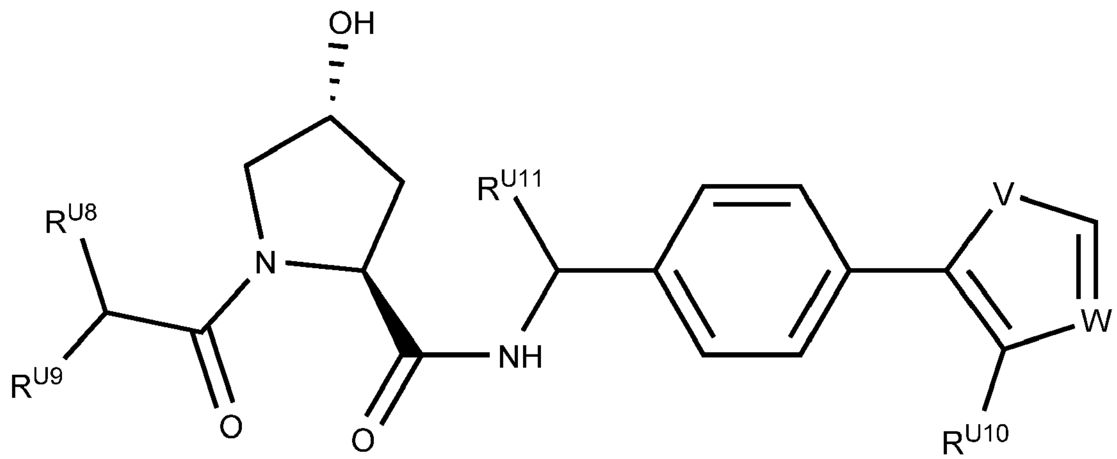

(U3)

wherein:

Vis S or O;
W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

Typically, in formula (U3), V is S.

Typically, in formula (U3), W is N or CH. Preferably, W is N.

Typically, in formula (U3), $R^{U10}$ is H or methyl. Preferably, $R^{U10}$ is methyl.

Typically, in formula (U3), $R^{U11}$ is H or methyl. Preferably, $R^{U11}$ is H.

Typically, in formula (U3), $R^{U8}$ is $C_{1-4}$ alkyl or phenyl. Preferably, $R^{U8}$ is t-butyl or phenyl, more preferably t-butyl.

Typically, therefore, in formula (U3), V is S, W is N or CH, $R^{U10}$ is H or methyl, $R^{U11}$ is H or methyl, and $R^{U8}$ is $C_{1-4}$ alkyl or phenyl. Preferably, V is S, W is N, $R^{U10}$ is methyl, $R^{U11}$ is H, $R^{U8}$ is t-butyl or phenyl, more preferably t-butyl.

*Linkers*

As discussed herein, in the compounds of the invention, the binder of MLLT1 and/or MLLT3 (M) is covalently attached to an E3 ubiquitin ligase binding moiety (U). This covalent attachment is labelled LINK and may be a direct single bond or a divalent chemical linker group that forms covalent bonds both to the binder of MLLT1 and/or MLLT3 (M), and to the E3 ubiquitin ligase binding moiety (U). Typically, LINK is a divalent chemical linker group that forms covalent bonds both to the binder of MLLT1 and/or MLLT3 (M), and to the E3 ubiquitin ligase binding moiety (U).

There is no particular limitation on the nature of LINK in the compounds of the present invention (beyond that the respective active moieties, e.g., U and M, are able to exert their desired function and LINK is capable of covalently attaching them together). Those skilled in the art would recognise that chemical linker groups are routinely used in the construction of bifunctional molecules and would be able routinely to provide appropriate chemical linker groups for attaching particular U and M moieties together. Typically, a chemical linker group for use in the present invention is an organic group.

When LINK is a divalent chemical linker group, it may be represented by formula (L):

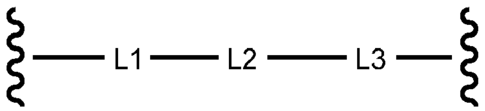

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-, $-S(O_2)N(R')-$, $-N(R')S(O_2)-$, -($C_{1-6}$ alkylene)-, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

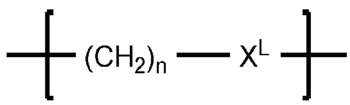
;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30; and
each R' is independently selected from H and $C_{1-4}$ alkyl.

Typically, in formula (L), L1 is selected from a single bond, -C(O)N(R')-, -N(R')C(O)-, -O-, $-S(O_2)N(R')-$, $-N(R')S(O_2)-$, and a divalent 4- to 7-membered heterocyclyl ring. When present, the divalent 4- to 7-membered heterocyclyl ring of L1 typically contains at least one N atom, for instance one or two N atoms. Preferably, when L1 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane. Preferably, in formula (L), L1 is selected from a single bond, -C(O)N(H)-, -N(H)C(O)-, and a divalent moiety of piperidine or diazinane.

Preferably, where the group $R^8$ within the MLLT1 and/or MLLT3 binder M is (i) a bond to LINK, then L1 within LINK is also a single bond. In this embodiment, L2 within LINK is direcly bonded to the group L within the MLLT1 and/or MLLT3 binder M.

Typically, in formula (L), L3 is selected from a single bond, -N(R')-, -C(O)N(R')-, -O-, -N(R')C(O)-, -S($O_2$)N(R')-, -N(R')S($O_2$)-, and a divalent 4- to 7-membered heterocyclyl ring. When present, the divalent 4- to 7-membered heterocyclyl ring of L3 typically contains at least one N atom, for instance one or two N atoms. Preferably, when L3 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane, preferably 1,4-diazinane. Preferably, in formula (L), L3 is selected from a single bond, -N(H)-, - C(O)N(H)-, -O-, -N(H)C(O)-, and a divalent moiety of piperidine or diazinane.

L2 is represented by the formula $-(L4)_m-$. Typically, in formula (L), each L4 is independently selected from a divalent 4- to 7-membered heterocyclyl ring and a unit of formula

$$-\left[(CH_2)_n - X^L\right]-$$

as described above. Typically, each $X^L$ is independently selected from a single bond, -O-, or -S-, and n is selected from 1 or 2. In one embodiment, each L4 is the same. Alternatively, L2 may comprise two or more blocks, wherein in each block, each L4 is the same. For instance, L2 may be represented by the formula

$-(L4')_p-(L4'')_q-(L4''')_r-$, wherein L4', L4" and L4‴ are selected from those moieties described above for L4, and wherein each L4' is the same, each L4" is the same and each L4‴ is the same. Preferably, L4', L4" and L4‴ are selected from $-CH_2-$, $-CH_2CH_2O-$, $-OCH_2CH_2-$, $-CH_2CH_2S-$ and $-SCH_2CH_2-$. More preferably, L4', L4" and L4‴ are selected from $-CH_2-$, $-CH_2CH_2O-$ and $-OCH_2CH_2-$. p, q and r are integers of from 0 to 30, wherein p+q+r=m.

Preferably, each L4 unit is the same. For instance, each L4 in formula (L) may be - $CH_2$-, each L4 in formula (L) may be $-CH_2CH_2O-$, each L4 in formula (L) may be - $OCH_2CH_2$-, each L4 in formula (L) may be $-CH_2CH_2S-$, or each L4 in formula (L) may be $-SCH_2CH_2-$. More preferably, each L4 in formula (L) is $-CH_2-$, each L4 in formula (L) is $-CH_2CH_2O-$ or each L4 in formula (L) is $-OCH_2CH_2-$. Most preferably, each L4 in formula (L) is $-CH_2-$.

Typically, in formula (L), m is selected from 1 to 10.

Typically, in formula (L), each R' is independently selected from H and methyl. Preferably, each R' is H.

Typically, therefore, in formula (L):

L1 is selected from a single bond, -C(O)N(R')-, -N(R')C(O)-, -O-, -S($O_2$)N(R')-, -N(R')S($O_2$)-, and a divalent 4- to 7-membered heterocyclyl ring typically containing at least one N atom, for instance one or two N atoms;
L3 is selected from a single bond, -N(R')-, -C(O)N(R')-, -O-, -N(R')C(O)-, -S($O_2$)N(R')-, -N(R')S($O_2$)-, and a divalent 4- to 7-membered heterocyclyl ring typically containing at least one N atom, for instance one or two N atoms;
each L4 is independently selected from $-CH_2-$, $-CH_2CH_2O-$, $-OCH_2CH_2-$, $-CH_2CH_2S-$, $-SCH_2CH_2-$, and a divalent 4- to 7-membered heterocyclyl ring;
m is selected from 1 to 10; and
each R' is independently selected from H and methyl.

Preferably, in formula (L):

L1 is selected from a single bond, -C(O)N(H)-, -N(H)C(O)-, and a divalent moiety of piperidine or diazinane;
L3 is selected from a single bond, -N(H)-, -C(O)N(H)-, -O-, -N(H)C(O)-, and a divalent moiety of piperidine or diazinane;
each L4 in formula (L) is $-CH_2-$, each L4 in formula (L) is $-CH_2CH_2O-$, each L4 in formula (L) is $-OCH_2CH_2-$, each L4 in formula (L) is $-CH_2CH_2S-$, or each L4 in formula (L) is $-SCH_2CH_2-$, with each L4 preferably being $-CH_2-$;
m is selected from 1 to 10; and
each R' is H.

In a typical embodiment, L2 is -($C_{1-10}$ alkylene)- or -($C_{1-10}$ alkylene)-C(O)-, preferably - ($C_{1-10}$ alkylene)-. In this embodiment, L1 is preferably selected from a single bond, - C(O)N(H)-, -N(H)C(O)-, and a divalent moiety of piperidine or diazinane. In this embodiment, L3 is preferably selected from a single bond, -N(H)-, -C(O)N(H)-, -O-, - N(H)C(O)-, and a divalent moiety of piperidine or diazinane. More preferably, L1 and L3 are selected from the following options:

- L1 is a single bond and L3 is -N(H)-;

- L1 is -C(O)N(H)- or -N(H)C(O)-, and L3 is -C(O)N(H)- or -N(H)C(O)-;
- L1 is a divalent moiety of piperidine and L3 is a divalent moiety of diazinane;
- L1 is -C(O)N(H)- or -N(H)C(O)-, and L3 is a divalent moiety of diazinane;
- L1 is a single bond and L3 is a divalent moiety of diazinane;
- L1 is a single bond and L3 is -O-; and
- L1 is -C(O)N(H)- or -N(H)C(O)-, and L3 is -O-.

For the avoidance of doubt, the skilled person would readily appreciate that each of the definitions of moieties U, M and LINK provided herein may be taken together in any combination to arrive at a definition of the compound of the present invention. As examples that are in no way limiting, definitions of moieties U, M and LINK labelled "typically" may be combined to arrive at a definition of a typical compound of the present invention, and definitions of moieties U, M and LINK labelled "preferably" may be combined to arrive at a definition of a preferred compound of the present invention. However, the skilled person would appreciate that a definition labelled "typically" of one moiety may be combined with a definition labelled "preferably" of another moiety to arrive at a definition of a compound of the present invention.

In preferred compounds of the present invention, M is of formula (III):

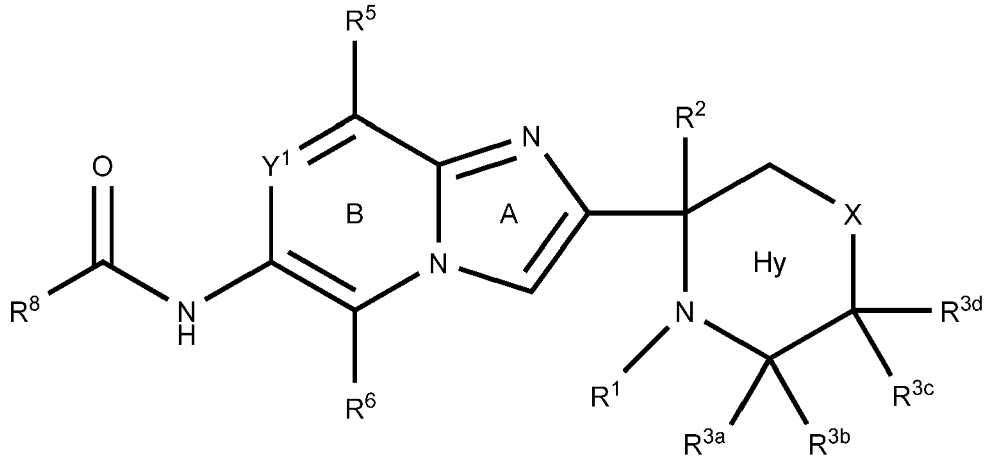

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
X is a bond, -N(Me)-, O or $-CH_2-$;
$Y^1$ is N or $-C(R^7)-$;
$R^5$, $R^6$ and $R^7$ are independently selected from H, halo $C_{1-4}$ alkoxy, and $C_{1-4}$ alkyl;
$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R_{10}$, $-(C_{1-4}$ alkylene$)-R_{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to

LINK; and LINK is (a) a single bond, or (b) a chemical linker group represented by formula (L):

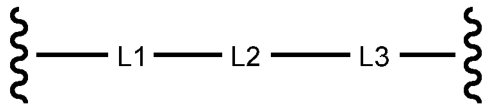

(L)

wherein:

LINK is attached to M via L1;
LINK is attached to U via L3;
when $R^8$ is (i) a bond to LINK, then L1 is a single bond and L3 is selected from a single bond, -N(R')-, -C(O)N(R')-, -O-, -N(R')C(O)-, -C(O)-, -$S(O_2)$N(R')-, -N(R')$S(O_2)$-, -($C_{1-6}$ alkylene)-, -($C_{2-6}$ alkenylene)-, a divalent 3- to 6-membered cycloalkyl ring and a divalent 4- to 7-membered heterocyclyl ring; and when $R^8$ is a group (ii), then L1 and L3 are each independently selected from a single bond, -N(R')-, -C(O)N(R')-, -O-, -N(R')C(O)-, -C(O)-, -$S(O_2)$N(R')-, -N(R')$S(O_2)$-, -($C_{1-6}$ alkylene)-, -($C_{2-6}$ alkenylene)-, a divalent 3- to 6-membered cycloalkyl ring and a divalent 4- to 7-membered heterocyclyl ring;
L2 is represented by the formula -$(L4)_m$-;
each L4 is independently selected from a divalent 3- to 6-membered cycloalkyl ring, a divalent 4- to 7-membered heterocyclyl ring and a unit of formula:

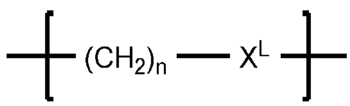

;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30;
each R' is independently selected from H and $C_{1-4}$ alkyl; and
Uis:

(a) a CRBN E3 ubiquitin ligase binding moiety of formula (U1):

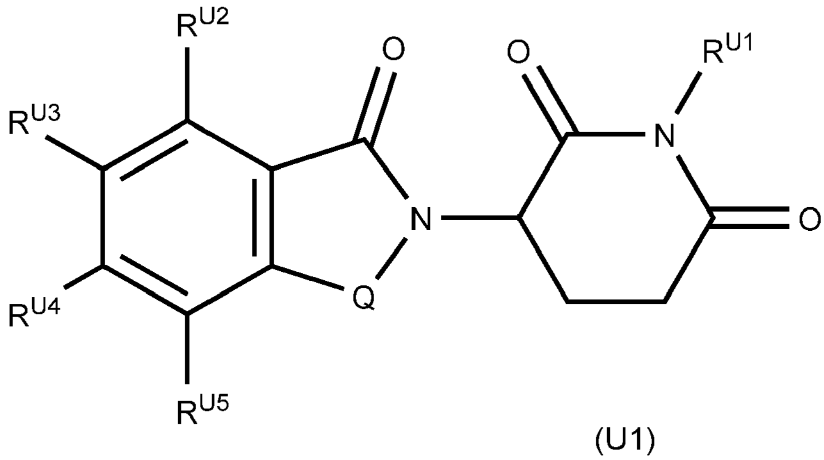

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;

$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

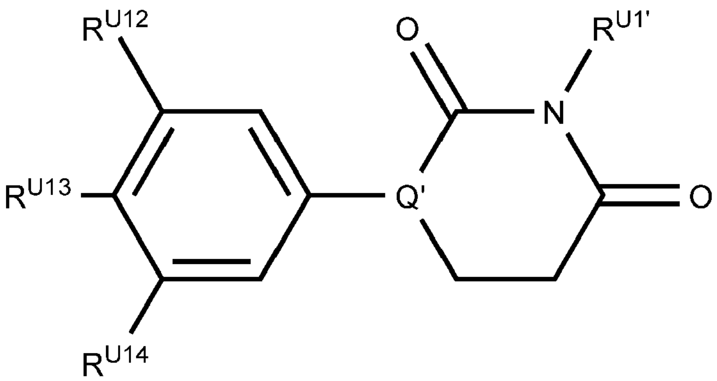

(U4)

wherein:

$R^{U1'}$ is H;
Q' is selected from CH and N;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

or (c) a VHL E3 ubiquitin ligase binding moiety of formula (U2):

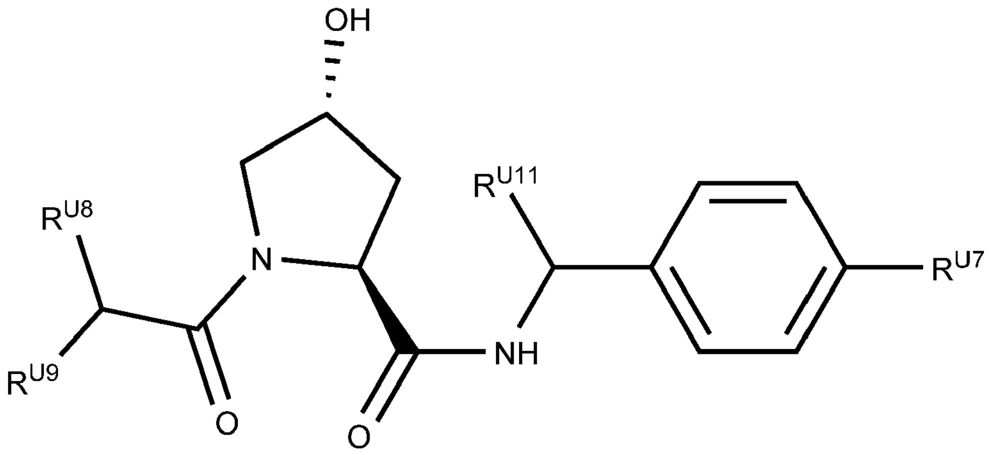

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and

$R^{U9}$ is a single bond to LINK.

In one preferred aspect of this embodiment, M is of formula (IV):

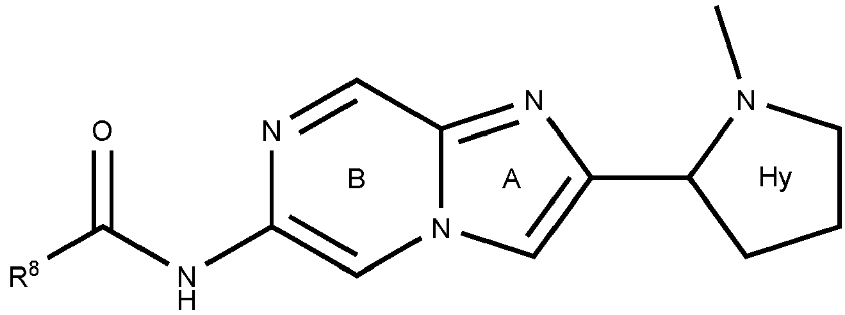

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;
wherein M is bonded to LINK via a C or N atom within group $R^8$ such that a hydrogen atom on the C or N atom within group $R^8$ is replaced with LINK.

In this embodiment, $R^8$ is preferably selected from one of the following structures:

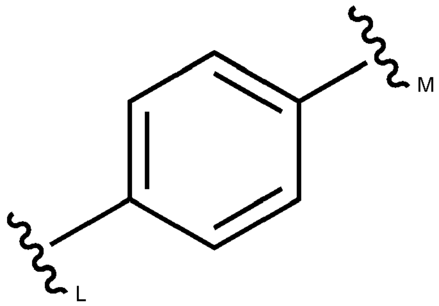

A

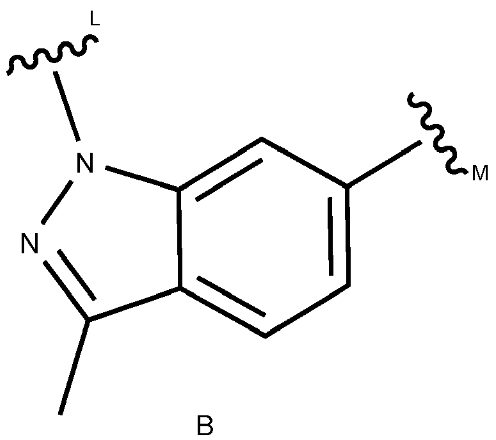

B

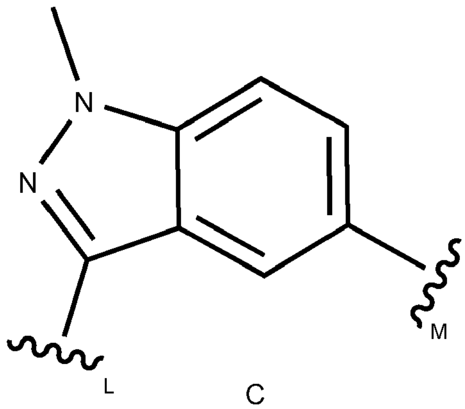

C

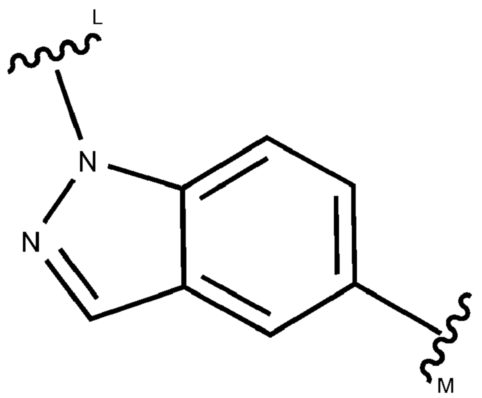

D

wherein:

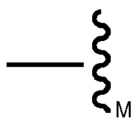

represents the point of attachment of $R^8$ to the rest of moiety M;

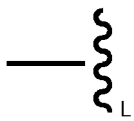

represents the point of attachment of $R^8$ to LINK; and

the group $R^8$ is further unsubstituted or substituted by one or two substituents independently selected from fluorine and methyl.

In this embodiment, L2 is preferably selected from $-(CH_2)_m-$, $-(CH_2CH_2O)_m-$, - $(OCH_2CH_2)_m-$, $-(CH_2CH_2S)_m-$, $-(SCH_2CH_2)_m-$, and a divalent 4- to 7-membered heterocyclyl ring.

In this embodiment, preferably L1 and L3 are each independently selected from a single bond, -N(R')-, -C(O)N(R')-, -N(R')C(O)-, -O-, and a divalent 4- to 7-membered heterocyclyl ring. Preferably in this embodiment, R' is H and the divalent 4- to 7-membered heterocyclyl ring is piperidine or diazinane.

In this embodiment, U is preferably (i) of formula (U1) wherein preferably Q is selected from $-CH_2-$ and -C(O)-, $R^{U6}$ is H, one of $R^{U2}$, $R^{u3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H, or (ii) of formula (U3) wherein preferably V is S, W is N, $R^{U10}$ is methyl, $R^{U11}$ is H, and $R^{U8}$ is t-butyl or phenyl, more preferably t-butyl.

In a further preferred aspect of this embodiment, M is of formula (IVA):

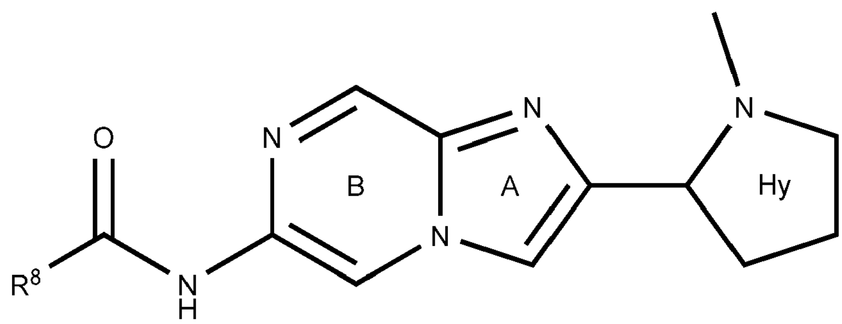

(IVA)

wherein $R^8$ is a bond to LINK;

L1 is a single bond;

L2 is selected from $-(CH_2)_m-$, $-(CH_2CH_2O)_m-$, $-(OCH_2CH_2)_m-$, $-(CH_2CH_2S)_m-$, -

$(SCH_2CH_2)_m$-, and a divalent 4- to 7-membered heterocyclyl ring;
L3 is selected from a single bond, -N(R')-, -C(O)N(R')-, -N(R')C(O)-, -O-, and a divalent 4- to 7-membered heterocyclyl ring, wherein R' is preferably H and the divalent 4- to 7-membered heterocyclyl ring is preferably piperidine or diazinane; and U is (i) of formula (U1) wherein Q is selected from $-CH_2$- and -C(O)-, $R^{U6}$ is H, one of $R^{U2}$, $R^{u3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H, or (ii) of formula (U3) wherein V is S, W is N, $R^{U10}$ is methyl, $R^{U11}$ is H, and $R^{U8}$ is *t*-butyl or phenyl, more preferably *t*-butyl.

Particularly preferred compounds of the invention may be selected from:

1-(8-{[2-(2,6-dioxopiperidin-3-yl)-1,3-dioxoisoindol-4-yl]amino}octyl)-3-methyl-N-{2-[(rel-2R)-1-methylpyrrolidin-2-yl]imidazo[1,2-a]pyrazin-6-yl}indazole-6-carboxamide;
1-(8-{[2-(2,6-dioxopiperidin-3-yl)-1,3-dioxoisoindol-4-yl]amino}octyl)-3-methyl-N-{2-[(rel-2S)-1-methylpyrrolidin-2-yl]imidazo[1,2-a]pyrazin-6-yl}indazole-6-carboxamide;
(2S,4R)-1-[(2S)-3,3-dimethyl-2-(11-{[4-({2-[(rel-2R)-1-methylpyrrolidin-2-yl]imidazo[1,2-a]pyrazin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide;
(2S,4R)-1-[(2S)-3,3-dimethyl-2-(11-{[4-({2-[(rel-2S)-1-methylpyrrolidin-2-yl]imidazo[1,2-a]pyrazin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide;
7-{[2-(2,6-dioxopiperidin-3-yl)-1,3-dioxoisoindol-4-yl]amino}-N-{2-[(2R)-1-methylpyrrolidin-2-yl]imidazo[1,2-a]pyrazin-6-yl}heptanamide;
and the pharmaceutically acceptable salts thereof.

In a separate aspect of the present invention, the following MLLT1 and/or MLLT3 inhibitors are provided:

N-[8-cyclopropyl-2-[(2R)-1-methylpyrrolidin-2-yl]imidazo[1,2-a]pyrazin-6-yl]-1-methyl-indazole-5-carboxamide;
N-[8-cyclopropyl-2-[(2R)-1-methylpyrrolidin-2-yl]imidazo[1,2-a]pyrazin-6-yl]-5-fluoro-1,3-dimethyl-indazole-6-carboxamide;
6-fluoro-1-methyl-N-[2-[(2R)-1-methylpyrrolidin-2-yllimidazo[1,2-alpyrazin-6-yl]indazole-5-carboxamide;
1-methyl-N-[2-[(6R)-5-methyl-5-azaspiro[2.4]heptan-6-yl]imidazo[1,2-a]pyrazin-6-yl]indazole-5-carboxamide;
N-{2-[(rel-2R)-1-ethylpyrrolidin-2-yl]imidazo[1,2-a]pyrazin-6-yl}-1,3-dimethylindazole-6-carboxamide;
N-{2-[(rel-2S)-1-ethylpyrrolidin-2-yl]imidazo[1,2-a]pyrazin-6-yl}-1,3-dimethylindazole-6-carboxamide;
N-(2-((2R, rel-4S)-1,4-dimethylpyrrolidin-2-yl)imidazo[1,2-a]pyrazin-6-yl)-1-methyl-1H-indazole-5-carboxamide;
N-(2-((2R,rel-4R)-1,4-dimethylpyrrolidin-2-yl)imidazo[1,2-a]pyrazin-6-yl)-1-methyl-1H-indazole-5-carboxamide;
N-{2-[(rel-2R,4R)-4-methoxy-1-methylpyrrolidin-2-yl]imidazo[1,2-a]pyrazin-6-yl}-1-methylindazole-5-carboxamide;
N-{2-[(rel-2S,4R)-4-methoxy-1-methylpyrrolidin-2-yl]imidazo[1,2-a]pyrazin-6-yl}-1-methylindazole-5-carboxamide;
N-(2-((2R,4R)-4-fluoro-1-methylpyrrolidin-2-yl)imidazo[1,2-a]pyrazin-6-yl)-1-methyl-1H-indazole-5-carboxamide;
and the pharmaceutically acceptable salts thereof.

*Therapeutic Efficacy*

The compounds of the present invention are therapeutically useful. The present invention therefore provides compounds as described herein, for use in medicine. The present invention provides compounds as described herein, for use in treating the human or animal body. For the avoidance of doubt, the agent may comprise a compound of the invention in the form of a solvate. Also provided is a pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.
The composition of the invention may be provided as a kit comprising instructions to enable the kit to be used in the methods described herein or details regarding which subjects the method may be used for.
As explained above, the compounds of the invention are useful in treating or preventing various disorders. Disorders for treatment using the compounds of the invention may include cancer. In particular, the compounds of the invention are useful for inducing selective degradation of MLLT1 and/or MLLT3.
Cancer, e.g. acute lymphoblastic leukemia, acute myeloid leukemia, adrenocortical carcinoma, aids-related lymphoma, primary CNS lymphoma, anal cancer, astrocytomas, brain cancer, basal cell carcinoma, bile duct cancer,

bladder cancer, bone cancer (e.g. ewing sarcoma, osteosarcoma and malignant fibrous histiocytoma), breast cancer, bronchial tumors, medulloblastoma and other CNS embryonal tumors, cervical cancer, chronic lymphocytic leukemia, chronic myelogenous leukemia, chronic myeloproliferative neoplasms, colorectal cancer, craniopharyngioma, endometrial cancer, ependymoma, esophageal cancer, esthesioneuroblastoma, ewing sarcoma, extragonadal germ cell tumor, intraocular melanoma, retinoblastoma, fallopian tube cancer, gallbladder cancer, gastric cancer, gastrointestinal carcinoid tumor, gastrointestinal stromal tumors (gist), germ cell tumors, extragonadal germ cell tumors, ovarian germ cell tumors, testicular cancer, gestational trophoblastic disease, hairy cell leukemia, hepatocellular cancer, histiocytosis, langerhans cell, hodgkin lymphoma, hypopharyngeal cancer, intraocular melanoma, islet cell tumors, pancreatic neuroendocrine tumors, kaposi sarcoma, kidney (renal cell) cancer, langerhans cell histiocytosis, laryngeal cancer, leukemia, liver cancer, lung cancer (non-small cell, small cell, pleuropulmonary blastoma, and tracheobronchial tumor), lymphoma, malignant fibrous histiocytoma of bone and osteosarcoma, merkel cell carcinoma, mesothelioma, mouth cancer, multiple endocrine neoplasia syndromes, multiple myeloma/plasma cell neoplasms, mycosis fungoides, myelodysplastic syndromes, myelodysplastic/ myeloproliferative neoplasms, myelogenous leukemia, neuroblastoma, non-hodgkin lymphoma, oropharyngeal cancer, osteosarcoma and undifferentiated pleomorphic sarcoma, pancreatic cancer, pancreatic neuroendocrine tumors (islet cell tumors), papillomatosis, paraganglioma, parathyroid cancer, penile cancer, pharyngeal cancer, pheochromocytoma, pituitary tumor, prostate cancer, rectal cancer, retinoblastoma, rhabdomyosarcoma, t-cell lymphoma, testicular cancer, throat cancer, thymoma and thymic carcinoma, thyroid cancer, tracheobronchial tumors and the like is particularly suitable to being treated with the compounds of the invention provided herein.

Typically, the compounds of the invention are for use in treating cancers which are transcriptionally addicted cancers, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma and sarcoma (Cell Rep, 2021, 16, 1087).

Preferably, the compounds of the invention are for use in treating acute myeloid leukaemia (AML) or acute lymphoblastic leukaemia (ALL), such as mixed-lineage leukaemia (MLL) rearranged acute leukemia (AML and ALL) and NPM1 mutant leukemia.

The compounds of the invention may be used as standalone therapeutic agents. Alternatively, they may be used in combination with other active agents such as chemotherapeutic agents, targeted precision medicines or immune oncology agents. For example, they may be used in combination with a Bcl2 targeted drug (for instance venetoclax), FLT3 inhibitor (for instance sorafenib, quizartinib or gilteritinib), EGFR inhibitor (for instance erlotinib, gefitinib, lapatinib or osimertinib), CDK4/6 inhibitor (for instance abemaciclib, palbociclib or ribociclib), a chemotherapy (for instance cytarabine, doxorubicin, gemcitabine, cisplatin or paclitaxel) or an immune checkpoint inhibitor (for instance pembrolizumab, nivolumab, durvalumab or cemiplimab).

When used to treat a cancer, the compounds of the invention may be used in alleviating, ameliorating or preventing aggravation of the symptoms of the cancer. Typically, treating a cancer may comprise reducing progression of the cancer, e.g. increasing progression free survival (PFS) and/or increasing survival e.g. increasing overall survival (OS). Treating a cancer may comprise preventing or inhibiting growth of a tumour associated with the cancer. Treating a cancer may comprise preventing metastasis of the cancer. Preferably, treating a cancer may comprise reducing the size of a tumour associated with the cancer. As such, the treatment may cause tumour regression in the cancer. Treating a cancer may comprise reducing the number of tumours or lesions present in the patient. When the treatment reduces the size of a tumour associated with the cancer, the size of the tumour is typically reduced from base line by at least 10%. Base line is the size of the tumour at the date treatment with the compound is first started. The size of the tumour is typically as measured in accordance with version 1.1 of the RECIST criteria (for instance as described in Eisenhauer et al, European Journal of Cancer 45 (2009) 228-247).

The response to the treatment with the compound may be complete response, partial response or stable disease, in accordance with version 1.1 of the RECIST criteria. Preferably, the response is partial response or complete response. The treatment may achieve progression free survival for at least 60 days, at least 120 days or at least 180 days.

The reduction in tumour size may be greater 20%, greater than 30% or greater than 50% reduction relative to base line. The reduction in tumour size may be observed after 30 days of treatment or after 60 days of treatment.

In haematological cancers, treating a cancer preferentially may lead to complete remission (CR) of the cancer or it may lead to complete remission with incomplete haematological recovery (CRh) or complete remission but with incomplete platelet recovery (CRp). Additionally, treatment may lead to an increase in duration of remission (DoR), an increase in minimal residual disease (MRD), or an increase in relapse free survival (RFS) (Blood 2007 109 1815 and Leukemia Res 2018, 68, 32).

As explained here, the compounds of the invention are useful in treating or preventing various disorders. The present invention therefore provides a compound of the invention for use in medicine. The invention also provides the

use of a compound of the invention in the manufacture of a medicament. The invention also provides compositions and products comprising the compounds of the invention. Such compositions and products are also useful in treating or preventing disorders. The present invention therefore provides a composition or product as defined herein for use in medicine. The invention also provides the use of a composition or product of the invention in the manufacture of a medicament. Also provided is a method of treating a subject in need of such treatment, said method comprising administering to the subject a compound of the invention. In some embodiments the subject suffers from or is at risk of suffering from one of the disorders disclosed herein.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

A subject is typically a human patient. The patient may be male or female. The age of the patient is typically at least 18 years, for instance from 30 to 70 years or from 40 to 60 years. Alternatively, the patient may be a child, for instance the patient may be under 18 years of age, or under 12 years of age. In one embodiment, the patient is a child under ten years of age or an infant under two years of age. In one embodiment the invention provides a compound as defined herein for use in treating cancer in paediatric patients.

A compound or composition of the invention can be administered to the subject in order to treat one or more symptoms of the disorder. In this embodiment, the subject is typically symptomatic. A therapeutically effective amount of the agent or formulation is administered to such a subject. A therapeutically effective amount is an amount effective to ameliorate one or more symptoms of the disorder.

The compound or composition of the invention may be administered in a variety of dosage forms. Thus, it can be administered orally, for example as tablets, troches, lozenges, aqueous or oily suspensions, dispersible powders or granules. The compound or composition of the invention may also be administered parenterally, whether subcutaneously, intravenously, intramuscularly, intrasternally, transdermally or by infusion techniques. The compound or composition may also be administered as a suppository. Preferably, the compound, composition or combination may be administered orally.

The compound or composition of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

A therapeutically effective amount of the compound or composition of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific compound, the age, weight and conditions of the subject to be treated, the type and severity of the disease and the frequency and route of administration. Preferably, daily dosage levels are from 5 mg to 2 g.

When the compound or composition of the invention is administered to a subject in combination with another active agent, the dose of the other active agent can be determined as described above. The dose may be determined according to various parameters, especially according to the agent used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the

activity of the specific agent, the age, weight and conditions of the subject to be treated, the type and severity of the disease and the frequency and route of administration. Preferably, daily dosage levels are from 5 mg to 2 g.

*Synthesis*

Compounds of the invention can be prepared by the synthetic methods described in the Examples that follow, or by analogy with such methods using appropriate starting materials and methodologies familiar to the skilled chemist.
The following examples illustrate the invention. They do not however limit the invention in any way. In this regard, it is important to understand that the particular assay used in the Examples section is designed only to provide an indication of biological activity. There are many assays available to determine biological activity, and a negative result in any one particular assay is therefore not determinative. The invention is defined according to the claims.

**Examples**

In the examples which follow below, compounds may be produced in racemic form. Compounds in racemic form are indicated by use of the term rac- in advance of the IUPAC name. In some instances, as indicated, enantiomers and/or diastereomers are separated. In general, the enantiomers and/or diastereomers of the invention can be separated using chiral chromatography. The separated enantiomers and/or diastereomers may be identified in the order in which they elute, for example the first, second, third or fourth eluting isomer respectively, as obtained by separation using chiral chromatography. The isomers may alternatively or additionally be identified using IUPAC naming conventions. Thus, where enantiomers and/or diastereomers have been assigned an absolute stereochemistry, this is indicated as R or S stereochemistry, as appropriate, and by use of a wedge-shaped bond (e.g. 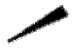 )in the chemical formula.
Where enantiomers have been separated but have undefined absolute stereochemistry the term "rel-R" or "rel-S" has been used. In these examples it is understood that the absolute configuration is unknown and the "rel-R" and "rel-S" labels are used merely to distinguish the two enantiomers form one another. The absolute stereochemistry of an enantiomer labelled as "rel-R" may be either R or S. In either case, the corresponding "rel-S" enantiomer has the opposite configuration to the "rel-R" enantiomer. In these examples the use of a rectangular bond (e.g. 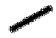 ) has been used to define the "rel" configuration.
Alternatively, where diastereomers have been separated and the absolute configuration is unknown they may be assigned a relative stereochemistry, which denotes the stereochemistry of a first chiral centre with respect to a second chiral centre. Where a relative stereochemistry has been assigned, this is indicated by the term "rel" in the IUPAC name, and by use of a rectangular-shaped bond (e.g. 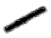 ). Where diastereomers have been separated and the relative and absolute configuration is unknown, the compounds have been labelled by the order in which they elute, for example the first, second, third or fourth eluting isomer respectively, as obtained by separation using chiral chromatography.
Temperatures are given in degrees Celsius (°C). The reactants used in the examples below may be obtained from commercial sources or they may be prepared from commercially available starting materials as described herein or by methods known in the art. All the compounds of the invention are synthesized according to the Examples described herein. The progress of the reactions described herein were followed as appropriate by e.g. LC, GC or TLC, and as the skilled person will readily realize, reaction times and temperatures may be adjusted accordingly.

**NMR spectroscopy:**

Solids/oils were solubilized in DMSO-$d_6$ or MeOH-$d_4$, vortexed vigorously until the solution was clear and transferred to an NMR tube for data acquisition.
Liquid-state NMR experiments were recorded using:

- 600 MHz (14.1 Tesla) Bruker Avance III NMR spectrometer (600 MHz for $^1$H, 151 MHz for $^{13}$C) using a triple-resonance $^1$H, $^{15}$ N, $^{13}$C CP-TCI 5 mm cryoprobe (Bruker Biospin, Germany)
- 500 MHz (11.75 Tesla) Bruker Avance I NMR spectrometer (500 MHz for $^1$H, 125 MHz for $^{13}$C) using a Dual Resonance BBI 5 mm probe (Bruker Biospin, Germany)
- 400 MHz (9.4 Tesla) Bruker Avance NEO NMR spectrometer (400 MHz for $^1$H, 100 MHz for $^{13}$C) using a SEI 5 mm probe (Bruker Biospin, Germany)
- 400 MHz (9.4 Tesla) Bruker Avance NEO NMR spectrometer using a PI HR-BBO400S1-BBF/H/D-5.0-Z SP probe (Bruker Biospin, Germany)
- 300 MHz Bruker AVANCE III HD NMR spectrometer using a PA BBO 300S1 BBF-H-D-05 Z probe (Bruker Biospin, Germany)

- 300 MHz Bruker Avance NEO NMR spectrometer using a PA BBO BBF-H-D-05 Z (Bruker Biospin, Germany)

All the experiments used for the resonance assignment procedure and the elucidation of the products structure (1D $^1H$, 2D 1H-$^1H$-COSY, 2D 1H-$^1H$-ROESY, 2D $^1H$-$^{13}C$-HSQC, 2D $^1H$-$^{13}C$-HMBC) were recorded at 300 K. $^1H$ chemical shifts are reported in δ ppm as s (singlet), d (doublet), t (triplet), q (quartet), dd (double doublet), m (multiplet) or br s (broad singlet).

**UPLC-MS chromatography:**

UPLC-MS chromatography analysis were recorded using the following apparatus using:

- Waters UPLC: Acquity, UV: Acquity PDA, MS: Qda, ELSD
- Waters UPLC: Acquity, UV: Acquity TUV, MS: Qda
- Waters UPLC: Acquity, UV: Acquity PDA, MS: QDa

The apparatus was tested using a CSH C18 Waters column (50 x 2.1 mm), 1.7 μm. It used a combination of the following eluents: $H_2O$ + 0.05% TFA (v/v) (solvent A) and MeCN + 0.035% TFA (v/v) (solvent B) and a positive electrospray ES+ as ionization mode. The UV detection was set at 220 and 254 nm. The methods used were the following:

- Polar method (1.7 min run): gradient t=0 2% B, t=0.5 min 2% B, t=1.5 min 98% B, t=1.52 min 2% B, t=1.7 min 2% B
- Polar method (3.5 min run): gradient t=0 2% B, t=1.0 min 2% B, t=2.4 min 98% B, t=3.00 min 98% B, t=3.03 min 2% B, t=3.5 min 2% B
- Normal method (1.7 min run): gradient t=0 2% B, t=1.0 min 98% B, t=1.5 min 98% B, t=1.52 min 2% B, t=1.7 min 2% B
- Normal method (3.5 min run): gradient t=0 2% B, t=2.4 min 98% B, t=3.0 min 98% B, t=3.03 min 2% B, t=3.5 min 2% B

Where it is observed mass spectra analysis is reported as $[M+H]^+$ for the molecular ion; Someone skilled in the art will also appreciate that isotope patterns may be reported where evident, for example $[M+H+2]^+$ represents the isotopic peak of Br if it is present in the molecule; Additionally, some fragmentation ions may be included in the analysis, for example [M+H-*t*Bu]$^+$, $[M+H-Boc]^+$ and $[M-Cl+MeOH]^+$.

**Abbreviations:**

In addition to the definitions above, the following abbreviations are used in the Example experimental procedures below. If an abbreviation used herein is not defined, it has its generally accepted meaning:

| | |
|---|---|
| Ac | Acetyl |
| AcOH | Acetic acid |
| MeCN | Acetonitrile |
| $BF_3.OEt_2$ | Boron trifluoride diethyl etherate |
| $Boc_2O$ | Di-*tert*butyl decarbonate |
| Boc | *tert*-butyloxycarbonyl |
| BrettPhos Pd G3 | [(2-Di-cyclohexylphosphino-3,6-dimethoxy-2',4',6'- triisopropyl-1,1'-biphenyl)-2-(2'-amino-1,1'-biphenyl)]palladium(II) methanesulfonate |
| $CBr_4$ | Carbon tetrabromide |
| $CHCl_3$ | Chloroform |
| $Cs_2CO_3$ | Cesium carbonate |
| DAST | (Diethylamino)sulfur trifluoride |
| DCM | Dichloromethane |
| DIPEA | Diisopropylethylamine |
| DMF | Dimethylformamide |
| DMSO | Dimethylsulfoxide |
| DMSO-$d_6$ | Hexadeuterodimethylsulfoxide |
| DPPA | Diphenyl phosphoryl azide |
| EDCI | N-Ethyl-N'-(3-dimethylaminopropyl)carbodiimide hydrochloride |
| ee% | Enantiomeric excess |
| Et | Ethyl |
| EtOAc | Ethyl acetate |
| $Et_3N$ | Triethylamine |

| | |
|---|---|
| EtOH | Ethanol |
| $Et_2O$ | Diethylether |
| EPhos Pd G4 | Methanesulfonato{Dicyclohexyl[3-(1-methylethoxy)-2',4',6'-tris(1-methylethyl)[1,1'-biphenyl]-2-yl]phosphine}(2'-methylamino-1,1'-biphenyl-2-yl)palladium(II) |
| $Et_3SiH$ | Triethylsilane |
| GC | Gas chromatography |
| GPhos | (3-(tert-Butoxy)-2',6'-diisopropyl-6-methoxy-[1,1'-biphenyl]-2-yl)dicyclohexylphosphane |
| Gphos Pd $G_6$ TES | GPhos OAC precatalyst TES |
| Grubbs 2nd | generation(1,3-Bis(2,4,6-trimethylphenyl)-2-imidazolidinylidene)dichloro(phenylmethylene)(tricyclohexylpho sphine)ruthenium, Benzylidene[1,3-bis(2,4,6-trimethylphenyl)-2-imidazolidinylidene]dichloro(tricyclohexylphosphine)ruthenium, Dichloro[1,3-bis(2,4,6-trimethylphenyl)-2-imidazolidinylidene] (benzylidene)(tricyclohexylphosphine)ruthen ium(II) |
| h | hour |
| HATU | [dimethylamino(triazolo[4,5-b]pyridin-3-yloxy)methylene]-dimethyl-ammonium hexafluorophosphate |
| $H_2O$ | water |
| Het | Heteroaromatic |
| HFIP | 1,1,1,3,3,3-Hexafluoro-2-propanol |
| HOBt | 1-Hydroxybenzotriazole |
| HPLC | High performance liquid chromatography |
| IPA | Isopropyl amine |
| $iPr_2O$ | Isopropyl ether |
| iPrOH | Isopropanol |
| iPrOAc | Isopropylacetate |
| KI | Potassium iodide |
| $K_3PO_4$ | Tripotassium phosphate |
| LC | Liquid chromatography |
| $LiBEt_3H$ | Lithium triethylborohydride |
| LiHMDS | Lithium bis(trimethylsilyl)amide |
| LiOH | Lithium hydroxide |
| min | Minutes |
| Me | Methyl |
| MeCN | Acetonitrile |
| MeMgBr | Methylmagnesium bromide |
| MeOH | Methanol |
| MeTHF | 2-Methyltetrahydrofuran |
| $MgSO_4$ | Magnesium sulfate |
| MS | Mass spectrometry |
| MtBE | Tert-butyl methylether |
| NaH | Sodium hydride |
| $Na_2SO_4$ | Sodium sulfate |
| $Na_2S_2O_3$ | Sodium thiosulfate |
| $NaBH_4$ | Sodium borohydride |
| $NaBH(OAc)_3$ | Sodium triacetoxyborohydride |
| $NaBH_3CN$ | Sodium cyanoborohydride |
| NBS | N-bromosuccinimide |
| NaCl | Sodium chloride |
| $NaHCO_3$ | Sodium bicarbonate |
| $Na_2CO_3$ | Sodium carbonate |
| $NH_4Cl$ | Ammonium chloride |
| $NH_4OA_C$ | Ammonium acetate |
| NMP | N-methyl-2-pyrrolidone |
| NMM | N-methylmorpholine |
| $N_2$ | Nitrogen |
| NaOH | Sodium hydroxide |
| $Pd(PPh_3)_4$ | Tetrakis(triphenylphosphine)palladium(0) |
| $Pd_2(dba)_3$ | Tris(dibenzylideneacetone)dipalladium(0) |
| $Pd(dppf)Cl_2$ | [1,1'-Bis(diphenylphosphino)ferrocene]dichloropalladium(II) |

| | |
|---|---|
| Pd/C | Palladium on carbon |
| PE | Petroleum ether |
| $POCl_3$ | Phosphorus oxychloride |
| $PPh_3$ | Triphenylphosphine |
| Rochelle's salt | Potassium sodium tartrate |
| rt | room temperature (18 to 22 °C) |
| $SOCl_2$ | Thionyl chloride |
| TBAF | Tetrabutylammonium fluoride |
| TBSCl | tert-Butyldimethylsilyl chloride |
| tBuLi | Tert-butyllithium |
| tBuOH | Tert-butanol |
| tBuOK | Potassium tert-butoxide |
| TCFH | N-[chloro(dimethylamino)methylidene]-N-methylmethanaminium hexafluorophosphate |
| TFA | Trifluoroacetic acid |
| TLC | Thin layer chromatography |
| THF | Tetrahydrofuran |
| TMSCl | Chlorotrimethylsilane |
| UPLC | Ultra Performance Liquid Chromatography |
| Xantphos | 4,5-Bis(diphenylphosphino)-9,9-dimethylxanthene |
| 9-BBN | 9-Borabicyclo[3.3.1]nonane |

**Procedures**

**Intermediates 6A and 6B were synthesized following Scheme 1.**

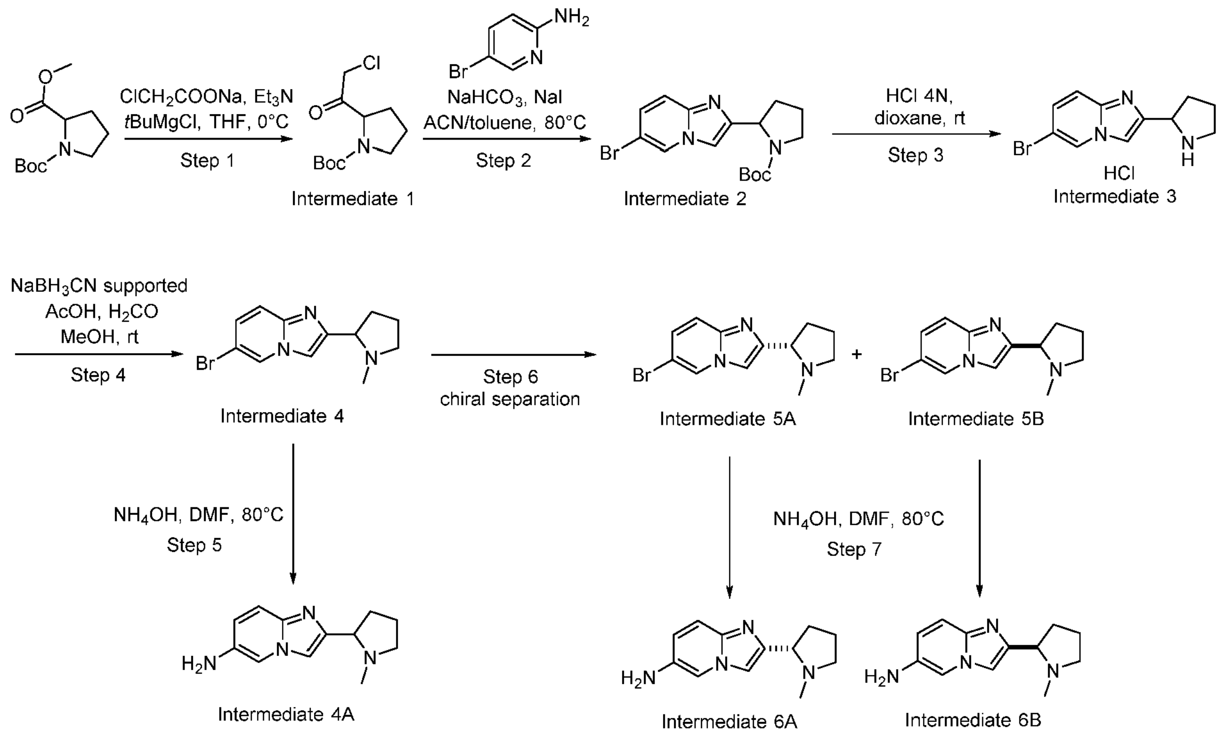

**Scheme 1**

**Step 1**

**Intermediate 1: rac-tert-Butyl 2-(2-chloroacetyl)pyrrolidine-1-carboxylate**

Under dry conditions and argon atmosphere, rac-1-tert-butyl 2-methyl pyrrolidine-1,2-dicarboxylate (CAS: 145681-01-2, 100 g, 0.406 mol) was charged followed by anhydrous THF (860 mL). Then, sodium chloroacetate (124.34 g, 1.01 mol) was added in one portion followed by the dry triethylamine (113 mL, 0.811 mol). While keeping the temperature of the media between 0 and 5°C, a solution of tert-butylmagnesium chloride (1.014 L, 1.01 mol, 1M in THF) was added over 2h. The white suspension was stirred at 2-5°C for 18h. Then, a solution of citric acid (190 g in 570 mL of water) was added over 1h into the reaction mixture while maintaining the temperature between 0-7°C using an ice bath. The organic layer was washed twice with an aqueous $NaHCO_3$ solution ($NaHCO_3$: 25 g, NaCl: 90 g, $H_2O$: 550 mL) and once with brine (NaCl: 100 g, $H_2O$: 400 mL). The organic layer was concentrated under pressure. $^1$PrOAc (300mL) was added to the residue, the organic layer was washed with an aqueous $NaHCO_3$ solution ($NaHCO_3$: 5g, NaCl: 18g, $H_2O$: 100mL) and the organic layer was dried over anhydrous $MgSO_4$, filtered, and concentrated under pressure to afford rac-tert-butyl 2-(2-chloroacetyl)pyrrolidine-1-carboxylate **(Intermediate 1:** 107.7 g, quant.) as a yellow oil which was used in Step 2 without further purification. Mass spec: m/z: 192 $[M+H-tBu]^+$, 148 $[M+H-Boc]^+$; $^1H$ NMR (400 MHz, DMSO-$d_6$) $\delta$ ppm 4.71 - 4.53 (m, 2H), 4.40 (ddd, J = 11.1, 8.7, 4.8 Hz, 1H), 3.34 (d, J = 7.2 Hz, 2H), 2.24 - 2.05 (m, 1H), 1.93 - 1.66 (m, 3H), 1.35 (d, J = 32.2 Hz, 9H).

**Step 2**

**Intermediate 2: rac-tert-Butyl 2-{6-bromoimidazo[1,2-a]pyrazin-2-yl}pyrrolidine-1-carboxylate**

In a round bottom flask, 5-bromopyridin-2-amine (CAS: 1072-97-5, 15 g, 86.7 mmol) was dissolved in toluene (75 mL)/MeCN (75 mL). Then, $NaHCO_3$ (8.74 g, 0.104 mol) was added, followed by rac-tert-butyl 2-(2-chloroacetyl)pyrrolidine-1-carboxylate **(Intermediate 1:** 23.34 g, 86.7 mmol) and sodium iodide (1.3 g, 8.67 mmol). The reaction mixture was stirred at 83°C for 18h. After cooling to rt, the mixture was concentrated under reduced pressure. The crude was diluted with $^i$PrOAc (250 mL) and water (250 mL). The layers were filtered, the aqueous and organic layer from the filtrate were separated and the organic layer was dried over anhydrous $MgSO_4$, filtered, and concentrated under reduced pressure. The crude material was purified by flash chromatography on silica gel using a gradient of acetone in DCM from 0% to 100% to afford rac-tert-Butyl 2-{6-bromoimidazo[1,2-a]pyrazin-2-yl}pyrrolidine-1-carboxylate **(Intermediate 2:** 14.3 g, 43%) as a white solid. Mass spec: m/z: 366 $[M+H]^+$, 368 $[M+H+2]^+$; $^1H$ NMR (400 MHz, DMSO-$d_6$) $\delta$ ppm 8.86 (d, J = 14.3 Hz, 1H), 7.67 (d, J = 10.3 Hz, 1H), 7.47 (d, J = 9.5 Hz, 1H), 7.30 (dd, J = 9.5, 2.0 Hz, 1H), 4.91 (d, J = 12.9 Hz, 1H), 3.44 (s, 1H), 3.36 (ddd, J = 10.4, 8.6, 7.2 Hz, 1H), 2.17 (s, 1H), 2.08 - 1.99 (m, 1H), 1.98 - 1.79 (m, 2H), 1.31 (d, J = 74.2 Hz, 9H).

**Step 3**

**Intermediate 3: rac-2-{6-Bromoimidazo[1,2-a]pyridin-2-yl}pyrrolidine hydrochloride**

In a round bottom flask under nitrogen atmosphere, rac-tert-butyl 2-(6-bromoimidazo[1,2-a]pyridin-2-yl)pyrrolidine-1-carboxylate **(Intermediate 2:** 14.30 g, 37.1 mmol) was solubilized in $^i$PrOAc (75 mL). A solution of hydrogen chloride (74 mL, 0.371 mol, 5M in $^i$PrOAc) was added to the mixture. The mixture was stirred for 2h at rt. A suspension appeared, the reaction mixture was filtered and the solid was washed with isopropanol (3x70 mL), dried under reduced pressure to afford rac-2-{6-Bromoimidazo[1,2-a]pyridin-2-yl}pyrrolidine hydrochloride **(Intermediate 3:** 10.89 g, 92%) as a brown solid which was used in Step 4 without further purification. Mass spec: m/z: 266 $[M+H]^+$, 268 $[M+H+2]^+$; $^1H$ NMR (400 MHz, DMSO-$d_6$) $\delta$ ppm 10.13 (s, 1H), 9.17 (s, 1H), 9.07 (dd, J = 2.0, 0.9 Hz, 1H), 8.15 (s, 1H), 7.67 (d, J = 9.5 Hz, 1H), 7.58 (dd, J = 9.5, 1.8 Hz, 1H), 4.84 (p, J = 6.7 Hz, 1H), 3.39 - 3.23 (m, 2H), 2.44 - 2.34 (m, 1H), 2.23 - 2.08 (m, 2H), 2.08 - 1.96 (m, 1H).

**Step 4**

**Intermediate 4: rac-2-{6-bromoimidazo[1,2-a]pyridin-2-yl}-1-methylpyrrolidine**

6-bromo-2-pyrrolidin-2-yl-imidazo[1,2-a]pyridine hydrochloride **(Intermediate 3** (10.20 g, 32.0 mmol) was diluted in MeOH (100 mL), then formaldehyde (37% aq, 4.8 mL, 64.0 mmol) and acetic acid (3.85 g, 64.0 mmol) were

added and the mixture was stirred for 1h at rt. Solid supported $NaBH_3CN$ (8.01 g, 16.0 mmol - loading 2 mmol/g) was then added and the mixture was stirred at rt for 1.5h. The reaction mixture was filtered, rinsed by MeOH and the filtrate was concentrated under pressure. The residue was diluted with EtOAc and a solution of saturated $NaHCO_3$. The aqueous layer was extracted with EtOAc (5x). The organic layers were combined, dried over anhydrous $MgSO_4$, filtered, and concentrated under reduced pressure to afford rac-2-{6-bromoimidazo[1,2-a]pyridin-2-yl}-1-methylpyrrolidine **(Intermediate 4:** 7.94 g, 89%) as a yellow oil. Mass spec: m/z: 280 $[M+H]^+$, 282 $[M+H+2]^+$, $^1H$ NMR (400 MHz, DMSO-$d_6$) δ ppm 8.82 (d, J = 1.9 Hz, 1H), 7.76 (s, 1H), 7.45 (d, J = 9.5 Hz, 1H), 7.29 (dd, J = 9.5, 2.0 Hz, 1H), 3.33 (d, J = 7.4 Hz, 1H), 3.09 (ddd, J = 10.0, 6.1, 2.5 Hz, 1H), 2.28 (p, J = 8.6 Hz, 1H), 2.21 (s, 3H), 2.19 - 2.11 (m, 1H), 1.88 - 1.70 (m, 3H).

**Step 5**

**Intermediate 4A: rac-2-(1-methylpyrrolidin-2-yl)imidazo[1,2-a]pyridin-6-amine**

To a solution of rac-2-{6-bromoimidazo[1,2-a]pyridin-2-yl}-1-methylpyrrolidine **(Intermediate 4:** 2.3 g, 7.06 mmol)) and copper(I) oxide (303 mg; 2.12 mmol)) in DMF (2.8 mL) was added 7.1 M ammonium hydroxide (35 mL, 247 mmol, 25% in H2O). The autoclave reactor was sealed and stirred at 80°C for 18 h (pressure of 3-4 bars observed). The mixture was cooled to rt and DCM/MeOH (9/1) and water were added, and the two phases were separated. The aqueous phase was extracted with DCM/MeOH (9/1) twice. The combined organic layers were dried over anhydrous MgSO4 and concentrated to dryness to afford 2-(1-methylpyrrolidin-2-yl)imidazo[1,2-a]pyridin-6-amine **(Intermediate 4A:** 1.6 g, 82%) as an oil. Mass spec: m/z: 217 [M+H]+; 1H NMR (400 MHz, DMSO-d6) δ ppm 7.61 (s, 2H), 7.22 (s, 1H), 6.82 (s, 1H), 4.84 (s, 2H), 3.26 (s, 1H), 3.13 - 3.02 (m, 1H), 2.14 (d, J = 46.3 Hz, 5H), 1.79 (s, 3H).

**Step 6**

**Intermediate 5A: rel-(2R)-{6-bromoimidazo[1,2-a]pyridin-2-yl}-1-methylpyrrolidine and Intermediate 5B: rel-(2S)-2-{6-bromoimidazo[1,2-a]pyridin-2-yl}-1-methylpyrrolidine**

rac-2-{6-bromoimidazo[1,2-a]pyridin-2-yl}-1-methylpyrrolidine **(Intermediate 4)** was separated into the enantiomeric **Intermediates 5A** and **5B** using the following conditions: Novasep Lab LC, column; Chiralcel IA 20μm, 300 x 78mm; Eluent: EtOH + 0.5% IPA; Flow rate: 275 mL/min to provide:

**Intermediate 5A: rel-(2S)-2-{6-bromoimidazo[1,2-a]pyridin-2-yl}-1-methylpyrrolidine**

First eluting compound (retention time = 9.5 to 10.5 min):
Mass spec: m/z: 280 $[M+H]^+$, 282 $[M+H+2]^+$; $^1H$ NMR (400 MHz, DMSO-$d_6$) δ ppm 8.83 (dd, J = 2.1, 0.9 Hz, 1H), 7.75 (s, 1H), 7.45 (dd, J = 9.5, 0.9 Hz, 1H), 7.29 (dd, J = 9.5, 1.9 Hz, 1H), 3.30 (d, J = 7.7 Hz, 1H), 3.08 (ddt, J = 7.9, 6.3, 2.6 Hz, 1H), 2.26 (q, J = 8.7 Hz, 1H), 2.20 (s, 3H), 2.19 - 2.10 (m, 1H), 1.87 - 1.69 (m, 3H). ee = 100%.

**Intermediate 5B: rel-(2R)-2-{6-bromoimidazo[1,2-a]pyridin-2-yl}-1-methylpyrrolidine**

Second eluting compound (retention time = 22 to 36 min)
Mass spec: m/z: 280 $[M+H]^+$, 282 $[M+H+2]^+$; $^1H$ NMR (400 MHz, DMSO-$d_6$) δ ppm 8.83 (dd, J = 2.1, 0.9 Hz, 1H), 7.75 (s, 1H), 7.45 (dd, J = 9.5, 0.9 Hz, 1H), 7.29 (dd, J = 9.5, 1.9 Hz, 1H), 3.30 (d, J = 7.7 Hz, 1H), 3.08 (ddt, J = 7.9, 6.3, 2.6 Hz, 1H), 2.26 (q, J = 8.7 Hz, 1H), 2.20 (s, 3H), 2.19 - 2.10 (m, 1H), 1.87 - 1.69 (m, 3H). ee = 100%.

**Step 7**

**Intermediate 6A: rel-2-[(2R)-1-methylpyrrolidin-2-yl]imidazo[1,2-a]pyridin-6-amine or Intermediate 6B: rel-2-[(2S)-1-methylpyrrolidin-2-yl]imidazo[1,2-a]pyridin-6-amine**

To a solution of **Intermediate 5A** or **5B** (1 eq) and copper(I) oxide (0.3 eq) in DMF (2.5 M) was added 7.1 M ammonium hydroxide (35 eq, 25% in $H_2O$). The autoclave reactor was sealed and stirred at 80°C for 18 h (pressure of 3-4 bars observed). The mixture was cooled to rt and DCM/MeOH (9/1) and water were added, and the two phases were separated. The aqueous phase was extracted with DCM/MeOH (9/1) twice. The combined organic layers were dried over anhydrous $MgSO_4$ and concentrated to dryness to afford **Intermediate 6A** or **6B:**

**Intermediate 6A: rel-2-[(2S)-1-methylpyrrolidin-2-yl]imidazo[1,2-a]pyridin-6-amine**

Mass spec: m/z: 217 [M+H]+; 1H NMR (400 MHz, DMSO-d6) δ ppm 7.63 (d, J = 8.7 Hz, 2H), 7.25 (d, J = 9.5 Hz, 1H), 6.81 (dd, J = 9.5, 2.1 Hz, 1H), 4.84 (s, 2H), 3.50 (s, 1H), 3.18 (s, 1H), 2.35 - 2.15 (m, 5H), 1.88 (d, J = 26.2 Hz, 3H).

**Intermediate 6B: rel-2-[(2R)-1-methylpyrrolidin-2-yl]imidazo[1,2-a]pyridin-6-**amine

Mass spec: m/z: 217 [M+H]+; 1H NMR (400 MHz, DMSO-d6) δ ppm 7.65 - 7.59 (m, 1H), 7.54 (s, 1H), 7.21 (d, J = 9.5 Hz, 1H), 6.77 (dd, J = 9.4, 2.1 Hz, 1H), 4.80 (s, 2H), 3.26 (t, J = 7.9 Hz, 1H), 3.13 - 3.03 (m, 1H), 2.31 - 2.22 (m, 1H), 2.20 (s, 3H), 2.16 - 2.06 (m, 1H), 1.93 - 1.67 (m, 3H).

**Intermediate 7: rac-2-{6-bromoimidazo[1,2-a]pyridin-2-yl}-1-(2-methoxyethyl)pyrrolidine**

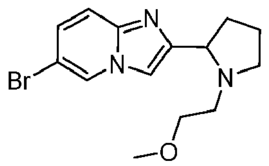

To a solution of rac-2-{6-bromoimidazo[1,2-a]pyridin-2-yl}pyrrolidine hydrochloride **(Intermediate 3;** 300 mg, 1.13 mmol) in DMF (4 mL) was added 2-bromoethyl methyl ether (313 mg, 2.25 mmol), tetrabutylammonium iodide $Bu_4NI$ (41.6 mg, 0.110 mmol) and DIPEA (583 mg, 4.51 mmol). The mixture was stirred at 80°C for 3h and then quenched with water (5 mL). The mixture was concentrated under reduced pressure. The crude material was purified by reverse phase flash chromatography (C18 Aq) using MeCN/water (84/16) as the eluent to provide rac-2-{6-bromoimidazo[1,2-a]pyridin-2-yl}-1-(2-methoxyethyl)pyrrolidine **(Intermediate 7:** 160 mg 39%) as a light yellow oil. Mass spec: m/z 324 $[M+H]^+$.

**Intermediate 10 was synthesized following Scheme 2.**

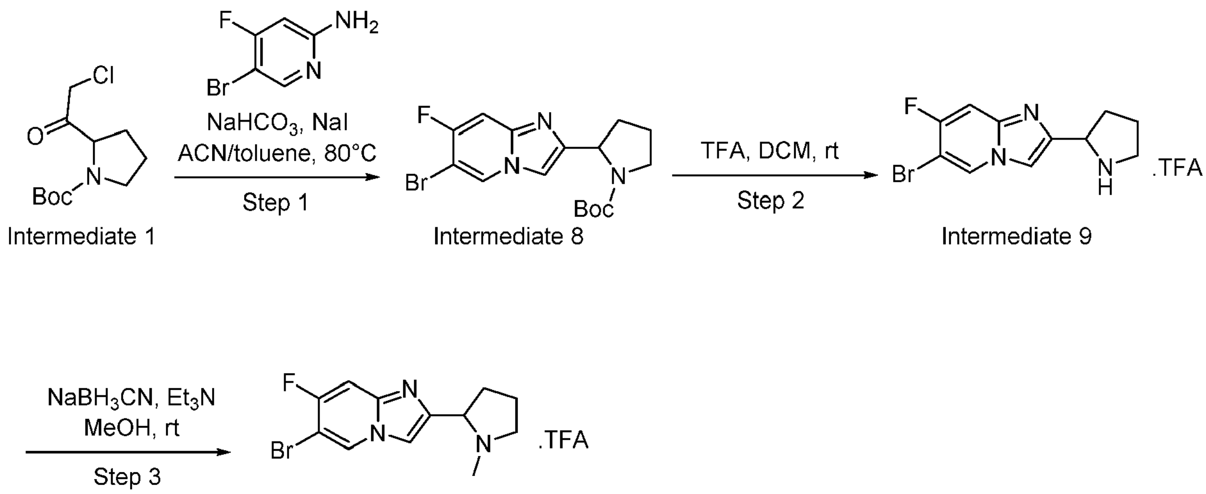

Intermediate 1

Intermediate 8

Intermediate 9

Intermediate 10

**Scheme 2**

**Step 1**

**Intermediate 8: rac-tert-butyl 2-{6-bromo-7-fluoroimidazo[1,2-a]pyridin-2-yl}pyrrolidine-1-carboxylate**

Using rac-tert-butyl 2-(2-chloroacetyl)pyrrolidine-1-carboxylate **(Intermediate 1)** as the chloro-ketone and 5-bromo-4-fluoropyridin-2-amine (CAS: 944401-69-8) as the amine and following an analogous procedure to **Step 2** of **Scheme 1,** followed by purification by flash chromatography (eluent: EtOAc/PE, 1/4) afforded rac-tert-butyl 2-{6-bromo-7-fluoroimidazo[1,2-a]pyridin-2-yl}pyrrolidine-1-carboxylate **(Intermediate 8:** 350 mg, 14%) as a yellow oil. Mass spec: m/z: 384 $[M+H]^+$.

**Step 2**

**Intermediate 9: rac-2-{6-bromo-7-fluoroimidazo[1,2-a]pyridin-2-yl}pyrrolidine trifluoroacetate**

A round-bottom flask was charged with rac-tert-butyl 2-{6-bromo-7-fluoroimidazo[1,2-a]pyridin-2-yl}pyrrolidine-1-carboxylate **(Intermediate 8:** 480 mg, 1.249 mmol), DCM (15 mL) and TFA (3 mL). The reaction was stirred for 2h at rt. The mixture was then concentrated under reduced pressure to provide rac-2-{6-bromo-7-fluoroimidazo[1,2-a]pyridin-2-yl}pyrrolidine trifluoroacetate **(Intermediate 9:** 380 mg, 96%) as a yellow oil. Mass spec: m/z: 284 $[M+H]^+$.

**Step 3**

**Intermediate 10: rac-2-{6-bromo-7-fluoroimidazo[1,2-a]pyridin-2-yl}-1-methylpyrrolidine trifluoroacetate**

A round-bottom flask was charged with rac-2-{6-bromo-7-fluoroimidazo[1,2-a]pyridin-2-yl}pyrrolidine trifluoroacetate **(Intermediate 9:** 380 mg, 1.34 mmol), paraformaldehyde (201 mg, 6.69 mmol), $Et_3N$ (406 mg, 4.01 mmol) and MeOH (20 mL). The reaction was stirred for 1h at rt. Then $NaBH_3CN$ (252 mg, 4.01 mmol) was added, and the reaction was stirred overnight at 60°C. The reaction was diluted $H_2O$ and extracted with EtOAc twice. The combined organic layers were washed with brine, dried over anhydrous $Na_2SO_4$, filtered, and concentrated under reduced pressure. The residue was purified by reverse-phase flash chromatography (C18 aq) using a gradient of MeCN in water from 0% to 100% (0.5% TFA) to afford rac-2-{6-bromo-7-fluoroimidazo [1,2-a]pyridin-2-yl} -1-methylpyrrolidine trifluoroacetate **(Intermediate 10:** 400 mg, 90% as a colorless oil. Mass spec: m/z: 298 $[M+H]^+$.

**Intermediate 15 was synthesized following Scheme 3.**

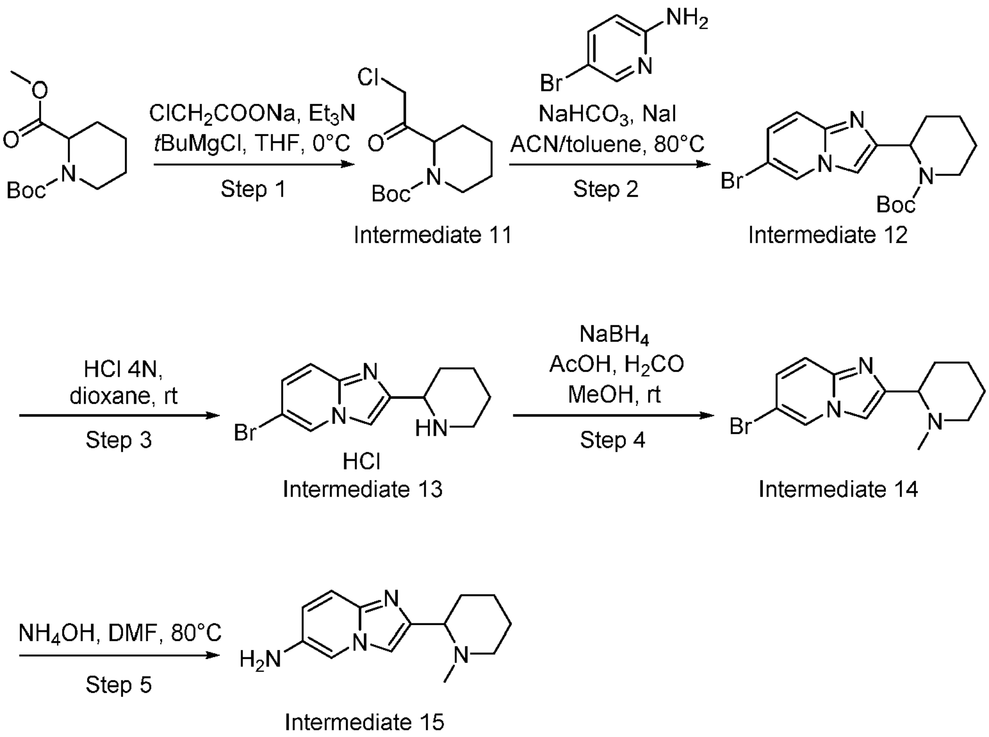


**Scheme 3**

**Step 1**

**Intermediate 11: rac-tert-butyl 2-(2-chloroacetyl)piperidine-1-carboxylate**

To a solution of rac-1-tert-butyl 2-methyl piperidine-1,2-dicarboxylate (CAS: 167423-93-0, 1.8 mL, 7.97 mmol), sodium chloroacetate (978 mg, 7.97 mmol) and triethylamine (1.1 mL, 7.97 mmol) in anhydrous THF (18 mL) was added dropwise tert-butylmagnesium chloride (1M in THF 20 mL, 19.9 mmol) at -7°C over 20 min. The reaction mixture was left stirring between 0-5°C for 2h. A solution of citric acid (3.54 g, 18.3 mmol) in water (10 mL) was slowly added to the reaction mixture while maintaining the temperature between 0-5°C. EtOAc was then added to the reaction mixture and the phases were separated. The organic layer was washed twice with a solution of $NaHCO_3$/NaCl (470 mg of $NaHCO_3$, 1.6 g of NaCl in 10mL $H_2O$) and once with brine. The combined organic layers were dried over anhydrous $MgSO_4$, filtered, and concentrated under reduced pressure to afford rac-tert-butyl 2-(2-chloroacetyl)piperidine-1-carboxylate **(Intermediate 11:** 1.9 g, 64%) as an orange oil which was used in Step 2 without further purification. Mass spec: m/z: 206 $[M-tBu+H]^+$, 162 $[M-Boc+H]^+$; $^1H$ NMR (400 MHz, DMSO-$d_6$) δ ppm 4.76 (dd, J = 6.3, 3.0 Hz, 1H), 4.69 - 4.55 (m, 2H), 3.89 - 3.69 (m, 1H), 3.01 - 2.63 (m, 1H), 2.14 - 2.03 (m, 1H), 1.57 (dtd, J = 17.2, 9.3, 3.9 Hz, 3H), 1.42 - 1.33 (m, 9H), 1.31 - 1.25 (m, 1H), 1.20 - 1.05 (m, 1H).

**Step 2**

**Intermediate 12: rac-tert-butyl 2-{6-bromoimidazo[1,2-a]pyridin-2-yl}piperidine-1-carboxylate**

To a solution of rac-tert-butyl 2-(2-chloroacetyl)piperidine-1-carboxylate **(Intermediate 11:** 1.69 g, 4.51 mmol) and 5-bromopyridin-2-amine (780 mg, 4.51 mmol) in toluene (32.5 mL) and MeCN (32.5 mL) was added sodium hydrogen carbonate (454 mg, 5.41 mmol) followed by sodium iodide (68 mg, 0.451 mmol). The reaction mixture was heated at 95°C for 3h. The reaction mixture was concentrated under reduced pressure. The residue was diluted in DCM, filtered and filtrate was concentrated in vacuo. The crude material was purified by flash chromatography on silica gel (120 g) using a gradient of DCM/Acetone (90/10) in DCM from 0% to 50% to afford rac-tert-butyl 2-{6-bromoimidazo[1,2-a]pyridin-2-yl}piperidine-1-carboxylate **(Intermediate 12:** 403 mg, 22%) as an orange oil. Mass spec: m/z: 380 $[M+H]^+$, 382 $[M+H+2]^+$.

**Step 3**

**Intermediate 13: rac-2-{6-bromoimidazo[1,2-a]pyridin-2-yl}piperidine hydrochloride**

To a solution of rac-tert-butyl 2-(6-bromoimidazo[1,2-a]pyridin-2-yl)piperidine-1-carboxylate **(Intermediate 12:** 400 mg, 0.968 mmol) in 1,4-dioxane (3.23 mL) was added a solution of HCl (3.6 mL, 14.5 mmol, 4N in dioxane). The reaction mixture was stirred at rt for 5h. The reaction mixture was filtered and washed with $iPr_2O$ to afford rac-2-{6-bromoimidazo[1,2-a]pyridin-2-yl}piperidine hydrochloride **(Intermediate 13:** 315 mg, 99%) as a white solid which was used in Step 4 without further purification. Mass spec: m/z: 280 $[M+H]^+$, 282 $[M+H+2]^+$; $^1H$ NMR (400 MHz, DMSO-$d_6$) $\delta$ ppm 9.39 (s, 1H), 9.13 (d, J = 2.5 Hz, 1H), 8.15 (d, J = 2.3 Hz, 1H), 7.68 (d, J = 9.5 Hz, 1H), 7.63 - 7.56 (m, 1H), 4.49 (t, J = 10.8 Hz, 1H), 3.27 (s, 1H), 3.04 (d, J = 11.8 Hz, 1H), 2.14 (dd, J = 13.6, 3.5 Hz, 1H), 2.03 - 1.59 (m, 5H).

**Step 4**

**Intermediate 14: rac-2-{6-bromoimidazo[1,2-a]pyridin-2-yl}-1-methylpiperidine**

To a solution of rac-2-{6-bromoimidazo[1,2-a]pyridin-2-yl}piperidine hydrochloride **(Intermediate 13:** 315 mg, 0.967 mmol) in MeOH (6.4 mL) was added formaldehyde (37% aq, 0.13 mL, 1.74 mmol) and acetic acid (0.083 mL, 1.45 mmol). The reaction mixture was stirred at rt for 1.5h before addition of $NaBH_4$ (774 mg, 1.93 mmol). The resulting reaction mixture was stirred at rt for 1h. The reaction mixture was filtered through a pad of celite, washed with MeOH and the filtrate was concentrated under reduced pressure. The residue was partitioned between a saturated solution of $NaHCO_3$ and EtOAc, the phases were separated, and aqueous phase was extracted with EtOAc twice. The combined organic layers were dried over anhydrous $Na_2SO_4$, filtered, and concentrated under reduced pressure to obtain rac-2-{6-bromoimidazo[1,2-a]pyridin-2-yl}-1-methylpiperidine **(Intermediate 14:** 225 mg, 74%) as a light beige solid which was used in Step 5 without further purification. Mass spec: m/z: 294 $[M+H]^+$, 296 $[M+H+2]^+$; $^1H$ NMR (400 MHz, DMSO-$d_6$) $\delta$ ppm 8.82 (dd, J = 2.1, 0.8 Hz, 1H), 7.75 (s, 1H), 7.46 (d, J = 9.5 Hz, 1H), 7.29 (dd, J = 9.5, 2.0 Hz, 1H), 3.28 (d, J = 3.1 Hz, 1H), 3.04 (d, J = 10.6 Hz, 1H), 2.91 (d, J = 11.5 Hz, 1H), 2.06 (t, J = 11.2 Hz, 1H), 1.97 (s, 3H), 1.79 - 1.69 (m, 2H), 1.68 - 1.50 (m, 2H), 1.39 - 1.23 (m, 1H).

**Step 5**

**Intermediate 15: rac-2-(1-methylpiperidin-2-yl)imidazo[1,2-a]pyridin-6-amine**

To a solution of rac-2-{6-bromoimidazo[1,2-a]pyridin-2-yl}-1-methylpiperidine **(Intermediate 14:** 225 mg, 0.711 mmol) in DMF (0.35 mL) was added copper(I) oxide (32 mg, 0.213 mmol) followed by a solution of ammonium hydroxide (3.5 mL, 24.9 mmol, 7.1 M in water). The reaction mixture was heated at 80°C overnight (sealed tube - pressure observed). The reaction mixture was partitioned between $H_2O$ and DCM/MeOH (3/1), the phases were separated, and the aqueous phase was extracted with DCM/MeOH (3/1) twice. The combined organic layers were dried over anhydrous $Na_2SO_4$, filtered, and concentrated under reduced pressure to afford rac-2-(1-methylpiperidin-2-yl)imidazo[1,2-a]pyridin-6-amine **(Intermediate 15**:158 mg, 81%) as a brown oil which was used without further purification. Mass spec: m/z: 231 $[M+H]^+$; $^1H$ NMR (400 MHz, DMSO-$d_6$) $\delta$ ppm 7.62 (dd, J = 2.2, 0.9 Hz, 1H), 7.52 (s, 1H), 7.21 (d, J = 9.4 Hz, 1H), 6.76 (dd, J = 9.4, 2.1 Hz, 1H), 4.79 (s, 2H), 2.96 - 2.90 (m, 1H), 2.87 (d, J = 3.5 Hz, 1H), 2.03 (td, J = 11.4, 3.3 Hz, 1H), 1.96 (s, 3H), 1.80 - 1.50 (m, 5H), 1.29 (dtd, J = 16.8, 12.3, 4.3 Hz, 1H).

**Intermediate 21 was synthesized following Scheme 4.**

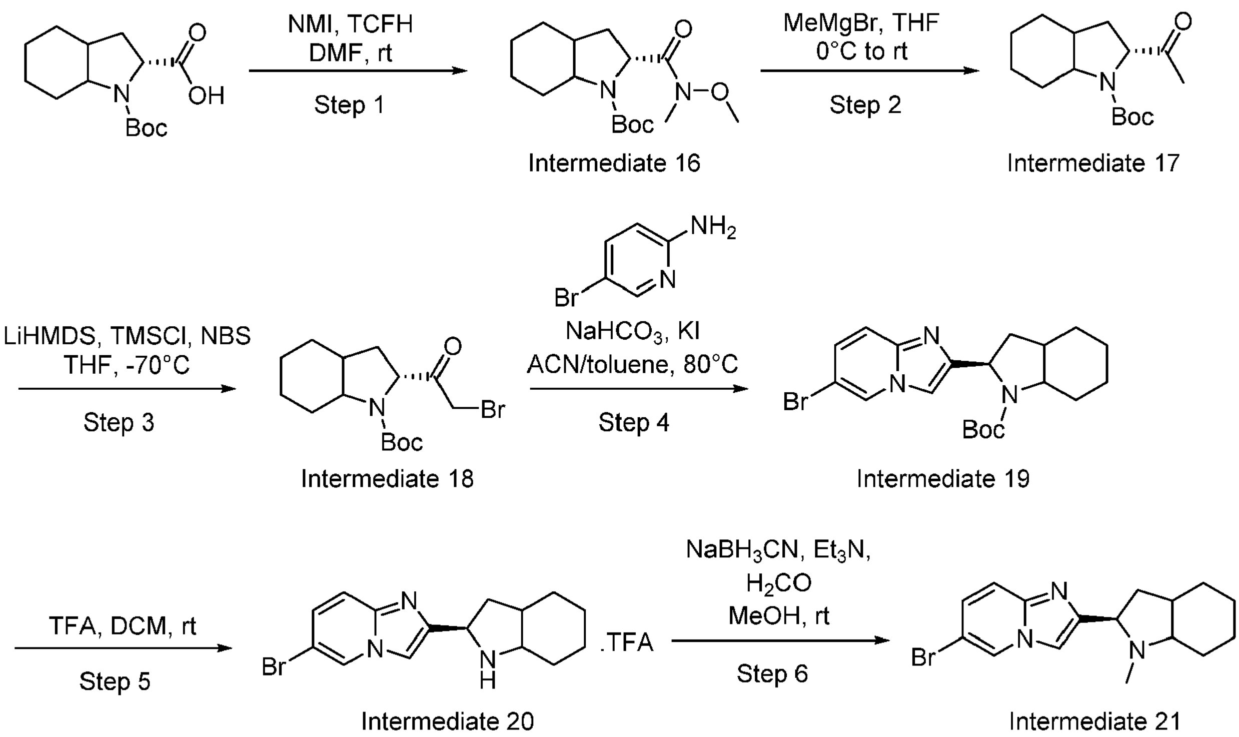


## Scheme 4

**Step 1**

**Intermediate 16: tert-butyl (2R)-2-[methoxy(methyl)carbamoyl]-octahydro-1H-indole-1-carboxylate**

To a stirred solution of (2R)-1-(tert-butoxycarbonyl)-octahydroindole-2-carboxylic acid (CAS: 1217525-04-6: 1 g, 3.71 mmol) and N-methyl imidazole (1.52 g, 18.7 mmol) in DMF (10 mL) was added TCFH (1.35 g, 4.82 mmol) at rt. The reaction was stirred at rt for 18h. The reaction mixture was quenched with $H_2O$ and extracted with EtOAc three times. The combined organic layers were washed with brine, dried over anhydrous $Na_2SO_4$, filtered, and concentrated under reduced pressure. The crude material was purified by flash chromatography on silica gel using DCM/MeOH (9/1)] as eluent to afford tert-butyl (2R)-2-[methoxy(methyl)carbamoyl]-octahydro-1H-indole-1-carboxylate **(Intermediate 16:** 600 mg, 44%) as a white solid. Mass spec: m/z: 313 $[M+H]^+$.

**Step 2**

**Intermediate 17: tert-butyl (2R)-2-[methoxy(methyl)carbamoyl]-octahydro-1H-indole-1-carboxylate**

To a stirred solution of tert-butyl (2R)-2-[methoxy(methyl)carbamoyl]-octahydroindole-1-carboxylate **(Intermediate 16:** 600 mg, 1.92 mmol) in THF (6 mL) were added a solution of MeMgBr (3.84mL, 3.84mmol, 1M in THF) dropwise at 0°C under nitrogen atmosphere. The reaction mixture was stirred for 3 days at rt. The reaction was quenched with water and extracted with EtOAc three times. The combined organic layers were washed with brine, dried over anhydrous $Na_2SO_4$, filtered, and concentrated under reduced pressure. The crude material was purified by flash chromatography on silica gel using PE/EtOAc (86/14) as eluent to afford tert-butyl (2R)-2-[methoxy(methyl)carbamoyl]-octahydro-1H-indole-1-carboxylate **(Intermediate 17:** 238 mg, 50%) as a colorless oil. Mass spec: m/z: 268 $[M+H]^+$.

**Step 3**

**Intermediate 18: tert-butyl (2R)-2-(2-bromoacetyl)-octahydro-1H-indole-1-carboxylate**

To a stirred solution of tert-butyl (2R)-2-acetyl-octahydroindole-1-carboxylate **(Intermediate 17:** 200 mg, 0.748 mmol) in THF (3 mL) was added a LiHMDS solution (1.5 mL, 1.496 mmol, 1M in THF) dropwise at -70°C under nitrogen

atmosphere. The resulting mixture was stirred at -70°C for 1h. To the above mixture was added TMSCl (162 mg, 1.50 mmol) at -70°C. The resulting mixture was warmed to rt and stirred for 1h. The mixture was cooled to -70°C and a solution of NBS (160 mg, 0.898 mmol) in THF (1 mL) was added dropwise over 1 min at -70°C. The mixture was warmed to rt and was stirred for 2h. The mixture was quenched with a saturated $NH_4Cl$ solution. The reaction was extracted three times with EtOAc. The combined organic layers were washed with brine, dried over anhydrous $Na_2SO_4$, filtered, and concentrated in vacuo. The crude material was purified by flash chromatography on silica gel using PE/EtOAc (92/8) as eluent to afford tert-butyl (2R)-2-(2-bromoacetyl)-octahydro-1H-indole-1-carboxylate **(Intermediate 18:** 168 mg, 64%) as a colorless oil. Mass spec: m/z: 346 $[M+H]^+$.

**Step 4**

**Intermediate 19: tert-butyl (2R)-2-{6-bromoimidazo[1,2-a]pyridin-2-yl}-boctahydro-1H-indole-1-carboxylate**

To a stirred solution of tert-butyl (2R)-2-(2-chloroacetyl)-octahydroindole-1-carboxylate **(Intermediate 18**:168 mg, 0.596 mmol) and 5-bromopyridin-2-amine (124 mg, 0.715 mmol) in Toluene (1 mL) and MeCN (3 mL) were added KI (9.90 mg, 0.06 mmol) and $NaHCO_3$ (60 mg, 0.715 mmol) at rt. The resulting mixture was stirred overnight at 100°C. The reaction was diluted with water and extracted with EtOAc three times. The combined organic layers were washed with brine, dried over anhydrous $Na_2SO_4$, filtered, and concentrated under reduced pressure. The crude material was purified by flash chromatography on silica gel using PE/EtOAc (81/19) to afford tert-butyl (2R)-2-{6-bromoimidazo[1,2-a]pyridin-2-yl}-octahydro-1H-indole-1-carboxylate **(Intermediate 19:** 110 mg, 73%) as a colorless oil. Mass spec: m/z: 420 $[M+H]^+$.

**Step 5**

**Intermediate 20: (2R)-2-{6-bromoimidazo[1,2-a]pyridin-2-yl}-octahydro-1H-indole trifluoroacetate**

To a stirred solution of tert-butyl (2R)-2-{6-bromoimidazo[1,2-a]pyridin-2-yl}-octahydroindole-1-carboxylate **(Intermediate 19**:110 mg, 0.262 mmol) in DCM (5 mL) was added TFA (1 mL). The mixture was stirred for 2h at rt. The reaction mixture was concentrated under reduced pressure to afford the title compound (2R)-2-{6-bromoimidazo[1,2-a]pyridin-2-yl}-octahydro-1H-indole trifluoroacetate **(Intermediate 20:** 80 mg, 95%) as a white solid which was used in Step 6 without further purification. Mass spec: m/z: 320 $[M+H]^+$.

**Step 6**

**Intermediate 21: (2R)-2-{6-bromoimidazo[1,2-a]pyridin-2-yl}-1-methyl-octahydro-1H-indole**

To a stirred solution of (2R)-2-{6-bromoimidazo[1,2-a]pyridin-2-yl}-octahydro-1H-indole trifluoroacetate **(Intermediate 20**: 60 mg, 0.187 mmol) in MeOH (3 mL) were added $Et_3N$ (0.26 mL, 1.87 mmol) and paraformaldehyde (39 mg, 1.31 mmol) . The resulting mixture was stirred for 1h at rt, then $NaBH_3CN$ (35 mg, 0.561 mmol) was added to the mixture which was stirred for 3h at 60°C. The mixture was concentrated under reduced pressure. The crude was purified by reverse-phase flash chromatography (C18 aq) using MeCN/$H_2O$ (85/15) as eluent to provide (2R)-2-{6-bromoimidazo[1,2-a]pyridin-2-yl}-1-methyl-octahydro-1H-indole **(Intermediate 21:** 60 mg, quant.) as a colorless oil. Mass spec: m/z: 334 $[M+H]^+$.

**Intermediate 27 was synthesized following Scheme 5.**

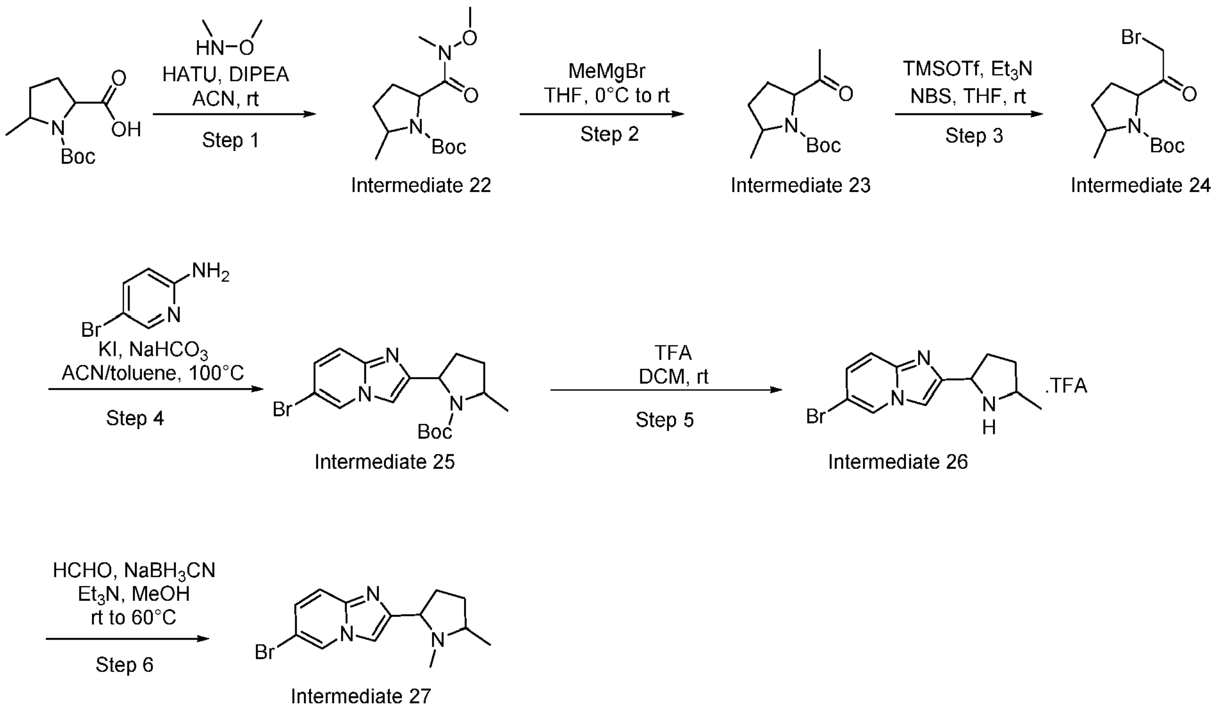


**Scheme 5**

**Step 1**

**Intermediate 22: rac-tert-butyl 2-[methoxy(methyl)carbamoyl]-5-methylpyrrolidine-1-carboxylate**

To a solution of rac-1-(tert-butoxycarbonyl)-5-methylpyrrolidine-2-carboxylic acid (CAS: 1248546-01-1, 1.50 g, 6.54 mmol) and N,O-dimethylhydroxylamine (799 mg, 13.1 mmol) in MeCN (30 mL) were added DIPEA (4.22 g, 32.7 mmol) and HATU (2.98 g, 7.85 mmol). The reaction mixture was stirred at rt overnight and then quenched with water (100 mL). The mixture was extracted three times with EtOAc. The combined organic layers were washed with brine, dried over anhydrous $Na_2SO_4$, filtered, and concentrated in vacuo to afford rac-tert-butyl 2-[methoxy(methyl)carbamoyl]-5-methylpyrrolidine-1-carboxylate **(Intermediate 22:** 1.8 g, 86%) as a colorless oil which was used in Step 2 without further purification. Mass spec: m/z: 173 $[M+H-Boc]^+$.

**Step 2**

**Intermediate 23: rac-tert-butyl 2-acetyl-5-methylpyrrolidine-1-carboxylate**

To a solution of rac-tert-butyl 2-[methoxy(methyl)carbamoyl]-5-methylpyrrolidine-1-carboxylate **(Intermediate 22**:1.80 g, 5.62 mmol) in THF (40 mL) was added a solution of MeMgBr (1.58 g, 13.2 mmol, 1M in THF). The reaction mixture was stirred at rt for 48h and then quenched with a saturated aqueous $NH_4Cl$ solution. The reaction mixture was extracted five times with EtOAc. The combined organic layers were washed brine, dried over anhydrous $Na_2SO_4$, filtered, and concentrated under reduced pressure. The crude material was purified by flash chromatography on silica gel using PE/EtOAc (4/1) to afford rac-tert-butyl 2-acetyl-5-methylpyrrolidine-1-carboxylate **(Intermediate 23:** 1.2 g, 76%) as a colorless oil. Mass spec: m/z: 128 $[M+H-Boc]^+$.

**Step 3**

**Intermediate 24: rac-tert-butyl 2-(2-bromoacetyl)-3-methylpyrrolidine-1-carboxylate**

To a solution of rac-tert-butyl 2-acetyl-3-methylpyrrolidine-1-carboxylate **(Intermediate 23:** 500 mg, 1.76 mmol) in THF (20 mL) at 0°C were added $Et_3N$ (890 mg, 8.80 mmol) and TMSOTf (733 mg, 3.30 mmol). The reaction mixture was stirred at rt for 1h and then a solution of NBS (587 mg, 3.300 mmol) in THF (5 mL) was added. The reaction mixture was stirred at rt for 4 h and quenched with water. The reaction mixture was extracted with EtOAc three times. The

combined organic layers were dried over anhydrous $Na_2SO_4$, filtered, and concentrated under reduced pressure. The crude material was purified by flash chromatography on silica gel using PE/EtOAc (3/1) to afford rac-tert-butyl 2-(2-bromoacetyl)-3-methylpyrrolidine-1-carboxylate **(Intermediate 24:** 120 mg, 18%) as a brown oil. Mass spec: m/z: 250 $[M+H-t-Bu]^+$.

**Step 4**

**Intermediate 25: rac-tert-butyl 2-{6-bromoimidazo[1,2-a]pyridin-2-yl}-5-methylpyrrolidine-1-carboxylate**

To a solution of rac-tert-butyl 2-(2-bromoacetyl)-3-methylpyrrolidine-1-carboxylate **(Intermediate 24:** 100 mg, 0.262 mmol) and 5-bromopyridin-2-amine (56.5 mg, 0.327 mmol) in toluene (1.25 mL) and MeCN (3.75 mL) were added KI (5.42 mg, 0.0330 mmol) and $NaHCO_3$ (32.9 mg, 0.392 mmol). The reaction mixture was stirred at 100°C for 3h and then concentrated under reduced pressure. The crude was purified by reverse-phase flash chromatography (C18 aq) using MeCN/$H_2O$ (0.05% TFA) (1/3) as eluent to provide rac-tert-butyl 2-{6-bromoimidazo[1,2-a]pyridin-2-yl}-5-methylpyrrolidine-1-carboxylate **(Intermediate 25:** 90 mg, 64%) as a brown solid. Mass spec: m/z: 380 $[M+H]^+$.

**Step 5**

**Intermediate 26: rac-2-{6-bromoimidazo[1,2-a]pyridin-2-yl}-5-methylpyrrolidine trifluoroacetate**

To a solution of rac-tert-butyl 2-{6-bromoimidazo[1,2-a]pyridin-2-yl}-5-methylpyrrolidine-1-carboxylate **(Intermediate 25:** 90 mg, 0.166 mmol) in DCM (1.5 mL) was added TFA (0.5 mL). The reaction mixture was stirred at rt for 2h and then concentrated under reduced pressure to afford rac-2-{6-bromoimidazo[1,2-a]pyridin-2-yl}-5-methylpyrrolidine trifluoroacetate **(Intermediate 26:** 80 mg, 97%) as a brown oil. Mass spec: m/z: 270 $[M+H]^+$.

**Step 6**

**Intermediate 27: rac-2-{6-bromoimidazo[1,2-a]pyridin-2-yl}-1,3-dimethylpyrrolidine**

A solution of rac-2-{6-bromoimidazo[1,2-a]pyridin-2-yl}-3-methylpyrrolidine trifluoroacetate **(Intermediate 26:** 50.0 mg, 0.125 mmol) and paraformaldehyde (42.9 mg, 1.42 mmol) in MeOH (3 mL) was stirred at rt for 1h and then $NaBH_3CN$ (33.6 mg, 0.534 mmol) was added. The reaction mixture was stirred at 60°C for 1h and then concentrated under reduced pressure. The crude was purified by reverse-phase flash chromatography (C18 aq) using MeCN/$H_2O$ (0.05% TFA) (1/3) as eluent to afford rac-2-{6-bromoimidazo[1,2-a]pyridin-2-yl}-l,3-dimethylpyrrolidine **(Intermediate 27:** 25 mg, 55%) as a white solid. Mass spec: m/z: 294 $[M+H]^+$.

**Intermediates 33 was synthesized following Scheme 6.**

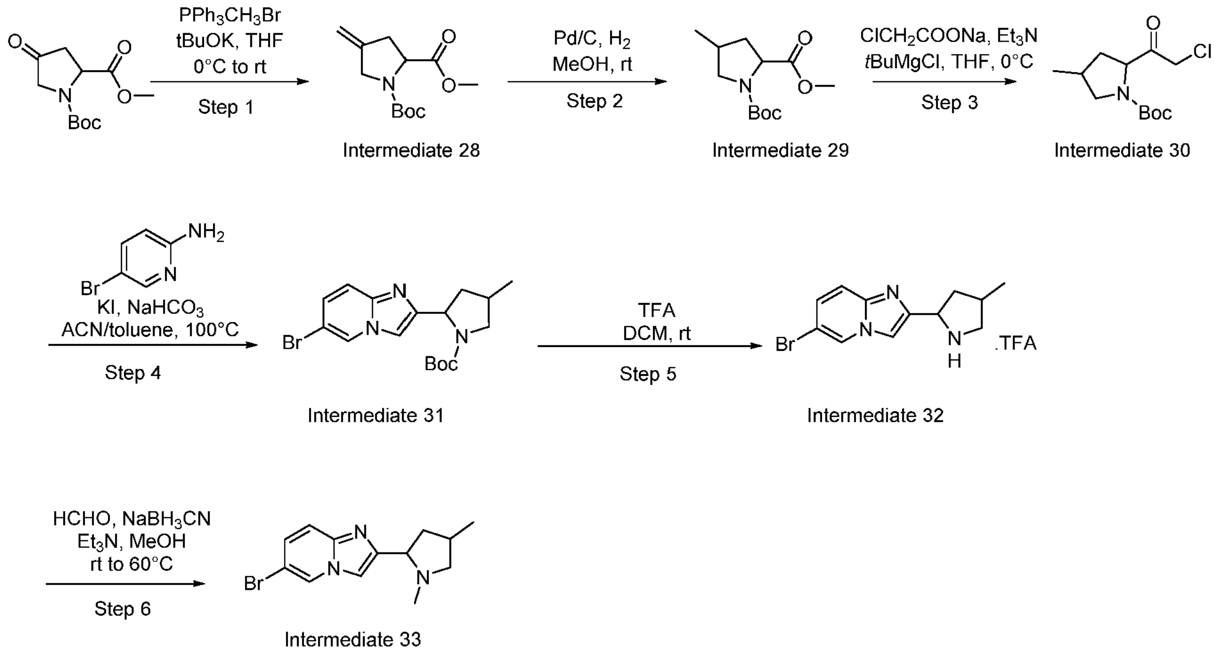


## Scheme 6

**Step 1**

**Intermediate 28: rac-1-tert-butyl 2-methyl 4-methylidenepyrrolidine-1,2-dicarboxylate**

A solution of tBuOK (2.77 g, 24.7 mmol) in THF (40 mL) was added to a suspension of methyltriphenylphosphonium bromide (8.81 g, 24.7 mmol) in THF (40 mL) at 0°C. The yellow suspension was stirred at 0°C for 2h before the addition of a solution of 1-tert-butyl 2-methyl 4-oxopyrrolidine-1,2-dicarboxylate (CAS: 362706-26-1, 3.00 g, 12.4 mmol) in THF (40 mL). The reaction mixture was stirred at rt for 3h and then quenched with water. The mixture was diluted with EtOAc and both phases were separated. The aqueous layer was extracted twice with EtOAc. The organic layers were combined, washed with brine dried over anhydrous $Na_2SO_4$, filtered, and concentrated under reduced pressure. The crude material was purified by flash chromatography on silica gel using PE/EtOAc (9/1) to afford rac-1-tert-butyl 2-methyl 4-methylidenepyrrolidine-1,2-dicarboxylate **(Intermediate 28:** 1.70 g, 51%) as colorless oil. Mass spec: m/z: 242 $[M+H]^+$.

**Step 2**

**Intermediate 29: rac-1-tert-butyl 2-methyl 4-methylpyrrolidine-1,2-dicarboxylate**

To a solution of rac-1-tert-butyl 2-methyl 4-methylidenepyrrolidine-1,2-dicarboxylate **(Intermediate 28:** 1.70 g, 7.34 mmol) in MeOH (40 mL) was added Pd/C (225 mg, 10% in carbon). The mixture was placed under a $H_2$ atmosphere (3 atm) and stirred at rt overnight. The solids were filtered out through a celite pad and the filtrate was concentrated under reduced pressure to provide rac-1-tert-butyl 2-methyl 4-methylpyrrolidine-1,2-dicarboxylate **(Intermediate 29:** 1.70 g, 99%) as colorless oil. Mass spec: m/z: 244 $[M+H]^+$.

**Step 3**

**Intermediate 30: rac-tert-butyl 2-(2-chloroacetyl)-4-methylpyrrolidine-1-carboxylate**

To a solution of rac-1-tert-butyl 2-methyl 4-methylpyrrolidine-1,2-dicarboxylate **(Intermediate 29:** 1.50 g, 5.55 mmol) and sodium 2-chloroacetate (1.80 g, 15.4 mmol) in THF (40 mL) was added a solution of tert-butylmagnesium chloride (1.80 g, 15.4 mmol, 1M in THF) dropwise at 0°C under $N_2$ atmosphere. The reaction mixture was stirred at 0-5 °C for 2h. Then, the mixture was allowed to warm to room temperature and stirred overnight. The reaction was quenched

with a citric acid solution and the mixture was extracted three times with EtOAc. The combined organic layers were washed with brine, dried over anhydrous $Na_2SO_4$, filtered, and concentrated under reduced pressure to provide rac-tert-butyl 2-(2-chloroacetyl)-4-methylpyrrolidine-1-carboxylate **(Intermediate 30:** 1.40 g, 48%) as a yellow oil which was used as such in Step 4. Mass spec: m/z: 262 $[M+H]^+$.

**Step 4**

**Intermediate 31: rac-tert-butyl 2-{6-bromoimidazo[1,2-a]pyridin-2-yl}-4-methylpyrrolidine-1-carboxylate**

To a solution of rac-tert-butyl 2-(2-chloroacetyl)-4-methylpyrrolidine-1-carboxylate **(Intermediate 30:** 700 mg, 0.670 mmol) and 5-bromopyridin-2-amine (278 mg, 1.61 mmol) in MeCN (4 mL) and toluene (1 mL) were added KI (23.0 mg, 0.134 mmol) and $NaHCO_3$ (225 mg, 2.68 mmol). The reaction mixture was stirred at 100°C for 18h and then quenched with water (8 mL). The crude concentrated under reduced pressure. The crude was purified by reverse-phase flash chromatography (C18 aq) using MeCN/$H_2O$ (neutral) (65/35) as eluent to provide rac-tert-butyl 2-{6-bromoimidazo[1,2-a]pyridin-2-yl}-4-methylpyrrolidine-1-carboxylate **(Intermediate 31:** 330 mg, 65%) as a yellow oil. Mass spec: m/z: 380 $[M+H]^+$.

**Step 5**

**Intermediate 32: rac-2-{6-bromoimidazo[1,2-a]pyridin-2-yl}-4-methylpyrrolidine trifluoroacetate**

To a solution of rac-tert-butyl 2-{6-bromoimidazo[l,2-a]pyridin-2-yl}-4-methylpyrrolidine-1-carboxylate **(Intermediate 31:** 330 mg, 0.764 mmol) in DCM (4 mL) was added TFA (1 mL). The reaction mixture was stirred at rt for 2h. The reaction mixture was concentrated under reduced pressure to afford rac-2-{6-bromoimidazo[1,2-a]pyridin-2-yl}-4-methylpyrrolidine trifluoroacetate **(Intermediate 32:** 220 mg, quant) as a yellow oil which was used in Step 6 without further purification. Mass spec: m/z: 280 $[M+H]^+$.

**Step 6**

**Intermediate 33: rac-2-{6-bromoimidazo[1,2-a]pyridin-2-yl}-1,4-dimethylpyrrolidine**

A solution of rac-tert-butyl 2-{6-bromoimidazo[1,2-a]pyridin-2-yl}-4-methylpyrrolidine-1-carboxylate trifluoroacetate **(Intermediate 32:** 220 mg, 0.675 mmol), $Et_3N$ (238 mg, 2.36 mmol) and paraformaldehyde (70.8 mg, 2.36 mmol) in MeOH (4 mL) was stirred at rt for 1h. $NaBH_3CN$ (49.3 mg, 0.785 mmol) was added and the reaction mixture was stirred at 60°C for 1h and then quenched with water. The mixture was concentrated under reduced pressure. The crude was purified by reverse-phase flash chromatography (C18 aq) using MeCN/$H_2O$ (neutral) (60/40) as eluent to afford rac-2-{ 6-bromoimidazo[1,2-a]pyridin-2-yl} -1,4-dimethylpyrrolidine **(Intermediate 33:** 140 mg, 69%) as a yellow oil. Mass spec: m/z: 294 $[M+H]^+$.

**Intermediate 38 was synthesized following Scheme 7.**

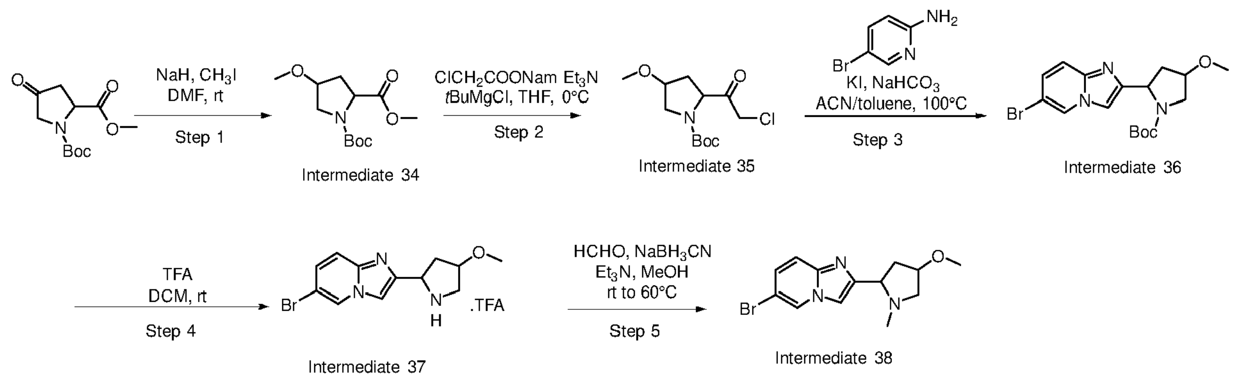


## Scheme 7

### Step 1

**Intermediate 34: rac-1-tert-butyl 2-methyl 4-methoxypyrrolidine-1,2-dicarboxylate**

To a solution of rac-1-tert-butyl 2-methyl 4-hydroxypyrrolidine-1,2-dicarboxylate (CAS: 897046-42-3, 4.00 g, 15.5 mmol) in DMF (100 mL) at 0°C was added NaH (0.720 g, 17.9 mmol, 60% in mineral oil). The reaction mixture was stirred at 0°C for 15 min. Iodomethane (3.47 g, 24.5 mmol) was added and the mixture was allowed to warm to rt and stirred for 2h. The reaction mixture was quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (1/9) to afford rac-1-tert-butyl 2-methyl 4-methoxypyrrolidine-1,2-dicarboxylate **(Intermediate 35:** 3.30 g, 82%) as colorless liquid. Mass spec: m/z 260 $[M+H]^+$.

### Step 2

**Intermediate 35: rac-tert-butyl 2-(2-chloroacetyl)-4-methylpyrrolidine-1-carboxylate**

To a solution of rac-1-tert-butyl 2-methyl 4-methoxypyrrolidine-1,2-dicarboxylate **(Intermediate 34**:1.50 g, 5.21 mmol), sodium 2-chloroacetate (1.68 g, 14.5 mmol) and $Et_3N$ (1.17 g, 11.6 mmol) in THF (30 mL) at 0°C was added dropwise a solution of tert-butylmagnesium chloride (15 mL, 14.5 mmol, 1M in THF). The reaction mixture was stirred at 0~5 °C for 2 h. The mixture was allowed to warm to rt and stirred overnight. The reaction was quenched with a citrate acid aqueous solution and the mixture was extracted with three times with EtOAc. The combined organic layers were washed with brine, dried over anhydrous $Na_2SO_4$, filtered, and concentrated under vacuum to afford rac-tert-butyl 2-(2-chloroacetyl)-4-methylpyrrolidine-1-carboxylate **(Intermediate 35:** 1.40 g, 48%) as yellow oil which was used in Step 3 without further purification. Mass spec: m/z 278 $[M+H]^+$.

### Step 3

**Intermediate 36: rac-tert-butyl 2-{6-bromoimidazo[1,2-a]pyridin-2-yl}-4-methoxypyrrolidine-1-carboxylate**

To a solution of rac-tert-butyl 2-(2-chloroacetyl)-4-methylpyrrolidine-1-carboxylate **(Intermediate 35:** 800 mg, 1.44 mmol) in MeCN (10 mL) and toluene (4 mL) was added 5-bromopyridin-2-amine (600 mg, 3.46 mmol), KI (100 mg, 0.576 mmol) and $NaHCO_3$ (480 mg, 5.76 mmol). The reaction mixture was stirred at 100°C overnight and then quenched with water. The mixture was extracted three times with EtOAc. The combined organic layers were washed with brine, dried over anhydrous $Na_2SO_4$, filtered, and concentrated under reduced pressure. The crude material was purified by flash chromatography on silica gel using DCM/MeOH (10/1) as eluent to afford rac-tert-butyl 2-{6-bromoimidazo[1,2-a]pyridin-2-yl}-4-methoxypyrrolidine-1-carboxylate **(Intermediate 36:** 460 mg, 64%) as a brown solid. Mass spec: m/z 396 $[M+H]^+$.

**Step 4**

**Intermediate 37: rac-2-{6-bromoimidazo[1,2-a]pyridine-2-yl}-4-methoxypyrrolidine trifluoroacetate**

To a solution of rac-tert-butyl 2-{6-bromoimidazo[l,2-a]pyridine-2-yl}-4-methoxypyrrolidine-1-carboxylate **(Intermediate 36:** 360 mg, 0.726 mmol) in DCM (8 mL) was added TFA (2 mL). The reaction mixture was stirred at rt for 2h, then concentrated under reduced pressure to give the rac-2-{6-bromoimidazo[1,2-a]pyridine-2-yl}-4-methoxypyrrolidine trifluoroacetate **(Intermediate 37:** 270 mg, quant.) as black oil which was used in Step 5 without further purification. Mass spec: m/z 296 [M+H]+.

**Step 5**

**Intermediate 38: rac-2-{6-bromoimidazo[1,2-a]pyridine-2-yl}-4-methoxy-1-methylpyrrolidine**

A solution of rac-2-{6-bromoimidazo[1,2-a]pyridine-2-yl}-4-methoxypyrrolidine trifluoroacetate **(Intermediate 37:** 270 mg, 0.730 mmol), $Et_3N$ (277 mg, 2.74 mmol) and paraformaldehyde (82.1 mg, 2.74 mmol) in MeOH (5 mL) was stirred at rt for 1h. $NaBH_3CN$ (57.3 mg, 0.912 mmol) was added and the reaction mixture was stirred at 60°C for 1h, and then quenched with water. The mixture was concentrated under reduced pressure. The crude was purified by reverse-phase flash chromatography (C18 aq) using CAN/$H_2O$ (neutral) (60/40) as eluent to afford rac-2-{6-bromoimidazo[1,2-a]pyridine-2-yl}-4-methoxy-1-methylpyrrolidine **(Intermediate** 38: 120 mg, 50%) as a yellow oil. Mass spec: m/z 312 $[M+H]^+$.

**General Procedure 1**

**Intermediate 43** was **synthesized following Scheme 8.**

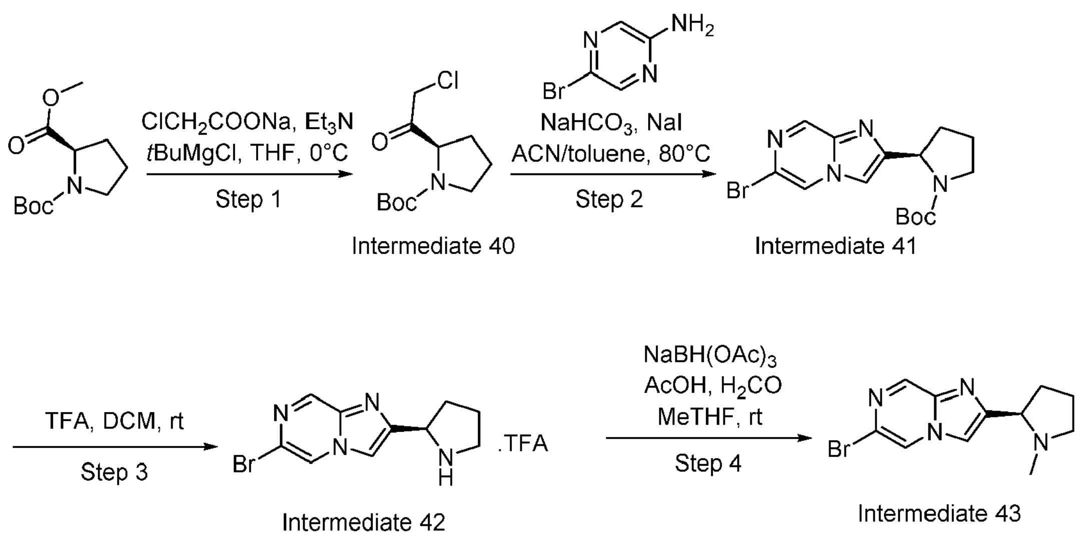


**Scheme 8**

**Step** 1

**Intermediate** 40: **tert-butyl (2R)-2-(2-chloroacetyl)pyrrolidine-1-carboxylate**

To a solution of 1-tert-butyl 2-methyl (2R)-pyrrolidine-1,2-dicarboxylate (CAS: 73323-65-6: $\alpha_D$ +66.7°, C=1 in MeOH; 2.00 g, 8.29 mmol), sodium chloroacetate (2.54 g, 20.7 mmol) and triethylamine (2.3 mL, 16.6 mmol) in THF-anhydrous (18.8 mL) was added dropwise a tert-butylmagnesium chloride solution (1M in THF, 21 mL, 20.7 mmol) at 2-3°C over 20 min. The reaction mixture was stirred between 0-5°C for 2h. A solution of citric acid (3.68 g, 19.1 mmol) in $H_2O$ (10.4 mL) was slowly added to the reaction mixture while maintaining the temperature between 0-5°C. EtOAc was added to the reaction mixture, the phases were separated. The organic layer was washed twice with a solution of

$NaHCO_3$/NaCl (470 mg of $NaHCO_3$, 1.6 g of NaCl in 10mL) and once with brine. The combined organic layers were dried over hydrophobic paper and concentrated under reduced pressure to afford tert-butyl (2R)-2-(2-chloroacetyl)pyrrolidine-1-carboxylate **(Intermediate 40:** 2.05 g, 95%) as a white solid which was used in Step 2 without further purification. Mass spec: m/z: 192 $[M+H-tBu]^+$, 148 $[M+H-Boc]^+$; $^1$H NMR (400 MHz, DMSO-$d_6$) $\delta$ ppm 4.71 - 4.53 (m, 2H), 4.40 (ddd, J = 11.1, 8.7, 4.8 Hz, 1H), 3.34 (d, J = 7.2 Hz, 2H), 2.24 - 2.05 (m, 1H), 1.93 - 1.66 (m, 3H), 1.35 (d, J = 32.2 Hz, 9H). Chiral analysis: Chiral purity = 98.6% ee (Column: IC 250x4.6 5$\mu$m; Eluent:5% MeOH+0.5% IPA; Flow rate: 2.4 mL/min; rt = 3.88 min).

**Step 2**

**Intermediate 41: tert-butyl (2R)-2-{6-bromoimidazo[1,2-a]pyrazin-2-yl}pyrrolidine-1-carboxylate**

In a round bottom flask, to a suspension of 5-bromopyrazin-2-amine (CAS: 59489-71-3, 0.92 g, 5.18 mmol) and tert-butyl (2R)-2-(2-chloroacetyl)pyrrolidine-1-carboxylate **(Intermediate 40:** 2.03 g, 7.77 mmol) in toluene (5.2 mL) and MeCN (5.2 mL) were added $NaHCO_3$ (0.52 g, 6.22 mmol) and sodium iodide (1.17 g, 7.77 mmol). The reaction mixture was heated at 80°C overnight. The solvents were removed under reduced pressure and the residue was taken up in EtOAc. The mixture was filtered through a pad of decalite, then the filtrate was washed with a saturated solution of $Na_2S_2O_3$. The organic layer was dried over anhydrous $Na_2SO_4$, filtered, and concentrated in vacuo. The crude material was purified by flash chromatography on silica gel using a gradient of EtOAc in heptane from 30% to 100% to afford tert-butyl (2R)-2-{6-bromoimidazo[1,2-a]pyrazin-2-yl}pyrrolidine-1-carboxylate **(Intermediate 41:** 485 mg, 26%) as a brown solid. Mass spec: m/z: 367 $[M+H]^+$, 369 $[M+H+2]^+$; $^1$H NMR (400 MHz, DMSO-$d_6$) $\delta$ ppm 8.92 (d, J = 16.1 Hz, 1H), 8.86 (s, 1H), 7.90 (d, J = 10.1 Hz, 1H), 4.99 (dd, J = 16.0, 7.9 Hz, 1H), 3.46 (ddd, J = 11.5, 7.7, 4.1 Hz, 1H), 3.39 (dt, J = 10.4, 8.1 Hz, 1H), 2.31 - 2.15 (m, 1H), 2.05 (s, 1H), 1.91 (dq, J = 18.0, 6.2 Hz, 2H), 1.30 (s, 9H). Chiral analysis: Chiral purity = 98.8% ee (Column: IA 250 x 4.6 5 $\mu$m; Eluent: 30% (iPrOH+0.5% IPA); Flow rate: 2.4 mL/min; rt = 3.03 min).

**Intermediate 41A: rac-tert-butyl 2-{6-bromoimidazo[1,2-a]pyrazin-2-yl}pyrrolidine-1-carboxylate**

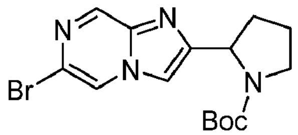

Following an analogous procedure to **Scheme 8, Step 2,** using 5-bromopyrazin-2-amine as the amine (25 g, 0.14 mol) and rac-tert-butyl-2-(2-chloroacetyl)pyrrolidine-1-carboxylate **(Intermediate 1:** 60 g, 0.206 mol) as the ketone, afforded rac-tert-butyl 2-{6-bromoimidazo[1,2-a]pyrazin-2-yl}pyrrolidine-1-carboxylate **(Intermediate 41A:** 18.65 g, 36%) as a brown solid. Mass spec: m/z: 367 $[M+H]^+$, 369 $[M+H+2]^+$; $^1$H NMR (400 MHz, DMSO-$d_6$) $\delta$ ppm 8.98 - 8.84 (m, 2H), 7.90 (d, J = 9.2 Hz, 1H), 4.99 (dd, J = 15.8, 7.8 Hz, 1H), 3.50 - 3.34 (m, 2H), 2.23 (s, 1H), 2.05 (s, 1H), 1.97 - 1.82 (m, 2H), 1.30 (s, 9H).

**Intermediate 41B: tert-butyl (2R)-2-(6-bromo-8-methyl-imidazo[1,2-a]pyrazin-2-yl)pyrrolidine-1-carboxylate**

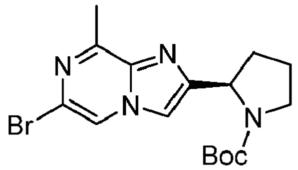

Following an analogous procedure to **Scheme 8, Step 2,** using 5-bromo-3-methylpyrazin-2-amine (CAS: 74290-67-8, 0.75 g, 3.90 mmol) as the amine and tert-butyl (2R)-2-(2-chloroacetyl)pyrrolidine-1-carboxylate **(Intermediate 40:** 1.53 g, 5.86 mmol) as the ketone, afforded tert-butyl (2R)-2-(6-bromo-8-methyl-imidazo[1,2-a]pyrazin-2-yl)pyrrolidine-1-carboxylate **(Intermediate 41B:** 400 mg, 27%) as an orange solid. Mass spec: m/z: 382 $[M+H]^+$, 384 $[M+H+2]^+$; $^1$H NMR (400 MHz, DMSO-$d_6$) $\delta$ ppm 8.75 (d, J = 15.5 Hz, 1H), 7.83 (d, J = 14.6 Hz, 1H), 4.97 (s, 1H), 3.45 (s, 1H),

3.42 - 3.36 (m, 1H), 2.70 (s, 3H), 2.24 (d, J = 13.8 Hz, 1H), 2.05 (dq, J = 12.4, 5.4 Hz, 1H), 1.93 - 1.80 (m, 2H), 1.50 - 1.17 (m, 9H).

**Intermediate 41C: (rel-2R)-tert-butyl 2-{6-bromoimidazo[1,2-a]pyrazin-2-yl}pyrrolidine-1-carboxylate and Intermediate 41D: (rel-2S)-tert-butyl 2-{6-bromoimidazo[1,2-a]pyrazin-2-yl}pyrrolidine-1-carboxylate**

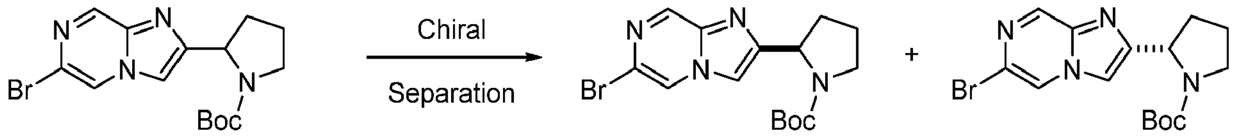


Intermediate 41A Intermediate 41C Intermediate 41D

rac-tert-Butyl 2-{6-bromoimidazo[1,2-a]pyrazin-2-yl}pyrrolidine-1-carboxylate **(Intermediate 41A)** was separated by chiral SFC using the following conditions:

Column: CHIRAL ART Amylose-C NEO 3x25 cm, 5μm; Mobile Phase A: CO2,
Mobile Phase B: MeOH (with 1% 2M $NH_3$ in MeOH); Flow rate: 85 mL/min; Gradient: isocratic 40% B.

**Intermediate 41C: (rel-2R)-tert-butyl 2-{6-bromoimidazo[1,2-a]pyrazin-2-yl}pyrrolidine-1-carboxylate**

First eluting compound (retention time: 2.7 mins)
Mass spec: m/z: 367 $[M+H]^+$, 369 $[M+H+2]^+$; $^1H$ NMR (400 MHz, DMSO-$d_6$) δ ppm 8.98 - 8.84 (m, 2H), 7.90 (d, J = 9.2 Hz, 1H), 4.99 (dd, J = 15.8, 7.8 Hz, 1H), 3.50 - 3.34 (m, 2H), 2.23 (s, 1H), 2.05 (s, 1H), 1.97 - 1.82 (m, 2H), 1.30 (s, 9H).

**Intermediate 41D: (rel-2S)-tert-butyl 2-{6-bromoimidazo[1,2-a]pyrazin-2-yl}pyrrolidine-1-carboxylate**

Second eluting compound (retention time: 6.57 mins)
Mass spec: m/z: 367 $[M+H]^+$, 369 $[M+H+2]^+$; $^1H$ NMR (400 MHz, DMSO-$d_6$) δ ppm 8.98 - 8.84 (m, 2H), 7.90 (d, J = 9.2 Hz, 1H), 4.99 (dd, J = 15.8, 7.8 Hz, 1H), 3.50 - 3.34 (m, 2H), 2.23 (s, 1H), 2.05 (s, 1H), 1.97 - 1.82 (m, 2H), 1.30 (s, 9H).

**Step 3**

**Intermediate 42: (2R)-2-{6-bromoimidazo[1,2-a]pyrazin-2-yl}pyrrolidine trifluoroacetate**

Under a nitrogen atmosphere, tert-butyl (2R)-2-(6-bromoimidazo[1,2-a]pyrazin-2-yl)pyrrolidine-1-carboxylate **(Intermediate 41:** 185 mg, 0.504 mmol) was solubilized in DCM (2.5 mL) and 2,2,2-trifluoroacetic acid (0.39 mL, 5.04 mmol ) was added. The mixture was stirred at rt for 2h. The reaction mixture was concentrated under reduced pressure to afford (2R)-2-{6-bromoimidazo[1,2-a]pyrazin-2-yl}pyrrolidine trifluoroacetate **(Intermediate 42:** 228 mg, quant.) as a brown oil which were used in Step 4 without further purification. Mass spec: m/z: 267 $[M+H]^+$; 269 $[M+H+2]^+$. 1H NMR (400 MHz, DMSO-$d_6$) δ ppm 9.62 (s, 1H), 9.05 (d, J = 1.4 Hz, 1H), 9.02 - 9.00 (m, 1H), 8.24 (s, 1H), 4.92 (p, J = 6.9 Hz, 1H), 3.33 (dq, J = 8.3, 5.1 Hz, 2H), 2.49 - 2.36 (m, 1H), 2.23 - 1.97 (m, 3H). Chiral analysis: Chiral purity = 98.5% ee (Column: AD 250x4.6 5μm; Eluent: 40% (EtOH + 0.5% IPA); Flow rate: 2.4 mL/min; rt = 4.74 min).

**Intermediate 42A: rac-2-{6-bromoimidazo[1,2-a]pyrazin-2-yl}pyrrolidine trifluoroacetate**

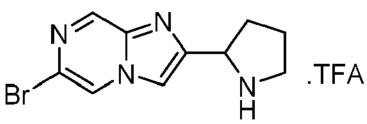

Following an analogous procedure to **Scheme 8, Step 3,** using rac-tert-butyl 2-(6-bromoimidazo[1,2-a]pyrazin-2-yl)pyrrolidine-1-carboxylate **(Intermediate 41A:** 21.86 g, 56.5 mmol) as the Boc-protected amine, afforded rac-2-{6-bromoimidazo[1,2-a]pyrazin-2-yl}pyrrolidine trifluoroacetate **(Intermediate 42A:** 22 g, quant.) as an orange oil. Mass

spec: m/z: 267 $[M+H]^+$, 269 $[M+H+2]^+$; $^1H$ NMR (400 MHz, DMSOd$_6$) $\delta$ ppm 9.62 (s, 1H), 9.05 (d, J = 1.4 Hz, 1H), 9.02 - 9.00 (m, 1H), 8.24 (s, 1H), 4.92 (p, J = 6.9 Hz, 1H), 3.33 (dq, J = 8.3, 5.1 Hz, 2H), 2.49 - 2.36 (m, 1H), 2.23 - 1.97 (m, 3H).

**Intermediate 42B: (2R)-2-{6-bromo-8-methylimidazo[1,2-a]pyrazin-2-yl}pyrrolidine trifluoroacetate**

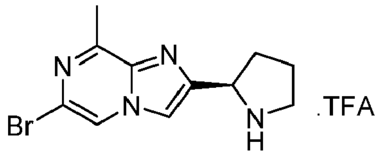

Following an analogous procedure to **Scheme 8, Step 3,** starting with tert-butyl (2R)-2-(6-bromo-8-methylimidazo[1,2-a]pyrazin-2-yl)pyrrolidine-1-carboxylate **(Intermediate 41B:** 400 mg, 1.05 mmol), afforded (2R)-2-{6-bromo-8-methylimidazo[1,2-a]pyrazin-2-yl}pyrrolidine trifluoroacetate **(Intermediate 42B:** 691 mg, quant.) as a red oil. Mass spec: m/z: 281 $[M+H]^+$, 283 $[M+H+2]^+$; $^1H$ NMR (400 MHz, DMSO-d$_6$) $\delta$ ppm 9.71 (s, 1H), 8.97 (s, 1H), 8.88 (d, J = 0.8 Hz, 1H), 8.20 (s, 1H), 4.88 (p, J = 7.0 Hz, 1H), 3.40 - 3.24 (m, 2H), 2.76 (s, 3H), 2.48 - 2.36 (m, 1H), 2.23 - 1.97 (m, 3H).

**Step 4**

**Intermediate 43: (2R)-2-{6-bromoimidazo[1,2-a]pyrazin-2-yl}-1-methylpyrrolidine**

(2R)-2-{6-bromoimidazo[1,2-a]pyrazin-2-yl}pyrrolidine trifluoroacetate **(Intermediate 42:** 228 mg, 0.508 mmol) was dissolved in 2-MeTHF (2.5 mL). Formaldehyde (37% aq, 0.076 mL, 1.02 mmol) was added followed by acetic acid (0.058 mL, 1.02 mmol). The reaction mixture was stirred for 20 min, then $NaBH(OAc)_3$ (167 mg, 0.763 mmol) was added portion wise (gas evolution). The mixture was stirred for 2h, then cooled to 0 °C and quenched slowly with a saturated solution of $NaHCO_3$. The aqueous layer was extracted with EtOAc three times. The organic layers were combined, washed with brine, dried over anhydrous $MgSO_4$ and the solvents were removed under reduced pressure to afford (2R)-2-{6-bromoimidazo[1,2-a]pyrazin-2-yl}-1-methylpyrrolidine **(Intermediate 43:** 140 mg, 94%) as a yellow oil. m/z: 281 $[M+H]^+$, 283 $[M+H+2]^+$; $^1H$ NMR (400 MHz, DMSO-d$_6$) $\delta$ ppm 8.91 (d, J = 1.4 Hz, 1H), 8.86 (d, J = 1.2 Hz, 1H), 8.02 (s, 1H), 3.64 (s, 1H), 3.18 (d, J = 9.0 Hz, 1H), 2.46 (d, J = 8.6 Hz, 1H), 2.30 (s, 4H), 1.95 - 1.80 (m, 3H). Chiral analysis: Chiral purity = 98.8% ee (Column: AD 250 $\times$ 4.6 5 $\mu$m N°317; Eluent: 25 % (iPrOH + 0.5% IPA); Flow rate: 2.4 mL/min; rt = 6.45 min).

**Intermediate 43A: rac-2-{6-bromoimidazo[1,2-a]pyrazin-2-yl}-1-methylpyrrolidine**

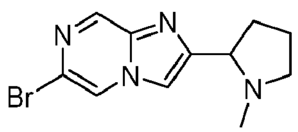

Following an analogous procedure to **Scheme 8, Step 4,** starting with rac-6-bromo-2-pyrrolidin-2-yl-imidazo[1,2-a]pyrazine trifluoroacetate **(Intermediate 42A:** 19.30 g, 48.1 mmol) as the amine, afforded rac-2-{6-bromoimidazo[1,2-a]pyrazin-2-yl}-1-methylpyrrolidine **(Intermediate 43A:** 8.13 g, 58%) as a brown oil. Mass spec: m/z: 281 $[M+H]^+$, 283 $[M+H+2]^+$; $^1H$ NMR (400 MHz, DMSO-d$_6$) $\delta$ ppm 8.91 (d, J = 1.4 Hz, 1H), 8.86 (d, J = 1.2 Hz, 1H), 8.02 (s, 1H), 3.64 (s, 1H), 3.18 (d, J = 9.0 Hz, 1H), 2.46 (d, J = 8.6 Hz, 1H), 2.30 (s, 4H), 1.95 - 1.80 (m, 3H).

**Intermediate 43B: (2R)-2-{6-bromo-8-methylimidazo[1,2-a]pyrazin-2-yl}-1-methylpyrrolidine**

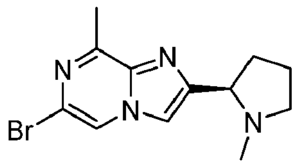

Following an analogous procedure to **Scheme 8, Step 4,** starting with (2R)-2-{6-bromo-8-methylimidazo[1,2-a]pyrazin-2-yl}pyrrolidine trifluoroacetate **(Intermediate 42B:** 691 mg, 1.75 mmol) as the amine, afforded (2R)-2-{6-bromo-8-methylimidazo[1,2-a]pyrazin-2-yl}-1-methylpyrrolidine **(Intermediate 43B:** 313 mg, 59%) as a yellow oil. m/z: 295 $[M+H]^+$, 297 $[M+H+2]^+$; $^1H$ NMR (400 MHz, DMSO-$d_6$) δ ppm 8.74 (s, 1H), 7.95 (s, 1H), 3.61 - 3.46 (m, 1H), 3.16 (s, 1H), 2.71 (s, 3H), 2.38 (s, 1H), 2.32 - 2.20 (m, 4H), 1.83 (d, J = 16.7 Hz, 3H).

**Intermediate 44: (2S)-2-{6-bromoimidazo[1,2-a]pyrazin-2-yl}-1-methylpyrrolidine**

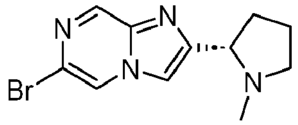

rac-2-{6-bromoimidazo[1,2-a]pyrazin-2-yl}-1-methylpyrrolidine **(Intermediate 43A:** 8.13 g, 28 mmol) was purified by chiral chromatography (Column: Chiralpak AD-H 250 × 30 mm, 5 μm; Eluent: $CO_2$/( iPrOH + 0.5% IPA) 65/35; rt = 2.5 to 3.5 min) to afford (2S)-2-{6-bromoimidazo[1,2-a]pyrazin-2-yl}-1-methylpyrrolidine **(Intermediate 44:** 3.95 g, 49%). m/z: 281 $[M+H]^+$, 283 $[M+H+2]^+$; $^1H$ NMR (400 MHz, DMSO-$d_6$) δ ppm 8.91 (d, J = 1.4 Hz, 1H), 8.86 (d, J = 1.2 Hz, 1H), 8.02 (s, 1H), 3.64 (s, 1H), 3.18 (d, J = 9.0 Hz, 1H), 2.46 (d, J = 8.6 Hz, 1H), 2.30 (s, 4H), 1.95 - 1.80 (m, 3H). Chiral analysis: Column: AD 250 × 4.6, 5 μm; Eluent: $CO_2$/(iPrOH + 0.5% IPA) 65/35; Flow rate: 2.4 mL/min; rt = 2.80 min, first eluting compound.

**General Procedure 2**

**Intermediates 47 and 48 were synthesized following Scheme 9.**

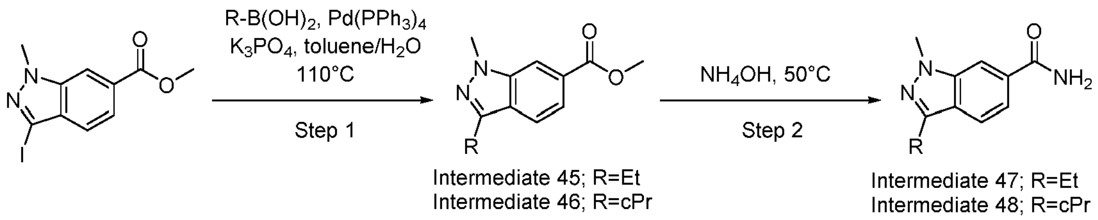


Intermediate 45; R=Et
Intermediate 46; R=cPr

Intermediate 47; R=Et
Intermediate 48; R=cPr

**Scheme 9**

**Step 1**

To a vial purged and maintained with an inert atmosphere of nitrogen, were added methyl 3-iodo-1-methyl-1H-indazole-6-carboxylate (CAS: 1041205-25-7, 1 eq), the boronic acid stated below (3 eq), $Pd(PPh_3)_4$ (0.1 eq) and $K_3PO_4$ (4 eq) in toluene/$H_2O$ (10/1) (0.15 M). The resulting mixture was stirred for 16 h at 110 °C. The solids were filtered through a pad of celite. The solution was concentrated under reduced pressure. The crude material was purified by flash chromatography on silica gel using a gradient of EtOAc in heptane from 0% to 50% to afford the intermediate detailed below.

**Intermediate 45: Methyl 3-ethyl-1-methyl-1H-indazole-6-carboxylate**

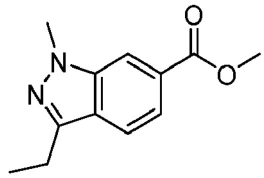

Following **Scheme 9, Step 1,** using ethylboronic acid (CAS: 4433-63-0) as the boronic acid, afforded methyl 3-ethyl-1-methyl-1H-indazole-6-carboxylate **(Intermediate 45:** 71 mg, 20%) as a colorless oil. Mass spec: m/z: 219 $[M+H]^+$; $^1H$ NMR (400 MHz, DMSO-$d_6$) δ ppm 8.21 (s, 1H), 7.85 (d, J = 8.4 Hz, 1H), 7.66 (dd, J = 8.5, 1,4 Hz, 1H), 4.05 (s, 3H), 3.91 (s, 3H), 2.94 (q, J = 7.5 Hz, 2H), 1.31 (t, J = 7.6 Hz, 3H).

**Intermediate 46: Methyl 3-cyclopropyl-1-methyl-1H-indazole-6-carboxylate**

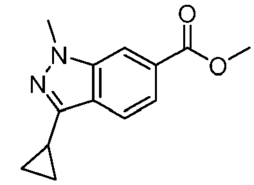

Following **Scheme 9, Step 1,** using cyclopropylboronic acid (CAS: 411235-57-9) as the boronic acid, afforded methyl 3-cyclopropyl-1-methyl-1H-indazole-6-carboxylate **(Intermediate 46:** 108 mg, 75%) as a pale yellow solid. Mass spec: m/z: 231 $[M+H]^+$; $^1H$ NMR (400 MHz, DMSO-$d_6$) δ ppm 8.19 (d, J = 1.4 Hz, 1H), 7.89 (dd, J = 8.4, 2.0 Hz, 1H), 7.65 (dd, J = 8.5, 1.5 Hz, 1H), 4.01 (s, 3H), 3.90 (s, 3H), 2.31 (tt, J = 8.3, 5.1 Hz, 1H), 1.01 (dtd, J = 8.6, 5.1, 2.2 Hz, 2H), 0.97 - 0.91 (m, 2H).

**Step 2**

A suspension of the appropriate intermediate prepared in **Scheme 9, Step 1** (1 eq) in ammonium hydroxide (20 eq) was stirred overnight at 50°C in a sealed tube. The reaction mixture was diluted in $H_2O$ and the precipitate was filtered to provide the expected products detailed below.

**Intermediate 47: 3-Ethyl-1-methyl-1H-indazole-6-carboxamide**

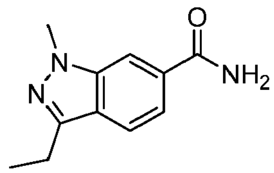

Following **Scheme 9, Step 2,** using methyl 3-ethyl-1-methyl-1H-indazole-6-carboxylate **(Intermediate 45),** afforded 3-ethyl-1-methyl-1H-indazole-6-carboxamide **(Intermediate 47:** 44 mg, 69%) as a white solid. Mass spec: m/z: 204 $[M+H]^+$; $^1H$ NMR (400 MHz, DMSO-$d_6$) δ ppm 8.11 (d, J = 1.2 Hz, 1H), 8.03 (d, J = 5.1 Hz, 1H), 7.80 - 7.74 (m, 1H), 7.59 (dd, J = 8.5, 1.4 Hz, 1H), 7.40 (s, 1H), 4.01 (s, 3H), 2.93 (q, J = 7.5 Hz, 2H), 1.31 (t, J = 7.6 Hz, 3H).

**Intermediate 48: 3-Cyclopropyl-1-methyl-1H-indazole-6-carboxamide**

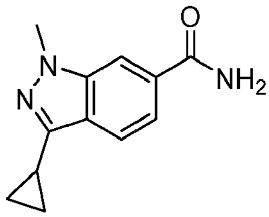

Following **Scheme 9, Step 2,** using afforded methyl 3-cyclopropyl-1-methyl-1H-indazole-6-carboxylate **(Intermediate 46)** afforded 3-cyclopropyl-1-methyl-1H-indazole-6-carboxamide **(Intermediate 48:** 33 mg, quant.) as a white solid. Mass spec: m/z: 217 $[M+H]^+$; $^1H$ NMR (400 MHz, DMSO-$d_6$) δ ppm 8.09 (s, 1H), 8.02 (s, 1H), 7.81 (d, J = 8.4 Hz, 1H), 7.59 (dd, J = 8.5, 1.4 Hz, 1H), 7.40 (s, 1H), 3.97 (s, 3H), 2.29 (tt, J = 8.3, 5.1 Hz, 1H), 1.05 - 0.90 (m, 4H).

**Intermediate 49: Methyl 1,3-dimethyl-1H-indazole-6-carboxylate**

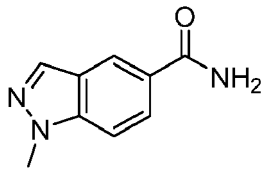

A stirred solution of 1-methyl-1H-indazole-5-carboxylic acid (CAS: 1176754-31-6, 2.50 g, 14.2 mmol) in anhydrous DCM (125 mL), with a few drops of DMF, was cooled to 0°C before addition of thionyl chloride (1.1 mL, 15.6 mmol). The reaction mixture was stirred 4h at rt until consumption of starting material observed. The mixture was concentrated to dryness and re-dissolved in DCM (110 mL). A solution of $NH_4OH$ (11 mL, 71.0 mmol, 25% in water) was added and the mixture was stirred vigorously overnight at rt. Water was added and the mixture was extracted three times with EtOAc. The combined organic layers were dried over a phase separator and concentrated to dryness. The residue was taken up in DCM and the precipitate was filtered, washed with DCM and dried under vaccum at 40°C to afford methyl 1,3-dimethyl-1H-indazole-6-carboxylate **(Intermediate 49:** 1.6 g, 64%) as a beige solid. Mass spec: m/z: 176 $[M+H]^+$; $^1H$ NMR (400 MHz, DMSO-$d_6$) δ ppm 8.35 (dd, *J* = 1.5, 0.7 Hz, 1H), 8.17 (d, *J* = 0.9 Hz, 1H), 7.98 (s, 1H), 7.93 (dd, *J* = 8.8, 1.6 Hz, 1H), 7.67 (d, *J* = 8.8 Hz, 1H), 7.27 (s, 1H), 4.07 (s, 3H).

**Intermediates 51 and 52 were synthesized following Scheme 10.**

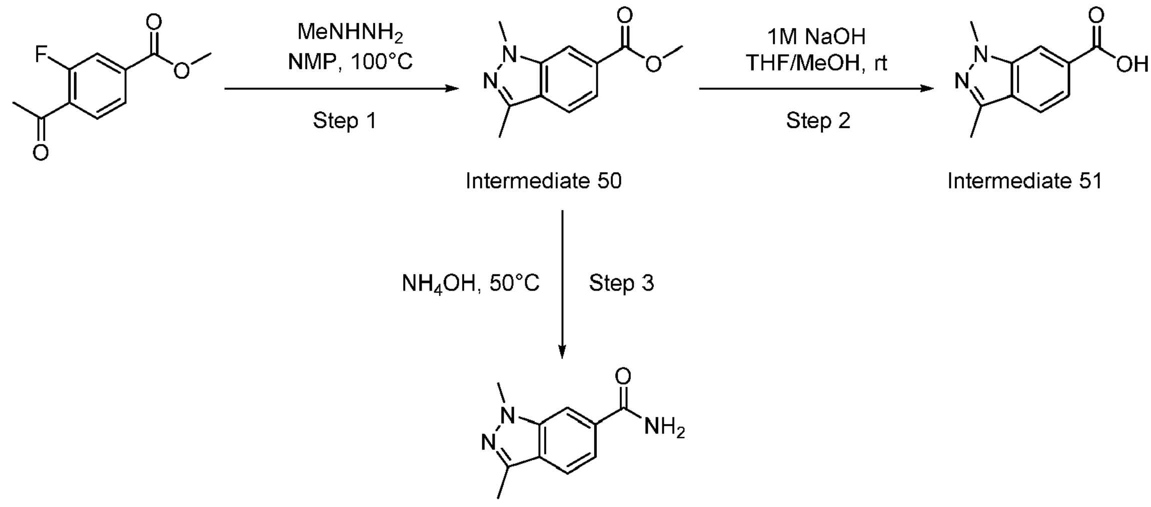


Intermediate 50

Intermediate 51

Intermediate 52

## Scheme 10

**Step 1**

**Intermediate 50: Methyl 1,3-dimethyl-1H-indazole-6-carboxylate**

To a solution of methyl 4-acetyl-3-fluorobenzoate (CAS: 1059549-72-2, 5.00 g, 24.2 mmol) in NMP (35 mL) was added methylhydrazine (1.5 mL, 29.1 mmol) and the mixture was stirred overnight at 100°C in a sealed tube. The solution was cooled to rt, diluted with $H_2O$ (35 mL) and the aqueous layer was extracted with EtOAc (40mL). The organic layer was washed three times with $H_2O$, dried over anhydrous $MgSO_4$, filtered, and concentrated to dryness to afford methyl 1,3-dimethyl-1H-indazole-6-carboxylate **(Intermediate 50:** 4.3 g, 87%) as an orange oil which was used in Step 2 without further purification. Mass spec: m/z: 205 $[M+H]^+$; $^1H$ NMR (400 MHz, DMSOd$_6$): δ ppm 8.21 (s, 1H), 7.81 (d, J=8.5 Hz, 1H), 7.65 (dd, J=8.5, 1.3 Hz, 1H), 4.03 (s, 3H), 3.90 (s, 3H), 2.50 (s, 3H).

**Step 2**

**Intermediate 51: 1,3-dimethyl-1H-indazole-6-carboxylic acid**

To a solution of methyl 1,3-dimethylindazole-6-carboxylate **(Intermediate 50:** 4.3 g, 20.0 mmol) in a mixture of THF (50 mL)/MeOH (50 mL) was added a NaOH solution (60 mL, 60.0 mmol, 1M in water) dropwise over 14 min. The resulting mixture was stirred at rt for 45 min. The solvents were removed under reduced pressure and the residue was acidified with a solution of HCl (60 mL, 1N in $H_2O$). The precipitate was filtered, rinsed with $H_2O$ (20 mL) and dried under reduced pressure at 40°C overnight to afford 1,3-dimethyl-1H-indazole-6-carboxylic acid **(Intermediate 51:** 3.85 g, quant.) as a yellow solid. Mass spec: m/z: 191 $[M+H]^+$; $^1H$ NMR (400 MHz, DMSO-d$_6$): δ ppm 13.04 (s, 1H), 8.19 (t, J=1.1 Hz, 1H), 7.79 (dd, J=8.3, 0.8 Hz, 1H), 7.65 (dd, J=8.4, 1.3 Hz, 1H), 4.03 (s, 3H), 2.51 (s, 3H).

**Step 3**

**Intermediate 52: 1,3-dimethylindazole-6-carboxamide**

Following the procedure exemplified by **Scheme 9, Step 2 (General Procedure 2),** using methyl 1,3-dimethyl-1H-indazole-6-carboxylate **(Intermediate 50)** as the ester, afforded 1,3-dimethylindazole-6-carboxamide **(Intermediate 52:** 2.15 g, 61%). Mass spec: m/z: 190 $[M+H]^+$; $^1H$ NMR (400 MHz, DMSO-d$_6$) δ ppm 8.11 (t, J = 1.1 Hz, 1H), 8.02 (s, 1H), 7.74 (dd, J = 8.4, 0.8 Hz, 1H), 7.60 (dd, J = 8.4, 1.4 Hz, 1H), 7.41 (s, 1H), 4.00 (s, 3H), 2.49 (s, 3H).

**Intermediate 55 was synthesized following Scheme 11.**

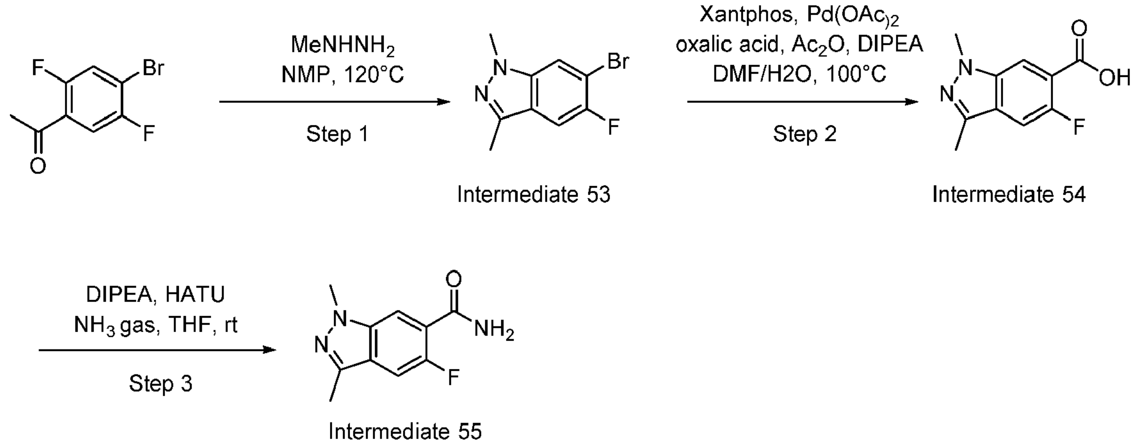


Intermediate 53

Intermediate 54

Intermediate 55

**Scheme 11**

**Step 1**

**Intermediate 53: 6-bromo-5-fluoro-1,3-dimethyl-1H-indazole**

To a stirred solution of 1-(4-bromo-2,5-difluorophenyl)ethan-1-one (CAS: 123942-11-0, 1.00 g, 4.04 mmol) in NMP (8 mL) was added methylhydrazine (0.26 mL, 4.85 mmol) and the mixture was stirred overnight at 120°C. After this time, water was added, and a precipitate was observed. This precipitate was filtered and dried under vacuum to afford 6-bromo-5-fluoro-1,3-dimethyl-1H-indazole **(Intermediate 53:** 859 mg, 87%) as a white solid. Mass spec: m/z: 243 $[M+H]^+$, 245 $[M+H+2]^+$; $^1H$ NMR (400 MHz, DMSO-$d_6$) δ ppm 8.07 (d, J = 5.6 Hz, 1H), 7.72 (d, J = 8.9 Hz, 1H), 3.95 (s, 3H), 2.44 (s, 3H).

**Step 2**

**Intermediate 54: 5-fluoro-1,3-dimethyl-1H-indazole-6-carboxylic acid**

In a sealed tube, to a stirred solution of Xantphos (409 mg, 0.707 mmol), 6-bromo-5-fluoro-1,3-dimethyl-indazole **(Intermediate 53:** 859 mg, 3.53 mmol), diacetoxypalladium (159 mg, 0.707 mmol), and oxalic acid (477 mg, 5.30 mmol) in DMF (5 mL) were added acetic anhydride (0.49 mL, 5.30 mmol), DIPEA (0.93 mL, 5.30 mmol) and water (0.19 mL, 10.6 mmol). The resulting mixture was heated at 100 °C for 2 h. The mixture was diluted with EtOAc and filtered through decalite. The filtrate was washed with brine, the organic layer was dried with hydrophobic paper and evaporated to dryness. The crude was purified by reverse-phase flash chromatography (50 g aq) using a gradient of MeCN in water from 0% to 100% (neutral) to afford 5-fluoro-1,3-dimethyl-1H-indazole-6-carboxylic acid **(Intermediate 54:** 264 mg, 27%) as a beige solid. Mass spec: m/z: 209 $[M+H]^+$; $^1H$ NMR (400 MHz, DMSO-$d_6$): δ ppm 13.28 (s, 1H), 8.10 (d, J=5.7 Hz, 1H), 7.62 (d, J=11.0 Hz, 1H), 4.02 (s, 3H), 2.47 (s, 3H).

**Step 3**

**Intermediate 55: 5-fluoro-1,3-dimethyl-1H-indazole-6-carboxamide**

A solution of 5-fluoro-1,3-dimethyl-indazole-6-carboxylic acid **(Intermediate 54:** 244 mg, 1.17 mmol), DIPEA (1.0 mL, 5.86 mmol) and HATU (546 mg, 1.41 mmol) in THF (12 mL) was stirred 30 min at rt in a sealed vial. After this time, $NH_3$ gas was injected, and the mixture was stirred overnight at rt. Then, the mixture was diluted with EtOAc and washed with brine. The organic layer was dried with hydrophobic paper and evaporated to dryness. The crude material was purified by flash chromatography on silica gel (12 g) using a gradient of EtOAc/EtOH (3/1) in heptane from 0% to 60% to afford 5-fluoro-1,3-dimethyl-1H-indazole-6-carboxamide **(Intermediate 55:** 230 mg, 94%) as a white solid. Mass spec: m/z: 208 $[M+H]^+$.

**General procedure 3**

**Intermediates 61-65 were synthesized following General Procedure 3 in Scheme 12.**

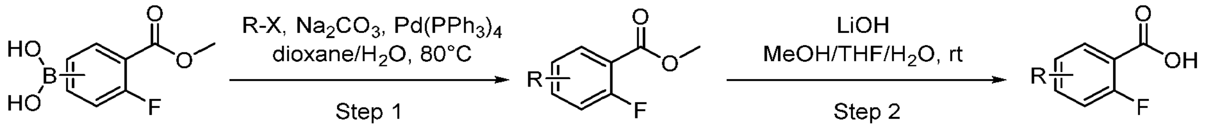


Intermediates 56-60

Intermediates 61-65

## Scheme 12

**Step 1**

To a solution of the appropriate boronic acid (1 eq), the appropriate aryl bromide or aryl chloride (R-X; 1 eq) and $Na_2CO_3$ (3 eq) in 1,4-dioxane/$H_2O$ 1/1 (0.6 M) was added $Pd(PPh_3)_4$ (0.1 eq). The reaction mixture was stirred at 80°C overnight. The reaction mixture was cooled to rt, diluted with EtOAc and filtered through a pad of celite. A saturated solution of $NaHCO_3$ was added to the filtrate. The aqueous layer was extracted with twice with EtOAc and the organic layer was washed with brine, dried over anhydrous $Na_2SO_4$, filtered, and concentrated under vacuum. The crude material was purified by flash chromatography on silica gel using a gradient of EtOAc/EtOH (3/1) in heptane from 0% to 100% to afford the corresponding Intermediates detailed below.

**Intermediate 56: Methyl 2-fluoro-4-(2-methyl-1,3-thiazol-5-yl)benzoate**

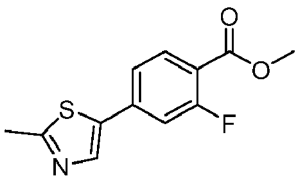

Following **Step 1** of **General Procedure 3** shown in **Scheme 12,** using 5-bromo-2-methyl-1,3-thiazole (CAS: 57268-16-3) as the aryl bromide (R-X) and [3-fluoro-4-(methoxycarbonyl)phenyl]boronic acid (CAS: 505083-04-5) as the boronic acid, afforded methyl 2-fluoro-4-(2-methyl-1,3-thiazol-5-yl)benzoate **(Intermediate 56:** 118 mg, 19%) was obtained as a beige solid. Mass spec: m/z: 252 $[M+H]^+$; $^1H$ NMR (400 MHz, DMSO-$d_6$) δ ppm 8.28 (s, 1H), 7.92 (t, J = 8.0 Hz, 1H), 7.71 (dd, J = 12.2, 1.8 Hz, 1H), 7.55 (dd, J = 8.2, 1.8 Hz, 1H), 3.86 (s, 3H), 2.71 (s, 3H).

**Intermediate 57: Methyl 2-fluoro-4-(pyrazin-2-yl)benzoate**

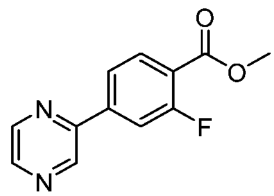

Following **Step 1** of **General Procedure 3** shown in **Scheme 12,** using 2-chloropyrazine (CAS: 14508-49-7) as the aryl chloride (R-X) and [3-fluoro-4-(methoxycarbonyl)phenyl]boronic acid as the boronic acid, afforded methyl 2-fluoro-4-(pyrazin-2-yl)benzoate **(Intermediate 57:** 91 mg, 40%) as a white solid. Mass spec: m/z: 233 $[M+H]^+$; $^1H$ NMR

(400 MHz, DMSO-$d_6$) δ ppm 9.40 (d, J = 1.6 Hz, 1H), 8.79 (dd, J = 2.5, 1.5 Hz, 1H), 8.72 (d, J = 2.5 Hz, 1H), 8.15 (d, J = 1.7 Hz, 1H), 8.12 (dd, J = 5.7, 1.5 Hz, 1H), 8.09 - 8.01 (m, 1H), 3.89 (s, 3H).

**Intermediate 58: Methyl 2-fluoro-4-(1,3-thiazol-5-yl)benzoate**

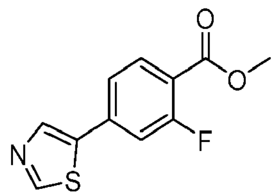

Following **Step 1** of **General Procedure 3** shown in **Scheme 12,** using 5-bromothiazole (CAS: 3034-55-7) as the aryl-bromide (R-X) and [3-fluoro-4-(methoxycarbonyl)phenyl]boronic acid as the boronic acid, afforded methyl 2-fluoro-4-(1,3-thiazol-5-yl)benzoate **(Intermediate 58:** 1.2 g, 36%) as a white solid. Mass spec: m/z: 238 $[M+H]^+$.

**Intermediate 59: Methyl 2-fluoro-5-(2-methyl-1,3-thiazol-5-yl)benzoate**

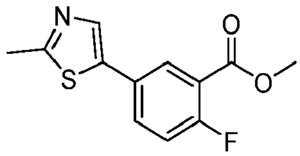

Following **Step 1** of **General Procedure** 3 shown in **Scheme 12,** using 5-bromo-2-methyl-1,3-thiazole (CAS: 57268-16-3) as the aryl-bromide (R-X) and [4-fluoro-3-(methoxycarbonyl)phenyl]boronic acid (CAS: 874219-35-9) as the boronic acid, afforded methyl 2-fluoro-5-(2-methyl-1,3-thiazol-5-yl)benzoate **(Intermediate 59:** 106 mg, 17%) was obtained as an off-white solid. Mass spec: m/z: 252 $[M+H]^+$; $^1H$ NMR (400 MHz, DMSO-$d_6$) δ ppm 8.09 (s, 1H), 8.00 (dd, J = 6.7, 2.5 Hz, 1H), 7.93 (ddd, J = 8.7, 4.5, 2.6 Hz, 1H), 7.44 (dd, J = 10.6, 8.7 Hz, 1H), 3.89 (s, 3H), 2.69 (s, 3H).

**Intermediate 60: Methyl 2-fluoro-5-(pyrazin-2-yl)benzoate**

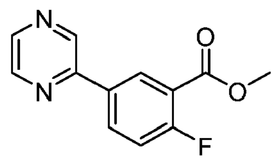

Following **Step 1** of **General Procedure 3** shown in **Scheme 12,** using 2-chloropyrazine as the aryl-chloride (R-X) and [4-fluoro-3-(methoxycarbonyl)phenyl]boronic acid, afforded methyl 2-fluoro-5-(pyrazin-2-yl)benzoate **(Intermediate 60:** 88 mg, 39%) as a white solid. Mass spec: m/z: 233 $[M+H]^+$; $^1H$ NMR (400 MHz, DMSO-$d_6$) δ ppm 9.33 (d, J = 1.6 Hz, 1H), 8.75 (dd, J = 2.5, 1.5 Hz, 1H), 8.70 - 8.63 (m, 2H), 8.45 (ddd, J = 8.7, 4.6, 2.5 Hz, 1H), 7.55 (dd, J = 10.7, 8.7 Hz, 1H), 3.91 (s, 3H).

**Step 2**

To a solution of the appropriate ester prepared in **Scheme 12, Step 1** (1 eq) in MeOH/THF/$H_2O$ 1/1/1 (0.15 M) was added lithium hydroxide hydrate (2 eq). The reaction mixture was stirred at rt for 18h. The reaction mixture was concentrated under reduced pressure and the residue was diluted with $H_2O$, acidified with citric acid to pH = 4. The resulting precipitate was filtered and dried under vacuum to afford the corresponding Intermediates as detailed below.

**Intermediate 61: 2-fluoro-4-(2-methyl-1,3-thiazol-5-yl)benzoic acid**

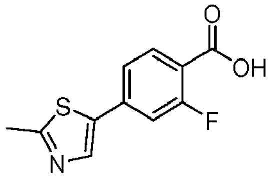

Following **Step 2** of **General Procedure 3** shown in **Scheme 12,** using methyl 2-fluoro-4-(2-methyl-1,3-thiazol-5-yl)benzoate **(Intermediate 56)** as the ester, afforded 2-fluoro-4-(2-methyl-1,3-thiazol-5-yl)benzoic acid **(Intermediate 61:** 114 mg, 93%) as a beige solid. Mass spec: m/z: 238 $[M+H]^+$; $^1H$ NMR (400 MHz, DMSO-$d_6$) δ ppm 13,17 (s, 1H), 8.26 (s, 1H), 7.90 (t, J = 8.0 Hz, 1H), 7.70 - 7.58 (m, 1H), 7.52 (dd, J = 8.2, 1.8 Hz, 1H), 2.71 (s, 3H).

**Intermediate 62: 2-fluoro-4-(pyrazin-2-yl)benzoic acid**

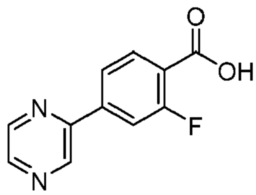

Following **Step 2** of **General Procedure 3** shown in **Scheme 12,** using methyl 2-fluoro-4-(pyrazin-2-yl)benzoate **(Intermediate 57)** as the ester, afforded 2-fluoro-4-(pyrazin-2-yl)benzoic acid **(Intermediate 62:** 86 mg, 88%) as a white solid. Mass spec: m/z: 219 $[M+H]^+$; $^1H$ NMR (400 MHz, DMSO-$d_6$) δ ppm 9.32 (d, J = 1.5 Hz, 1H), 8.75 (dd, J = 2.5, 1.5 Hz, 1H), 8.66 (d, J = 2.5 Hz, 1H), 7.95 (ddt, J = 17.1, 11.6, 1.9 Hz, 2H), 7.88 - 7.78 (m, 1H), 3.37 (s, 2H).

**Intermediate 63: 2-fluoro-4-(1,3-thiazol-5-yl)benzoic acid**

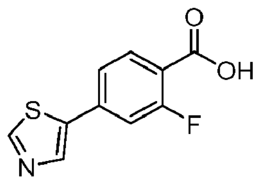

Following **Step 2** of **General Procedure 3** shown in **Scheme 12,** using methyl 2-fluoro-4-(1,3-thiazol-5-yl)benzoate **(Intermediate 58)** as the ester, afforded 2-fluoro-4-(1,3-thiazol-5-yl)benzoic acid **(Intermediate 63:** 353 mg, 83%) as an off-white solid. Mass spec: m/z: 224 $[M+H]^+$; $^1H$ NMR (400 MHz, DMSO-$d_6$) δ ppm 13.31 (s, 1H), 9.20 (d, J = 0.9 Hz, 1H), 8.53 (d, J = 0.8 Hz, 1H), 7.92 (t, J = 8.0 Hz, 1H), 7.75 (dd, J = 12.0, 1.8 Hz, 1H), 7.64 - 7.57 (m, 1H).

**Intermediate 64: 2-fluoro-5-(2-methyl-1,3-thiazol-5-yl)benzoic acid**

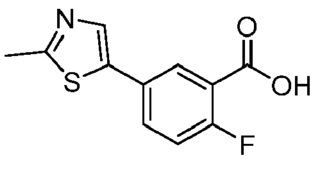

Following **Step 2** of **General Procedure 3** shown in **Scheme 12,** using methyl 2-fluoro-5-(2-methyl-1,3-thiazol-5-yl)benzoate **(Intermediate 59)** as the ester, afforded 2-fluoro-5-(2-methyl-1,3-thiazol-5-yl)benzoic acid **(Intermediate 64:** 83 mg, 83%) as an off-white solid. Mass spec: m/z: 238 $[M+H]^+$; $^1H$ NMR (400 MHz, DMSO-$d_6$) δ ppm 13.28 (s, 1H), 7.89 (s, 1H), 7.80 (dd, J = 6.8, 2.6 Hz, 1H), 7.70 (ddd, J = 8.7, 4.5, 2.6 Hz, 1H), 7.21 (dd, J = 10.6, 8.6 Hz, 1H), 2.50 (s, 3H).

**Intermediate 65: 2-fluoro-5-(pyrazin-2-yl)benzoic acid**

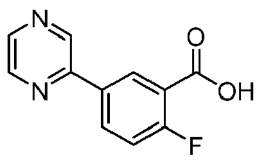

Following **Step 2** of **General Procedure 3** shown in **Scheme 12, ,** using methyl 2-fluoro-5-(pyrazin-2-yl)benzoate **(Intermediate 60)** as the ester, afforded 2-fluoro-5-(pyrazin-2-yl)benzoic acid **(Intermediate 65:** 66 mg, 75%) as a white solid. Mass spec: m/z: 219 $[M+H]^+$; $^1H$ NMR (400 MHz, DMSO-$d_6$) δ ppm 13.43 (s, 1H), 9.31 (d, J = 1.6 Hz, 1H), 8.74 (dd, J = 2.5, 1.5 Hz, 1H), 8.69 - 8.59 (m, 2H), 8.40 (ddd, J = 8.6, 4.6, 2.5 Hz, 1H), 7.50 (dd, J = 10.6, 8.7 Hz, 1H).

**General procedure 4**

**Intermediates 66 and 67 was synthesized following General Procedure 4 in Scheme 13.**

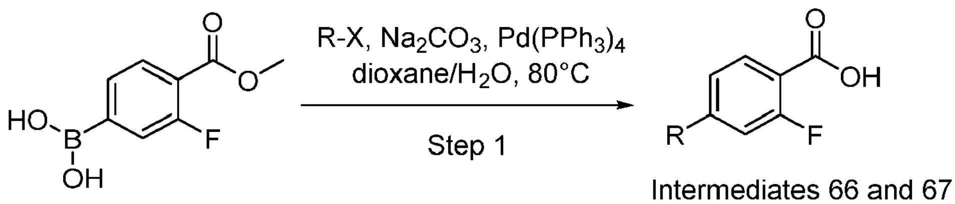


Intermediates 66 and 67

**Scheme 13**

**Step 1**

To a solution of [3-fluoro-4-(methoxycarbonyl)phenyl]boronic acid (CAS: 505083-04-5; 1 eq), the appropriate aryl-bromide or aryl-chloride (R-X; 1 eq) and $Na_2CO_3$ (3 eq) in 1,4-dioxane/$H_2O$ 1/1 (0.6 M) was added $Pd(PPh_3)_4$ (0.1 eq). The reaction mixture was stirred at 80°C overnight. The reaction mixture was cooled to rt, diluted in $CHCl_3$/iPrOH [3:1] and filtered through a pad of celite. The solution was then washed twice with brine. The aqueous layer was acidified with a 37% aqueous solution of HCl until a pH = 1-2 was achieved. The aqueous layer was extracted three times with $CHCl_3$/iPrOH (3/1). The combined layers were dried over anhydrous $MgSO_4$, filtered, and concentrated under vacuo to afford the desired products detailed below.

**Intermediate 66: 2-fluoro-4-(4-methyl-1,3-thiazol-5-yl)benzoic acid**

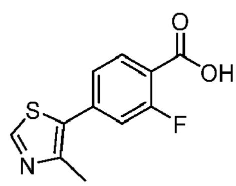

Following **Step 1** of **General Procedure 4** shown in **Scheme 13,** using 5-bromo-4-methyl-1,3-thiazole (CAS:

111600-83-0) as the aryl-bromide (R-X) and [3-fluoro-4-(methoxycarbonyl)phenyl]boronic acid as the boronic acid, afforded 2-fluoro-4-(4-methyl-1,3-thiazol-5-yl)benzoic acid **(Intermediate 66:** 190 mg, 64%) as an off-white solid. Mass spec: m/z: 238 $[M+H]^+$.

**Intermediate 67: 2-fluoro-4-(pyrimidin-2-yl)benzoic acid**

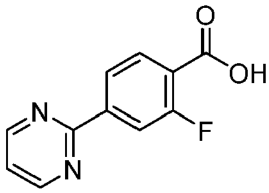

Following **Step 1** of **General Procedure 4** shown in **Scheme 13,** using 2-chloropyrimidine (CAS: 1722-12-9) as the aryl-chloride (R-X) and [3-fluoro-4-(methoxycarbonyl)phenyl]boronic acid as the boronic acid, afforded 2-fluoro-4-(pyrimidin-2-yl)benzoic acid **(Intermediate 67:** 104 mg, 30%) as a pale yellow solid. Mass spec: m/z: 219 $[M+H]^+$; $^1H$ NMR (400 MHz, DMSO-$d_6$) δ ppm 13.40 (s, 1H), 8.99 (d, J = 4.8 Hz, 2H), 8.31 (dd, J = 8.2, 1.6 Hz, 1H), 8.17 (dd, J = 12.2, 1.6 Hz, 1H), 8.04 (t, J = 7.9 Hz, 1H), 7.56 (t, J = 4.9 Hz, 1H).

**Intermediate 71 was synthesized following Scheme 14.**

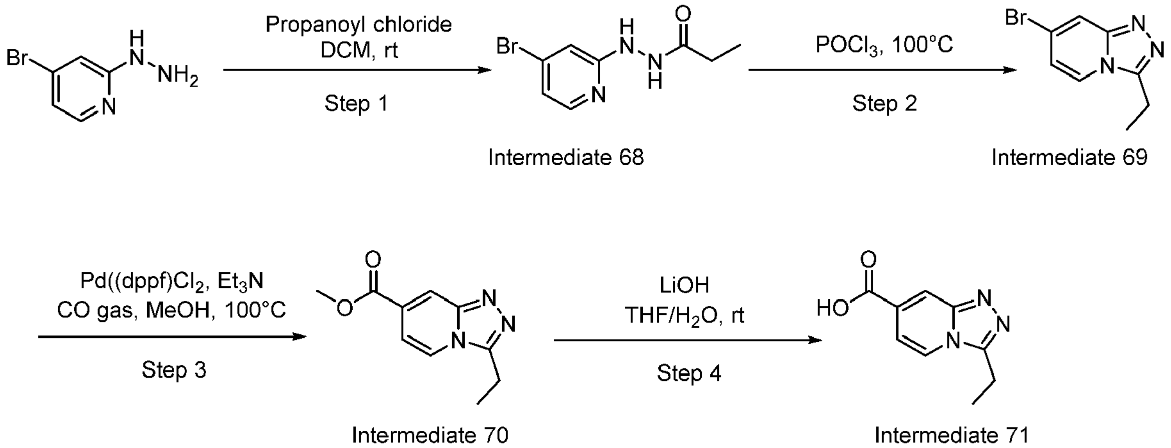


Scheme 14

**Step 1**

**Intermediate 68: N'-(4-bromopyridin-2-yl)propanehydrazide**

To a solution of 4-bromo-2-diazenylpyridine (CAS: 1019918-39-8, 1.00 g, 5.32 mmol) in DCM (15 mL) was added propanoyl chloride (0.750 g, 8.06 mmol). The reaction was stirred at rt for 2h. The reaction mixture was filtered, and the filter cake was washed with DCM and dried to provide N'-(4-bromopyridin-2-yl)propanehydrazide **(Intermediate 68:** 1.30 g, 97%) as a white solid. Mass spec: m/z: 244 $[M+H]^+$.

**Step 2**

**Intermediate 69: 7-bromo-3-ethyl-[1,2,4]triazolo[4,3-a]pyridine**

A solution of N'-(4-bromopyridin-2-yl)propanehydrazide **(Intermediate 68:** 1.30 g, 5.06 mmol) in $POCl_3$ (8 mL) was heated to 100°C for 2h. The reaction mixture was concentrated under reduced pressure. The crude material was purified by flash chromatography on silica gel using EtOAc/PE (2/3) as eluent to afford 7-bromo-3-ethyl-[1,2,4]triazolo[4,3-a]pyridine **(Intermediate 69:** 1.00 g, 22%) as a yellow solid which was used in step 3 without further purification. Mass spec: m/z: 226 $[M+H]^+$.

**Step 3**

**Intermediate 70: methyl 3-ethyl-[1,2,4]triazolo[4,3-a]pyridine-7-carboxylate**

To a solution of 7-bromo-3-ethyl-[1,2,4]triazolo[4,3-a]pyridine **(Intermediate 69:** 1.00 g, 1.11 mmol) in MeOH (20 mL) was added $Et_3N$ (134 mg, 1.33 mmol) and $Pd(dppf)Cl_2$ (32.4 mg, 0.0442 mmol,). The reaction was stirred at 100°C for 16h under CO (30 atm) in a sealed tube and concentrated under reduced pressure. The crude material was purified by flash chromatography on silica gel using MeOH/DCM (1115) as eluent to provide methyl 3-ethyl-[1,2,4]triazolo[4,3-a]pyridine-7-carboxylate **(Intermediate 70:** 80.0 mg, 30%) as a yellow solid. Mass spec: m/z: 206 $[M+H]^+$.

**Step 4**

**Intermediate 71: 3-ethyl-[1,2,4]triazolo[4,3-a]pyridine-7-carboxylic acid**

To a solution of methyl 3-ethyl-[1,2,4]triazolo[4,3-a]pyridine-7-carboxylate **(Intermediate 70:** 80.0 mg, 0.332 mmol) in THF (1 mL) and $H_2O$ (1 mL) was added LiOH (46.7 mg, 1.95 mmol). The reaction was stirred at rt for 2h. The resulting mixture was extracted with EtOAc twice. The pH value was adjusted to 3 with HCl 7N solution. Then the mixture was extracted with iPrOH/$CHCl_3$ [1:3] three times. The combined organic layers were washed with brine, dried over anhydrous $Na_2SO_4$ and concentrated under reduced pressure to provide 3-ethyl-[1,2,4]triazolo[4,3-a]pyridine-7-carboxylic acid **(Intermediate 71:** 65.0 mg, 87%) as a yellow solid which was used in the next step without further purification. Mass spec: m/z: 192 $[M+H]^+$.

**Intermediate 74 was synthesized following Scheme 15.**

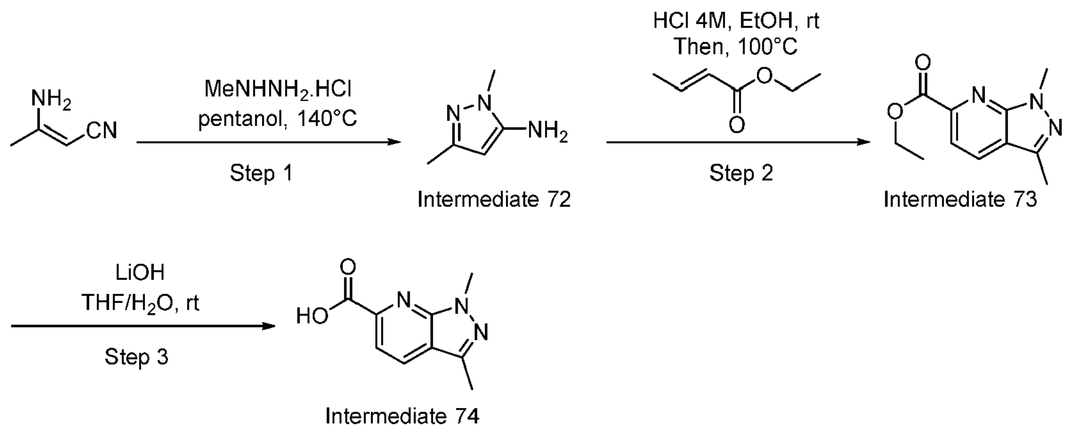


**Scheme 15**

**Step 1**

**Intermediate 72: 1,3-dimethyl-1H-pyrazol-5-amine**

To a solution of (2Z)-3-aminobut-2-enenitrile (CAS: 1118-61-2, 1 g, 12.1 mmol) in pentan-1-ol (7 mL) was added

methyl hydrazine dihydrochloride (2.03 g, 17.1 mmol). The resulting mixture was stirred for 3h at 140°C and cooled to rt. The precipitated solids were collected by filtration and washed with n-hexane (3 × 150 mL) and dried to provide 1,3-dimethyl-1H-pyrazol-5-amine **(Intermediate 72:** 910 mg, 18%) as a white solid which was used in Step 2 without further purification. Mass spec: m/z: 112 $[M+H]^+$.

**Step 2**

**Intermediate 73: ethyl 1,3-dimethyl-1H-pyrazolo[3,4-b]pyridine-6-carboxylate**

To a solution of 1,3-dimethyl-1H-pyrazol-5-amine **(Intermediate 72:** 892 mg, 8.02 mmol) in EtOH (5 mL) was added hydrogen chloride solution (5 mL, 4M in 1,4-dioxane). The mixture was stirred at rt for 15 min. Then ethyl (3E)-4-ethoxy-2-oxobut-3-enoate (1.66 g, 9.63 mmol) was added. The resulting mixture was stirred overnight at 100°C. The mixture was allowed to cool down to rt and quenched with $H_2O$ (30 mL). The resulting solution was extracted with DCM (3 × 50 mL) and the organic layers were combined, washed with brine (50 mL), dried over anhydrous $Na_2SO_4$, filtered, and concentrated under reduced pressure to provide ethyl 1,3-dimethyl-1H-pyrazolo[3,4-b]pyridine-6-carboxylate **(Intermediate 73:** 100 mg, 15%) as a brown oil which was used in step 3 without further purification. Mass spec: m/z: 220 $[M+H]^+$.

**Step 3**

**Intermediate 74: 1,3-dimethyl-1H-pyrazolo[3,4-b]pyridine-6-carboxylic acid**

To a solution of ethyl 1,3-dimethylpyrazolo[3,4-b]pyridine-6-carboxylate **(Intermediate 73:** 90 mg, 0.411 mmol,) in THF (2 mL)/$H_2O$ (2 mL) was added LiOH (49.2 mg, 2.05 mmol). The resulting mixture was stirred for 2h at rt. The mixture was acidified to pH = 6 with a 1M HCl solution and extracted three times with $CHCl_3$/iPrOH (3/1). The organic layers were combined, washed with brine, dried over anhydrous $Na_2SO_4$, filtered, and concentrated under reduced pressure to provide 1,3-dimethyl-1H-pyrazolo[3,4-b]pyridine-6-carboxylic acid **(Intermediate 74:** 40 mg, 40%) as a brown oil which was used without further purification. Mass spec: m/z: 192 $[M+H]^+$.

**Intermediate 79 was synthesized following Scheme 16.**

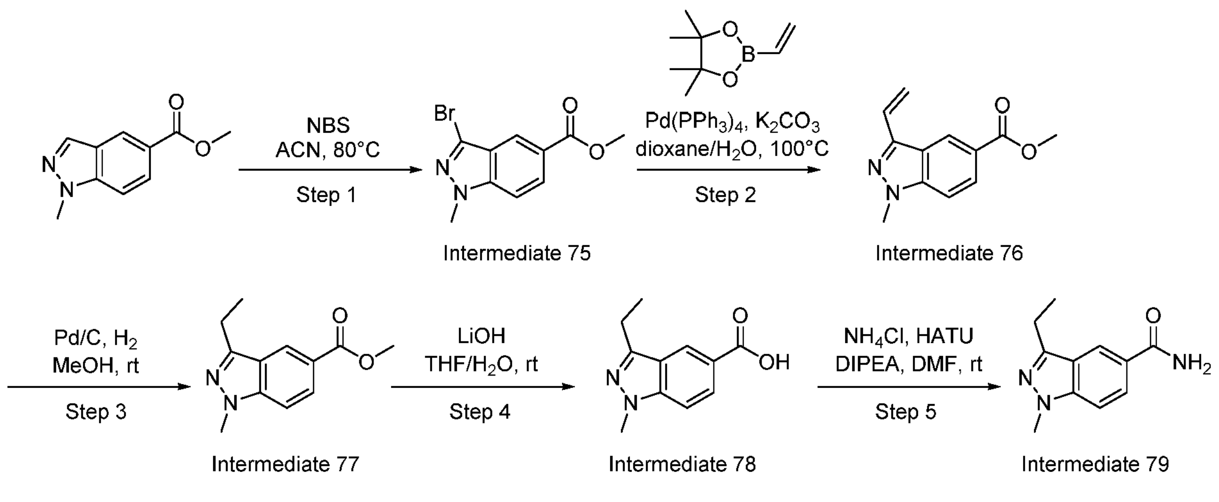


Intermediate 75 Intermediate 76 Intermediate 77 Intermediate 78 Intermediate 79

## Scheme 16

**Step 1**

**Intermediate 75: Methyl 3-bromo-1-methyl-1H-indazole-5-carboxylate**

To a solution of methyl 1-methyl-1H-indazole-5-carboxylate (CAS: 1092351-82-0, 4.0 g, 22.16 mmol) in MeCN

(150 mL) was added NBS (5.92 g, 33.24 mmol). The reaction mixture was stirred overnight at 80°C. Water was added and extracted with EtOAc three times. The combined organic layers were washed with brine, dried over anhydrous $Na_2SO_4$, filtered, and concentrated under reduced pressure. The crude material was purified by flash chromatography on silica gel using EtOAc/PE (3/7) as eluent to afford methyl 3-bromo-1-methyl-1H-indazole-5-carboxylate **(Intermediate 75:** 5.0 g, 76%) as a white solid. Mass spec: m/z: 269 $[M+H]^+$.

**Step 2**

**Intermediate 76: Methyl 1-methyl-3-vinyl-1H-indazole-5-carboxylate**

A round-bottom flask was charged with methyl 3-bromo-1-methyl-1H-indazole-5-carboxylate **(Intermediate 75:** 1.0 g, 4.15 mmol), 4,4,5,5-tetramethyl-2-vinyl-1,3,2-dioxaborolane (1.01 g, 6.22 mmol), $Pd(PPh_3)_4$.DCM adduct (479 mg, 0.42 mmol), $K_2CO_3$ (1.72 g, 12.44 mmol), 1,4-dioxane (15 mL) and water (3 mL).The reaction mixture was stirred for 3h at 100°C under a nitrogen atmosphere. The mixture was taken up in water and extracted three times with EtOAc. The combined organic layers were washed with brine, dried over anhydrous $Na_2SO_4$, filtered, and concentrated under reduced pressure. The crude material was purified by flash chromatography on silica gel using EtOAc/PE (5/95) as eluent to afford methyl 1-methyl-3-vinyl-1H-indazole-5-carboxylate **(Intermediate 76:** 511 mg, 53%) as a yellow solid. Mass spec: m/z: 217 $[M+H]^+$.

**Step 3**

**Intermediate 77: Methyl 3-ethyl-1-methylindazole-5-carboxylate**

A round-bottom flask was charged with methyl 3-ethenyl-1-methylindazole-5-carboxylate **(Intermediate 76:** 511 mg, 2.55 mmol), Methanol (20 mL) and Pd/C (250 mg, 10% on carbon). The reaction mixture was stirred under an atmosphere of hydrogen (3 atm) for 3h at rt. The solids were filtered, and the organic layer was concentrated under reduced pressure to provide methyl 3-ethyl-1-methylindazole-5-carboxylate **(Intermediate 77:** 421 mg, 74%) as a white solid. Mass spec: m/z: 219 $[M+H]^+$.

**Step 4**

**Intermediate 78: 3-ethyl-1-methylindazole-5-carboxylic acid**

To a solution of methyl 3-ethyl-1-methylindazole-5-carboxylate **(Intermediate 77:** 421 mg, 1.93 mmol) in THF (20 mL) and water (5 mL) was added lithium hydroxide (231 mg, 9.65 mmol). The reaction mixture was stirred overnight at 60°C. The pH value of the reaction mixture was adjusted to 5 with a solution of HCl (6 N in water). The resulting solution was extracted three times with DCM. The combined organic layers were washed with brine, dried over anhydrous $Na_2SO_4$, filtered, and concentrated under reduced pressure to afford 3-ethyl-1-methylindazole-5-carboxylic acid **(Intermediate 78:** 390 mg, 79%) as a white solid which was used in Step 5 without further purification. Mass spec: m/z: 205 $[M+H]^+$.

**Step 5**

**Intermediate 79: 3-ethyl-1-methyl-1H-indazole-5-carboxamide**

A solution of 3-ethyl-1-methylindazole-5-carboxylic acid **(Intermediate 78:** 200 mg, 0.833 mmol), $NH_4Cl$ (225 mg, 4.17 mmol), HATU (475 mg, 1.25 mmol), DIPEA (322 mg, 2.50 mmol) in DMF (5 mL) was stirred overnight at rt. The mixture was concentrated in vacuo. The crude material was purified by reverse-phase flash chromatography (C18) using a MeCN/water (0.5% TFA) (87/17) as eluent to afford 3-ethyl-1-methyl-1H-indazole-5-carboxamide **(Intermediate 79:** 140 mg, 67%) as a white solid. Mass spec: m/z: 204 $[M+H]^+$.

**Intermediate 82 was synthesized following Scheme 17.**

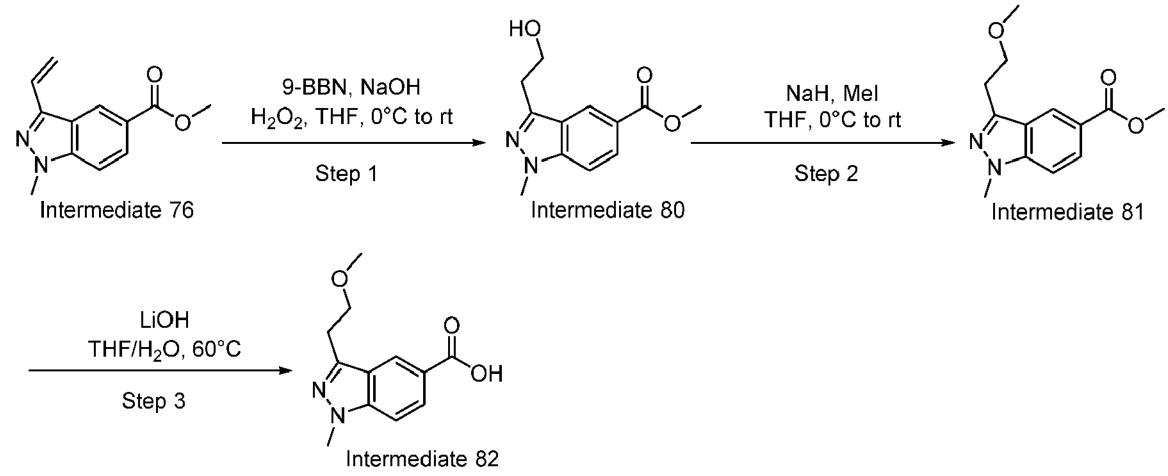


## Scheme 17

**Step 1**

**Intermediate 80: Methyl 3-(2-hydroxyethyl)-1-methylindazole-5-carboxylate**

To a solution of methyl 1-methyl-3-vinyl-1H-indazole-5-carboxylate **(Intermediate 76:** 1.00 g, 4.62 mmol) in THF (20 mL) was added 9-borabicyclo[3.3.1]nonane 9-BBN (37.0 mL, 18.5 mmol) at 0°C. The mixture was stirred at rt for 18h. A NaOH solution (3M in water, 12.3 mL, 37.0 mmol) and $H_2O_2$ (4.19 g, 37.0 mmol, 30% in water) were added. The reaction mixture was stirred overnight and quenched with a saturated aqueous solution of sodium thiosulfate solution (20 mL). The aqueous layer was extracted with EtOAc three times, and the organic layers were combined, washed with brine, dried over anhydrous $Na_2SO_4$, filtered, and concentrated under reduced pressure to afford methyl 3-(2-hydroxyethyl)-1-methylindazole-5-carboxylate **(Intermediate 80:** 1.1 g, 90%) as a yellow oil which was used in Step 2 without further purification. Mass spec: m/z 235 $[M+H]^+$.

**Step 2**

**Intermediate 81: Methyl 3-(2-methoxyethyl)-1-methylindazole-5-carboxylate**

To a solution of methyl 3-(2-hydroxyethyl)-1-methylindazole-5-carboxylate **(Intermediate 80:** 600 mg, 2.56 mmol) in THF (10 mL) was added NaH (79.9 mg, 3.33 mmol, 30% in mineral oil) at 0°C. The mixture was stirred for 30 min, then $CH_3I$ (545 mg, 3.84 mmo) was added. The mixture was stirred at rt for 18h, then quenched with water (10 mL). The resulting solution was extracted with EtOAc three times and the organic layers were combined, washed with brine, dried over anhydrous $Na_2SO_4$, filtered and concentrated under reduced pressure. The crude material was purified by flash chromatography on silica gel using EtOAc/PE (2/8) as eluent to afford methyl 3-(2-methoxyethyl)-1-methylindazole-5-carboxylate **(Intermediate 81:** 80 mg, 16%) as a yellow oil. Mass spec: m/z 249 $[M+H]^+$.

**Step 3**

**Intermediate 82: 3-(2-methoxyethyl)-1-methylindazole-5-carboxylic acid**

To a solution of methyl 3-(2-methoxyethyl)-1-methylindazole-5-carboxylate **(Intermediate 81:** 80.0 mg, 0.32 mmol) in THF (2 mL) was added LiOH (38.6 mg, 1.61 mmol) in water (1 mL). The mixture was stirred at 60°C for 3h. The pH value of the mixture was adjusted to 6 with an aqueous solution of 1M HCl. The resulting solution was extracted with $CHCl_3$/iPrOH 3/1 three times, and the organic layers were combined, washed with brine, dried over anhydrous $Na_2SO_4$, filtered, and concentrated under reduced pressure to afford 3-(2-methoxyethyl)-1-methylindazole-5-carboxylic acid **(Intermediate 82:** 50 mg, 63%) as a white solid which was used without further purification. Mass spec: m/z 235 $[M+H]^+$.

**Intermediate 86 was synthesized following Scheme 18.**

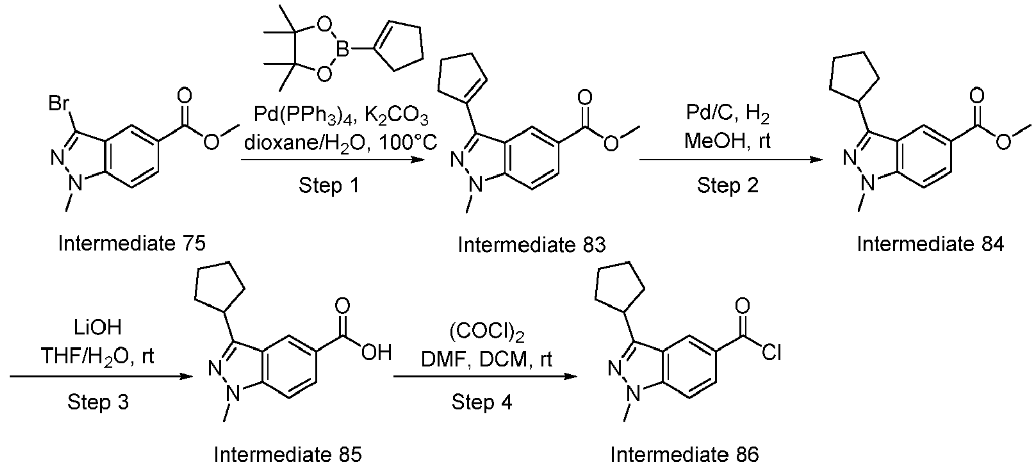


Intermediate 75, Intermediate 83, Intermediate 84, Intermediate 85, Intermediate 86

**Scheme 18**

**Step 1**

**Intermediate 83: Methyl 3-(cyclopent-1-en-1-yl)-1-methylindazole-5-carboxylate**

A solution of methyl 3-bromo-1-methyl-1H-indazole-5-carboxylate **(Intermediate 75:** 400 mg, 1.418 mmol), 2-(cyclopent-1-en-1-yl)-4,4,5,5-tetramethyl-1,3,2-dioxaborolane (319 mg, 1.56 mmol), $Pd(PPh_3)_4$ (266 mg, 0.230 mmol) and $K_2CO_3$ (392 mg, 2.84 mmol) in dioxane (15 mL) and water (5 mL) was stirred for 3h at 100°C under nitrogen atmosphere. The reaction was taken in water and extracted with EtOAc three times. The combined organic layers were washed with brine, dried over anhydrous $Na_2SO_4$, filtered, and concentrated under reduced pressure. The crude material was purified by flash chromatography on silica gel using EtOAc/PE (3/2) as eluent to afford methyl 3-(cyclopent-1-en-1-yl)-1-methylindazole-5-carboxylate **(Intermediate 83:** 340 mg, 80%) as a yellow solid. Mass spec: m/z: 257 $[M+H]^+$.

**Step 2**

**Intermediate 84: Methyl 3-cyclopentyl-1-methylindazole-5-carboxylate**

A round-bottom flask was charged with methyl 3-(cyclopent-1-en-1-yl)-1-methylindazole-5-carboxylate **(Intermediate 83:** 330 mg, 1.160 mmol), MeOH (15 mL) and Pd/C (300 mg, 10% on carbon). The reaction was placed under an atmosphere of hydrogen (3 atm). The reaction mixture was stirred under an atmosphere of hydrogen (3 atm) for 3h at rt. The solids were filtered, and the organic layer was concentrated under reduced pressure to provide the title compound methyl 3-cyclopentyl-1-methylindazole-5-carboxylate **(Intermediate 84:** 310 mg, 79%) as a yellow oil which was used in Step 3 without further purification. Mass spec: m/z: 259 $[M+H]^+$.

**Step 3**

**Intermediate 85: 3-cyclopentyl-1-methylindazole-5-carboxylic acid**

To a solution of methyl 3-cyclopentyl-1-methylindazole-5-carboxylate **(Intermediate 84:** 310 mg, 1.02 mmol) in THF (20 mL) and water (5 mL) was added LiOH (98.2 mg, 4.09 mmol). The reaction was stirred for 5h at rt. The pH value of the reaction mixture was adjusted to 5 with a solution of HCl (6mol/L in water). The resulting solution was extracted three times with DCM. The combined organic layers were washed with brine, dried over anhydrous $Na_2SO_4$, filtered, and concentrated under reduced pressure to provide the title compound 3-cyclopentyl-1-methylindazole-5-carboxylic acid **(Intermediate 85:** 300 mg, 82%) as a white solid which was used in Step 4 without further purification. Mass spec: m/z: 245 $[M+H]^+$.

**Step 4**

**Intermediate 86: 3-cyclopentyl-1-methyl-1H-indazole-5-carbonyl chloride**

To a solution of 3-cyclopentyl-1-methylindazole-5-carboxylic acid **(Intermediate 85:** 120 mg, 0.393 mmol) in DCM (8 mL) were added DMF (0.05 mL) and oxalyl chloride (100 mg, 0.787 mmol). The reaction mixture was stirred for 2h at rt. The mixture was concentrated under reduced pressure to afford 3-cyclopentyl-1-methyl-1H-indazole-5-carbonyl chloride **(Intermediate 86:** 60 mg, 37%) as a yellow oil which was used without further purification. Mass spec: m/z: 263 $[M+H]^+$.

**Intermediate 91 was synthesized following Scheme 19**

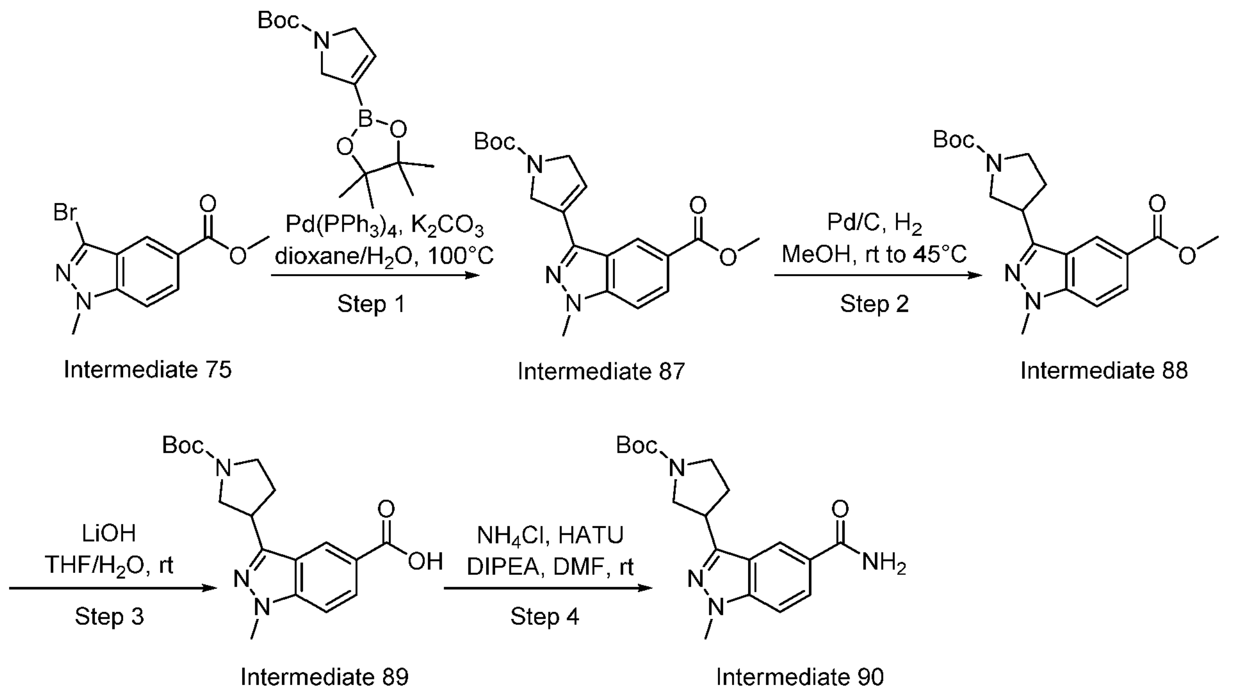


**Scheme 19**

**Step 1**

**Intermediate 87: Methyl 3-[1-(tert-butoxycarbonyl)-2,5-dihydropyrrol-3-yl]-1-methylindazole-5-carboxylate**

A solution of methyl 3-bromo-1-methylindazole-5-carboxylate **(Intermediate 75:** 400 mg, 1.42 mmol), tert-butyl 3-(4,4,5,5-tetramethyl-1,3,2-dioxaborolan-2-yl)-2,5-dihydropyrrole-1-carboxylate (484 mg, 1.56 mmol), $Pd(PPh_3)_4$ (329 mg, 0.284 mmol) and $K_2CO_3$ (392 mg, 2.84 mmol) in 1,4-dioxane (15 mL) and water (5 mL) under nitrogen atmosphere. The reaction mixture was stirred for 3h at 100°C. The mixture was diluted with water and extracted with EtOAc three times. The combined organic layers were washed with brine, dried over anhydrous $Na_2SO_4$, filtered, and concentrated under reduced pressure. The crude material was purified by flash chromatography on silica gel using EtOAc/PE (1/4) as eluent to afford methyl 3-[1-(tert-butoxycarbonyl)-2,5-dihydropyrrol-3-yl]-1-methylindazole-5-carboxylate **(Intermediate 87:** 410 mg, 70%) as a yellow solid. Mass spec: m/z: 358 $[M+H]^+$.

**Step 2**

**Intermediate 88: rac-methyl 3-[1-(tert-butoxycarbonyl)pyrrolidin-3-yl]-1-methylindazole-5-carboxylate**

To a solution of methyl 3-[1-(tert-butoxycarbonyl)-2,5-dihydropyrrol-3-yl]-1-methylindazole-5-carboxylate **(Intermediate 87:** 410 mg, 1.05 mmol) in MeOH (30 mL) was added Pd/C (200 mg, 10% on carbon). The mixture was placed under an atmosphere of hydrogen (3 atm) and stirred for 5h at 45°C. The solids were filtered and the filtrate was concentrated under reduced pressure to afford rac-methyl 3-[1-(tert-butoxycarbonyl)pyrrolidin-3-yl]-1-methylindazole-5-carboxylate **(Intermediate 88:** 350 mg, 72%) as a colorless oil which was used in Step 3 without further purification. Mass spec: m/z: 360 $[M+H]^+$.

**Step 3**

**Intermediate 89: rac-3-[1-(tert-butoxycarbonyl)pyrrolidin-3-yl]-1-methylindazole-5-carboxylic acid**

To a solution of methyl 3-[1-(tert-butoxycarbonyl)pyrrolidin-3-yl]-1-methylindazole-5-carboxylate **(Intermediate 88:** 351 mg, 0.831 mmol) in THF (10 mL) and water (3 mL) was added LiOH (99.7 mg, 4.16 mmol). The reaction mixture was stirred overnight at 60°C. The pH of the mixture was then adjusted to 5 by the addition of aqueous solution of HCl (6 N). The aqueous layer was extracted with DCM three times. The combined organic layers were washed with brine, dried over anhydrous $Na_2SO_4$, filtered, and concentrated under reduced pressure to afford rac-3-[1-(tert-butoxycarbonyl)pyrrolidin-3-yl]-1-methylindazole-5-carboxylic acid **(Intermediate 89:** 320 mg, 85%) as a white solid which was used in the next step without further purification. Mass spec: m/z: 346 $[M+H]^+$.

**Step 4**

**Intermediate 90: rac-tert-butyl 3-(5-carbamoyl-1-methylindazol-3-yl)pyrrolidine-1-carboxylate**

To a solution of 3-[1-(tert-butoxycarbonyl)pyrrolidin-3-yl]-1-methylindazole-5-carboxylic acid **(Intermediate 89:** 290 mg, 0.757 mmol) in DMF (5 mL) were added HATU (431 mg, 1.14 mmol), $NH_4Cl$ (162 mg, 3.03 mmol) and DIPEA (293 mg, 2.27 mmol) . The reaction mixture was stirred overnight at rt. The mixture was concentrated under reduced pressure. The crude material was purified by reverse phase flash chromatography (C18 Aq) using MeCN/water (33/67) as eluent to rac-tert-butyl 3-(5-carbamoyl-1-methylindazol-3-yl)pyrrolidine-1-carboxylate **(Intermediate 90:** 150 mg, 49%) as a yellow oil. Mass spec: m/z: 345 $[M+H]^+$.

**Intermediate 92 was synthesized following Scheme 20.**

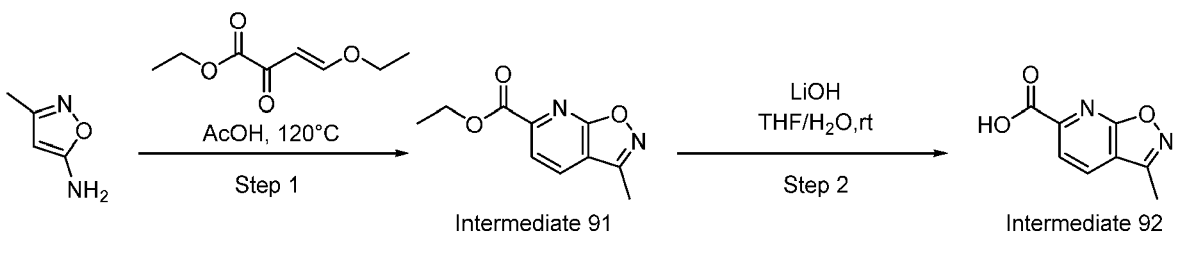


**Scheme 20**

**Step 1**

**Intermediate 91: ethyl 3-methyl-[1,2]oxazolo[5,4-b]pyridine-6-carboxylate**

A solution of 3-methyl-1,2-oxazol-5-amine (CAS: 14678-02-5, 400 mg, 4.08 mmol) and ethyl (3E)-4-ethoxy-2-oxobut-3-enoate (CAS: 65260-58-4, 702 mg, 4.08 mmol) in acetic acid was stirred at 120C for 18h, then quenched with water (50 ml). The mixture was extracted with EtOAc three times. The organic layers were combined, dried over anhydrous

$Na_2SO_4$, filtered, and concentrated under reduced pressure. The crude material was purified by flash chromatography on silica gel using EtOAc/PE (13/87) as eluent to afford ethyl 3-methyl-[1,2]oxazolo[5,4-b]pyridine-6-carboxylate **(Intermediate 91:** 324 mg, 31%) as a yellow solid. Mass spec: m/z: 207 $[M+H]^+$.

**Step 2**

**Intermediate 92: 3-methyl-[1,2]oxazolo[5,4-b]pyridine-6-carboxylic acid**

To a solution of ethyl 3-methyl-[1,2]oxazolo[5,4-b]pyridine-6-carboxylate **(Intermediate 91:** 300 mg, 1.46 mmol) in THF/$H_2O$ (4mL/4mL) was added LiOH (174 mg, 7.27 mmol). The mixture was stirred at rt for 1h. The resulting solution was acidified to pH = 6 with an aqueous 1M HCl solution. The mixture was extracted with EtOAc three times, the organic layers were combined, dried over anhydrous $Na_2SO_4$, filtered, and concentrated under reduced pressure to afford 3-methyl-[1,2]oxazolo[5,4-b]pyridine-6-carboxylic acid **(Intermediate 92:** 45 mg, 18%) as a brown oil. Mass spec: m/z: 179 $[M+H]^+$.

**Intermediate 95 was synthesized following Scheme 21**

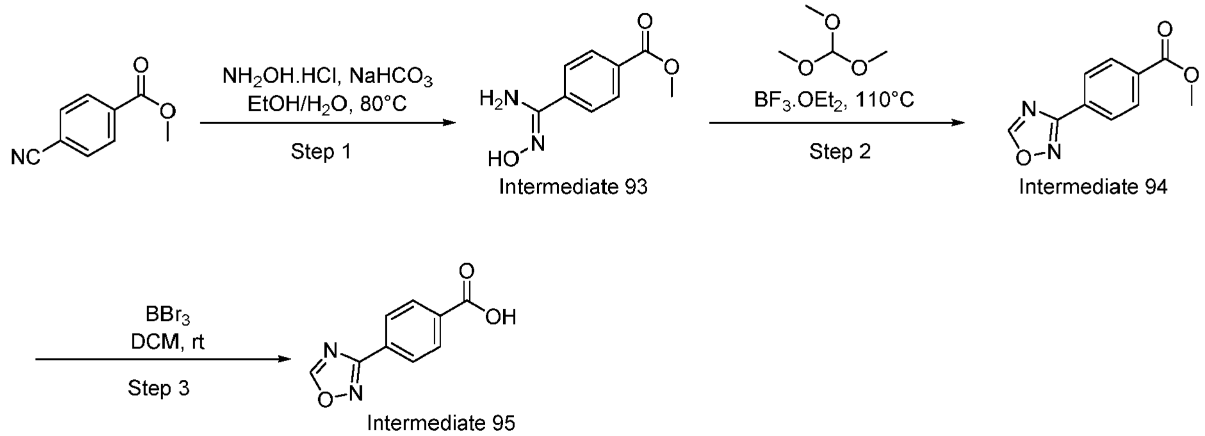


## Scheme 21

**Step 1**

**Intermediate 93: Methyl 4-[(Z)-N'-hydroxycarbamimidoyl]benzoate**

To a solution of methyl 4-cyanobenzoate (CAS: 1129-35-7, 1.00 g, 6.21 mmol) in Ethanol (10 mL) and $H_2O$ (1 mL) were added hydroxylamine hydrochloride (0.47 g, 6.83 mmol) and $NaHCO_3$ (0.57 g, 6.83 mmol). The mixture was stirred at 80°C for 3h and then quenched with water (10 mL). The resulting solution was extracted with EtOAc three times, and the organic layers were combined, washed with brine, dried over anhydrous $Na_2SO_4$, filtered, and concentrated under reduced pressure to afford methyl 4-[(Z)-N'-hydroxycarbamimidoyl]benzoate **(Intermediate 93:** 1 g, 74%) as a white solid. Mass spec: m/z 195 $[M+H]^+$.

**Step 2**

**Intermediate 94: Methyl 4-(1,2,4-oxadiazol-3-yl)benzoate**

To a solution of methyl 4-[(Z)-N'-hydroxycarbamimidoyl]benzoate **(Intermediate 93:** 1.05 g, 5.41 mmol) in trimethyl orthoformate (10 mL) was added $BF_3.Et_2O$ (40.0 mg, 0.280 mmol). The reaction mixture was heated under microwave conditions for 40 min at 110°C and then quenched with water (10 mL). The aqueous layer was extracted with EtOAc three times, and the organic layers were combined, washed with brine, dried over anhydrous $Na_2SO_4$, filtered, and concentrated under reduced pressure. The crude material was purified by flash chromatography on silica

gel using EtOAc/PE (1/9) as eluent to afford methyl 4-(1,2,4-oxadiazol-3-yl)benzoate **(Intermediate 94:** 750 mg 76%) as a yellow oil. Mass spec: m/z 205 $[M+H]^+$.

**Step 3**

**Intermediate 95: 4-(1,2,4-oxadiazol-3-yl)benzoic acid**

To a solution of methyl 4-(1,2,4-oxadiazol-3-yl)benzoate **(Intermediate 94:** 300 mg, 1.47 mmol) in DCM (10 mL) was added $BBr_3$ (29.4 mL, 29.4 mmol, 1 M in DCM). The mixture was stirred at rt for 3 days and then quenched with a saturated $NaHCO_3$ aqueous solution. The aqueous layer was extracted with EtOAc three times and the organic layers were combined, washed with brine, dried over anhydrous $Na_2SO_4$, filtered, and concentrated under reduced pressure to afford 4-(1,2,4-oxadiazol-3-yl)benzoic acid **(Intermediate 95:** 160 mg 59%) as a light yellow oil. Mass spec: m/z 191 $[M+H]^+$.

**Intermediate 97 was synthesized following Scheme 22.**

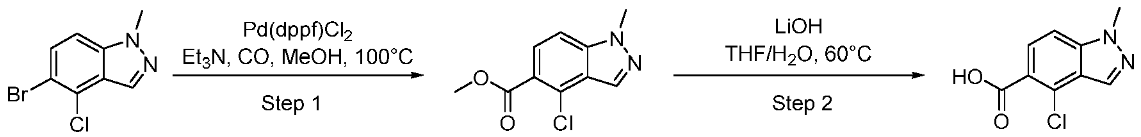


Intermediate 96

Intermediate 97

**Scheme 22**

**Step 1**

**Intermediate 96: Methyl 4-chloro-1-methylindazole-5-carboxylate**

To a solution of 5-bromo-4-chloro-1-methylindazole (CAS: 1785427-70-4, 300 mg, 1.22 mmol) in MeOH (6 mL) were added $Pd(dppf)Cl_2$ (358 mg, 0.49 mmol) and $Et_3N$ (989 mg, 9.78 mmol). Carbon monoxide (30 atm) was introduced into the autoclave. The solution was then stirred at 100°C for 3 days and then quenched with water (10 mL). Both layers were separated, and the aqueous layer was extracted with EtOAc twice. The organic layers were combined, washed with brine, dried over anhydrous $Na_2SO_4$, filtered, and concentrated under reduced pressure. The crude material was purified by flash chromatography on silica gel using EtOAc/PE (18/82) as eluent to afford methyl 4-chloro-1-methylindazole-5-carboxylate **(Intermediate 96:** 80 mg 13%) as a light yellow oil. Mass spec: m/z 225 $[M+H]^+$.

**Step 2**

**Intermediate 97: 4-chloro-1-methylindazole-5-carboxylic acid**

To a solution of methyl 4-chloro-1-methylindazole-5-carboxylate **(Intermediate 96:** 38.0 mg, 0.170 mmol) in THF (2 mL) was added LiOH (20.3 mg, 0.850 mmol) in $H_2O$ (1 mL). The mixture was stirred at 60°C for 3h. The pH was adjusted to 6 with an aqueous solution of HCl (6M). The resulting layers were separated and the aqueous layer was extracted twice with $CHCl_3$/iPrOH (3/1). The organic layers were combined, washed with brine, dried over anhydrous $Na_2SO_4$, filtered and concentrated under reduced pressure to 4-chloro-1-methylindazole-5-carboxylic acid **(Intermediate 97:** 30 mg 84%) as a light yellow oil. Mass spec: m/z 211 $[M+H]^+$.

**General Procedures 5A, 5B and 5C**

**Intermediates 98-107 were prepared using the appropriate General Procedure 5 in Scheme 23.**

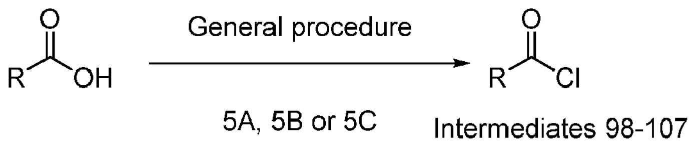


**Scheme 23**

**General procedure 5A**

To a solution of the appropriate carboxylic acid (1 eq) in DCM (0.25 M) were added $SOCl_2$ (2 eq) and DMF (0.5 eq). The reaction mixture was stirred at rt until completion was observed by TLC or LCMS (addition of MeOH into the aliquot to observe the corresponding methyl ester). The mixture was concentrated under vacuo to provide the corresponding acid chloride.

**General procedure 5B**

A solution of the appropriate carboxylic acid (1 eq) in $SOCl_2$ (0.4 M) was stirred at 80°C until completion was observed by TLC or LCMS (addition of MeOH into the aliquot to observe the corresponding methyl ester). The mixture was concentrated under vacuo to provide the corresponding acid chloride.

**General procedure 5C**

To a solution of the carboxylic acid (1 eq) in MeCN (0.15 M) was added $SOCl_2$ (2 eq). The reaction mixture was stirred at 80°C until completion was observed by TLC or LCMS (addition of MeOH in the aliquot to observe the corresponding methyl ester). The mixture was concentrated under vacuo to provide the corresponding acid chloride.

**Intermediate 98: 6-methoxy-1H-indole-3-carbonyl chloride**

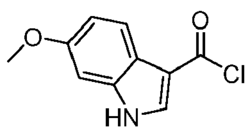

Following General Procedure **5A,** using 6-methoxy-1H-indole-3-carboxylic acid (CAS: 90924-43-9) as the carboxylic acid, afforded 6-methoxy-1H-indole-3-carbonyl chloride **(Intermediate 98:** 120 mg, 99%). Mass spec: m/z: 206 $[M-Cl+MeOH]^+$.

**Intermediate 99: 4-(pyrazol-1-yl)benzoyl chloride**

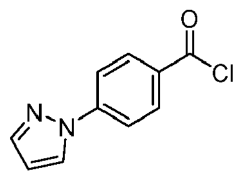

Following **General Procedure 5A,** using 4-(pyrazol-1-yl)benzoic acid (CAS: 16209-00-0) as the carboxylic acid, afforded 4-(pyrazol-1-yl)benzoyl chloride **(Intermediate 99:** 100 mg, 97%) as a brown oil. Mass spec: m/z: 203 $[M-Cl+MeOH]^+$.

**Intermediate 100: 1,3-benzothiazole-5-carbonyl chloride**

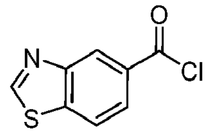

Following **General Procedure 5B,** using 1,3-benzothiazole-5-carboxylic acid (CAS: 68867-17-4) as the carboxylic acid, afforded 1,3-benzothiazole-5-carbonyl chloride **(Intermediate 100:** (160 mg, 92%) as a yellow solid. Mass spec: m/z: 198 $[M+H]^+$.

**Intermediate 101: 4-(pyrazol-1-ylmethyl)benzoyl chloride**

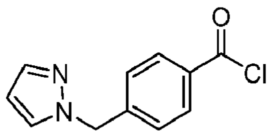

Following **General Procedure 5A,** using 4-(pyrazol-1-ylmethyl)benzoic acid (CAS: 160388-53-4) as the carboxylic acid, afforded 4-(pyrazol-1-ylmethyl)benzoyl chloride **(Intermediate 101:** 100 mg, 97%) as a brown solid. Mass spec: m/z: 217 $[M-Cl+MeOH]^+$.

**Intermediate 102: 3-(pyrazol-1-yl)benzoyl chloride**

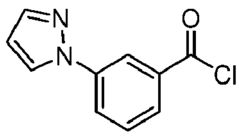

Following **General Procedure 5A,** using 3-(pyrazol-1-yl)benzoic acid (CAS: 264264-33-7) as the carboxylic acid, afforded 3-(pyrazol-1-yl)benzoyl chloride **(Intermediate 102:** 100 mg, 97%) as a brown oil. Mass spec: m/z: 203 $[M-Cl+MeOH]^+$.

**Intermediate 103: 5-methyl-4-oxo-1H-thieno[2,3-d]pyrimidine-6-carbonyl chloride**

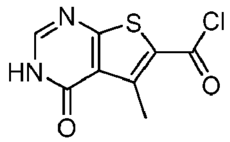

Following **General Procedure 5B,** using 5-methyl-4-oxo-1H-thieno[2,3-d]pyrimidine-6-carboxylic acid (CAS: 101667-97-4) as the carboxylic acid, afforded 5-methyl-4-oxo-1H-thieno[2,3-d]pyrimidine-6-carbonyl chloride **(Intermediate 103:** 60 mg, 62%) as a light yellow solid. Mass spec: m/z: 229 $[M+H]^+$.

**Intermediate 104: 1-cyclohexyl-1,2,3-benzotriazole-5-carbonyl chloride**

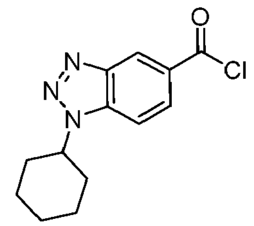

Following **General Procedure 5A,** using 1-cyclohexyl-1,2,3-benzotriazole-5-carboxylic acid (CAS: 691363-11-8) as carboxylic acid, afforded 1-cyclohexyl-1,2,3-benzotriazole-5-carbonyl chloride **(Intermediate 104:** 70 mg, 98%) as a brown solid. Mass spec: m/z: 260 $[M-Cl+MeOH]^+$.

**Intermediate 105: 2-phenyl-1,3-oxazole-5-carbonyl chloride**

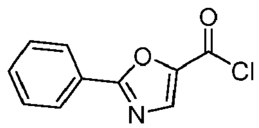

Following **General Procedure 5C,** using 2-phenyl-1,3-oxazole-5-carboxylic acid (CAS: 106833-79-8) as the carboxylic acid, afforded 2-phenyl-1,3-oxazole-5-carbonyl chloride **(Intermediate 105:** 130 mg, 63%) as a yellow oil. Mass spec: m/z: 208 $[M+H]^+$.

**Intermediate 106: Quinazoline-7-carbonyl chloride**

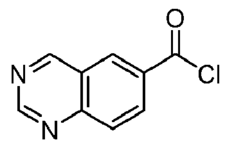

Following **General Procedure 5A,** using quinazoline-7-carboxylic acid (CAS: 1234616-41-1) as the carboxylic acid, afforded quinazoline-7-carbonyl chloride **(Intermediate 106:** 60 mg, 98%) as a brown solid. Mass spec: m/z: 189 $[M-Cl+MeOH]^+$.

**Intermediate 107: 1-methylindazole-5-carbonyl chloride**

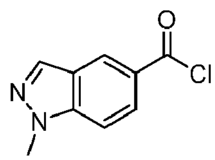

Following **General Procedure 5A,** using 1-methyl-1H-indazole-5-carboxylic acid (CAS: 1176754-31-6) as the carboxylic acid, afforded 1-methylindazole-5-carbonyl chloride **(Intermediate 107:** 5.5 g, quant.). Mass spec: m/z: 191 $[M-Cl+MeOH]^+$.

Examples 1 to 99 describe compounds which are MLLT1 and/or MLLT3 inhibitors and which may be joined to LINK-U to form a PROTAC of the present invention.

**Example** 1 was **synthesized following Scheme** 24.

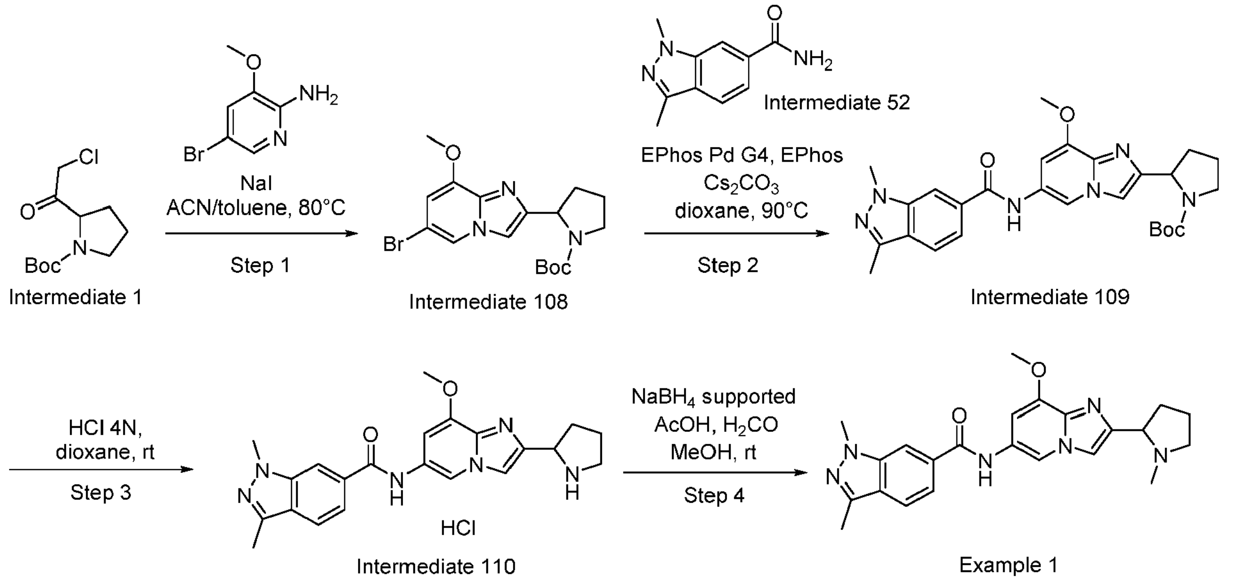


## Scheme 24

**Step 1**

**Intermediate 108: rac-tert-butyl 2-(6-bromo-8-methoxy-imidazo[1,2-pyridine-2-yl)pyrrolidine-1-carboxylate**

To a stirred solution of 5-bromo-3-methoxypyridin-2-amine (CAS: 42409-58-5, 45 mg, 0.215 mmol) in a 1:1 mixture of dry toluene (200 μL) and dry MeCN (200 μL) were added rac-tert-butyl 2-(2-chloroacetyl)pyrrolidine-1-carboxylate (**Intermediate 1**: 50 mg, 0.196 mmol) and sodium iodide (3.0 mg, 0.02 mmol). The reaction mixture was stirred at 80°C for 16h. The reaction mixture was concentrated under reduced pressure to give the crude product which was purified by reverse phase flash chromatography (C18 aq) using a gradient of MeCN/water from 0% to 100% to afford rac-tert-butyl 2-(6-bromo-8-methoxy-imidazo[1,2-pyridine-2-yl)pyrrolidine-1-carboxylate **(Intermediate 108:** 60 mg, 71%) as a yellow oil. Mass spec: m/z 396 $[M+H]^+$, 398 $[M+H+2]^+$; $^1H$ NMR (400 MHz, DMSO-$d_6$) δ ppm 8.46 (d, *J* = 12.6 Hz, 1H), 7.63 (d, *J* = 8.2 Hz, 1H), 6.76 (d, *J* = 1.6 Hz, 1H), 4.94 - 4.83 (m, 1H), 3.93 (s, 3H), 3.46 - 3.39 (m, 1H), 2.20 - 2.12 (m, 1H), 2.06 - 2.00 (m, 1H), 1.95 - 1.76 (m, 3H), 1.40 (s, 6H), 1.23 (s, 3H).

**Step 2**

**Intermediate 109: rac-tert-butyl 2-[6-[(1,3-dimethylindazole-6-carbonyl)amino]-8-methoxy-imidazo[1,2-a]pyridin-2-yl]pyrrolidine-1-carboxylate**

To a stirred solution of rac-tert-butyl 2-(6-bromo-8-methoxy-imidazo[1,2-pyridine-2-yl)pyrrolidine-1-carboxylate **(Intermediate 108:** 250 mg, 0.574 mmol) in anhydrous 1,4-dioxane (0.1 M) were added 1,3-dimethylindazole-6-carboxamide (**Intermediate 52**: 163 mg, 0.861 mmol) and $Cs_2CO_3$ (561 mg, 1.72 mmol). The reaction mixture was degassed with argon for 10 min then EPhos Pd G4 (53 mg, 0.0574 mmol) and Dicyclohexyl(3-isopropoxy-2',4',6'-triisopropyl-[1,1'-biphenyl]-2-yl)phosphine (31 mg, 0.057 mmol) were added. The reaction mixture was stirred at 90°C for 16 h. The mixture was cooled to rt and concentrated to dryness to give the crude compound which was purified by reverse-phase flash chromatography (C18 aq) using a gradient of MeCN/water (0.1% AcOH) from 5% to 70% to afford rac-tert-butyl 2-[6-[(1,3-dimethylindazole-6-carbonyl)amino]-8-methoxy-imidazo[1,2-a]pyridin-2-yl]pyrrolidine-1-carboxylate (**Intermediate**

**109:** 75 mg, 23%) as a brown solid. Mass spec: m/z 505 $[M+H]^+$; $^1H$ NMR (400 MHz, DMSO-$d_6$) $\delta$ ppm 10.31 (s, 1H), 8.94 (s, 1H), 8.21 (t, *J* = 1.1 Hz, 1H), 7.85 (dd, *J* = 8.4, 0.8 Hz, 1H), 7.76 (d, *J* = 10.6 Hz, 1H), 7.69 (dd, *J* = 8.4, 1.4 Hz, 1H), 6.92 (d, *J* = 1.6 Hz, 1H), 4.90 (d, *J* = 13.4 Hz, 1H), 4.07 (s, 3H), 3.94 (s, 3H), 3.56 - 3.41 (m, 1H), 3.41 - 3.36 (m, 1H), 2.53 (s, 3H), 2.17 (s, 1H), 2.01 (s, 2H), 1.85 (s, 1H), 1.41 (s, 3H), 1.24 (s, 6H).

**Step 3**

**Intermediate 110: rac-N-(8-methoxy-2-pyrrolidin-2-yl-imidazo[1,2-a]pyridin-6-yl)-1,3-dimethyl-indazole-6-carboxamide trihydrochloride**

To a stirred solution of rac-tert-butyl 2-[6-[(1,3-dimethylindazole-6-carbonyl)amino]-8-methoxy-imidazo[1,2-a]pyridin-2-yl]pyrrolidine-1-carboxylate **(Intermediate 109:** 75 mg, 0.134 mmol) in dioxane (2.1 mL) was added dropwise a solution of hydrogen chloride (4N in dioxane, 334 µL, 1.34 mmol). The reaction mixture was stirred at rt for 16 h. Additional hydrogen chloride (4N in dioxane, 334 µL, 1.34 mmol) was added at rt and the reaction mixture was stirred at rt for 72 h. The reaction mixture was concentrated to dryness to afford rac-N-(8-methoxy-2-pyrrolidin-2-yl-imidazo[1,2-a]pyridin-6-yl)-1,3-dimethyl-indazole-6-carboxamide trihydrochloride (**Intermediate 110:** 75mg, 96%) which was directly used in step 4 without further purification. Mass spec: m/z 405 $[M+H]^+$; $^1H$ NMR (400 MHz, DMSO-$d_6$) $\delta$ ppm 10.76 (s, 1H), 10.00 (s, 1H), 9.34 - 9.21 (m, 2H), 8.43 - 8.37 (m, 2H), 7.87 (dd, *J* = 8.5, 0.8 Hz, 1H), 7.73 (dd, *J* = 8.4, 1.4 Hz, 1H), 7.53 (s, 1H), 4.87 - 4.82 (m, 1H), 4.09 (s, 3H), 4.04 (s, 3H), 3.35 - 3.29 (m, 2H), 2.54 (s, 3H), 2.45 - 2.39 (m, 1H), 2.17 (ddd, *J* = 17.3, 9.8, 4.6 Hz, 2H), 2.09 - 2.01 (m, 1H).

**Step 4**

**Example 1: rac-N-[8-methoxy-2-(1-methylpyrrolidin-2-yl)imidazo[1,2-a]pyridin-6-yl]-1,3-dimethyl-indazole-6-carboxamide**

To a stirred solution of N-(8-methoxy-2-pyrrolidin-2-yl-imidazo[1,2-a]pyridin-6-yl)-1,3-dimethyl-indazole-6-carboxamide trihydrochloride (**Intermediate 110:** 75 mg, 0.128 mmol) in MeOH (1.3 mL) were added formaldehyde (37% aq, 48 µL, 0.642 mmol), acetic acid (15 µL, 0.257 mmol) and supported sodium borohydride (103 mg, 0.257 mmol). The reaction mixture was stirred at rt for 16h. Additional supported sodium borohydride (103 mg, 0.257 mmol) was added, and the reaction mixture was stirred at rt for 16h. The reaction mixture was filtered and dried using a phase separator then diluted in anhydrous methanol (1.3 mL). Supported sodium borohydride (103 mg, 0.257 mmol) was added and the reaction mixture was stirred at rt for 16h. The reaction mixture was filtered and dried using a phase separator, washed with MeOH then concentrated to dryness. The crude material was purified by reverse-phase flash chromatography (C18 Aq) using a gradient of MeCN/water (0.1% AcOH) from 0% to 80% to afford rac-N-[8-methoxy-2-(1-methylpyrrolidin-2-yl)imidazo[1,2-a]pyridin-6-yl]-1,3-dimethyl-indazole-6-carboxamide (**Example 1:** 5 mg, 9%) as a white foam. Mass spec: m/z 419 $[M+H]^+$; $^1H$ NMR (600 MHz, DMSO-$d_6$): $\delta$ ppm 10.29 (s, 1H), 8.93 (d, *J* = 1.6 Hz, 1H), 8.19-8.22 (m, 1H), 7.82-7.86 (m, 2H), 7.68 (dd, *J* = 8.4, 1.3 Hz, 1H), 6.90 (d, *J* = 1.6 Hz, 1H), 4.06 (s, 3H), 3.93 (s, 3H), 3.24-3.28 (m, 1H), 3.06-3.10 (m, 1H), 2.51-2.54 (m, 3H), 2.09-2.26 (m, 5H), 1.72-1.90 (m, 3H).

**Example 2 was synthesized following Scheme 25**

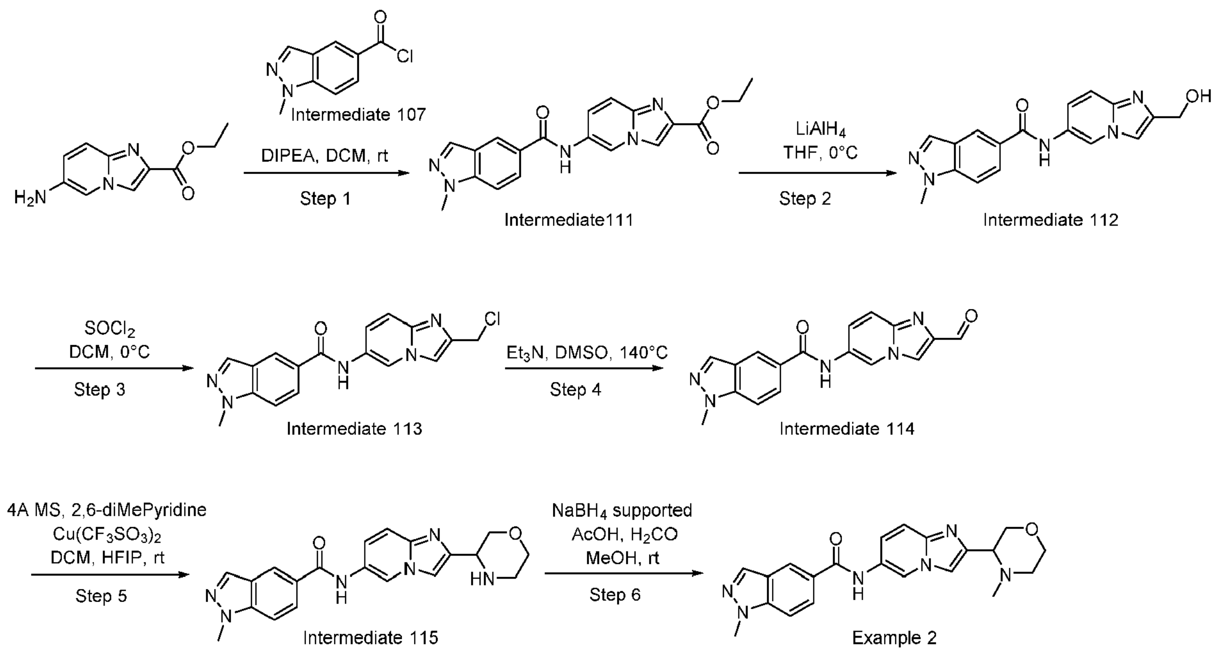


## Scheme 25

### Step 1

**Intermediate 111: Ethyl 6-[(1-methylindazole-5-carbonyl)amino]imidazo[1,2-a]pyridine-2-carboxylate**

To a solution of ethyl 6-aminoimidazo[1,2-a]pyridine-2-carboxylate (CAS: 158980-21-3,5.00 g, 23.1 mmol) and N-ethyl-N-isopropyl-propan-2-amine (24 mL, 0.139 mol) in DCM (225 mL) was added 1-methylindazole-5-carbonyl chloride (**Intermediate 107:** 5.41 g, 27.8 mmol). The reaction mixture was stirred for 1h at rt. The mixture was diluted with DCM and washed with water. The resulting solid was filtered to give the title product. The aqueous phase of the filtrate was extracted with DCM, dried with a phase separator and concentrated under reduced pressure. The crude material was purified by flash chromatography on silica gel using a gradient of MeOH in DCM from 0% to 10% and was combined with the solid obtained previously to afford ethyl 6-[(1-methylindazole-5-carbonyl)amino]imidazo[1,2-a]pyridine-2-carboxylate (**Intermediate 111:** 5.75 g, 56%) as a beige solid. Mass spec: m/z 364 $[M+H]^+$; $^1H$ NMR (400 MHz, DMSO-$d_6$) $\delta$ ppm 10.45 (s, 1H), 9.44 (s, 1H), 8.67 (s, 1H), 8.51 (s, 1H), 8.28 (s, 1H), 8.03 (d, $J$ = 8.8 Hz, 1H), 7.80 (d, $J$ = 8.9 Hz, 1H), 7.69 - 7.56 (m, 2H), 4.32 (q, $J$ = 7.1 Hz, 2H), 4.11 (s, 3H), 1.33 (t, $J$ = 7.1 Hz, 3H).

### Step 2

**Intermediate 112: N-[2-(hydroxymethyl)imidazo[1,2-a]pyridin-6-yl]-1-methylindazole-5-carboxamide**

To a solution of ethyl 6-[(1-methylindazole-5-carbonyl)amino]imidazo[1,2-a]pyridine-2-carboxylate (**Intermediate 111:** 5.00 g, 13.8 mmol) in anhydrous THF (130 mL) cooled to 0°C was added dropwise a solution of lithium aluminium hydride (2 M in THF, 14 mL, 27.5 mmol). The reaction mixture was stirred for 3h at rt before being quenched with a saturated solution of Rochelle's salt. The resulting precipitate was filtered and dried under vacuum to afford N-[2-(hydroxymethyl)imidazo[1,2-a]pyridin-6-yl]-1-methyl-indazole-5-carboxamide (**Intermediate 112:** 5.0 g, quant) as a brown solid that was used in the next step without any further purification. Mass spec: m/z 322 $[M+H]^+$.

### Step 3

**Intermediate 113: N-[2-(chloromethyl)imidazo[1,2-a]pyridin-6-yl]-1-methylindazole-5-carboxamide**

To a solution of N-[2-(hydroxymethyl)imidazo[1,2-a]pyridin-6-yl]-1-methyl-indazole-5-carboxamide (**Intermediate 112:** 5.00 g, 15.2 mmol) in anhydrous DCM (188 mL) cooled to 0°C was added thionyl dichloride (1.4 mL, 19.8 mmol). The mixture was stirred for 16h at rt. Additional thionyl dichloride (1.4 mL, 19.8 mmol) was added and stirring was stirred for another 2h. The observed precipitate was filtered to give the crude product that was purified by flash

column chromatography on silica gel using a gradient of MeOH in DCM from 0% to 10% to afford N-[2-(chloromethyl)imidazo[1,2-a]pyridin-6-yl]-1-methyl-indazole-5-carboxamide (**Intermediate 113:** 1.5 g, 28%) as a yellow solid. Mass spec: m/z 340 $[M+H]^+$.

**Step 4**

**Intermediate 114: N-(2-formylimidazo[1,2-a]pyridin-6-yl)-1-methyl-indazole-5-carboxamide**

A solution of N-[2-(chloromethyl)imidazo[1,2-a]pyridin-6-yl]-1-methyl-indazole-5-carboxamide (**Intermediate 113**: 2.00 g, 5.89 mmol), triethylamine (0.82 mL, 5.89 mmol) in anhydrous DMSO (29 mL) was stirred at 140°C for 8h. The reaction mixture was diluted with DCM and quenched with a saturated solution of $NaHCO_3$. The resulting white precipitate was dissolved in chloroform/isopropanol (3:1) and organic layer was washed 3 times with a saturated solution of $NaHCO_3$. The organic layer was dried over anhydrous $Na_2SO_4$, filtered, and concentrated under vacuum. The crude material was purified by flash chromatography on silica gel using a gradient of MeOH in DCM from 0% to 15% to afford N-(2-formylimidazo[1,2-a]pyridin-6-yl)-1-methylindazole-5-carboxamide (**Intermediate 114**: 222 mg, 12%) as a brown solid. Mass spec: m/z 320 $[M+H]^+$; $^1H$ NMR (400 MHz, DMSO-$d_6$) δ ppm 10.50 (s, 1H), 10.00 (s, 1H), 9.52 (dd, *J* = 2.1, 1.0 Hz, 1H), 8.77 (d, *J* = 0.7 Hz, 1H), 8.51 (dd, *J* = 1.7, 0.8 Hz, 1H), 8.28 (d, *J* = 0.9 Hz, 1H), 8.02 (dd, *J* = 8.9, 1.7 Hz, 1H), 7.80 (dt, *J* = 8.9, 0.9 Hz, 1H), 7.72 (dt, *J* = 9.7, 0.9 Hz, 1H), 7.64 (dd, *J* = 9.8, 2.0 Hz, 1H), 4.11 (s, 3H).

**Step 5.**

**Intermediate 115: rac-1-Methyl-N-(2-morpholin-3-ylimidazo[1,2-a]pyridin-6-yl)indazole-5-carboxamide**

A mixture of N-(2-formylimidazo[1,2-a]pyridin-6-yl)-1-methyl-indazole-5-carboxamide (**Intermediate 114**: 110 mg, 0.338 mmol), 4Å molecular sieves and [(2-aminoethoxy)methyl]tributylstannane (129 mg, 0.338 mmol) in anhydrous DCM (2 mL) was stirred at rt for 2h. A separate vial was loaded with 2,6-dimethylpyridine (0.040 mL, 0.338 mmol) and copper bis(trifluoromethanesulfonate) (122 mg, 0.338 mmol) in HFIP (1.6 mL). The reaction mixture was stirred at rt for 1h. The pre-formed imine was filtered through a pad of celite, concentrated under reduced pressure then added to the copper salt solution. The mixture was stirred at rt for 16h. Additional [(2-aminoethoxy)methyl]tributylstannane (129 mg, 0.338 mmol) and preformed copperbis(trifluoromethanesulfonate) (122 mg, 0.338 mmol) were added and the reaction mixture was stirred at rt for 16h. The reaction mixture was filtered through a pad of celite, then the filtrate was concentrated to dryness to afford rac-1-Methyl-N-(2-morpholin-3-ylimidazo[1,2-a]pyridin-6-yl)indazole-5-carboxamide (**Intermediate 115**: 23 mg, 0.061 mmol) used in Step 6 without purification. Mass spec: m/z 377 $[M+H]^+$.

**Step 6**

**Example 2: rac-1-methyl-N-[2-(4-methylmorpholin-3-yl)imidazo[1,2-a]pyridin-6-yl]indazole-5-carboxamide**

A solution of rac-1-methyl-N-(2-morpholin-3-ylimidazo[1,2-a]pyridin-6-yl)indazole-5-carboxamide (**Intermediate 115**: 23 mg, 0.0611 mmol), acetic acid (0.0035 mL, 0.0611 mmol) and formaldehyde (37% aq, 0.0055 mL, 0.0733 mmol) in MeOH (0.4 mL) was stirred for 1h at rt. Supported $NaBH_4$ (24 mg, 0.0611 mmol, loading 2.5 mmol/g) was added and the reaction mixture was stirred for 6h at rt. Additional formaldehyde (37% Aq, 0.0055 mL, 0.0733 mmol) and supported $NaBH_4$ (24 mg, 0.0611 mmol, loading 2.5 mmol/g) were added and the mixture was stirred for 4h at rt. The reaction mixture was filtered, and the filtrate was concentrated under vacuum before addition of HCl in dioxane (4N, 5 mL). The solution was concentrated to dryness to give the crude product which was purified by preparative HPLC (column: Fuji Silysia Chromatorex THE C18 300 × 50 mm, 10 μm; Eluent: $NH_4OAc$ aq 10mM/(MeCN/$NH_4OAc$ aq 100mM (90/10)) from 80/20 to 0/100; flow rate: 120 mL/min) to afford rac-1-methyl-N-[2-(4-methylmorpholin-3-yl)imidazo[1,2-a]pyridin-6-yl]indazole-5-carboxamide (**Example 2:** 2 mg, 9%) as a yellow solid. Mass spec: m/z 391 $[M+H]^+$; $^1H$ NMR (600 MHz, DMSO-$d_6$) δ ppm 10.34 (s, 1 H) 9.13 - 9.35 (m, 1 H) 8.49 (dd, *J* = 1.6, 0.9 Hz, 1 H) 8.26 (d, *J* = 1.0 Hz, 1 H) 8.02 (dd, *J* = 8.9, 1.7 Hz, 1 H) 7.92 (s, 1 H) 7.74 - 7.83 (m, 1 H) 7.47 - 7.55 (m, 1 H) 7.44 (dd, *J* = 9.6, 1.9 Hz, 1 H) 4.10 (s, 3 H) 3.79 - 3.86 (m, 1 H) 3.69 - 3.76 (m, 1 H) 3.58 - 3.67 (m, 1 H) 3.43 - 3.50 (m, 1 H) 3.20 - 3.23 (m, 1 H) 2.75 - 2.82 (m, 1 H) 2.22 - 2.30 (m, 1 H) 2.04 - 2.11 (m, 3 H).

**Example 3 was synthesized following Scheme 26**

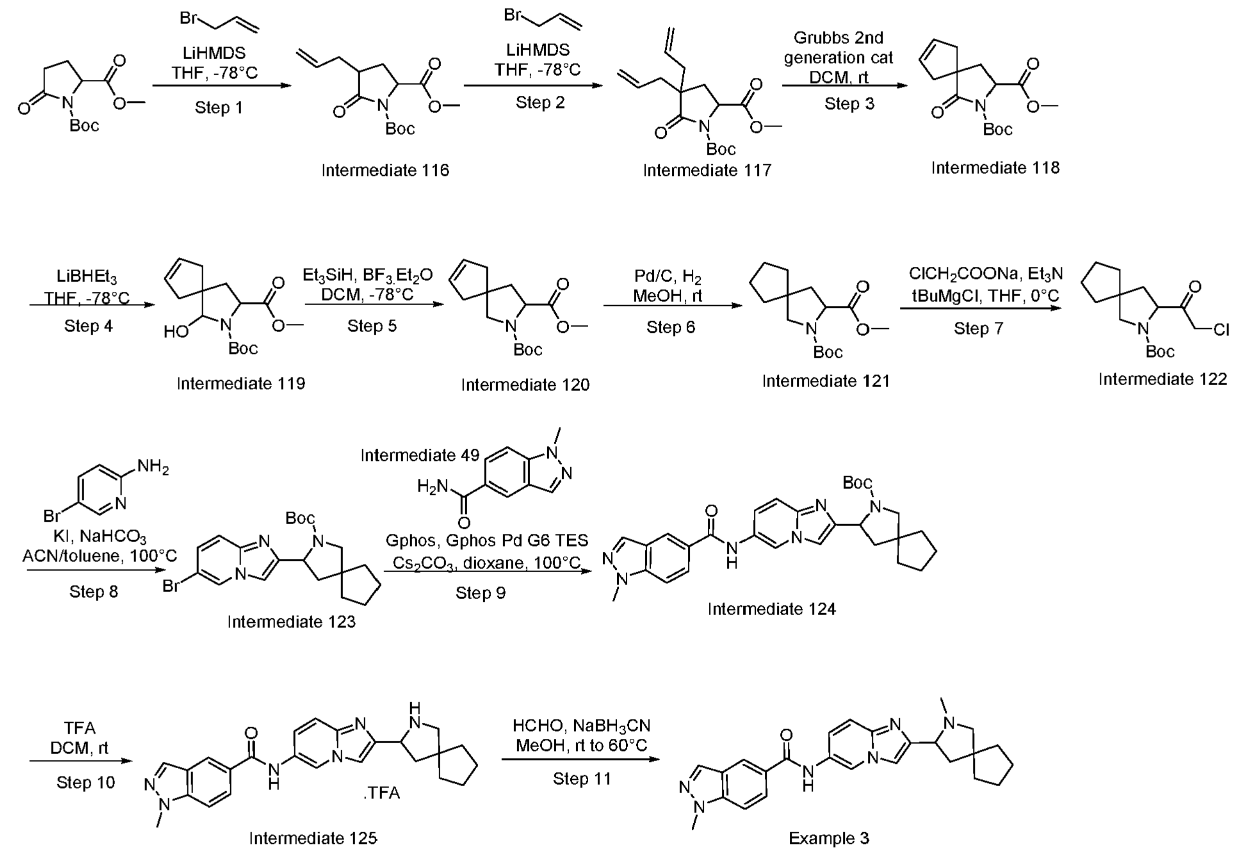


## Scheme 26

### Step 1

**Intermediate 116: rac-1-tert-butyl 2-methyl 5-oxo-4-(prop-2-en-1-yl)pyrrolidine-1,2-dicarboxylate**

To a solution of rac-1-tert-butyl 2-methyl 5-oxopyrrolidine-1,2-dicarboxylate (CAS: 861657-91-2, 5.00 g, 19.5 mmol) in THF (75 mL) was added a solution of LiHMDS (21.6 mL, 1M in THF, 21.6 mmol) at -78°C. The reaction was stirred at -78°C for 1h and allyl bromide (4.97 g, 41.1mmol) in THF (10 mL) was added. The reaction was stirred at -78°C for 2h and then quenched with a saturated $NH_4Cl$ solution (200 mL). The reaction mixture was extracted three times with EtOAc. The combined organic layers were washed with brine, dried over anhydrous $Na_2SO_4$, filtered, and concentrated to dryness. The crude material was purified by flash chromatography on silica gel using PE/EtOAc (3/1) to afford rac-1-tert-butyl 2-methyl 5-oxo-4-(prop-2-en-1-yl)pyrrolidine-1,2-dicarboxylate (**Intermediate 116**: 2.2 g, 30%) as a colorless oil. Mass spec: m/z: 184 $[M+H-Boc]^+$.

### Step 2

**Intermediate 117: rac-1-tert-butyl 2-methyl 5-oxo-4,4-bis(prop-2-en-1-yl)pyrrolidine-1,2-dicarboxylate**

To a solution of rac-1-tert-butyl 2-methyl 5-oxo-4-(prop-2-en-1-yl)pyrrolidine-1,2-dicarboxylate (**Intermediate 116**: 1.50 g, 4.50 mmol) in THF (30 mL) was added a solution of LiHMDS (5.56 mL, 1M in THF, 5.56mmol) at -78°C. The reaction was stirred at -78°C for 1h and allyl bromide (1.28 g, 10.6 mmol) in THF (5 mL) was added. The reaction was stirred at -78°C for 2h and quenched with a saturated $NH_4Cl$ solution (100 mL). The reaction mixture was extracted three times with EtOAc. The combined organic layers were washed with brine, dried over anhydrous $Na_2SO_4$, filtered, and concentrated to dryness. The crude material was purified by flash chromatography on silica gel using PE/EtOAc (3/1) to afford rac-1-tert-butyl 2-methyl 5-oxo-4,4-bis(prop-2-en-1-yl)pyrrolidine-1,2-dicarboxylate (**Intermediate 117**: 900 mg, 53%) as a colourless oil. Mass spec: m/z: 224 $[M+H-Boc]^+$.

**Step 3**

**Intermediate 118: rac-2-tert-butyl 3-methyl 1-oxo-2-azaspiro[4.4]non-7-ene-2,3-dicarboxylate**

To a solution of rac-1-tert-butyl 2-methyl 5-oxo-4,4-bis(prop-2-en-1-yl)pyrrolidine-1,2-dicarboxylate (**Intermediate 117**: 900 mg, 2.36 mmol) in DCM (30 mL) was added Grubbs 2$^{nd}$ generation catalyst (354 mg, 0.417 mmol). The reaction was stirred at rt overnight. The mixture was filtered, and the filtrate was concentrated under reduced pressure. The crude material was purified by flash chromatography on silica gel using PE/EtOAc (/:1) to afford rac-2-tert-butyl 3-methyl 1-oxo-2-azaspiro[4.4]non-7-ene-2,3-dicarboxylate (**Intermediate 118**: 700 mg, 85%) as a colourless oil. Mass spec: m/z: 196 $[M+H-Boc]^+$.

**Step 4**

**Intermediate 119: rac-2-tert-butyl 3-methyl 1-hydroxy-2-azaspiro[4.4]non-7-ene-2,3-dicarboxylate**

To a solution of rac-2-tert-butyl 3-methyl 1-oxo-2-azaspiro[4.4]non-7-ene-2,3-dicarboxylate (**Intermediate 118**: 500 mg, 1.44 mmol) in THF (13 mL) was added $LiBHEt_3$ (179 mg, 1.69 mmol). The reaction was stirred at -78°C for 1h and then quenched with a saturated $NaHCO_3$ solution (50 mL). The mixture was extracted three times with EtOAc. The combined organic layers were washed with brine, dried over anhydrous $Na_2SO_4$, filtered, and concentrated to dryness to afford rac-2-tert-butyl 3-methyl 1-hydroxy-2-azaspiro[4.4]non-7-ene-2,3-dicarboxylate (**Intermediate 119:** 500 mg, 93%) as a colorless oil which was used in step 5 without further purification. Mass spec: m/z: 180 $[M+H-Boc-OH]^+$.

**Step 5**

**Intermediate 120: rac-2-tert-butyl 3-methyl 2-azaspiro[4.4]non-7-ene-2,3-dicarboxylate**

To a solution of rac-2-tert-butyl 3-methyl 1-hydroxy-2-azaspiro[4.4]non-7-ene-2,3-dicarboxylate (**Intermediate 119:** 500 mg, 2.02 mmol) in DCM (20 mL) was added $Et_3SiH$ (616 mg, 5.30 mmol). Then the reaction was cooled to -78°C and $BF_3.Et_2O$ (859 mg, 6.05 mmol) was added. The reaction was allowed to warm to rt over 2h and then quenched with a saturated $NaHCO_3$ solution (50 mL). The reaction mixture was extracted three times with EtOAc. The combined organic layers were washed with brine, dried over anhydrous $Na_2SO_4$, filtered, and concentrated to dryness to afford rac-2-tert-butyl 3-methyl 2-azaspiro[4.4]non-7-ene-2,3-dicarboxylate (**Intermediate 120:** 400 mg, 85%) as a yellow oil which was used in step 6 without further purification. Mass spec: m/z: 226 $[M+H-t-Bu]^+$.

**Step 6**

**Intermediate 121: rac-2-tert-butyl 3-methyl 2-azaspiro[4.4]nonane-2,3-dicarboxylate**

To a solution of rac-2-tert-butyl 3-methyl 2-azaspiro[4.4]non-7-ene-2,3-dicarboxylate (**Intermediate 120:** 400 mg, 1.14 mmol) in MeOH (10 mL) was added Pd/C (200 mg, 10% in carbon). Hydrogen (3 atm) was introduced and the mixture which was stirred at rt overnight. The mixture was filtered, and the filtrate was concentrated under reduced pressure to afford rac-2-tert-butyl 3-methyl 2-azaspiro[4.4]nonane-2,3-dicarboxylate (**Intermediate 121:** 350 mg, 88 %) as a brown oil which was used in step 6 without further purification. Mass spec: m/z: 228 $[M+H-t-Bu]^+$.

**Step 7**

**Intermediate 122: rac-tert-butyl 3-(2-chloroacetyl)-2-azaspiro[4.4]nonane-2-carboxylate**

To a solution of rac-2-tert-butyl 3-methyl 2-azaspiro[4.4]nonane-2,3-dicarboxylate (**Intermediate 121**: 200 mg, 0.568 mmol) in THF (7 mL) was added $Et_3N$ (286 mg, 2.82 mmol) and sodium 2-chloroacetate (411 mg, 3.53 mmol). The reaction mixture was cooled to 0°C and tert-butyl(chloro)magnesium (412 mg, 3.53 mmol) was added dropwise under nitrogen atmosphere. The reaction mixture was stirred at 0°C for 2h and then quenched with a saturated $NH_4Cl$ solution (50 mL). The mixture was extracted three times with EtOAc. The combined organic layers were washed with brine, dried over anhydrous $Na_2SO_4$, filtered, and concentrated to dryness to afford rac-tert-butyl 3-(2-chloroacetyl)-2-azaspiro[4.4]nonane-2-carboxylate (**Intermediate 122:** 200 mg, 94%) as a colorless oil which was used in step 6 without further purification. Mass spec: m/z: 246 $[M+H-t-Bu]^+$.

**Step 8**

**Intermediate 123: rac-tert-butyl 3-{6-bromoimidazo[1,2-a]pyridin-2-yl}-2-azaspiro[4.4]nonane-2-carboxylate**

To a solution of rac-tert-butyl 3-(2-chloroacetyl)-2-azaspiro[4.4]nonane-2-carboxylate (**Intermediate 122**: 180 mg, 0.477 mmol) and 5-bromopyridin-2-amine (103 mg, 0.596 mmol) in toluene (1.8 mL) and MeCN (5.4 mL) were added $NaHCO_3$ (60.1 mg, 0.715 mmol) and KI (9.90 mg, 0.06 mmol). The reaction was stirred at 100 °C for 16h. The mixture was concentrated under reduced pressure and the resulting crude material was purified by reverse-phase flash chromatography (C18 aq) using MeCN/$H_2O$ (2/3) to afford rac-tert-butyl 3-{6-bromoimidazo[1,2-a]pyridin-2-yl}-2-azaspiro[4.4]nonane-2-carboxylate (**Intermediate 123:** 80.0 mg, 32%) as a brown solid. Mass spec: m/z: 420 $[M+H]^+$.

**Step 9**

**Intermediate 124: rac-tert-butyl 3-[6-(1-methylindazole-5-amido)imidazo[1,2-a]pyridin-2-yl]-2-azaspiro[4.4]nonane-2-carboxylate**

To a solution of rac-tert-butyl 3-{6-bromoimidazo[1,2-a]pyridin-2-yl}-2-azaspiro[4.4]nonane-2-carboxylate (**Intermediate 123:** 75.0 mg, 0.142 mmol) and 1-methylindazole-5-carboxamide (**Intermediate 49:** 31.3 mg, 0.178 mmol) in dioxane (1.88 mL) were added $Cs_2CO_3$ (145 mg, 0.445 mmol), Gphos (9.56 mg, 0.0180 mmol) and Gphos Pd G6 TES (16.8 mg, 0.0180 mmol) under nitrogen atmosphere. The reaction was stirred at 100 °C for 2h. The mixture was concentrated under reduced pressure. The crude material was purified by reverse-phase flash chromatography (C18) using MeCN/$H_2O$ (1/1, 0.05% formic acid) to afford rac-tert-butyl 3-[6-(1-methylindazole-5-amido)imidazo[1,2-a]pyridin-2-yl]-2-azaspiro[4.4]nonane-2-carboxylate (**Intermediate 124:** 60 mg, 65%) as a brown solid. Mass spec: m/z: 515 $[M+H]^+$.

**Step 10**

**Intermediate 125: rac-N-(2-{2-azaspiro[4.4]nonan-3-yl}imidazo[1,2-a]pyridin-6-yl)-1-methylindazole-5-carboxamide trifluoroacetate**

To a solution of rac-tert-butyl 3-[6-(1-methylindazole-5-amido)imidazo[1,2-a]pyridin-2-yl]-2-azaspiro[4.4]nonane-2-carboxylate (**Intermediate 124:** 50.0 mg, 0.082 mmol) in DCM (1 mL) was added TFA (0.5 mL). The reaction was stirred at rt for 1h and then concentrated under reduced pressure to afford rac-N-(2-{2-azaspiro[4.4]nonan-3-yl}imidazo[1,2-a]pyridin-6-yl)-1-methylindazole-5-carboxamide trifluoroacetate (**Intermediate 125:** 40 mg, quant.) as a brown oil which was used in step 11 without further purification. Mass spec: m/z: 415 $[M+H]^+$.

**Step 11**

**Example 3: rac-1-methyl-N-(2-{2-methyl-2-azaspiro[4.4]nonan-3-yl}imidazo[1,2-a]pyridin-6-yl)indazole-5-carboxamide**

A solution of rac-N-(2-{2-azaspiro[4.4]nonan-3-yl}imidazo[1,2-a]pyridin-6-yl)-1-methylindazole-5-carboxamide trifluoroacetate (**Intermediate 125:** 40.0 mg, 0.0768 mmol) and paraformaldehyde (20.3 mg, 0.672 mmol) in MeOH (2 mL) was stirred at rt for 1h. Then, $NaBH_3CN$ (18.2 mg, 0.288 mmol) was added. The reaction solution was stirred at 60°C for 1h. The mixture was concentrated under reduced pressure. The crude material was purified by preparative HPLC (column: Xselect CSH Prep OBD C18, 300 × 50 mm, 5 $\mu$m; Eluent: MeCN in $H_2O$ (0.1% formic acid) from 5 to 30%; flow rate: 60 mL/min) to afford rac-1-methyl-N-(2-{2-methyl-2-azaspiro[4.4]nonan-3-yl}imidazo[1,2-a]pyridin-6-yl)indazole-5-carboxamide (**Example 3**: 10.7 mg, 31%) as a white solid. Mass spec: m/z: 429 $[M+H]^+$; $^1H$ NMR (300 MHz, MeOH-$d_4$) $\delta$ ppm 9.21 (s, 1H), 8.45 (s, 1H), 8.16 (s, 1H), 8.02 (d, J = 8.8 Hz, 1H), 7.83 (s, 1H), 7.67 (d, J = 8.8 Hz, 1H), 7.43 - 7.48 (m, 2H), 4.12 (s, 3H), 3.62 - 3.66 (m, 1H), 3.15 (d, J = 10.0 Hz, 1H), 2.45 (d, J = 9.6 Hz, 1H), 2.37 (s, 3H), 2.21 - 2.26 (m, 1H), 2.02 - 2.08 (m, 1H), 1.70 - 1.85 (m, 8H).

**Example 4 was synthesized following Scheme 27**

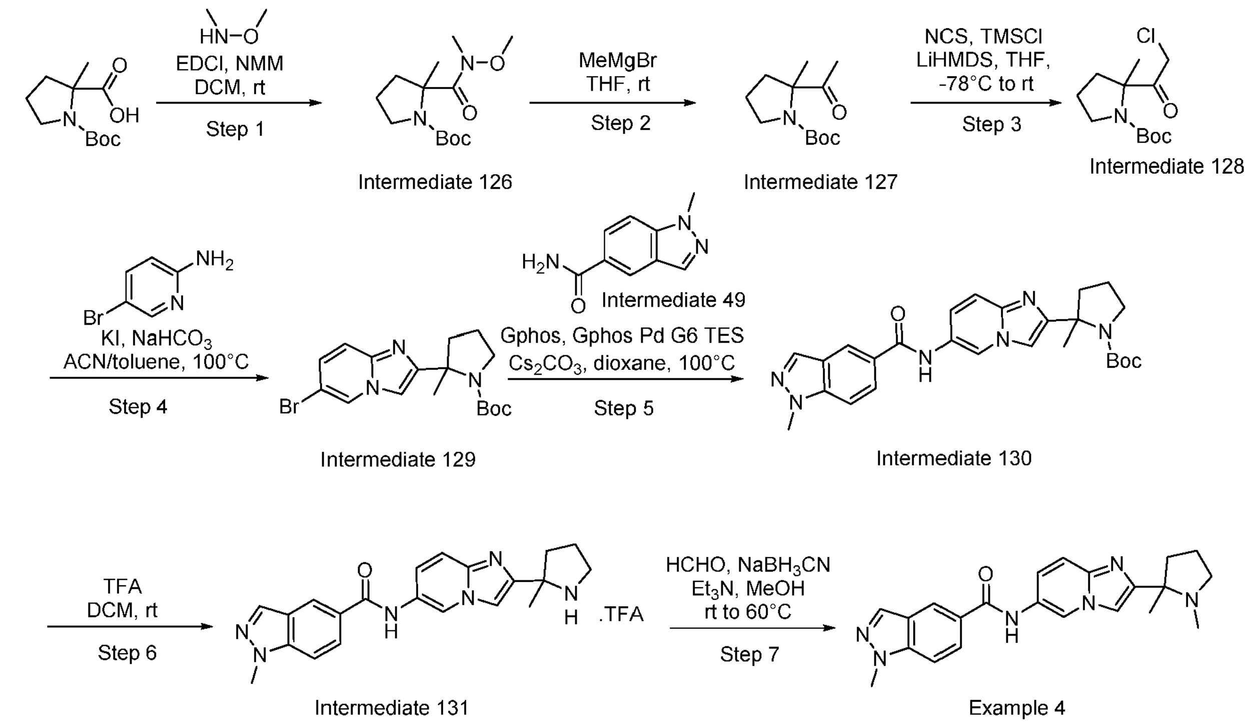


## Scheme 27

**Step 1**

**Intermediate 126: rac-tert-butyl 2-[methoxy(methyl)carbamoyl]-2-methylpyrrolidine-1-carboxylate**

To a solution of rac-1-(tert-butoxycarbonyl)-2-methylpyrrolidine-2-carboxylic acid (CAS: 203869-80-1, 2.00 g, 8.72 mmol) in DCM (40 mL) at 0°C were added N, O-dimethylhydroxylamine (0.800 g, 13.0 mmol), EDCI (2.01 g, 10.4 mmol) and NMM (1.06 g, 10.5 mmol). The reaction mixture was stirred at rt for 2h and quenched with water. The aqueous layer was extracted three times with EtOAc and the organic layers were combined, washed with brine, dried over anhydrous $Na_2SO_4$, filtered, and concentrated under reduced pressure to afford rac-tert-butyl 2-[methoxy(methyl)carbamoyl]-2-methylpyrrolidine-1-carboxylate (**Intermediate 126**: 1.91 g, 80%) as a colorless oil which was used in Step 2 without further purification. Mass spec: m/z 273 $[M+H]^+$.

**Step 2**

**Intermediate 127: rac-tert-butyl 2-acetyl-2-methylpyrrolidine-1-carboxylate**

To a solution of rac-tert-butyl 2-[methoxy(methyl)carbamoyl]-2-methylpyrrolidine-1-carboxylate (**Intermediate 126**: 1.90 g, 6.98 mmol) in THF (20 mL) at 0°C was added a solution of MeMgBr (17.4 mL, 17.4 mmol, 1M in THF). The reaction mixture was stirred at rt for 48h and then quenched with water. The resulting solution was extracted with EtOAc three times and the organic layers were combined, washed with brine, dried over anhydrous $Na_2SO_4$, filtered, and concentrated under reduced pressure to afford rac-tert-butyl 2-acetyl-2-methylpyrrolidine-1-carboxylate (**Intermediate 127:** 1.27 g, 72%) as a yellow oil which was used in step 3 without further purification. Mass spec: m/z 228 $[M+H]^+$.

**Step 3**

**Intermediate 128: rac-tert-butyl 2-(2-chloroacetyl)-2-methylpyrrolidine-1-carboxylate**

To a solution of rac-tert-butyl 2-acetyl-2-methylpyrrolidine-1-carboxylate (**Intermediate 127**: 500 mg, 2.20 mmol) in THF (20 mL) was added a solution of LiHMDS (4.40 mL, 4.40 mmol, 1M in THF) for 1.5h at -78°C under nitrogen atmosphere, followed by the dropwise addition of TMSCl (478 mg, 4.40 mmol). The reaction mixture was stirred at 0°C

for 1h, cooled to -78°C and NCS (441 mg, 3.30 mmol) was added. The reaction mixture was warmed to rt and stirred overnight. The reaction mixture was quenched with water (100 mL). The phases were separated, and the aqueous phase was extracted with EtOAc three times. The combined organic layers were washed with brine (3 × 100 mL), dried over anhydrous $Na_2SO_4$, filtered, and concentrated under reduced pressure. The crude material was purified by flash chromatography on silica gel using PE/EtOAc (8/2) to afford rac-tert-butyl 2-(2-chloroacetyl)-2-methylpyrrolidine-1-carboxylate (**Intermediate 128**: 500 mg, 87%) as a yellow oil. Mass spec: m/z 262 $[M+H]^+$.

**Step 4**

**Intermediate 129: rac-tert-butyl 2-{6-bromoimidazo[1,2-a]pyridin-2-yl}-2-methylpyrrolidine-1-carboxylate**

To a solution of rac-tert-butyl 2-(2-bromoacetyl)-2-methylpyrrolidine-1-carboxylate (**Intermediate 128**: 350 mg, 1.14 mmol) in MeCN (3 mL) and toluene (1 mL) were added 5-bromopyridin-2-amine (198 mg, 1.14 mmol), $NaHCO_3$ (115 mg, 1.37mmol) and KI (19.0 mg, 0.114 mmol). The reaction mixture was stirred at 100°C for 3h and then quenched with water. The phases were separated, and the aqueous phase was extracted with EtOAc three times. The combined organic layers were combined, washed with brine (3 x 50 mL), dried over anhydrous $Na_2SO_4$, filtered, and concentrated under reduced pressure. The crude material was purified by flash chromatography on silica gel using PE/EtOAc (8/2) to afford rac-tert-butyl 2-{6-bromoimidazo[1,2-a]pyridin-2-yl}-2-methylpyrrolidine-1-carboxylate (**Intermediate 129:** 60.0 mg, 14%) as an off-white solid. Mass spec: m/z 380 $[M+H]^+$.

**Step 5**

**Intermediate 130: rac-tert-butyl 2-methyl-2-[6-(1-methylindazole-5-amido)imidazo[1,2-a]pyridin-2-yl]pyrrolidine-1-carboxylate**

To a solution of rac-tert-butyl 2-{6-bromoimidazo[1,2-a]pyridin-2-yl}-2-methylpyrrolidine-1-carboxylate (**Intermediate 129**: 40.0 mg, 0.142 mmol) and 1-methylindazole-5-carboxamide (**Intermediate 49**: 27.3 mg, 0.156 mmol) in dioxane were added $Cs_2CO_3$ (138 mg, 0.426 mmol), Gphos (1.90 mg, 0.004 mmol) and Gphos Pd G6 TES (3.35 mg, 0.004 mmol). The reaction mixture was stirred at 100°C for 3h under nitrogen atmosphere. The mixture was concentrated under reduced pressure. The crude material was purified by reverse-phase flash chromatography (C18) using $MeCN/H_2O$ (75/25, 0.05% formic acid) to afford rac-tert-butyl 2-methyl-2-[6-(1-methylindazole-5-amido)imidazo[1,2-a]pyridin-2-yl]pyrrolidine-1-carboxylate (**Intermediate 130:** 43.0 mg, 86%) as an off-white solid. Mass spec: m/z 389 $[M+H]^+$.

**Step 6.**

**Intermediate 131: rac-1-methyl-N-[2-(2-methylpyrrolidin-2-yl)imidazo[1,2-a]pyridin-6-yl]indazole-5-carboxamide trifluoroacetate**

To a solution of rac-tert-butyl 2-methyl-2-[6-(1-methylindazole-5-amido)imidazo[1,2-a]pyridin-2-yl]pyrrolidine-1-carboxylate (**Intermediate 130**: 40.0 mg, 0.106 mmol) in DCM (0.6 mL) was added TFA (0.2 mL). The reaction mixture was stirred at rt for 2h and then concentrated under reduced pressure to afford rac-1-methyl-N-[2-(2-methylpyrrolidin-2-yl)imidazo[1,2-a]pyridin-6-yl]indazole-5-carboxamide trifluoroacetate (**Intermediate 131:** 14.0 mg, 95%) as a yellow oil. Mass spec: m/z 280 $[M+H]^+$.

**Step 7**

**Example 4: rac-N-[2-(1,2-dimethylpyrrolidin-2-yl)imidazo[1,2-a]pyridin-6-yl]-1-methylindazole-5-carboxamide**

To a solution of rac-1-methyl-N-[2-(2-methylpyrrolidin-2-yl)imidazo[1,2-a]pyridin-6-yl]indazole-5-carboxamide trifluoroacetate (**Intermediate 131**: 5.0 mg, 0.054 mmol) in MeOH (1 mL) were added $Et_3N$ (1 mL) and paraformaldehyde (8.04 mg, 0.270 mmol). The reaction mixture was stirred at rt for 1h. $NaBH_3CN$ (10.1 mg, 0.162 mmol) was added. The reaction mixture was stirred at 60°C for 1h and then concentrated under reduced pressure. The residue was purified by preparative HPLC (Column: XBridge Prep OBD C18 Column, 30x150 mm, 5 μm; Eluent: MeCN in water (10mmol/L $NH_4HCO_3$ + 0.05% $NH_4OH$) from 30 to 60%; Flow rate: 60 mL/min) to provide rac-N-[2-(1,2-dimethylpyrrolidin-2-yl)imidazo[1,2-a]pyridin-6-yl]-1-methylindazole-5-carboxamide (**Example 4**: 2.4 mg, 4%) as an off-white solid. Mass spec: m/z: 389 $[M+H]^+$. $^1H$ NMR (400 MHz, DMSO-$d_6$) δ ppm 10.34 (s, 1H), 9.23 (s,1H), 8.49 (s,1H), 8.26 (s, 1H), 8.00 - 8.03 (m, 1H), 7.86 (s, 1H), 7.77 - 7.79 (m, 1H), 7.50 -7.52 (m, 1H), 7.39 - 7.42 (m, 1H), 4.10 (s, 3H), 2.74 - 2.80 (m, 2H), 2.20 - 2.23 (m, 1H), 2.16 (s, 3H), 1.85 - 1.92 (m, 3H), 1.84 (s, 3H).

**General Procedures 6A, 6B, 6C, 6D and 6E**

**Examples 5-55**, shown in **Table 1,** were prepared as shown **Scheme 28** and following the appropriate **General Procedure 6A, 6B, 6C, 6D or 6E.** Where carboxylic acid intermediates are not indicated in **Table 1** these are available from commercial sources and well known in the art.

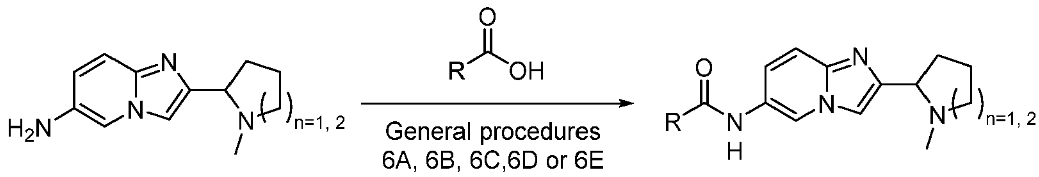


**Scheme 28**

**General Procedure 6A**

To a solution of the appropriate carboxylic acid (1.1 eq) in DCM (0.12 M) were added TCFH (1.2 eq), 1-methylimidazole (10 eq) and the appropriate aryl amine tabulated below (1 eq). The reaction mixture was stirred 18 h at rt. The mixture was partitioned between EtOAc and $H_2O$, the phases were separated, and the aqueous phase was extracted twice with EtOAc. The organic layer was dried over anhydrous $MgSO_4$ and concentrated under reduced pressure. The crude was purified by reverse-phase flash chromatography (C18 aq) using a gradient of MeCN in water from 0% to 100% (neutral) to afford the expected product. For some of the Examples the racemic products were separated by one of the chiral preparative reverse phase HPLC chromatography methods below and indicated in **Table** 1. The order of elution and retention time is also indicated where chiral separation has been performed.

Chiral purification methods by reverse phase HPLC chromatography:

Method A: Column: Chiralcel OD-H 20 μm, 300 × 76.6 mm; Eluent: $CH_3CN$ + 0.5% IPA;
Method B: Column: Chiralpak IA 20 μm, 76.5 × 300 mm; Eluent: MeOH + 0.5% IPA;
Method C: Column: Chiralpak AS 20 μm, 300 × 4.6 mm; Eluent: $CH_3CN$ + 0.5% IPA

**General Procedure 6B**

To a solution of the appropriate carboxylic acid (1 eq) in DCM (0.25 M) were added TCFH (1.2 eq), 1-methylimidazole (3.5 eq) and the appropriate aryl amine tabulated below (1.2 eq). The reaction mixture was stirred at rt until reaction completion. A saturated solution of $NaHCO_3$ and DCM (or EtOAc) were added, and layers were separated. The aqueous layer was extracted with DCM (or EtOAc) and the organic phase was washed with brine, dried over anhydrous $Na_2SO_4$, filtered, and concentrated to dryness. The crude was purified by flash chromatography ($SiO_2$) using an appropriate gradient of solvents to afford the Example shown in **Table 1.**

**General Procedure 6C**

To a solution of the appropriate carboxylic acid (1 eq) in DMF (0.1 M) were added TCFH (1.2 eq), 1-methylimidazole (5 eq) and the appropriate aryl amine tabulated below (1 eq). The reaction mixture was stirred 18 h at rt. The mixture was concentrated in vacuo. The crude was purified by preparative HPLC (C18 aq) using an appropriated gradient of solvents (MeCN/water, neutral, acidic or basic) to afford the desired Examples shown in **Table 1.**

**General procedure 6D**

To a solution of the appropriate carboxylic acid (1 eq) in DMF (0.1 M) was added the appropriate aryl amine tabulated below (1 eq), HATU (1.5 equiv) and DIPEA (3.0 eq). The mixture was stirred at rt for 3-18h. The mixture was then concentrated in vacuo. The crude was purified by preparative HPLC (C18 aq) using an appropriate gradient of solvents (MeCN/water, neutral, acidic or basic) to afford the desired Examples shown in **Table 1.**

**General procedure 6E**

To a solution of the appropriate aryl amine (1 eq) and appropriate acid chloride tabulated below (1.3 eq) in DCM (0.1 M) was added pyridine (4 eq). The reaction mixture was stirred at rt or 60°C for 18h. The mixture was

concentrated in vacuo. The crude was purified by preparative HPLC (C18 aq) using an appropriate gradient of solvents (MeCN/water, neutral, acidic or basic) to afford the desired Example.

**Table 1**

| Example Number | Example Name & Structure | Intermediates | Procedure and Chiral Purification method | NMR and Mass Spec |
|---|---|---|---|---|
| **5** | 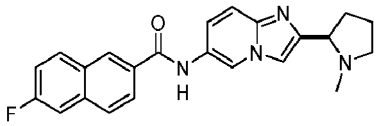 rel-6-fluoro-N-{2-[(2R)-1-methylpyrrolidin-2-yl]imidazo[1,2-a] pyridin-6-yl}naphthalene-2-carboxamide | **4A** | **6A** and purification method A 1st eluting isomer (rt = 11.85 min) | Mass spec: m/z: 389 $[M+H]^+$, $^1H$ NMR (600 MHz, DMSO-$d_6$) δ ppm 1.71 - 1.92 (m, 3 H) 2.13-2.19 (m, 1 H) 2.20 - 2.28 (m, 4 H) 3.05 - 3.12 (m, 1 H) 3.26 - 3.30 (m, 1 H) 7.42 (dd, J = 9.68, 2.05 Hz, 1 H) 7.48 - 7.51 (m, 1 H) 7.55 (td, J = 8.88, 2.64 Hz, 1 H) 7.83 (dd, J = 10.12, 2.64 Hz, 1 H) 7.87 (s, 1 H) 8.04 - 8.12 (m, 2 H) 8.20 (dd, J = 9.24, 5.72 Hz, 1 H) 8.64 (d, J = 0.73 Hz, 1 H) 9.26 (dd, J = 1.91, 0.88 Hz, 1 H) 10.49 (s, 1 H) |
| **6** | 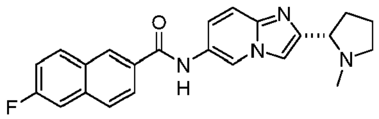 rel-6-fluoro-N-{ 2-[(2S)-1-methylpyrrolidin-2-yl]imidazo[1,2-a] pyridin-6-yl}naphthalene-2-carboxamide | **4A** | **6A** and purification method A 2nd eluting isomer (rt = 13.20 min) | Mass spec: m/z: 389 $[M+H]^+$, $^1H$ NMR (600 MHz, DMSO-$d_6$): δ ppm 10.50 (s, 1H), 9.27 (s, 1H), 8.64 (s, 1H), 8.20 (dd, J = 9.2, 5.8 Hz, 1H), 8.03-8.12 (m, 2H), 7.90 (s, 1H), 7.83 (dd, J = 10.1, 2.6 Hz, 1H), 7.49-7.58 (m, 2H), 7.43 (dd, J = 9.6, 2.0 Hz, 1H), 3.33-3.40 (m, 1H), 3.12 (br t, J = 7.3 Hz, 1H), 2.14-2.36 (m, 5H), 1.76-1.94 (m, 3H) |
| **7** | 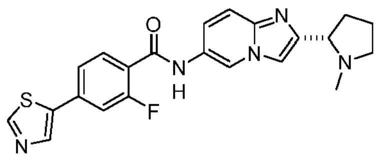 rel-2-fluoro-N-{ 2-[(2S)-1-methylpyrrolidin-2-yl]imidazo[1,2-a] pyridin-6-yl}-4-(1,3-thiazol-5-yl) benzamide | **4A & 63** | **6A** and purification method B 1st eluting isomer (rt = 10.9 min) | Mass spec: m/z: 422 $[M+H]^+$, $^1H$ NMR (500 MHz, DMSO-$d_6$) δ ppm 1.72 - 1.93 (m, 3 H) 2.13 - 2.20 (m, 1 H) 2.22 (s, 3 H) 2.27 (q, J = 8.80 Hz, 1 H) 3.06 - 3.14 (m, 1 H) 3.31 (s, 1 H) 7.27 (dd, J = 9.54, 1.96 Hz, 1 H) 7.48 (d, J = 9.54 Hz, 1 H) 7.66 (dd, J = 8.07, 1.71 Hz, 1 H) 7.78 (t, J = 7.82 Hz, 1 H) 7.82 (dd, J = 11.37, 1.59 Hz, 1 H) 7.88 (s, 1 H) 8.53 (s, 1 H) 9.20 (s, 1 H) 9.23 (d, J = 1.22 Hz, 1 H) 10.51 (s, 1 H) |

(continued)

| Example Number | Example Name & Structure | Intermediates | Procedure and Chiral Purification method | NMR and Mass Spec |
|---|---|---|---|---|
| **8** | 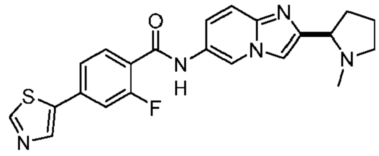 rel-2-fluoro-N-{ 2-[(2R)-1-methylpyrrolidin-2-yl]imidazo[1,2-a] pyridin-6-yl}-4-(1,3-thiazol-5-yl) benzamide | **4A & 63** | **6A** and purification method B 2nd eluting isomer (rt = 12.6 min) | Mass spec: m/z: 422 $[M+H]^+$, $^1H$ NMR (500 MHz, DMSO-$d_6$) δ ppm 10.51 (s, 1H), 9.23 (d, 1H, J = 1.2 Hz), 9.20 (s, 1H), 8.53 (s, 1H), 7.88 (s, 1H), 7.82 (dd, 1H, J = 1.7, 11.2 Hz), 7.7-7.8 (m, 1H), 7.66 (dd, 1H, J = 1.7, 7.8 Hz), 7.48 (d, 1H, J = 9.5 Hz), 7.27 (dd, 1H, J = 2.1, 9.7 Hz), 3.31 (s, 1H), 3.0-3.1 (m, 1H), 2.1-2.3 (m, 5H), 1.7-1.9 (m, 3H) |
| **9** | 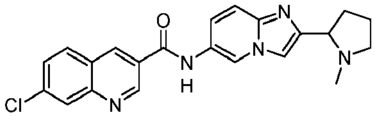 rac-7-chloro-N-[2-(1-methylpyrrolidin-2-yl)imidazo[1,2-a]pyridin-6-yl] quinoline-3-carboxamide | **4A** | **6A** | Mass spec: m/z: 406 $[M+H]^+$, $^1H$ NMR (500 MHz, DMSO-$d_6$) δ ppm 1.72 - 1.95 (m, 3 H) 2.13-2.21 (m, 1 H) 2.22 - 2.24 (m, 3 H) 2.24 - 2.31 (m, 1 H) 3.06 - 3.13 (m, 1 H) 3.28 - 3.32 (m, 1 H) 7.40 (dd, J = 9.54, 1.96 Hz, 1 H) 7.52 (d, J = 9.54 Hz, 1 H) 7.79 (dd, J = 8.68, 2.08 Hz, 1 H) 7.90 (s, 1 H) 8.20 (d, J = 2.20 Hz, 1 H) 8.23 (d, J = 8.80 Hz, 1 H) 9.02 (d, J = 2.20 Hz, 1 H) 9.27 (d, J = 1.22 Hz, 1 H) 9.41 (d, J = 2.20 Hz, 1 H) 10.71 (s, 1 H) |
| **10** | 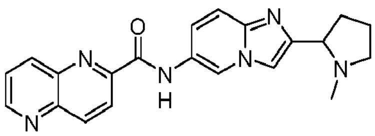 rac-N-[2-(1-methylpyrrolidin-2-yl) imidazo[1,2-a]pyridin-6-yl]-1,5-naphthyridine-2-carboxamide | **4A** | **6A** | Mass spec: m/z: 373 $[M+H]^+$, $^1H$ NMR (600 MHz, DMSO-$d_6$) δ ppm 1.67 - 1.96 (m, 3 H) 2.14-2.35 (m, 5 H) 3.05 - 3.18 (m, 1 H) 3.33 - 3.42 (m, 1 H) 7.41 (dd, J = 9.54, 1.91 Hz, 1 H) 7.53 (d, J = 9.54 Hz, 1 H) 7.93 (s, 1 H) 8.03 (d, J = 6.02 Hz, 1 H) 8.87 (d, J = 5.87 Hz, 1 H) 9.18 (dd, J = 2.20, 0.73 Hz, 1 H) 9.28 (d, J = 1.17 Hz, 1 H) 9.58 (d, J =0.88 Hz, 1 H) 9.59 (d, J = 2.35 Hz, 1 H) 10.79 (s, 1 H) |

(continued)

| Example Number | Example Name & Structure | Intermediates | Procedure and Chiral Purification method | NMR and Mass Spec |
|---|---|---|---|---|
| **11** | 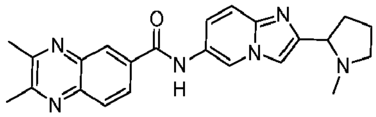 rac-2,3-dimethyl-N-[2-(1-methylpyrrolidin-2-yl)imidazo[1,2-a] pyridin-6-yl] quinoxaline-6-carboxamide | **4A** | **6C** | Mass spec: m/z: 401 $[M+H]^+$, $^1H$ NMR (500 MHz, DMSO-$d_6$) δ ppm 10.58 (s, 1H), 9.30 (d, 1H, J = 1.0 Hz), 8.68 (d, 1H, J = 1.7 Hz), 8.25 (dd, 1H, J = 2.0, 8.6 Hz), 8.10 (d, 1H, J = 8.6 Hz), 7.88 (s, 1H), 7.5-7.5 (m, 1H), 7.4-7.5 (m, 1H), 3.31 (s, 1H), 3.0-3.1 (m, 1H), 2.74 (d, 6H, J = 1.2 Hz), 2.1-2.3 (m, 5H), 1.6-2.0 (m, 3H) |
| **12** | 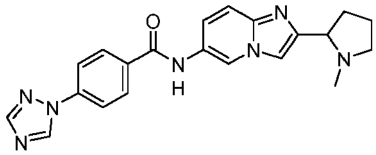 rac-N-[2-(1- methylpyrrolidin-2-yl) imidazo[1,2-a]pyridin-6-yl]-4-(1H-1,2,4-triazol-1-yl)benzamide | **4A** | **6A** | Mass spec: m/z: 388 $[M+H]^+$, $^1H$ NMR (500 MHz, DMSO-$d_6$) δ ppm 10.41 (s, 1 H) 9.45 (s, 1 H) 9.23 (d, J = 1.22 Hz, 1 H) 8.31 (s, 1 H) 8.18 (d, J = 8.80 Hz, 2 H) 8.07 (d, J = 8.80 Hz, 2 H) 7.87 (s, 1 H) 7.47 - 7.53 (m, 1 H) 7.40 (dd, J = 9.54, 1.96 Hz, 1 H) 3.26 - 3.30 (m, 1 H) 3.05 - 3.15 (m, 1 H) 2.23 - 2.30 (m, 1 H) 2.22 (s, 3 H) |
| **13** | 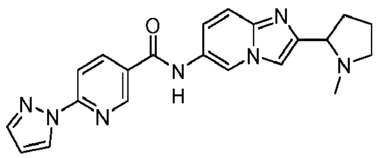 rac-N-[2-(1-methylpyrrolidin-2-yl) imidazo[1,2-a]pyridin-6-yl]-6-(1H-pyrazol-1-yl)pyridine-3-carboxamide | **4A** | **6A** | Mass spec: m/z: 388 [M+H] +, $^1H$ NMR (500 MHz, DMSO-$d_6$) δ ppm 10.52 (s, 1 H) 9.17 - 9.31 (m, 1 H) 8.98 - 9.09 (m, 1 H) 8.66 - 8.78 (m, 1 H) 8.53 (dd, J = 8.68, 2.32 Hz, 1 H) 8.08 (dd, J = 8.56, 0.73 Hz, 1 H) 7.90 - 7.93 (m, 1 H) 7.88 (s, 1 H) 7.50 (d, J = 9.54 Hz, 1 H) 7.38 (dd, J = 9.54, 1.96 Hz, 1 H) 6.57 - 6.73 (m, 1 H) 3.26 - 3.30 (m, 1 H) 3.09 (td, J = 8.31, 2.20 Hz, 1 H) 2.24 - 2.32 (m, 1 H) 2.22 (s, 3 H) 2.11 - 2.20 (m, 1 H) 1.86 - 1.93 (m, 1 H) 1.70 - 1.86 (m, 2H) |

(continued)

| Example Number | Example Name & Structure | Intermediates | Procedure and Chiral Purification method | NMR and Mass Spec |
|---|---|---|---|---|
| **14** | 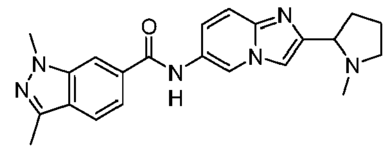 rac-1,3-dimethyl-N-[2-(1-methylpyrrolidin-2-yl)imidazo[1,2-a]pyridin-6- yl]-1H-indazole-6-carboxamide | **4A** | **6A** | Mass spec: m/z: 389 $[M+H]^+$, $^1H$ NMR (400 MHz, DMSO-$d_6$) δ ppm 10.39 (s, 1H), 9.24 (t, J = 1.5 Hz, 1H), 8.22 (d, J = 1.2 Hz, 1H), 7.90 - 7.81 (m, 2H), 7.68 (dd, J = 8.4, 1.4 Hz, 1H), 7.50 (d, J = 9.5 Hz, 1H), 7.41 (dd, J = 9.7, 2.0 Hz, 1H), 4.06 (s, 3H), 3.30 (s, 1H), 3.10 (t, J = 7.9 Hz, 1H), 2.53 (s, 3H), 2.30 (d, J = 18.9 Hz, 1H), 2.23 (s, 3H), 2.20 - 2.13 (m, 1H), 1.96 - 1.75 (m, 3H) |
| **15** | 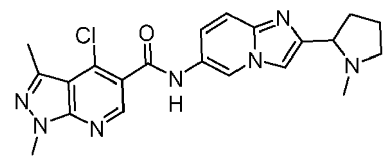 rac-4-chloro-1,3-dimethyl-N-[2-(1-methylpyrrolidin-2-yl)imidazo[1,2-a]pyridin-6-yl]-1H-pyrazolo[3,4-b]pyridine-5-carboxamide | **4A** | **6A** | Mass spec: m/z: 424 $[M+H]^+$, $^1H$ NMR (500 MHz, DMSO-$d_6$) δ ppm 1.68 - 1.96 (m, 3 H) 2.12 - 2.34 (m, 5 H) 2.69 (s, 3 H) 3.10 (br t, J = 7.46 Hz, 1 H) 3.31 (s, 1 H) 4.03 (s, 3 H) 7.23 (dd, J = 9.54, 1.96 Hz, 1 H) 7.50 (d, J = 9.54 Hz, 1 H) 7.91 (s, 1 H) 8.72 (s, 1 H) 9.28 (d, J = 1.47 Hz, 1 H) 10.72 (s, 1 H) |
| **16** | 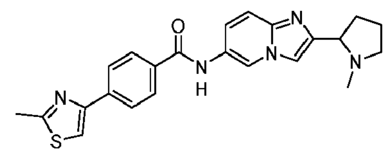 rac-4-(2-methyl-1,3-thiazol-4-yl)-N-[2-(1-methylpyrrolidin-2-yl)imidazo[1,2-a]pyridin-6-yl]benzamide | **4A** | **6A** | Mass spec: m/z: 418 $[M+H]^+$, $^1H$ NMR (600 MHz, DMSO-$d_6$) δ ppm 10.22 - 10.43 (m, 1 H) 9.24 (s, 1 H) 8.13 (s, 1 H) 8.08 - 8.12 (m, 2 H) 8.03 - 8.08 (m, 2 H) 7.88 (s, 1 H) 7.45 - 7.53 (m, 1 H) 7.41 (dd, J=9.68, 1.91 Hz, 1 H) 3.34 - 3.44 (m, 1 H) 3.08 - 3.17 (m, 1 H) 2.74 (s, 3 H) 2.12-2.37 (m, 3 H) 1.73 - 1.98 (m, 3 H) |
| **17** | 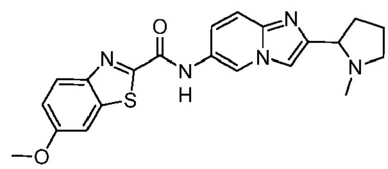 rac-6-methoxy-N-[2-(1-methylpyrrolidin-2-yl)imidazo[1,2-a]pyridin-6-yl]-1,3-benzothiazole-2-carboxamide | **4A** | **6A** | Mass spec: m/z: 408 $[M+H]^+$, $^1H$ NMR (600 MHz, DMSO-$d_6$) δ ppm 11.17 (s, 1 H) 9.24 (dd, J = 2.05, 0.88 Hz, 1 H) 8.09 (d, J = 8.95 Hz, 1 H) 7.82 - 7.88 (m, 2 H) 7.58 (dd, J = 9.54, 2.05 Hz, 1 H) 7.49 (dt, J = 9.57, 0.79 Hz, 1 H) 7.26 (dd, J = 9.10, 2.64 Hz, 1 H) 3.89 (s, 3 H) 3.27 - 3.30 (m, 1 H) 3.06 - 3.12 (m, 1 H) 2.13 - 2.29 (m, 5 H) 1.71 - 1.92 (m, 3 H) |

(continued)

| Example Number | Example Name & Structure | Intermediates | Procedure and Chiral Purification method | NMR and Mass Spec |
|---|---|---|---|---|
| **18** | 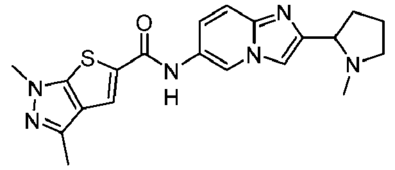 rac-1,3-dimethyl-N-[2-(1-methylpyrrolidin-2-yl)imidazo[1,2-a]pyridin-6-yl]-1H-thieno[2,3-c]pyrazole-5-carboxamide | **4A** | **6A** | Mass spec: m/z: 395 [M+H]+, $^{1}$H NMR (500 MHz, DMSO-$d_6$) δ ppm 10.40 (s, 1 H) 9.44 - 10.24 (m, 1 H) 9.24 - 9.36 (m, 1 H) 8.24 (s, 1 H) 8.06 (s, 1 H) 7.65 (d, J = 9.78 Hz, 1 H) 7.50 (dd, J = 9.78, 1.96 Hz, 1 H) 4.47 - 4.67 (m, 1 H) 3.90 (s, 3 H) 3.58 - 3.73 (m, 1 H) 3.16 - 3.26 (m, 1 H) 2.72 - 2.90 (m, 3 H) 2.41 (s, 4 H) 2.24 - 2.38 (m, 1 H) 2.04 - 2.23 (m, 2 H) |
| **19** | 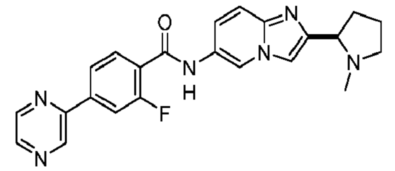 rel-2-fluoro-N-{ 2-[(2R)-1-methylpyrrolidin-2-yl]imidazo[1,2-a]pyridin-6-yl}-4-(pyrazin-2-yl)benzamide | **6B & 62** | **6A** | Mass spec: m/z: 417 [M+H]+, $^{1}$H NMR (500 MHz, DMSO-$d_6$) δ ppm 1.72 - 1.93 (m, 3 H) 2.12-2.20 (m, 1 H) 2.22 (s, 3 H) 2.26 (q, J = 8.88 Hz, 1 H) 3.03 - 3.15 (m, 1 H) 3.27 - 3.31 (m, 1 H) 7.28 (dd, J = 9.66, 2.08 Hz, 1 H) 7.49 (d, J = 9.54 Hz, 1 H) 7.86 - 7.90 (m, 2 H) 8.13 - 8.20 (m, 2 H) 8.72 (d, J = 2.69 Hz, 1 H) 8.80 (dd, J = 2.45, 1.71 Hz, 1 H) 9.26 (s, 1 H) 9.41 (d, J = 1.47 Hz, 1 H) 10.59 (s, 1 H) |
| **20** | 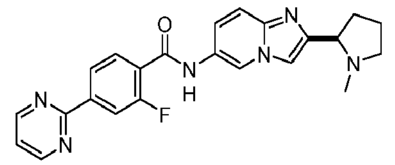 rel-2-fluoro-N-{2-[(2R)-1-methylpyrrolidin-2-yl]imidazo[1,2-a]pyridin-6-yl}-4-(pyrimidin-2-yl)benzamide | **6B & 67** | **6A** | Mass spec: m/z: 417 [M+H]+, $^{1}$H NMR (500 MHz, DMSO-$d_6$) δ ppm 1.73 - 1.92 (m, 3 H) 2.12-2.20 (m, 1 H) 2.22 (s, 3 H) 2.23 - 2.30 (m, 1 H) 3.07 - 3.11 (m, 1 H) 3.27 - 3.30 (m, 1 H) 7.28 (dd, J = 9.54, 1.96 Hz, 1 H) 7.49 (d, J = 9.54 Hz, 1 H) 7.56 (t, J = 4.89 Hz, 1 H) 7.86 - 7.90 (m, 2 H) 8.23 (dd, J = 11.49, 1.47 Hz, 1 H) 8.36 (dd, J = 8.07, 1.47 Hz, 1 H) 8.99 (d, J = 4.89 Hz, 2 H) 9.26 (d, J = 1.22 Hz, 1 H) 10.61 (s, 1 H) |

(continued)

| Example Number | Example Name & Structure | Intermediates | Procedure and Chiral Purification method | NMR and Mass Spec |
|---|---|---|---|---|
| **21** | 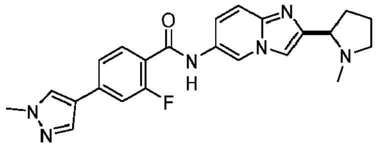 rel-2-fluoro-4-(1-methyl-1H-pyrazol-4-yl)-N-{2-[(2R)-1-methylpyrrolidin-2-yl] imidazo[1,2-a]pyridin-6-yl}benzamide | **6B** | **6A** | Mass spec: m/z: 419 $[M+H]^+$, $^1H$ NMR (500 MHz, DMSO-$d_6$) δ ppm 1.71 - 1.92 (m, 3 H) 2.11 - 2.20 (m, 1 H) 2.21 (s, 3 H) 2.22 - 2.28 (m, 1 H) 3.02 - 3.15 (m, 1 H) 3.27 -3.30 (m, 1 H) 3.88 (s, 3 H) 7.27 (dd, J = 9.66, 2.08 Hz, 1 H) 7.47 (d, J = 9.54 Hz, 1 H) 7.55 (dd, J = 7.95, 1.59 Hz, 1 H) 7.61 (dd, J = 11.74, 1.47 Hz, 1 H) 7.69 (t, J = 7.95 Hz, 1 H) 7.80 - 7.94 (m, 1 H) 7.97 - 8.10 (m, 1 H) 8.32 (s, 1 H) 9.22 (d, J = 0.98 Hz, 1 H) 10.36 (s, 1 H) |
| **22** | 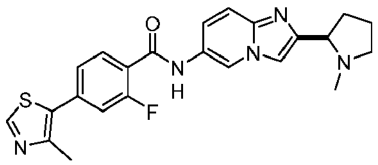 rel-2-fluoro-4-(4-methyl-1,3-thiazol-5-yl)-N-{2-[(2R)-1-methylpyrrolidin-2-yl] imidazo[1,2-a]pyridin-6-yl}benzamide | **6B & 66** | **6A** | Mass spec: m/z: 436 $[M+H]^+$, $^1H$ NMR (500 MHz, DMSO-$d_6$) δ ppm 10.55 (s, 1H), 9.25 (d, 1H, J = 1.2 Hz), 9.10 (s, 1H), 7.89 (s, 1H), 7.80 (t, 1H, J = 7.7 Hz), 7.56 (dd, 1H, J = 1.5, 11.0 Hz), 7.4-7.5 (m, 2H), 7.27 (dd, 1H, J = 2.1, 9.7 Hz), 3.31 (s, 1H), 3.0-3.2 (m, 1H), 2.53 (s, 3H), 2.0-2.4 (m, 5H), 1.7-2.0 (m, 3H) |
| **23** | 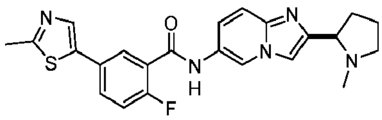 rel-2-fluoro-5-(2-methyl-1,3-thiazol-5-yl)-N-{2-[(2R)-1-methylpyrrolidin-2-yl] imidazo[1,2-a]pyridin-6-yl}benzamide | **6B & 64** | **6A** | Mass spec: m/z: 436 $[M+H]^+$, $^1H$ NMR (500 MHz, DMSO-$d_6$) δ ppm 10.60 (s, 1H), 9.24 (d, 1H, J = 1.2 Hz), 8.11 (s, 1H), 7.9-8.0 (m, 2H), 7.82 (ddd, 1H, J = 2.4, 4.6, 8.6 Hz), 7.49 (d, 1H, J = 9.5 Hz), 7.45 (dd, 1H, J = 8.7, 9.7 Hz), 7.27 (dd, 1H, J = 2.1, 9.7 Hz), 3.31 (s, 1H), 3.0-3.2 (m, 1H), 2.69 (s, 3H), 2.1-2.4 (m, 5H), 1.6-2.0 (m, 3H) |

(continued)

| Example Number | Example Name & Structure | Intermediates | Procedure and Chiral Purification method | NMR and Mass Spec |
|---|---|---|---|---|
| **24** | 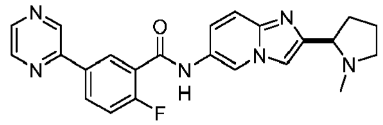 rel-2-fluoro-N-{ 2-[(2R)-1-methylpyrrolidin-2-yl]imidazo[1,2-a] pyridin-6-yl}-5-(pyrazin-2-yl) benzamide | **6B & 65** | **6A** | Mass spec: m/z: 417 $[M+H]^+$, $^1$H NMR (600 MHz, DMSO-$d_6$) δ ppm 2.03 - 2.20 (m, 2 H) 2.24 - 2.36 (m, 1 H) 2.43 - 2.48 (m, 1 H) 2.62 - 2.95 (m, 3 H) 3.19 (br s, 1 H) 3.46 - 3.69 (m, 1 H) 4.36 - 4.75 (m, 1 H) 7.42 (dd, J = 9.61, 1.98 Hz, 1 H) 7.58 (dd, J = 9.61, 8.88 Hz, 1 H) 7.64 (d, J = 9.68 Hz, 1 H) 8.26 (s, 1 H) 8.39 (ddd, J = 8.69, 4.88, 2.42 Hz, 1 H) 8.47 (dd, J = 6.75, 2.35 Hz, 1 H) 8.67 (d, J = 2.35 Hz, 1 H) 8.76 (dd, J = 2.49, 1.47 Hz, 1 H) 9.36 (d, J = 1.61 Hz, 1 H) 9.40 - 9.42 (m, 1 H) 9.56 - 10.22 (m, 1 H) 10.75 (s, 1 H) |
| **25** | 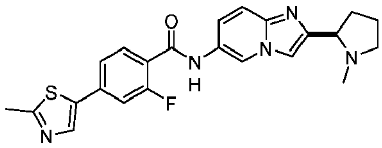 rel-2-fluoro-4-(2-methyl-1,3-thiazol-5-yl)-N-{2-[(2R)-1-methylpyrrolidin-2-yl] imidazo[1,2-a]pyridin-6-yl}benzamide | **6B & 61** | **6A** | Mass spec: m/z: 436 $[M+H]^+$, $^1$H NMR (600 MHz, DMSO-$d_6$) δ ppm 1.74 - 1.79 (m, 1 H) 1.79 - 1.91 (m, 2 H) 2.13 - 2.21 (m, 1 H) 2.22 (s, 3 H) 2.23 - 2.30 (m, 1 H) 2.71 (s, 3 H) 3.09 (br t, J = 7.04 Hz, 1 H) 7.26 (dd, J = 9.61, 1.98 Hz, 1 H) 7.48 (d, J = 9.54 Hz, 1 H) 7.53 - 7.59 (m, 1 H) 7.59 - 7.64 (m, 1 H) 7.68 - 7.80 (m, 2 H) 7.87 (s, 1 H) 8.25 (s, 1 H) 9.23 (s, 1 H) 10.48 (s, 1 H) |
| **26** | 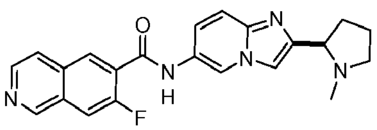 7-fluoro-N-[2-[rel-(2R)-1-methylpyrrolidin-2-yl]imidazo[1,2-a] pyridin-6-yl]isoquinoline-6-carboxamide | **6B** | **6A** | Mass spec: m/z: 390 [M+H] +, $^1$H NMR (600 MHz, DMSO-$d_6$) δ ppm 10.80 (s, 1 H) 9.40 (s, 1 H) 9.28 (dd, J = 1.98, 0.81 Hz, 1 H) 8.60 (d, J = 5.58 Hz, 1 H) 8.42 (d, J = 6.75 Hz, 1 H) 8.12 (d, J = 10.27 Hz, 1 H) 8.00 (d, J = 5.87 Hz, 1 H) 7.91 (s, 1 H) 7.50 (d, J = 9.54 Hz, 1 H) 7.27 (dd, J = 9.54, 2.05 Hz, 1 H) 3.25 - 3.29 (m, 1 H) 2.98 - 3.17 (m, 1 H) 2.26 - 2.33 (m, 1 H) 2.23 (s, 3 H) 2.13-2.21 (m, 1 H) 1.86 - 1.94 (m, 1 H) 1.68 - 1.86 (m, 2 H) |

(continued)

| Example Number | Example Name & Structure | Intermediates | Procedure and Chiral Purification method | NMR and Mass Spec |
|---|---|---|---|---|
| **27** | 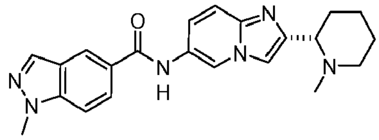 rel-1-methyl-N-{2-[(2S)-1-methylpiperidin-2-yl]imidazo[1,2-a] pyridin-6-yl}-1H-indazole-5-carboxamide | **15** | **6A** then purification method C $1^{st}$ eluting isomer (rt = 11.8 min) | Mass spec: m/z: 389 $[M+H]^+$, $^1H$ NMR (600 MHz, DMSO-$d_6$) $\delta$ ppm 1.29 - 1.37 (m, 1 H) 1.56 - 1.69 (m, 3 H) 1.72 - 1.79 (m, 2 H) 2.00 (br s, 3 H) 2.03 - 2.14 (m, 1 H) 2.93 (br d, J = 9.10 Hz, 1 H) 2.98 - 3.13 (m, 1 H) 4.10 (s, 3 H) 7.41 (dd, J = 9.54, 1.91 Hz, 1 H) 7.48 (d, J = 9.54 Hz, 1 H) 7.77 (d, J = 8.67 Hz, 1 H) 7.86 (s, 1 H) 8.01 (dd, J = 8.88, 1.69 Hz, 1 H) 8.26 (d, J = 0.88 Hz, 1 H) 8.49 (dd, J = 1.61, 0.88 Hz, 1 H) 9.25 (d, J = 0.59 Hz, 1 H) 10.32 (s, 1 H) |
| **28** | 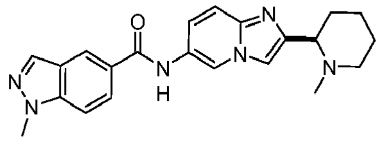 rel-1 -methyl-N- { 2-[(2R)-1-methylpiperidin-2-yl]imidazo[1,2-a] pyridin-6-yl}-1H-indazole-5-carboxamide | **15** | **6A** then purification method C $2^{nd}$ eluting isomer (rt = 15 min) | Mass spec: m/z: 389 [M+H] +, $^1H$ NMR (600 MHz, DMSO-$d_6$) $\delta$ ppm 10.32 (s, 1H), 9.25 (d, 1H, J = 0.6 Hz), 8.4-8.6 (m, 1H), 8.26 (d, 1H, J = 0.9 Hz), 8.01 (dd, 1H, J = 1.7, 8.9 Hz), 7.86 (s, 1H), 7.78 (d, 1H, J = 8.8 Hz), 7.5-7.5 (m, 1H), 7.41 (dd, 1H, J = 2.0, 9.6 Hz), 4.10 (s, 3H), 2.8-3.2 (m, 2H), 1.9-2.2 (m, 4H), 1.5-1.8 (m, 5H), 1.3-1.4 (m, 1H) |
| **29** | 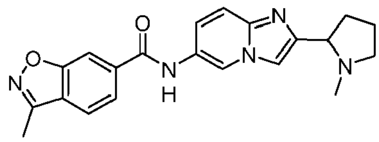 rac-3-methyl-N-[2-(1-methylpyrrolidin-2-yl)imidazo[1,2-a]pyridin-6-yl]-1,2-benzoxazole-6-carboxamide | **4A** | **6B** | Mass spec: m/z: 376 $[M+H]^+$, $^1H$ NMR (600 MHz, DMSO-$d_6$) $\delta$ ppm 1.72 - 1.94 (m, 3 H) 2.13 - 2.20 (m, 1 H) 2.22 (s, 3 H) 2.25 (q, *J* = 8.7 Hz, 1 H) 2.57 - 2.67 (m, 3 H) 3.04 - 3.14 (m, 1 H) 3.27 - 3.30 (m, 1 H) 7.40 (dd, J = 9.5, 2.05 Hz, 1 H) 7.50 (d, *J* = 9.5 Hz, 1 H) 7.87 (s, 1 H) 7.97 (dd, J = 8.2, 1.3 Hz, 1 H) 8.03 (dd, J = 8.2, 0.6 Hz, 1 H) 8.30 (s, 1 H) 9.15 - 9.31 (m, 1 H) 10.52 (s, 1 H) |

(continued)

| Example Number | Example Name & Structure | Intermediates | Procedure and Chiral Purification method | NMR and Mass Spec |
|---|---|---|---|---|
| **30** | 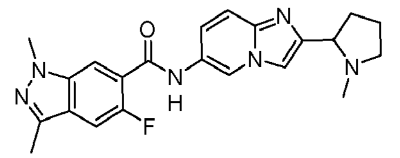 rac-5-fluoro-1,3-dimethyl-N-[2-(1-methylpyrrolidin-2-yl)imidazo[1,2-a]pyridin-6-yl]indazole-6-carboxamide | **4A & 54** | **6B** | Mass spec: m/z: 406 $[M+H]^+$, $^1H$ NMR (600 MHz, DMSO-$d_6$) δ ppm 1.75 - 2.48 (m, 8 H) 2.50 (s, 3 H) 2.53 - 2.92 (m, 2 H) 4.03 (s, 3 H) 7.33 (br d, J = 8.8 Hz, 1 H) 7.55 (br d, J = 9.4 Hz, 1 H) 7.69 (d, J = 10.1 Hz, 1 H) 7.97 (d, J = 5.3 Hz, 1 H) 7.99 - 8.10 (m, 1 H) 9.32 (brs, 1 H) 10.62 (s, 1 H) |
| **31** | 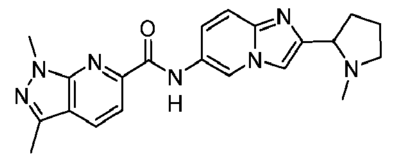 rac-1,3-dimethyl-N-[2-(1-methylpyrrolidin-2-yl)imidazo[1,2-a]pyridin-6-yl]-1H-pyrazolo[3,4-b]pyridine-6-carboxamide | **4A & 74** | **6C** | Mass spec: m/z: 390 $[M+H]^+$, $^1H$ NMR (300 MHz, MeOH-$d_4$) δ 9.35 (d, J = 8.7 Hz, 1H), 8.37 (d, J = 8.1 Hz, 1H), 8.04 (d, J = 8.4 Hz, 1H), 7.84 (s, 1H), 7.52 - 7.61 (m, 2H), 4.19 (s, 3H), 3.46 - 3.54 (m, 1H), 3.30 - 3.32 (m, 1H), 2.60 (s, 3H), 2.36 - 2.46 (m, 5H), 1.94 - 2.27 (m, 3H) |
| **32** | 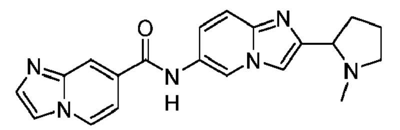 rac-N-[2-(1-methylpyrrolidin-2-yl)imidazo[1,2-a]pyridin-6-yl]imidazo[1,2-a]pyridine-7-carboxamide | **4A** | **6C** | Mass spec: m/z: 361 $[M+H]^+$, $^1H$ NMR (300 MHz, MeOH-$d_4$) δ ppm 9.31 (s, 1H), 8.47 (s, 1H), 8.33 (t, J = 8.4 Hz, 2H), 8.05 (s, 1H), 7.68 (s, 1H), 7.59 (d, J = 9.9 Hz, 1H), 7.48 - 7.52 (m, 1H), 7.19 - 7.22 (m, 1H), 4.41 (t, J = 8.7 Hz, 1H), 3.62 - 3.74 (m, 1H), 3.14 - 3.20 (m, 1H), 2.79 (s, 3H), 2.36 - 2.65 (m, 2H), 2.26 - 2.29 (m, 2H) |
| **33** | 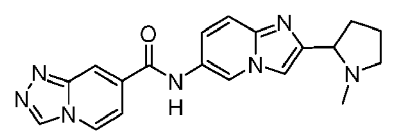 rac-N-[2-(1-methylpyrrolidin-2-yl)imidazo[1,2-a]pyridin-6-yl]-[1,2,4]triazolo[4,3-alpyridine-7-carboxamide | **4A** | **6C** | Mass spec: m/z: 362 $[M+H]^+$, $^1H$ NMR (300 MHz, MeOH-$d_4$) δ ppm 9.33 (d, J = 12.9 Hz, 2H), 8.63 (d, J = 6.9 Hz, 1H), 8.42 (s, 2H), 8.01 (s, 1H), 7.49 - 7.60 (m, 3H), 4.17 (t, J = 7.8 Hz, 1H), 3.50 - 3.59 (m, 1H), 2.95 - 3.07 (m, 1H), 2.68 (s, 3H), 2.38 - 2.56 (m, 1H), 2.27 - 2.36 (m, 1H), 2.20 - 2.22 (m, 2H) |

(continued)

| Example Number | Example Name & Structure | Intermediates | Procedure and Chiral Purification method | NMR and Mass Spec |
|---|---|---|---|---|
| **34** | 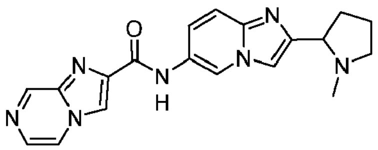 rac-N-[2-(1-methylpyrrolidin-2-yl) imidazo[1,2-a]pyridin-6-yl]imidazo[1,2-a]pyrazine-2-carboxamide | **4A** | **6D** | Mass spec: m/z: 362 $[M+H]^+$, $^1H$ NMR (400 MHz, MeOH-$d_4$) δ 9.30 (s, 1H), 9.17 (s, 1H), 8.57 - 8.61 (m, 2H), 7.97 (d, J = 4.4 Hz, 1H), 7.85 (s, 1H), 7.54 (d, J = 1.6 Hz, 2H), 3.40 - 3.49 (m, 1H), 3.20 - 3.32 (m, 1H), 2.42 - 2.51 (m, 1H), 2.30 - 2.37 (m, 4H), 2.04 - 2.06 (m, 3H) |
| **35** | 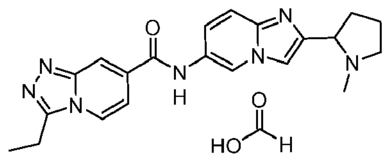 rac-3-ethyl-N-[2-(1-methylpyrrolidin-2-yl)imidazo[1,2-a]pyridin-6-yl]-[1,2,4] triazolo[4,3-a]pyridine-7-carboxamide formate | **4A** | **6D** | Mass spec: m/z: 390 $[M+H]^+$, $^1H$ NMR (300 MHz, MeOH-$d_4$) δ 9.30 (s, 1H), 8.42 - 8.55 (m, 2H), 8.32 (s, 1H), 8.07 (s, 1H), 7.48 - 7.56 (m, 3H), 4.43 - 4.46 (m, 1H), 3.68 - 3.76 (m, 1H), 3.15 - 3.28 (m, 3H), 2.84 (s, 3H), 2.32 - 2.62 (m, 2H), 2.20 - 2.30 (m, 2H), 1.45 - 1.50 (m, 3H) |
| **36** | 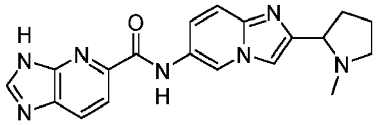 rac-N-[2-(1-methylpyrrolidin-2-yl) imidazo[1,2-a]pyridin-6-yl]-3H-imidazo [4,5-b]pyridine-5-carboxamide | **4A** | **6D** | Mass spec: m/z: 362 $[M+H]^+$, $^1H$ NMR (300 MHz, MeOH-$d_4$) δ ppm 9.40 (s, 1H), 8.62 (s, 1H), 8.52 (s, 1H), 8.19 - 8.27 (m, 2H), 8.02 (s, 1H), 7.57 - 7.66 (m, 2H), 4.20 (t, J = 8.4 Hz, 1H), 3.53 - 3.60 (m, 1H), 2.97 - 3.06 (m, 1H), 2.65 - 2.73 (m, 3H), 2.41 - 2.57 (m, 1H), 2.31 - 2.38 (m, 1H), 2.21 - 2.28 (m, 2H) |
| **37** | 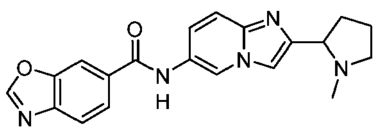 rac-N-[2-(1-methylpyrrolidin-2-yl) imidazo[1,2-a]pyridin-6-yl]-1,3-benzoxazole-6-carboxamide | **4A** | **6D** | Mass spec: m/z: 362 $[M+H]^+$, $^1H$ NMR (300 MHz, DMSO-$d_6$) δ ppm 10.45 (s, 1H), 9.27 (s, 1H), 8.95 (s, 1H), 8.42 (s, 1H), 8.06 - 8.06 (m, 1H), 7.97 - 7.99 (m, 1H), 7.89 (s, 1H), 7.43 - 7.50 (m, 2H), 3.10 - 3.16 (m, 1H), 2.13 - 2.40 (m, 6H), 1.75 - 1.95 (m, 3H) |

(continued)

| Example Number | Example Name & Structure | Intermediates | Procedure and Chiral Purification method | NMR and Mass Spec |
|---|---|---|---|---|
| **38** | 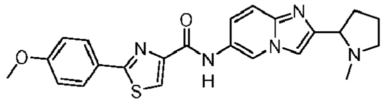 rac-2-(4-methoxyphenyl)-N-[2-(1-methylpyrrolidin-2-yl)imidazo[1,2-a] pyridin-6-yl]-1,3-thiazole-4-carboxamide | **4A** | **6D** | Mass spec: m/z: 434 $[M+H]^+$, $^1H$ NMR (300 MHz, MeOH-$d_4$) δ ppm 9.27 (s, 1H), 8.28 (s, 1H), 8.06 (d, J = 8.7 Hz, 2H), 7.90 (s, 1H), 7.52 - 7.58 (m, 2H), 7.07 (d, J = 9.0 Hz, 2H), 3.88 (s, 3H), 3.70 - 3.80 (m, 1H), 3.29 - 3.35 (m, 1H), 2.62 - 2.70 (m, 1H), 2.50 (s, 3H), 2.37 - 2.48 (m, 1H), 2.02 - 2.23 (m, 3H) |
| **39** | 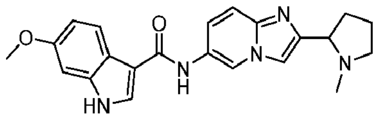 rac-6-methoxy-N-[2-(1-methylpyrrolidin-2-yl)imidazo[1,2-a] pyridin-6-yl]-1H-indole-3-carboxamide | **4A & 98** | **6E** | Mass spec: m/z: 390 $[M+H]^+$, $^1H$ NMR (300 MHz, MeOH-$d_4$): δ ppm 9.25 (s, 1H), 8.01 - 8.07 (m, 3H), 7.46 - 7.56 (m, 2H), 6.98 (s, 1H), 6.85 (d, J = 8.7 Hz, 1H), 4.32 - 4.86 (m, 1H), 2.84 (s, 3H), 3.60 - 3.68 (m, 1H), 3.12 - 3.19 (m, 1H), 2.77 (s, 3H), 2.34 - 2.57 (m, 2H), 2.17 - 2.27 (m, 2H) |
| **40** | 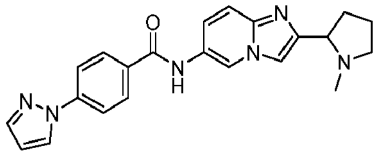 rac-N-[2-(1-methylpyrrolidin-2-yl) imidazo[1,2-a]pyridin-6-yl]-4-(1H-pyrazol-1-yl)benzamide | **4A & 99** | **6E** | Mass spec: m/z: 387 $[M+H]^+$, $^1H$ NMR (300 MHz, MeOH-$d_4$): δ ppm 9.33 (s, 1H), 8.37 (d, J = 2.4Hz, 1H), 8.07 - 8.12 (m, 3H), 7.94 - 7.97 (m, 2H), 7.79 (d, J = 1.5Hz, 1H), 7.50 - 7.61 (m, 2H), 6.59 (t, J = 2.1Hz, 1H), 4.44 - 4.50 (m, 1H), 3.65 - 3.73 (m, 1H), 3.18 -3.27 (m, 1H), 2.82 (s, 3H), 2.31 - 2.60 (m, 2H), 2.20 - 2.28 (m, 2H) |
| **41** | 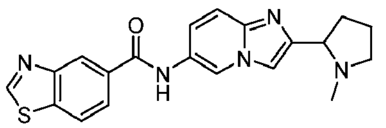 rac- N-[2-(1-methylpyrrolidin-2-yl) imidazo[1,2-a]pyridin-6-yl]-1,3-benzothiazole-5-carboxamide | **4A & 100** | **6E** | Mass spec: m/z: 378 $[M+H]^+$, $^1H$ NMR (300 MHz, MeOH-$d_4$): δ (ppm) 9.38 (s, 2H), 8.69 (s, 1H), 8.25 (d, J = 8.4 Hz, 1H), 8.07 - 8.11 (m, 2H), 7.54 - 7.63 (m, 2H), 4.57 - 4.63 (m, 1H), 3.64 - 3.79 (m, 1H), 3.30 - 3.38 (m, 1H), 2.88 (s, 3H), 2.43 - 2.66 (m, 2H), 2.20 - 2.35 (m, 2H) |

(continued)

| Example Number | Example Name & Structure | Intermediates | Procedure and Chiral Purification method | NMR and Mass Spec |
|---|---|---|---|---|
| **42** | 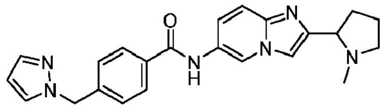<br>rac-N-[2-(1-methylpyrrolidin-2-yl) imidazo[1,2-a]pyridin-6-yl]-4-[(1H-pyrazol-1-yl)methyl]benzamide | **4A & 101** | **6E** | Mass spec: m/z: 401 $[M+H]^+$, $^1H$ NMR 300 MHz, MeOH-$d_4$): δ ppm 9.19 (s, 1H), 7.91 - 7.98 (m, 2H), 7.80 (s, 1H), 7.76 (d, J = 2.1 Hz, 1H), 7.55 (d, J = 1.5 Hz, 1H), 7.39 - 7.50 (m, 2H), 7.31 - 7.34 (m, 2H), 7.37 (t, J = 2.1 Hz, 1H), 5.46 (s, 2H), 3.44 - 3.49 (m, 1H), 3.21 - 3.24 (m, 1H), 2.39 - 2.48 (m, 1H), 2.34 (s, 3H), 2.29 - 2.30 (m, 1H), 1.80 - 2.10 (m, 3H) |
| **43** | 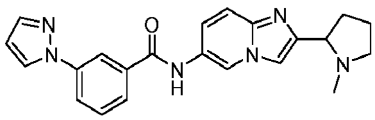<br>rac-N-[2-(1-methylpyrrolidin-2-yl) imidazo[1,2-a]pyridin-6-yl]-3-(1H-pyrazol-1-yl)benzamide | **4A & 102** | **6E** | Mass spec: m/z: 387 $[M+H]^+$, $^1H$ NMR (300 MHz, MeOH-$d_4$): δ ppm 9.43 (s, 1H), 8.34 - 8.38 (m, 2H), 8.20 (s, 1H), 7.98 - 8.02 (m, 1H), 7.90 - 7.93 (m, 1H), 7.78 (s, 1H), 7.64 - 7.69 (m, 3H), 6.59 (s, 1H), 4.70 - 4.80 (m, 1H), 3.81 - 3.89 (m, 1H), 3.32 - 3.89 (m, 1H), 2.93 (s, 3H), 2.41 - 2.69 (m, 2H), 2.30 - 2.37 (m, 2H) |
| **44** | 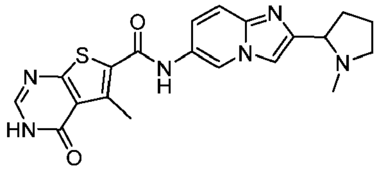<br>rac-5-methyl-N-[2-(1-methylpyrrolidin-2-yl)imidazo[1,2-a]pyridin-6-yl]-4-oxo-3H,4H-thieno[2,3-d]pyrimidine-6-carboxamide | **4A & 103** | **6E** | Mass spec: m/z: 409 $[M+H]^+$, $^1H$ NMR (400 MHz, MeOH-$d_4$): δ ppm 9.28 (s, 1H), 8.52 (br, 1H), 8.07 - 8.15 (m, 2H), 7.59 - 7.62 (m, 1H), 7.47 - 7.48 (m, 1H), 4.38 - 4.40 (m, 1H), 3.64 - 3.68 (m, 1H), 3.16 - 3.20 (m, 1H), 2.80 - 2.86 (m, 6H), 2.40 - 2.56 (m, 2H), 2.20 - 2.28 (m, 2H) |
| **45** | 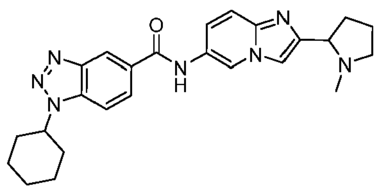<br>rac-1-cyclohexyl-N-[2-(1-methylpyrrolidin-2-yl)imidazo[1,2-a] pyridin-6-yl]-1H-1,2,3-benzotriazole-5-carboxamide | **4A & 104** | **6E** | Mass spec: m/z: 444 $[M+H]^+$, $^1H$ NMR (300 MHz, MeOH-$d_4$): δ ppm 9.30 (s, 1H), 8.65 (s, 1H), 8.13 - 8.16 (m, 1H), 7.98 - 7.98 (m, 2H), 7.49 - 7.58 (m, 2H), 4.87 - 4.92 (m, 1H), 4.01 - 4.05 (m, 1H), 3.47 - 3.53 (m, 1H), 2.91 - 2.97 (m, 1H), 2.64 (s, 3H), 2.45 - 2.49 (m, 1H), 2.04 - 2.33 (m, 9H), 1.42 - 1.86 (m, 4H) |

(continued)

| Example Number | Example Name & Structure | Intermediates | Procedure and Chiral Purification method | NMR and Mass Spec |
|---|---|---|---|---|
| **46** | 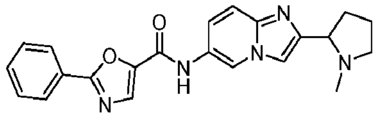 rac-N-[2-(1-methylpyrrolidin-2-yl) imidazo[1,2-a]pyridin-6-yl]-2-phenyl-1,3-oxazole-5-carboxamide | **4A & 105** | **6E** | Mass spec: m/z: 388 $[M+H]^+$, $^1H$ NMR (300 MHz, MeOH-$d_4$): δ ppm 9.23 (s, 1H), 8.21 - 8.24 (m, 2H), 8.05 (s, 1H), 7.86 (s, 1H), 7.47 - 7.77 (m, 5H), 3.54 - 3.59 (m, 1H), 2.50 - 2.53 (m, 1H), 2.21 - 2.39 (m, 4H), 1.97 - 2.17 (m, 4H) |
| **47** | 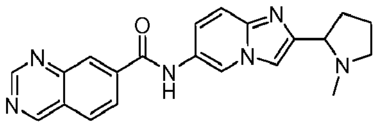 rac-N-[2-(1-methylpyrrolidin-2-yl) imidazo[1,2-a]pyridin-6-yl]quinazoline-7-carboxamide | **4A & 106** | **6E** | Mass spec: m/z: 373 $[M+H]^+$, $^1H$ NMR (300 MHz, MeOH-$d_4$) δ ppm 9.50 (s, 1H), 8.70 (s, 1H), 8.19 (s, 1H), 8.03 - 8.06 (m, 1H), 7.90 (s, 1H), 7.59 - 7.74 (m, 3H), 6.27 (s, 1H), 4.68 (br, 1H), 3.80 (br, 1H), 3.30 - 3.40 (m, 1H), 2.92 (s, 3H), 2.24 - 2.69 (m, 2H), 2.03 - 2.34 (m, 2H) |
| **48** | 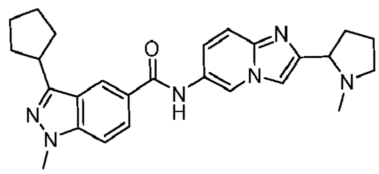 rac-3-cyclopentyl-1-methyl-N-[2-(1-methylpyrrolidin-2-yl)imidazo[1,2-a] pyridin-6-yl]-1H-indazole-5-carboxamide | **4A & 86** | **6E** | Mass spec: m/z: 443 $[M+H]^+$, $^1H$ NMR (300 MHz, MeOH-$d_4$): δ ppm 9.40 (s, 1H), 8.48 (s, 1H), 8.20(s, 1H), 8.00 - 8.04 (m, 1H), 7.66 (s, 2H), 7.59 (d, J = 8.7 Hz, 1H), 4.70 (br, 1H), 4.04 (s, 3H), 3.80 (br, 1H), 3.50 - 3.59 (m, 1H), 3.30 - 3.32 (m, 1H), 2.93 (br, 3H), 2.46 - 2.67 (m, 2H), 2.18 - 2.37 (m, 4H), 1.99 - 2.05 (m, 4H), 1.70 - 1.85 (m, 2H) |
| **49** | 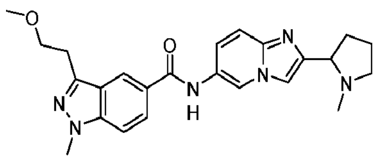 rac-3-(2-methoxyethyl)-1-methyl-N-[2-(1-methylpyrrolidin-2-yl)imidazo [1,2-a]pyridin-6-yl]-1H-indazole-5-carboxamide | **4A & 82** | **6C** | Mass spec: m/z: 433 $[M+H]^+$, $^1H$ NMR (400 MHz, MeOH-$d_4$) δ ppm 9.24 (s, 1H), 8.51 (s, 1H), 8.05 - 8.10 (m, 1H), 7.84 (s, 1H), 7.64 - 7.65 (m, 1H), 7.50 - 7.52 (m, 2H), 4.08 (s, 3H), 3.84 - 3.87 (m, 2H), 3.45 - 3.52 (m, 1H), 3.39 (s, 3H), 3.15 - 3.30 (m, 3H), 2.30 - 2.50 (m, 5H), 1.90 - 2.15 (m, 3H) |

(continued)

| Example Number | Example Name & Structure | Intermediates | Procedure and Chiral Purification method | NMR and Mass Spec |
|---|---|---|---|---|
| **50** | 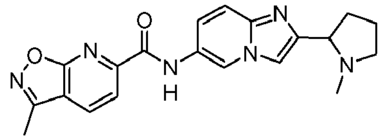 rac-3-methyl-N-[2-(1-methylpyrrolidin-2-yl)imidazo[1,2-a]pyridin-6-yl]-[1,2]oxazolo[5,4-b]pyridine-6-carboxamide | **4A & 92** | **6D** | Mass spec: m/z: 377 $[M+H]^+$, $^1H$ NMR (300 MHz, MeOH-$d_4$) δ ppm 9.42 (s, 1H), 8.53 (d, J = 8.1 Hz, 1H), 8.31 (d, J = 8.1 Hz, 1H), 8.11 (s, 1H), 7.59 - 7.70 (m, 2H), 4.55 - 4.60 (m, 1H), 3.70 - 3.78 (m, 1H), 3.30 - 3.32 (m, 1H), 2.87 (s, 3H), 2.75 (s, 3H), 2.41 - 2.66 (m, 2H), 2.21- 2.39 (m, 2H) |
| **51** | 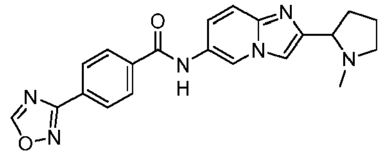 rac-N-[2-(1-methylpyrrolidin-2-yl)imidazo[1,2-a]pyridin-6-yl]-4-(1,2,4-oxadiazol-3-yl)benzamide | **4A & 95** | **6C** | Mass spec: m/z: 389 $[M+H]^+$, $^1H$ NMR (400 MHz, MeOH-$d_4$) δ 9.37 (s, 2H), 8.29 - 8.31 (m, 2H), 8.14 - 8.16 (m, 2H), 8.07 (s, 1H), 7.52 - 7.62 (m, 2H), 4.35 - 4.40 (m, 1H), 3.64 - 3.70 (m, 1H), 3.10 - 3.20 (m, 1H), 2.79 (s, 3H), 2.50 - 2.60 (m, 1H), 2.35 - 2.42 (m, 1H), 2.23 - 2.27 (m, 2H) |
| **52** | 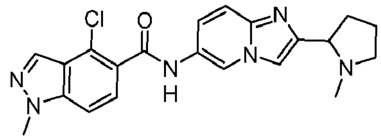 rac-4-chloro-1-methyl-N-[2-(1-methylpyrrolidin-2-yl)imidazo[1,2-a]pyridin-6-yl]-1H-indazole-5-carboxamide | **4A & 97** | **6C** | Mass spec: m/z: 409 $[M+H]^+$, $^1H$ NMR (400 MHz, MeOH-$d_4$) δ ppm 9.41 (s, 1H), 8.22 (s, 1H), 8.07 (s, 1H), 7.59 - 7.70 (m, 3H), 7.38 - 7.41 (m, 1H), 4.30 - 4.36 (m, 1H), 4.16 (s, 3H), 3.60 - 3.70 (m, 1H), 3.05 - 3.15 (m, 1H), 2.76 (s, 3H), 2.50 - 2.60 (m, 1H), 2.33 - 2.42 (m, 1H), 2.21 - 2.25 (m, 2H). |
| **53** | 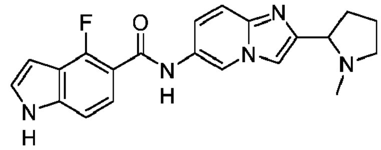 rac-4-fluoro-N-[2-(1-methylpyrrolidin-2-yl)imidazo[1,2-a]pyridin-6-yl]-1H-indole-5-carboxamide | **4A** | **6C** | Mass spec: m/z: 388 $[M+H]^+$, $^1H$ NMR (300 MHz, MeOH-$d_4$) δ ppm 9.57 (s, 1H), 8.36 (s, 1H), 7.78 (s, 2H), 7.540 (t, J = 7.2 Hz, 1H), 7.32 - 7.38 (m, 2H), 6.68 (d, J = 3.3 Hz, 1H), 4.60 - 4.76 (m, 1H), 3.84 (br, 1H), 3.40 - 3.54 (m, 1H), 2.98 (br, 3H), 2.60 - 2.78 (m, 1H), 2.50 - 2.58 (m, 1H), 2.32 - 2.47 (m, 2H) |

(continued)

| Example Number | Example Name & Structure | Intermediates | Procedure and Chiral Purification method | NMR and Mass Spec |
|---|---|---|---|---|
| **54** | 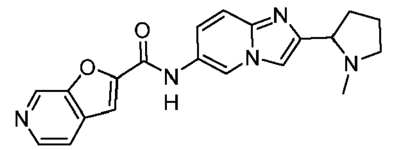 rac-N-[2-(1-methylpyrrolidin-2-yl) imidazo[1,2-a]pyridin-6-yl]furo[2,3-c] pyridine-2-carboxamide | **4A** | **6C** | Mass spec: m/z: 362 $[M+H]^+$, $^1H$ NMR (300 MHz, MeOH-$d_4$) δ ppm 9.35 (s, 1H), 9.05 (s, 1H), 8.49 - 8.51 (m, 1H), 8.03 (s, 1H), 7.88 - 7.90 (m, 1H), 7.77 - 7.78 (m, 1H), 7.60 - 7.61 (m, 2H), 4.15 - 4.25 (m, 1H), 3.52 - 3.60 (m, 1H), 2.95 - 3.05 (m, 1H), 2.70 (s, 3H), 2.48 - 2.53 (m, 1H), 2.29 - 2.36 (m, 1H), 2.13 - 2.20 (m, 2H) |
| **55** | 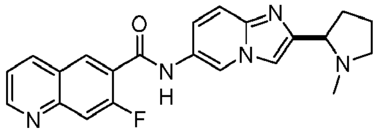 rel-7-fluoro-N-[2-[(2R)-1-methylpyrrolidin-2-yl]imidazo[1,2-a] pyridin-6-yl]quinoline-6-carboxamide | **6B** | **6A** | Mass spec: m/z: 390 $[M+H]^+$, $^1H$ NMR (600 MHz, DMSO-$d_6$) δ ppm 10.74 (s, 1 H) 9.30 (d, J = 0.88 Hz, 1 H) 9.02 (dd, J = 4.26, 1.76 Hz, 1 H) 8.56 (dt, J = 7.92, 0.88 Hz, 1 H) 8.46 (d, J = 7.78 Hz, 1 H) 7.93 (d, J = 11.30 Hz, 2 H) 7.63 (dd, J = 8.22, 4.25 Hz, 1 H) 7.51 (d, J = 9.54 Hz, 1 H) 7.29 (dd, J = 9.54, 1.91 Hz, 1 H) 3.35 - 3.54 (m, 1 H) 3.06 - 3.22 (m, 1 H) 2.11 - 2.42 (m, 5 H) 1.73 - 1.97 (m, 3 H) |

**Example 56 was synthesized as shown in Scheme 29.**

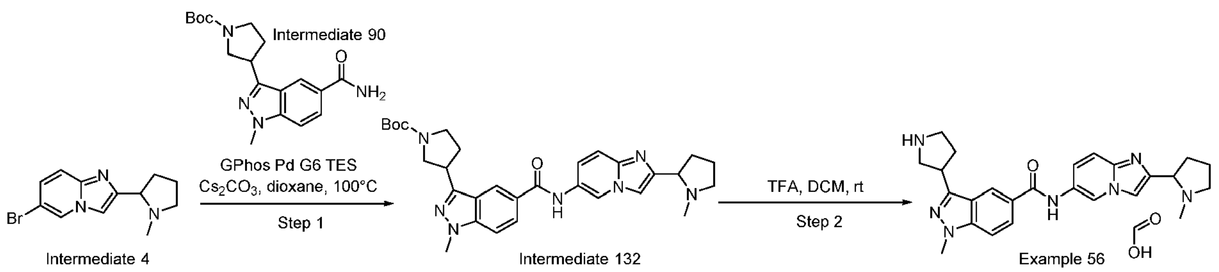


## Scheme 29

**Step 1**

**Intermediate 132: rac-tert-butyl 3-(1-methyl-5-((2-(1-methylpyrrolidin-2-yl)imidazo[1,2-a]pyridin-6-yl)carbamoyl)-1H-indazol-3-yl)pyrrolidine-1-carboxylate**

A solution of rac-tert-butyl 3-(5-carbamoyl-1-methylindazol-3-yl)pyrrolidine-1-carboxylate **(Intermediate 90**:

100 mg, 0.276 mmol), rac-2-{6-bromoimidazo[1,2-a]pyridin-2-yl}-1-methylpyrrolidine **(Intermediate 4**: 97.3 mg, 0.331 mmol), $Cs_2CO_3$ (180 mg, 0.552 mmol), GPhos Pd G6 (48.5 mg, 0.055 mmol) in dioxane (3 mL) was stirred for 3h at 100°C. The mixture was concentrated under reduced pressure. The crude material was purified by reverse phase flash chromatography (C18 Aq) using MeCN/water (55/45) as the eluent to afford rac-tert-butyl 3-(1-methyl-5-((2-(1-methylpyrrolidin-2-yl)imidazo[1,2-a]pyridin-6-yl)carbamoyl)-1H-indazol-3-yl)pyrrolidine-1-carboxylate **(Intermediate 132**: 30 mg, 18%) as a light-yellow oil. Mass spec: m/z 544 $[M+H]^+$.

**Step 2**

**Example 56: rac-1-methyl-N-(2-(1-methylpyrrolidin-2-yl)imidazo[1,2-a]pyridin-6-yl)-3-(pyrrolidin-3-yl)-1H-indazole-5-carboxamide formate**

To a solution of rac-tert-butyl 3-(1-methyl-5-((2-(1-methylpyrrolidin-2-yl)imidazo[1,2-a]pyridin-6-yl)carbamoyl)-1H-indazol-3-yl)pyrrolidine-1-carboxylate (**Intermediate 132**: 30 mg, 0.053 mmol) in DCM (5 mL) was added TFA (1 mL). The reaction mixture was stirred for 2h at rt, then the mixture was concentrated under reduced pressure. The residue was purified by preparative HPLC (Column: Xselect CSH C18 OBD Column 30x150 mm, 5 μm; Eluent: MeCN on water (0.1% Formic acid) from 3 to 20%; Flow rate: 60 mL/min) to rac-1-methyl-N-(2-(1-methylpyrrolidin-2-yl)imidazo[1,2-a]pyridin-6-yl)-3-(pyrrolidin-3-yl)-1H-indazole-5-carboxamide formate (**Example 56**: 2.5 mg, 10%) as a light-yellow solid. Mass spec: m/z 444 $[M+H]^+$. $^1H$ NMR (300 MHz, MeOH-$d_4$): δ ppm 9.31 (s, 1H), 8.52 (s, 1H), 8.04 - 8.12 (m, 2H), 7.61 - 7.68 (m, 1H), 7.52 - 7.57 (m, 2H), 4.32 - 4.35 (m, 1H), 4.14 - 4.29 (m, 1H), 4.08 (s, 3H), 3.76 - 3.78 (m, 2H), 3.51 - 3.76 (m, 4H), 3.01 - 3.13 (m, 1H), 2.75 (s, 3H), 2.51 - 2.66 (m, 2H), 2.39 - 2.48 (m, 2H), 2.10 - 2.24 (m, 2H).

**General Procedures 7A, 7B and 7C**

**Examples 57-65,** shown in **Table 2**, were prepared as shown **Scheme 30** and following the appropriate **General Procedure 7A**, **7B or 7C**. Where primary carboxamide intermediates are not indicated in **Table 1** these are available from commercial sources or well known in the art.

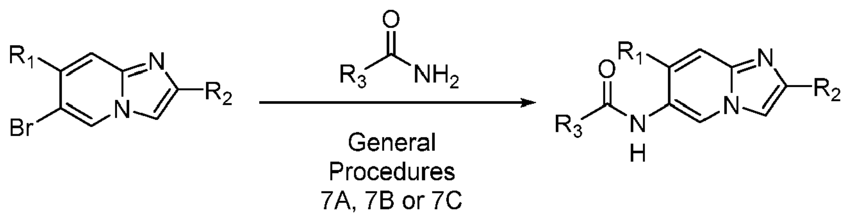


**Scheme 30**

**General procedure 7A**

A round-bottom flask was charged with the appropriate primary amide intermediate tabulated below or commercially available (1 eq), and appropriate aromatic bromides tabulated below (1.2 eq), $Cs_2CO_3$ (3 eq), 1,4-dioxane (0.05 M), Gphos Pd $G_6$ TES (0.1 eq), Gphos (0.2 eq) under nitrogen atmosphere. The reaction was stirred at 100°C until complete conversion of the amide was observed by LCMS or TLC, then cooled to rt and the reaction mixture was concentrated under reduced pressure. The crude was purified by preparative HPLC using a gradient of MeCN in water from 0% to 100% (0.1% formic acid) to afford the expected Example as shown in **Table 2.**

**General procedure 7B**

A round-bottom flask was charged with the appropriate primary amide intermediate tabulated below (1 eq), and appropriate aromatic bromides tabulated below (1.2 eq), $Cs_2CO_3$ (3 eq), 1,4-dioxane (0.2 M), Ephos Pd G4 (0.1 eq), Ephos (0.2 eq) under nitrogen atmosphere. The reaction was stirred at 100°C until complete conversion was observed by LCMS or TLC, then cooled to rt and the reaction mixture was concentrated under reduced pressure. The crude was purified by preparative HPLC using a gradient of MeCN in water from 0% to 100% (0.1% formic acid) to afford the expected Example. For some examples the racemic products were separated by the chiral preparative reverse phase HPLC chromatography method below and indicated in **Table 2**.

Chiral purification method by reverse phase HPLC chromatography:

Method A: Column: Chiralpak ID, 2×25 cm, 5 μm; Eluent: EtOH/(Hexane/DCM 3:1 (0.5% 2M $NH_3$-MeOH)) 15:85; Flow

rate: 20 mL/min.

**General procedure 7C**

A round-bottom flask was charged with the appropriate primary amide intermediate tabulated below (1.1 eq), and appropriate aromatic bromides tabulated below (1 eq), $Cs_2CO_3$ (3 eq), 1,4-dioxane (0.05 M), BrettPhos Pd G3 (0.1 eq), BrettPhos (0.2 eq) under nitrogen atmosphere. The reaction was stirred at 100°C until complete conversion was observed by LCMS or TLC, then cooled to rt and reaction mixture was concentrated under reduced pressure. The crude was purified by preparative HPLC using a gradient of MeCN in water from 0% to 100% (neutral, acid or basic condition) to afford the expected Example as shown in **Table 2.**

**Table 2**

| Example Number | Example Name & Structure | Intermediates | Procedure and Chiral Purification method | NMR and Mass Spec |
|---|---|---|---|---|
| **57** | 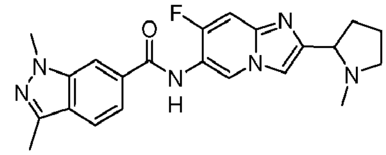 rac-N-[7-fluoro-2-(1-methylpyrrolidin-2-yl)imidazo[1,2-a]pyridin-6-yl]-1,3-dimethyl-1H-indazole-6-carboxamide | **10 & 52** | **7A** | Mass spec: m/z: 407 $[M+H]^+$, $^1H$ NMR (300 MHz, MeOH-$d_4$) δ ppm9.15 (d, J = 6.9Hz, 1H), 8.14 (d, J = 4.5 Hz, 2H), 7.81 - 7.85 (m, 1H), 7.69 - 7.72 (m, 1H), 7.48 (d, J = 10.5 Hz, 1H), 4.65 (br, 1H), 4.07 (s, 3H), 4.07 (br, 1H), 3.30 - 3.38 (m, 1H), 2.93 (br, 3H), 2.60 - 2.67 (m, 1H), 2.56 - 2.57 (m, 3H), 2.44 - 2.54 (m, 1H), 2.29 - 2.43 (m, 2H) |
| **58** | 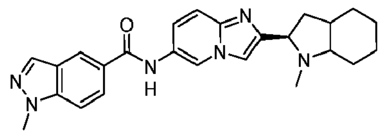 1-methyl-N-{2-[(2R)-1-methyl-octahydro-1H-indol-2-yl]imidazo[1,2-a]pyridin-6-yl}-1H-indazole-5-carboxamide | **21 & 49** | **7A** | Mass spec: m/z: 429 $[M+H]^+$, $^1H$ NMR (400 MHz, DMSO-$d_6$) δ ppm 10.34 (s, 1H), 9.22 (s, 1H), 8.50 (s, 1H), 8.27 (s, 1H), 8.00 - 8.01 (m, 1H), 7.79 -7.89 (m, 1H), 7.77 - 7.79 (m, 1H), 7.43 - 7.47 (m, 1H), 7.42 - 7.43 (m, 1H), 4.11 (s, 3H), 3.92-3.99 (m, 1H), 2.91-2.95 (m, 1H), 2.31-2.38 (m, 1H), 2.06 - 2.16 (m, 4H), 1.86 - 1.92 (m, 1H), 1.54 - 1.70 (m, 4H), 1.30 - 1.54 (m, 3H), 1.08 - 1.20 (m, 1H) |
| **59** | 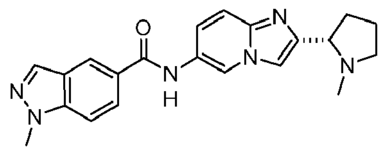 rel-1-methyl-N-{2-[(2S)-1-methylpyrrolidin-2-yl]imidazo[1,2-a] pyridin-6-yl}-1H-indazole-5-carboxamide | **4 & 49** | **7B** then purification method A 1st eluting isomer (rt = 6.7 min) | Mass spec: m/z: 375 $[M+H]^+$, $^1H$ NMR (300 MHz, MeOH-$d_4$) δ ppm 9.25 (s, 1H), 8.44 (s, 1H), 8.15 (s, 1H), 8.01 (d, J = 8.7 Hz, 1H), 7.99 (s, 1H), 7.65 (d, J = 9.0 Hz, 1H), 7.46 - 7.55 (m, 2H), 4.10 (s, 3H), 4.04 - 4.08 (m, 1H), 3.45 - 3.53 (m, 1H), 2.85 - 2.94 (m, 1H), 2.62 (s, 3H), 2.44 - 2.48 (m, 1H), 2.09 - 2.31 (m, 3H) |

(continued)

| Example Number | Example Name & Structure | Intermediates | Procedure and Chiral Purification method | NMR and Mass Spec |
|---|---|---|---|---|
| **60** | 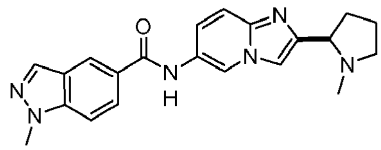 rel-1-methyl-N-{2-[(2R)-1-methylpyrrolidin-2-yl]imidazo[1,2-a] pyridin-6-yl}-1H-indazole-5-carboxamide | **4 & 49** | **7B** then purification method A 2nd eluting isomer (rt = 12.3 min) | Mass spec: m/z: 375 $[M+H]^+$, $^1H$ NMR (300 MHz, MeOH-$d_4$) δ ppm 9.25 (s, 1H), 8.44 (s, 1H), 8.16 (s, 1H), 8.02 (d, J = 9.0 Hz, 1H), 7.94 (s, 1H), 7.66 (d, J = 8.7 Hz, 1H), 7.46 - 7.55 (m, 2H), 4.11 (s, 3H), 4.02 - 4.05 (m, 1H), 3.44 - 3.52 (m, 1H), 2.83 - 2.92 (m, 1H), 2.61 (s, 3H), 2.43 - 2.48 (m, 1H), 2.08 - 2.30 (m, 3H). |
| **61** | 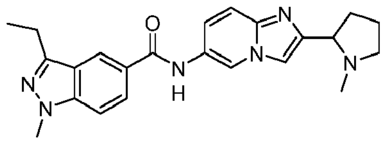 rac-3-ethyl-1-methyl-N-[2-(1-methylpyrrolidin-2-yl)imidazo[1,2-a] pyridin-6-yl]-1H-indazole-5-carboxamide | **4 & 79** | **7B** | Mass spec: m/z: 403 $[M+H]^+$, $^1H$ NMR (300 MHz, MeOH-$d_4$) δ ppm 9.23(s, 1H), 8.47 (s, 1H), 8.01 - 8.05 (m, 1H), 7.82 (s, 1H), 7.58 - 7.62 (m, 1H), 7.44 - 7.53 (m, 2H), 4.05 (s, 3H), 3.45 - 3.54 (m, 1H), 3.20 - 3.28 (m, 1H), 3.00 - 3.12 (m, 2H), 2.30 - 2.50 (m, 5H), 1.85 - 2.10 (m, 3H), 1.43 (t, J = 7.8 Hz, 3H) |
| **62** | 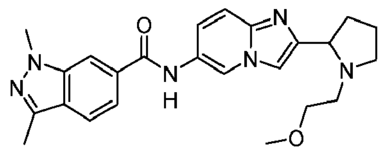 rac-N-{2-[1-(2-methoxyethyl) pyrrolidin-2-yl]imidazo[1,2-a]pyridin-6-yl}-1,3-dimethyl-1H-indazole-6-carboxamide | **7 & 52** | **7A** | Mass spec: m/z: 433 $[M+H]^+$, $^1H$ NMR (400 MHz, MeOH-$d_4$) δ ppm 9.25 (s, 1H), 8.16 (s, 1H), 7.83 - 7.86 (m, 2H), 7.71 - 7.73 (m, 1H), 7.48 - 7.53 (m, 2H), 4.10 (s, 3H), 3.60 - 3.70 (m, 1H), 3.51 - 3.53 (m, 2H), 3.35 - 3.40 (m, 1H), 3.32 - 3.34 (m, 3H), 2.95 - 3.05 (m, 1H), 2.60 (s, 3H), 2.44 - 2.51 (m, 2H), 2.30 - 2.35 (m, 1H), 1.90 - 2.05 (m, 3H) |
| **63** | 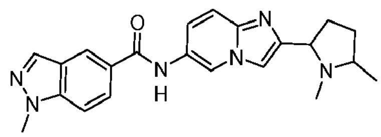 rac-N-[2-(1,5-dimethylpyrrolidin-2-yl) imidazo[1,2-a]pyridin-6-yl]-1-methyl-1H-indazole-5-carboxamide | **27** & **49** | **7A** | Mass spec: m/z: 389 $[M+H]^+$, $^1H$ NMR (300 MHz, MeOH-$d_4$) δ ppm 10.26 (s, 1H), 9.23 (s, 1H), 8.50 (s, 1H), 8.26 (s, 1H), 8.00 - 8.04 (m, 1H), 7.85 (s, 1H), 7.76 - 7.79 (m, 1H), 7.39 - 7.52 (m, 2H), 4.11 (s, 3H), 4.08 - 4.10 (m, 1H), 2.17-2.18 (m, 2H), 2.06 (s, 3H), 1.90 - 1.96 (m, 1H), 1.40 - 1.50 (m, 1H), 1.11 - 1.13 (m, 1H), 1.00 (s, 3H) |

(continued)

| Example Number | Example Name & Structure | Intermediates | Procedure and Chiral Purification method | NMR and Mass Spec |
|---|---|---|---|---|
| **64** | 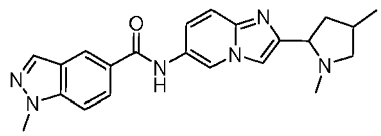 rac-N-[2-(1,4-dimethylpyrrolidin-2-yl) imidazo[1,2-a]pyridin-6-yl]-1-methyl-1H-indazole-5-carboxamide | **33** & **49** | **7C** | Mass spec: m/z: 389 $[M+H]^+$, $^1H$ NMR (300 MHz, MeOHd$_4$) δ ppm 9.23 (s, 1H), 8.48 (s, 1H), 8.19 (s, 1H), 8.07 - 8.03 (m, 1H), 7.83 (s, 1H), 7.71 - 7.68 (m, 1H), 7.54 - 7.48 (m, 2H), 4.14 (s, 3H), 3.58 - 3.52 (m, 1H), 2.92 - 2.88 (m, 1H), 2.71 - 2.64 (m, 1H), 2.58 - 2.47 (m, 1H), 3.34 (s, 1H), 2.32 (s, 3H), 2.05 (m, 1H), 1.72 - 1.97(m, 1H), 1.22 - 1.18 (m, 3H) |
| **65** | 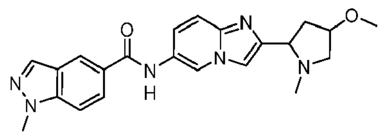 rac-N-[2-(4-methoxy-1-methylpyrrolidin-2-yl)imidazo[1,2-a] pyridin-6-yl]-1-methyl-1H-indazole-5-carboxamide | **38** & **49** | **7A** | Mass spec: m/z: 405 $[M+H]^+$, $^1H$ NMR (400 MHz, MeOHd$_4$) δ 9.20 (s, 1H), 8.45 (s, 1H), 8.16 (s, 1H), 8.01 - 8.02 (m, 1H), 7.84 - 7.79 (m, 1H), 7.69 - 7.65 (m, 1H), 7.51 - 7.43 (m, 2H), 4.12 -4.11 (m, 4H), 3.71 - 3.52 (m, 2H), 3.34 (s, 3H), 2.75 - 1.98 (m, 6H) |

**Example 66 and Example 67 were synthesized following Scheme 31**

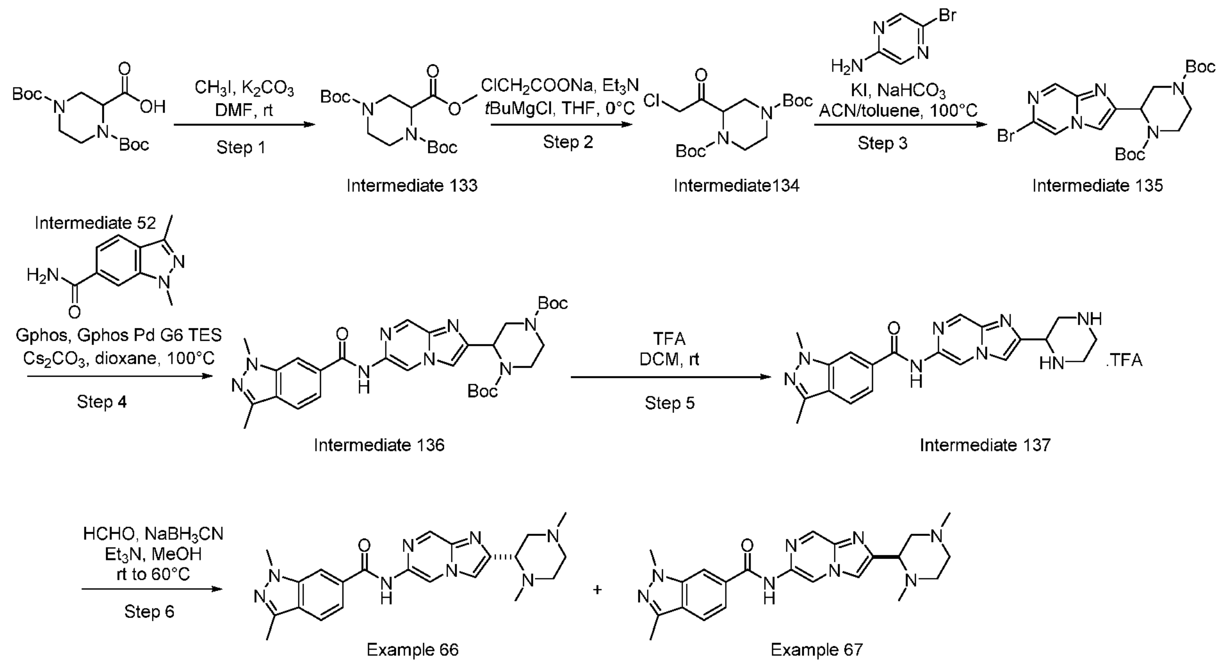


## Scheme 31

**Step 1**

**Intermediate 133: rac-1,4-di-tert-butyl 2-methyl piperazine-1,2,4-tricarboxylate**

To a solution of rac-1,4-bis(tert-butoxycarbonyl)piperazine-2-carboxylic acid (CAS: 181955-79-3, 5 g, 15.13 mmol) in DMF (70 mL) was added methyl iodide (4.30 g, 30.26 mmol) and $K_2CO_3$ (6.32 g, 45.40 mmol). The reaction mixture was stirred overnight at rt and then quenched with water. The resulting solution was extracted three times with EtOAc and the combined organic layers were washed with brine, dried over anhydrous $Na_2SO_4$, filtered, and concentrated under reduced pressure to afford rac-1,4-di-tert-butyl 2-methyl piperazine-1,2,4-tricarboxylate **(Intermediate 133:** 5.2 g, 95%) as a white solid which was used in Step 2 without further purification. Mass spec: m/z 345 $[M+H]^+$.

**Step 2**

**Intermediate 134: rac-1,4-di-tert-butyl 2-(2-chloroacetyl)piperazine-1,4-dicarboxylate**

To a solution of rac-1,4-di-tert-butyl 2-methyl piperazine-1,2,4-tricarboxylate **(Intermediate 133:** 5.2 g, 15.09 mmol) in THF (100 mL) was added sodium 2-chloroacetate (8.79 g, 75.49 mmol) and $Et_3N$ (6.1 g, 60.3945.40 mmol). The mixture was cooled to 0°C and a solution of tert-butylmagnesium chloride (44 mL, 75.49 mmol, 1.7 M in THF) was added dropwise at 0°C. The reaction mixture was stirred at 0°C for 3h, overnight at rt and then quenched with water. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using PE/EtOAc (15/85) as eluent to afford rac-1,4-di-tert-butyl 2-(2-chloroacetyl)piperazine-1,4-dicarboxylate **(Intermediate 134:** 4.8 g, 58%) as a white solid. Mass spec: m/z 363 $[M+H]^+$.

**Step 3**

**Intermediate 135: rac-1,4-di-tert-butyl 2-{6-bromoimidazo[1,2-a]pyrazin-2-yl}piperazine-1,4-dicarboxylate**

To a solution of rac-1,4-di-tert-butyl 2-(2-chloroacetyl)piperazine-1,4-dicarboxylate **(Intermediate 134:** 1.50 g, 4.13 mmol) in MeCN (18 mL) and toluene (6 mL) were added 5-bromopyrazin-2-amine (599 mg, 3.45 mmol), KI (858 mg, 5.17 mmol) and $NaHCO_3$ (347 mg, 4.13 mmol) under a nitrogen atmosphere. The reaction mixture was stirred at 80°C for 5h. The mixture was concentrated under reduced pressure. The crude was purified by reverse-phase flash

chromatography (C18 aq) using $H_2O$/MeCN (61/39) as eluent to afford rac-1,4-di-tert-butyl 2-{6-bromoimidazo[1,2-a]pyrazin-2-yl}piperazine-1,4-dicarboxylate **(Intermediate 135:** 1.60 g, 56%) as a yellow solid. Mass spec: m/z 482 $[M+H]^+$.

**Step 4**

**Intermediate 136: rac-1,4-di-tert-butyl 2-[6-(1,3-dimethylindazole-6-amido)imidazo[1,2-a]pyrazin-2-yl]piperazine-1,4-dicarboxylate**

To a solution of rac-1,4-di-tert-butyl 2-{6-bromoimidazo[1,2-a]pyrazin-2-yl}piperazine-1,4-dicarboxylate **(Intermediate 135:** 200 mg, 0.415 mmol) in dioxane (10 mL) were added 1,3-dimethylindazole-6-carboxamide **(Intermediate 52:** 78.45 mg, 0.415 mmol), Gphos (31.2 mg, 0.058 mmol), Gphos Pd G6 TES (27.5 mg, 0.029 mmol) and $Cs_2CO_3$ (405 mg, 1.25 mmol) under a nitrogen atmosphere. The reaction mixture was stirred at 100°C for 3h, then concentrated under reduced pressure. The crude was purified by reverse-phase flash chromatography (C18 aq) using $H_2O$/ACN (25/75) as eluent to afford rac-1,4-di-tert-butyl 2-[6-(1,3-dimethylindazole-6-amido)imidazo[1,2-a]pyrazin-2-yl]piperazine-1,4-dicarboxylate **(Intermediate 136:** 171 mg, 97%) as a white solid. Mass spec: m/z 591 $[M+H]^+$.

**Step 5**

**Intermediate 137: rac-1,3-dimethyl-N-[2-(piperazin-2-yl)imidazo[1,2-a]pyrazin-6-yl]indazole-6-carboxamide trifluoroacetate**

To a solution of rac-1,4-di-tert-butyl 2-[6-(1,3-dimethylindazole-6-amido)imidazo[1,2-a]pyrazin-2-yl]piperazine-1,4-dicarboxylate **(Intermediate 136:** 170 mg, 0.288 mmol) in DCM (4 mL) was added TFA (2 mL). The reaction mixture was stirred at rt for 1h and then concentrated under reduced pressure to provide rac-1,3-dimethyl-N-[2-(piperazin-2-yl)imidazo[1,2-a]pyrazin-6-yl]indazole-6-carboxamide trifluoroacetate **(Intermediate 137:** 110 mg, 93%) as a yellow solid. Mass spec: m/z 391 $[M+H]^+$.

**Step 6**

**Example 66: rel-N-{2-[(2S)-1,4-dimethylpiperazin-2-yl]imidazo[1,2-a]pyrazin-6-yl}-1,3-dimethyl-1H-indazole-6-carboxamide and Example 67: rel-N-{2-[(2R)-1,4-dimethylpiperazin-2-yl]imidazo[1,2-a]pyrazin-6-yl}-1,3-dimethyl-1H-indazole-6-carboxamide**

To a solution of rac-1,3-dimethyl-N-[2-(piperazin-2-yl)imidazo[1,2-a]pyrazin-6-yl]indazole-6-carboxamide trifluoroacetate **(Intermediate 137:** 50 mg, 0.128 mmol) in MeOH (7 mL) was added paraformaldehyde (38.4 mg, 1.28 mmol). The reaction mixture was stirred at rt for 1h. Then $NaBH_3CN$ (40.24 mg, 0.640 mmol) was added to the reaction mixture which was stirred at 60°C for 1h and then quenched with water. The mixture concentrated under reduced pressure and then purified by preparative HPLC (column: CHIRAL ART Cellulose-SB, 2x25 cm, 5 $\mu$m; Eluent: MtBE (0.5% 2M $NH_3$ in MeOH)/EtOH 40/60; flow rate: 20 mL/min) to afford:

**Example 66: rel-N-{2-[(2S)-1,4-dimethylpiperazin-2-yl]imidazo[1,2-a]pyrazin-6-yl}-1,3-dimethyl-1H-indazole-6-carboxamide**

First eluting compound (retention time = 4.79 min):
Mass spec: m/z: 419 $[M+H]^+$; $^1H$ NMR (400 MHz, MeOH-$d_4$) $\delta$ ppm 9.43 (s, 1H), 8.85 (s, 1H), 8.19 (s, 1H), 8.09 (s, 1H), 7.83 - 7.85 (m, 1H), 7.72 - 7.74 (m, 1H), 4.08 (s, 3H), 3.49 - 3.52 (m, 1H), 2.8 - 3.00 (m, 3H), 2.58 (s, 3H), 2.40 - 2.52 (m, 3H), 2.35 (s, 3H), 2.17 (s, 3H).

**Example 67: rel-N-{2-[(2R)-1,4-dimethylpiperazin-2-yl]imidazo[1,2-a]pyrazin-6-yl}-1,3-dimethyl-1H-indazole-6-carboxamide**

Second eluting compound (retention time = 6.10 min):
Mass spec: m/z: 419 $[M+H]^+$; $^1H$ NMR (400 MHz, MeOH-$d_4$) $\delta$ ppm 9.43 (s, 1H), 8.85 (s, 1H), 8.19 (s, 1H), 8.09 (s, 1H), 7.83 - 7.85 (m, 1H), 7.72 - 7.74 (m, 1H), 4.08 (s, 3H), 3.49 - 3.52 (m, 1H), 2.8 - 3.00 (m, 3H), 2.58 (s, 3H), 2.40 - 2.52 (m, 3H), 2.35 (s, 3H), 2.17 (s, 3H).

**General Procedure 8**

**Examples 68-75,** shown in **Table 3,** were prepared as shown **Scheme 32** and following General Procedure 8. Where primary carboxamides intermediates are not shown in the **Table 3** they are commercially available or well known in the art.

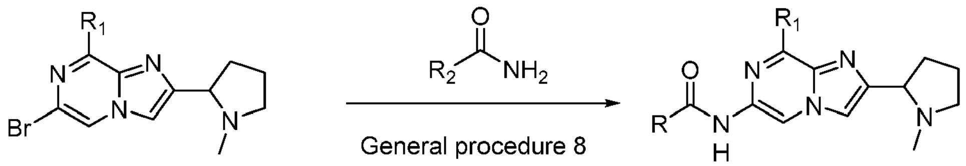


**Scheme 32**

**General procedure 8**

A solution of the appropriate aryl bromide (1 eq), BrettPhos Pd G3 (0.15 eq), $Cs_2CO_3$ (5 eq) and the appropriate primary amide intermediate tabulated below (1 eq) in 1,4-dioxane-anhydrous (0.1 M) was stirred at 110°C for 18h. The reaction mixture was filtered through a pad of celite. The filtrate was diluted with EtOAc and $H_2O$. The phases were separated, and the aqueous phase was extracted twice with EtOAc. The combined organic layers were washed with brine, dried using a phase separator and concentrated under vacuum. The crude was purified by reverse-phase flash chromatography using a gradient of MeCN in water from 0% to 100% (neutral) to afford the expected product. For some examples the racemic products were separated by the chiral preparative reverse phase HPLC chromatography method below and indicated in **Table 3.**

Chiral purification method by reverse phase HPLC chromatography:

Method A: Column: AD 250 x 4.6 5μm; Eluent: $CO_2$/(iPrOH + 0.5 % IPA) 70/30; Flow rate : 50 mL/min

**Table 3**

| Example Number | Example Name & Structure | Intermediates | Procedure and Chiral Purification method | NMR and Mass Spec |
|---|---|---|---|---|
| **68** | 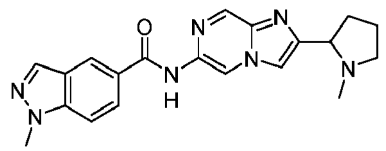 rac-1-methyl-N-[2-(1-methylpyrrolidin-2-yl)imidazo[1,2-a] pyrazin-6-yl]-1H-indazole-5-carboxamide | **43A** & **49** | **8** | Mass spec: m/z: 376 $[M+H]^+$, $^1H$ NMR (600 MHz, DMSO-$d_6$) δ ppm 1.74 - 1.92 (m, 3 H) 2.17 - 2.25 (m, 1 H) 2.22 (s, 3 H) 2.28 (q, J = 8.61 Hz, 1 H) 3.04 - 3.18 (m, 1 H) 3.34 - 3.42 (m, 1 H) 4.09 (s, 3 H) 7.73 (d, J = 8.95 Hz, 1 H) 8.04 - 8.13 (m, 2 H) 8.22 (s, 1 H) 8.57 (d, J = 0.73 Hz, 1 H) 8.87 (s, 1 H) 9.37 (d, J = 1.61 Hz, 1 H) 10.80 (br s, 1 H) |

(continued)

| Example Number | Example Name & Structure | Intermediates | Procedure and Chiral Purification method | NMR and Mass Spec |
|---|---|---|---|---|
| **69** | 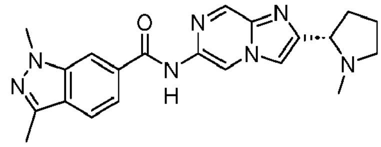 1,3-dimethyl-N-{2-[(2S)-1-methylpyrrolidin-2-yl]imidazo[1,2-a] pyrazin-6-yl}-1H-indazole-6-carboxamide | **44 & 52** | **8** and purification method A 1st eluting isomer (rt = 10.9 min) | Mass spec: m/z: 390 $[M+H]^+$, $^1H$ NMR (400 MHz, DMSO-$d_6$) δ ppm 10.88 (s, 1H), 9.41 (d, J = 1.5 Hz, 1H), 8.91 (d, J = 1.4 Hz, 1H), 8.43 (t, J = 1.1 Hz, 1H), 8.17 (s, 1H), 7.82 (d, J = 8.2 Hz, 1H), 7.74 (dd, J = 8.5, 1.4 Hz, 1H), 4.06 (s, 3H), 3.45 - 3.37 (m, 1H), 3.18 - 3.09 (m, 1H), 2.53 (s, 3H), 2.35 - 2.17 (m, 5H), 1.95 - 1.76 (m, 3H) |
| **70** | 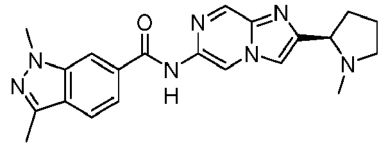 1,3-dimethyl-N-{2-[(2R)-1-methylpyrrolidin-2-yl]imidazo[1,2-a] pyrazin-6-yl}-1H-indazole-6-carboxamide | **43 & 52** | **8** | Mass spec: m/z: 390 [M+H]+, $^1H$ NMR (400 MHz, DMSO-$d_6$) δ ppm 10.88 (s, 1H), 9.41 (d, J = 1.5 Hz, 1H), 8.91 (d, J = 1.4 Hz, 1H), 8.43 (t, J = 1.1 Hz, 1H), 8.16 (s, 1H), 7.82 (d, J = 8.6 Hz, 1H), 7.74 (dd, J = 8.5, 1.4 Hz, 1H), 4.06 (s, 3H), 3.40 (t, J = 7.7 Hz, 1H), 3.12 (td, J = 8.2, 2.6 Hz, 1H), 2.53 (s, 3H), 2.35 - 2.19 (m, 5H), 1.95 - 1.76 (m, 3H) |
| **71** | 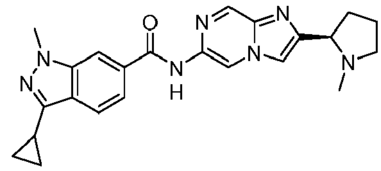 3-cyclopropyl-1-methyl-N-{2-[(2R)-1-methylpyrrolidin-2-yl]imidazo[1,2-a] pyrazin-6-yl}-1H-indazole-6-carboxamide | **43 & 48** | **8** | Mass spec: m/z: 416 $[M+H]^+$, $^1H$ NMR (500 MHz, DMSO-$d_6$) δ ppm 10.87 (s, 1H), 9.41 (d, 1H, J = 1.5 Hz), 8.91 (d, 1H, J = 0.9 Hz), 8.3-8.5 (m, 1H), 8.17 (s, 1H), 7.89 (dd, 1H, J = 0.7, 8.4 Hz), 7.73 (dd, 1H, J = 1.4, 8.5 Hz), 4.02 (s, 3H), 3.4-3.5 (m, 1H), 3.0-3.2 (m, 1H), 2.32 (tt, 2H, J = 5.1, 8.3 Hz), 2.2-2.3 (m, 4H), 1.7-2.0 (m, 3H), 0.9-1.1 (m, 4H) |
| **72** | 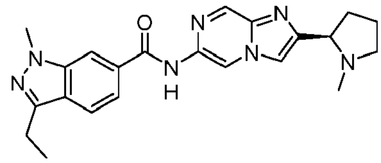 3-ethyl-1-methyl-N-{2-[(2R)-1-methylpyrrolidin-2-yl]imidazo[1,2-a] pyrazin-6-yl}-1H-indazole-6-carboxamide | **43 & 47** | **8** | Mass spec: m/z: 404 $[M+H]^+$, $^1H$ NMR (600 MHz, DMSO-$d_6$) δ ppm 1.32 (t, J = 7.56 Hz, 3 H) 1.73 - 1.92 (m, 3 H) 2.19-2.26 (m, 4 H) 2.26 - 2.34 (m, 1 H) 2.95 (d, J = 7.48 Hz, 2 H) 3.12 (br s, 1 H) 3.40 (t, J = 6.90 Hz, 1 H) 4.06 (s, 3 H) 7.72 (dd, J = 8.44, 1.39 Hz, 1 H) 7.85 (dd, J = 8.36, 0.73 Hz, 1 H) 8.15 (s, 1 H) 8.42 (s, 1 H) 8.78 - 8.98 (m, 1 H) 9.40 (d, J = 1.47 Hz, 1 H) 10.86 (s, 1 H) |

(continued)

| Example Number | Example Name & Structure | Intermediates | Procedure and Chiral Purification method | NMR and Mass Spec |
|---|---|---|---|---|
| **73** | 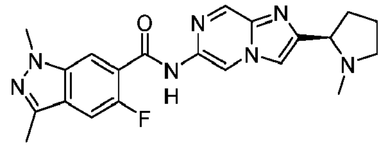 5-fluoro-1,3-dimethyl-N-{2-[(2R)-1-methylpyrrolidin-2-yl]imidazo[1,2-a]pyrazin-6-yl}-1H-indazole-6-carboxamide | **43 & 55** | **8** | Mass spec: m/z: 408 [M+H]+, $^1H$ NMR (600 MHz, DMSO-$d_6$) δ ppm 10.93 (s, 1 H) 9.38 (d, J = 1.03 Hz, 1 H) 8.86 (d, J = 0.73 Hz, 1 H) 8.16 (s, 1 H) 7.98 (d, J = 5.43 Hz, 1 H) 7.65 (d, J = 10.42 Hz, 1 H) 4.02 (s, 3 H) 3.35 - 3.40 (m, 1 H) 3.09 - 3.14 (m, 1 H) 2.48 (s, 3 H) 2.29 (d, J = 8.36 Hz, 1 H) 2.17-2.25 (m, 4 H) 1.73 - 1.92 (m, 3 H) |
| **74** | 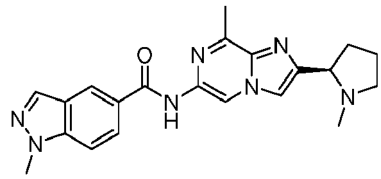 1-methyl-N-[8-methyl-2-[(2R)-1-methylpyrrolidin-2-yl]imidazo[1,2-a]pyrazin-6-yl]indazole-5-carboxamide | **43B & 49** | **8** | Mass spec: m/z : 390 $[M+H]^+$, $^1H$ NMR (500 MHz, DMSO-$d_6$) δ ppm 10.72 (s, 1 H) 9.24 (s, 1 H) 8.58 (dd, J= 1.63, 0.75 Hz, 1 H) 8.22 (d, J = 1.00 Hz, 1 H) 8.05 - 8.11 (m, 2 H) 7.72 - 7.76 (m, 1 H) 4.10 (s, 3 H) 3.34 - 3.40 (m, 1 H) 3.09 - 3.15 (m, 1 H) 2.74 (s, 3 H) 2.21 -2.31 (m, 5 H) 1.76 - 1.90 (m, 3 H) |
| **75** | 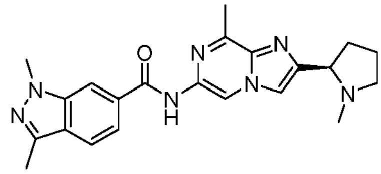 1,3-dimethyl-N-[8-methyl-2-[(2R)-1-methylpyrrolidin-2-yl]imidazo[1,2-a]pyrazin-6-yl]indazole-6-carboxamide | **43B & 52** | **8** | Mass spec: m/z : 404 $[M+H]^+$, $^1H$ NMR (500 MHz, DMSO-$d_6$) δ ppm 10.79 (s, 1 H) 9.27 (d, J=0.63 Hz, 1 H) 8.42 - 8.45 (m, 1 H) 8.10 (s, 1 H) 7.80 (dd, J=8.50, 0.75 Hz, 1 H) 7.73 (dd, J=8.38, 1.38 Hz, 1 H) 4.06 (s, 3 H) 3.35 - 3.40 (m, 1 H) 3.10-3.15 (m, 1 H) 2.75 (s, 3 H) 2.52 (s, 3 H) 2.20 - 2.32 (m, 5 H) 1.77 - 1.89 (m, 3 H) |

**Example 76 was synthesized following Scheme 33**

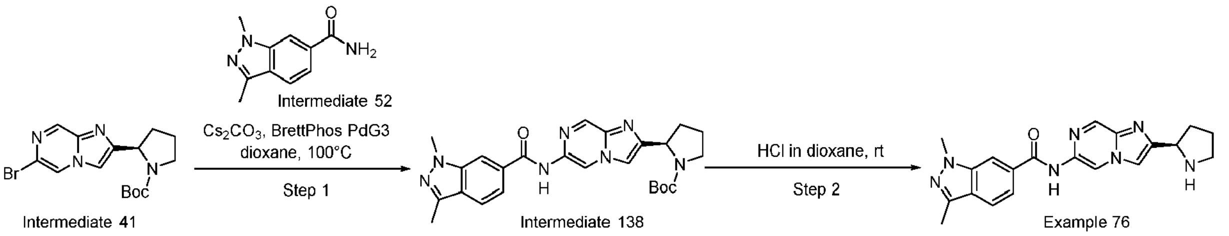


## Scheme 33

**Step 1**

**Intermediate 138: tert-butyl (2R)-2-[6-[(1,3-dimethylindazole-6-carbonyl)amino]imidazo[1,2-a]pyrazin-2-yl]pyrrolidine-1-carboxylate**

A solution of tert-butyl (2R)-2-(6-bromoimidazo[1,2-a]pyrazin-2-yl)pyrrolidine-1-carboxylate **(Intermediate 41**: (300 mg, 0.817 mmol), BrettPhos Pd G3 (234 mg, 0.245 mmol), $Cs_2CO_3$ (1331 mg, 4.08 mmol) and 1,3-dimethylindazole-6-carboxamide **(Intermediate 52:** 155 mg, 0.817 mmol) in dioxane (6.9 mL) was stirred at 100°C for 3h. The reaction mixture was filtered through a pad of celite. The filtrate was diluted with EtOAc and water. The phases were separated, and the aqueous phase was extracted with EtOAc twice. The combined organic layers were washed with brine, dried using a hydrophobic paper and concentrated under vacuum. The crude material was purified by flash chromatography on silica gel using a gradient of EtOAc/EtOH (3/1) in heptane from 20% to 100%, then by reverse-phase flash chromatography (C18 aq) using a gradient of MeCN in water from 0% to 100% (neutral) to afford tert-butyl (2R)-2-[6-[(1,3-dimethylindazole-6-carbonyl)amino]imidazo[1,2-a]pyrazin-2-yl]pyrrolidine-1-carboxylate **(Intermediate 138:** 109 mg, 28%) as a light yellow solid. Mass spec: m/z 476 $[M+H]^+$; $^1H$ NMR (400 MHz, DMSO-$d_6$) $\delta$ 10.87 (s, 1H), 9.42 (d, J = 14.6 Hz, 1H), 8.93 (s, 1H), 8.42 (d, J = 1.2 Hz, 1H), 8.09 (d, J = 16.3 Hz, 1H), 7.82 (d, J = 8.5 Hz, 1H), 7.73 (dd, J = 8.5, 1.4 Hz, 1H), 5.04 - 4.95 (m, 1H), 4.05 (s, 3H), 3.50 (s, 1H), 3.40 (dt, J = 9.9, 7.2 Hz, 1H), 2.52 (s, 6H), 2.33 - 2.14 (m, 1H), 2.10 - 1.84 (m, 3H), 1.30 - 1.19 (m, 9H).

**Step 2**

**Example 76: 1,3-dimethyl-N-[2-[(2R)-pyrrolidin-2-yl]imidazo[1,2-a]pyrazin-6-yl]indazole-6-carboxamide**

To a solution of tert-butyl (2R)-2-[6-[(1,3-dimethylindazole-6-carbonyl)amino]imidazo[1,2-a]pyrazin-2-yl]pyrrolidine-1-carboxylate (Intermediate **138:** 109 mg, 0.229 mmol) in dioxane (1.1 mL) was added a solution of HCl (0.57 mL, 2.29 mmol, 4N in dioxane). The mixture was stirred at rt for 18h. The mixture was washed with a saturated solution of $NaHCO_3$ until a basic pH was achieved. The phases were separated, and the aqueous phase was extracted with $CHCl_3$/iPrOH (3/1) three times. The combined organic layers were dried using a hydrophobic paper and concentrated under vacuum. The crude was purified by reverse-phase flash chromatography (C18 aq) using a gradient of MeCN in water from 0% to 100% (neutral) to afford 1,3-dimethyl-N-[2-[(2R)-pyrrolidin-2-yl]imidazo[1,2-a]pyrazin-6-yl]indazole-6-carboxamide **(Example 76:** 36.7 mg, 42%) as a light yellow solid. Mass spec: m/z : 376 $[M+H]^+$; $^1H$ NMR (500 MHz, DMSO-$d_6$) $\delta$ ppm 10.86 (br s, 1 H) 9.39 (d, J = 1.5 Hz, 1 H) 8.89 (d, J = 1.5 Hz, 1 H) 8.42 (d, J = 1.0 Hz, 1 H) 8.14 (s, 1 H) 7.79 - 7.86 (m, 1 H) 7.73 (dd, J = 8.5, 1.4 Hz, 1 H) 4.21 - 4.41 (m, 1 H) 4.05 (s, 3 H) 3.31 (br s, 1 H) 2.78 - 3.09 (m, 2 H) 2.52 (s, 3 H) 2.13 (dtd, J = 11.7, 7.8, 7.8, 5.7 Hz, 1 H) 1.58 - 1.94 (m, 3 H).

**Examples 77, 78, 79 and 80 were made following the route shown in Scheme 34.**

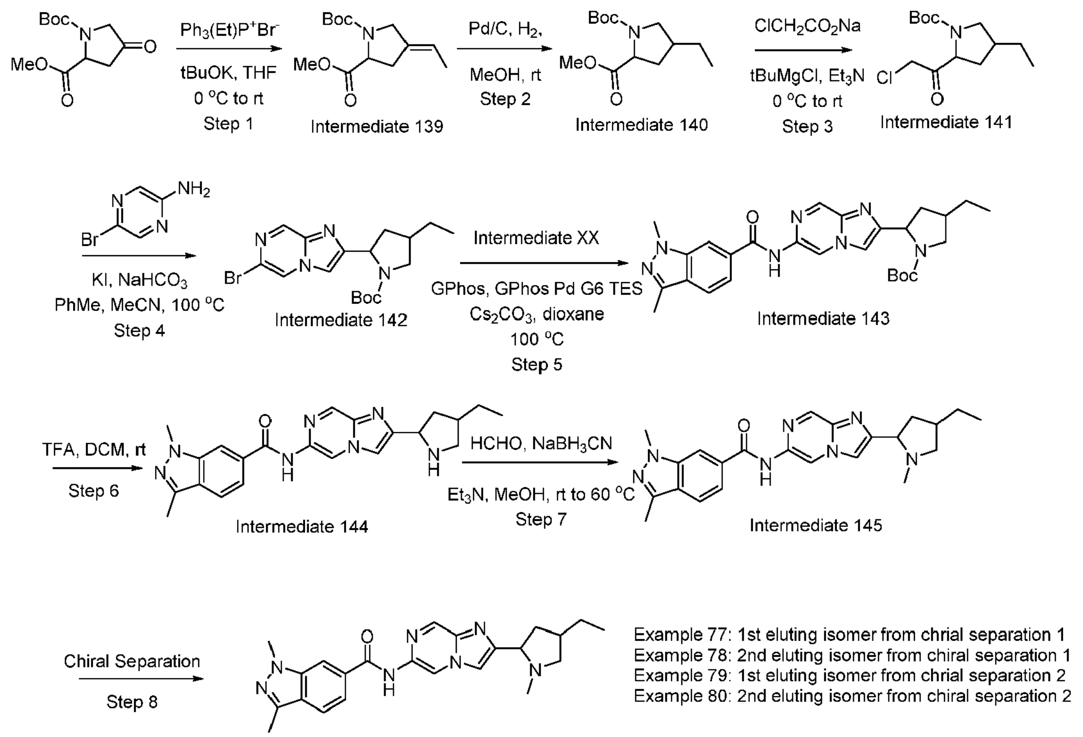


## Scheme 34

### Step 1

**Intermediate 139: 1-tert-butyl 2-methyl (4Z)-4-ethylidenepyrrolidine-1,2-dicarboxylate**

To a solution of ethyltriphenylphosphonium bromide (CAS: 1530-32-1, 24.4 g, 65.7 mmol) in THF (300 mL) was added t-BuOK (7.38 g, 65.7 mmol) in THF (300 mL) at 0°C. The resulting solution was stirred at 0°C for 1 h. Then 1-tert-butyl 2-methyl 4-oxopyrrolidine-1,2-dicarboxylate (CAS: 194924-96-4, 8 g, 32.9 mmol) in THF (300 mL) was added. The resulting mixture was stirred for 4h at room temperature and quenched with water (100 mL). The resulting mixture was extracted with EtOAc (3 x 300 mL). The combined organic layers were washed with NaHCOa (400 mL) and brine (400 mL), dried over anhydrous $Na_2SO_4$, filtered, and concentrated under reduced pressure. The crude material was purified by flash chromatography on silica gel using PE/EtOAc (9/1) as eluent to afford 1-tert-butyl 2-methyl (4Z)-4-ethylidenepyrrolidine-1,2-dicarboxylate **(Intermediate 139:** 1.8 g, 11.2%) as a yellow oil which was used in the next step without further purification. Mass spec: m/z: 256 $[M+H]^+$.

### Step 2

**Intermediate 140: rac-1-tert-butyl 2-methyl 4-ethylpyrrolidine-1,2-dicarboxylate**

To a solution of 1-tert-butyl 2-methyl (4Z)-4-ethylidenepyrrolidine-1,2-dicarboxylate **(Intermediate 139:** 1.8 g, 7.05 mmol) in MeOH (20 mL) was added Pd/C (0.53 g, 10%, in carbon). Hydrogen (3atm) was introduced and the reaction mixture was stirred at room temperature overnight. The mixture was filtered through a celite pad and the filtrate was concentrated under reduced pressure to give rac-1-tert-butyl 2-methyl 4-ethylpyrrolidine-1,2-dicarboxylate **(Intermediate 140:** 1.8 g) as a colorless oil which was used in Step 3 with further purification. Mass spec: m/z: 258 $[M+H]^+$.

### Step 3

**Intermediate 141: rac-tert-butyl 2-(2-chloroacetyl)-4-ethylpyrrolidine-1-carboxylate**

To a stirred mixture of rac-1-tert-butyl 2-methyl 4-ethylpyrrolidine-1,2-dicarboxylate **(Intermediate 140:** 1.8 g, 6.70 mmol) and $Et_3N$ (2.83 g, 27.9 mmol) in THF (40 mL) was added a solution of tert-butylmagnesium chloride (20.57 mL, 34.9 mmol, 1.7 M in THF) dropwise at 0°C under a nitrogen atmosphere. The resulting mixture was stirred overnight at rt. The mixture was then quenched with a saturated solution $NH_4Cl$ (100 mL) and extracted with EtOAc (3 x 200 mL).

The combined organic layers were washed with brine, dried over anhydrous $Na_2SO_4$, filtered, and concentrated under reduced pressure. The crude material was purified by flash chromatography on silica gel using PE/EtOAc (8/2) as eluent to afford rac-tert-butyl 2-(2-chloroacetyl)-4-ethylpyrrolidine-1-carboxylate **(Intermediate 141:** 1.6 g, 80%) as a yellow oil which was used in Step 4 without further purification. Mass spec: m/z: 276 $[M+H]^+$.

**Step 4**

**Intermediate 142: rac-tert-butyl 2-{6-bromoimidazo[1,2-a]pyrazin-2-yl}-4-ethylpyrrolidine-1-carboxylate**

A solution of rac-tert-butyl 2-(2-chloroacetyl)-4-ethylpyrrolidine-1-carboxylate **(Intermediate 141:** 1.6 g, 5.80 mmol), 5-bromopyrazin-2-amine (CAS: 59489-71-3, 1.01 g, 5.80 mmol), KI (1.93 g, 11.6 mmol) and $NaHCO_3$ (0.97 g, 11.6 mmol) in anhydrous toluene (6 mL) and MeCN (20 mL) was stirred overnight at 100°C. The mixture was concentrated in vacuo. The crude was purified by reverse-phase flash chromatography (C18 aq) using $H_2O$/MeCN (70/30) as eluent to afford rac-tert-butyl 2-{6-bromoimidazo [1,2-a]pyrazin-2-yl}-4-ethylpyrrolidine-1-carboxylate **(Intermediate 142:** 230 mg, 17%) as a yellow oil. Mass spec: m/z: 395 $[M+H]^+$.

**Step 5**

**Intermediate 143: rac-tert-butyl 2-[6-(1,3-dimethylindazole-6-amido)imidazo[1,2-a]pyrazin-2-yl]-4-ethylpyrrolidine-1-carboxylate**

To a solution of rac-tert-butyl 2-{6-bromoimidazo[1,2-a]pyrazin-2-yl}-4-ethylpyrrolidine-1-carboxylate **(Intermediate 142:** 230 mg, 0.582 mmol) and 1,3-dimethylindazole-6-carboxamide **(Intermediate 52:** 121 mg, 0.640 mmol) in dioxane (6 mL) were added Gphos (31.2 mg, 0.058 mmol), Gphos Pd G6 TES (27.5 mg, 0.029 mmol) and $Cs_2CO_3$ (568 mg, 1.75 mmol). The resulting mixture was stirred for 3h at 100°C under a nitrogen atmosphere. The reaction mixture was concentrated under reduced pressure and the residue was purified by reverse-phase flash chromatography (C18 aq) using MeCN/$H_2O$ (40/60) as eluent to afford rac-tert-butyl 2-[6-(1,3-dimethylindazole-6-amido)imidazo[1,2-a]pyrazin-2-yl]-4-ethylpyrrolidine-1-carboxylate **(Intermediate 143:** 190 mg, 69%) as a yellow oil. Mass spec: m/z: 504 $[M+H]^+$.

**Step 6**

**Intermediate 144: rac-N-[2-(4-ethylpyrrolidin-2-yl)imidazo[1,2-a]pyrazin-6-yl]-1,3-dimethylindazole-6-carboxamide trifluoroacetate**

To a solution of tert-butyl 2-[6-(1,3-dimethylindazole-6-amido)imidazo[1,2-a]pyrazin-2-yl]-4-ethylpyrrolidine-1-carboxylate **(Intermediate 143:** 190 mg, 0.377 mmol) in DCM (10 mL) was added TFA (3 mL) at rt and stirred for 2h. The resulting mixture was concentrated under reduced pressure to afford rac-N-[2-(4-ethylpyrrolidin-2-yl)imidazo[1,2-a]pyrazin-6-yl]-1,3-dimethylindazole-6-carboxamide trifluoroacetate **(Intermediate 144:** 160 mg) as a yellow oil which was used in Step 7 without further purification. Mass spec: m/z: 404.0 $[M+H]^+$.

**Step 7**

**Intermediate 145: rac-N-[2-(4-ethyl-1-methylpyrrolidin-2-yl)imidazo[1,2-a]pyrazin-6-yl]-1,3-dimethylindazole-6-carboxamide**

A solution of rac-N-[2-(4-ethylpyrrolidin-2-yl)imidazo[1,2-a]pyrazin-6-yl]-1,3-dimethylindazole-6-carboxamide trifluoroacetate **(Intermediate 144:** 160 mg, 0.397 mmol), $Et_3N$ (192 mg, 1.19 mmol) and paraformaldehyde (35.7 mg, 1.19 mmol) in MeOH (5 mL) was stirred at rt for 15min. $NaBH_3CN$ (74.76 mg, 1.19 mmol) was then added and the resulting mixture was stirred for 1h at 60°C. The solvent was then removed under reduced pressure and the residue was purified by reverse-phase flash chromatography (C18 aq) using MeCN/$H_2O$ (47/53) as eluent to afford rac-N-[2-(4-ethyl-1-methylpyrrolidin-2-yl)imidazo[1,2-a]pyrazin-6-yl]-1,3-dimethylindazole-6-carboxamide **(Intermediate 145:** 65 mg, 78%) as a yellow solid. Mass spec: m/z: 418 $[M+H]^+$.

**Step 8**

**Examples 77, 78, 79 and 80**

**Chiral Separation 1**

rac-N-[2-(4-ethyl-1-methylpyrrolidin-2-yl)imidazo[1,2-a]pyrazin-6-yl]-1,3-dimethylindazole-6-carboxamide **(Intermediate 145)** was subjected to chiral preparative HPLC (Column: Chiral Art Amylose-SA, 2x25 cm, 5 μm; Eluent: (MtBE (0.5% 2M $NH_3$ in MeOH))/EtOH 70/30; flow rate: 20 mL/min) to isolate the following examples:

**Example 77: N-{2-[4-ethyl-1-methylpyrrolidin-2-yl]imidazo[1,2-a]pyrazin-6-yl}-1,3-dimethyl-1H-indazole-6-carboxamide**

First eluting compound (retention time = 8.6 min):
Mass spec: m/z: 418 $[M+H]^+$; $^1H$ NMR (400 MHz, MeOH-$d_4$) δ ppm 9.43 (s, 1H), 8.85 (s, 1H), 8.18 (s, 1H), 8.09 (s, 1H), 7.83 - 7.85 (m, 1H), 7.72 - 7.74 (m, 1H), 4.04 (s, 3H), 3.75 - 3.98 (m, 1H), 3.05 - 3.07 (m, 1H), 2.74 - 2.77 (m, 1H), 2.63 (s, 3H), 2.52 - 2.58 (m, 1H), 2.29 - 2.40 (m, 3H), 2.11 - 2.25 (m, 1H),1.25 - 1.82 (m, 3H), 0.89 - 1.00 (m, 3H).

**Example 78: N-{2-[4-ethyl-1-methylpyrrolidin-2-yl]imidazo[1,2-a]pyrazin-6-yl}-1,3-dimethyl-1H-indazole-6-carboxamide**

Second eluting compound (retention time = 11.1 min)
Mass spec: m/z: 418 $[M+H]^+$; $^1H$ NMR (400 MHz, MeOH-$d_4$) δ ppm 9.44 (s, 1H), 8.86 (s, 1H), 8.18 (s, 1H), 8.12 (s, 1H), 7.83 - 7.85 (m, 1H), 7.72 - 7.74 (m, 1H), 4.09 (s, 3H), 3.91 - 3.97 (m, 1H), 3.48 - 3.51 (m, 1H), 2.58 (s, 3H), 2.55 (s, 3H), 2.45 (s, 3H), 2.09 - 2.11 (m, 1H), 1.52 - 1.55 (m, 2H), 0.97 - 1.00 (m, 3H).

**Examples 79 and 80** were isolated as a mixture (3rd eluting mixture of compounds from **Chiral Separation 1).** This mixture which was purified further, in **Chiral Separation 2,** using a chiral preparative HPLC column: Chiral Art Amylose-SA, 2x25 cm, 5 μm; Eluent: (MtBE (0.5% 2M $NH_3$ in MeOH))/IPA 90/10; flow rate: 20 mL/min) to isolate the following examples:

**Chiral Separation 2**

**Example 79: N-{2-[4-ethyl-1-methylpyrrolidin-2-yl]imidazo[1,2-a]pyrazin-6-yl}-1,3-dimethyl-1H-indazole-6-carboxamide**

First eluting compound (retention time = 7.3 min):
Mass spec: m/z: 418 $[M+H]^+$; $^1H$ NMR (400 MHz, MeOH-$d_4$) δ ppm 9.41 (s, 1H), 8.82 (s, 1H), 8.19 (s, 1H), 8.05 (s, 1H), 7.81 - 7.82 (m, 1H), 7.71 - 7.73 (m, 1H), 4.08 (s, 3H), 3.57 - 3.58 (m, 1H), 2.98 - 2.99 (m, 1H), 2.45 - 2.47 (m, 1H), 2.62 (s, 3H), 2.47-2.49 (m, 1H), 2.32 (s, 3H), 2.18 - 2.21 (m, 1H), 1.75 - 1.77 (m, 1H), 1.56 - 1.58 (m, 2H), 0.94 - 0.98 (m, 3H).

**Example 80: N-{2-[4-ethyl-1-methylpyrrolidin-2-yl]imidazo[1,2-a]pyrazin-6-yl}-1,3-dimethyl-1H-indazole-6-carboxamide**

Second eluting compound (retention time = 9.7 min):
Mass spec: m/z: 418 $[M+H]^+$; $^1H$ NMR (400 MHz, MeOH-$d_4$) δ ppm 9.40 (s, 1H), 8.81 (s, 1H), 8.17 (s, 1H), 8.04 (s, 1H), 7.81 - 7.83 (m, 1H), 7.71 - 7.73 (m, 1H), 4.08 (s, 3H), 3.58 - 3.59 (m, 1H), 3.31 - 3.32 (m, 1H), 2.57 (s, 3H), 2.23 - 2.50 (m, 5H), 2.11 - 2.13 (m, 1H), 1.95 - 1.98 (m, 1H), 1.50 - 1.52 (m, 2H), 0.96 - 0.98 (m, 3H).

**Examples 81 and 82 were synthesized following Scheme 35.**

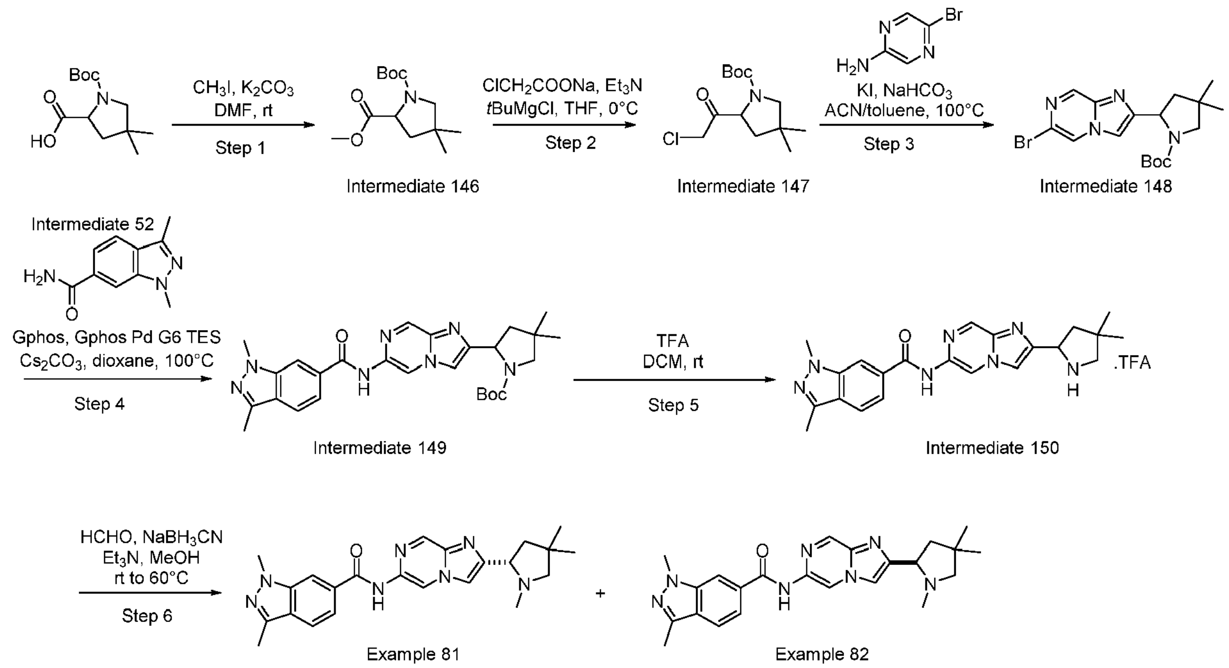


## Scheme 35

**Step 1**

**Intermediate 146: rac-1-tert-butyl 2-methyl 4,4-dimethylpyrrolidine-1,2-dicarboxylate**

To a solution of rac-1-(tert-butoxycarbonyl)-4,4-dimethylpyrrolidine-2-carboxylic acid (CAS: 1613115-21-1, 1.00 g, 4.11 mmol) in DMF (20 mL) was added $K_2CO_3$ (1.70 g, 12.3 mmol). The reaction mixture was stirred at rt for 15 min. Then, MeI (0.88 g, 6.17 mmol) was added. The mixture was stirred at rt overnight and quenched with water. The resulting solution was extracted three times with EtOAc and the combined organic layers were washed with brine, dried over anhydrous $Na_2SO_4$, filtered, and concentrated under reduced pressure to provide rac-1-tert-butyl 2-methyl 4,4-dimethylpyrrolidine-1,2-dicarboxylate **(Intermediate 146:** 1.00 g, 85%) as a yellow oil which was used in Step 2 without further purification. Mass spec: m/z 258 $[M+H]^+$.

**Step 2**

**Intermediate 147: rac-tert-butyl 2-(2-chloroacetyl)-4,4-dimethylpyrrolidine-1-carboxylate**

To a solution of rac-1-tert-butyl 2-methyl 4,4-dimethylpyrrolidine-1,2-dicarboxylate **(Intermediate 146:** 1.00 g, 3.50 mmol), sodium 2-chloroacetate (2.04 g, 17.5 mmol) and $Et_3N$ (1.42 g, 14.0 mmol) in THF (30 mL) was added a solution of tert-butylmagnesium chloride (10.3 mL, 17.5 mmol, 1.7M in THF,) dropwise at 0°C. The reaction mixture was stirred at rt for 18h and then quenched with saturated aqueous $NH_4Cl$ solution. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using PE/EtOAc (2/8) as eluent to afford rac-tert-butyl 2-(2-chloroacetyl)-4,4-dimethylpyrrolidine-1-carboxylate **(Intermediate 147:** 960 mg, 80%) as a yellow oil. Mass spec: m/z 276 $[M+H]^+$.

**Step 3**

**Intermediate 148: rac-tert-butyl 2-{6-bromoimidazo[1,2-a]pyrazin-2-yl}-4,4-dimethylpyrrolidine-1-carboxylate**

To a solution of rac-tert-butyl 2-(2-chloroacetyl)-4,4-dimethylpyrrolidine-1-carboxylate **(Intermediate 147:** 960 mg, 2.79 mmol) in MeCN (10 mL) and toluene (4 mL) were added 5-bromopyrazin-2-amine (582 mg, 3.34 mmol), KI

(693 mg, 4.18 mmol) and $NaHCO_3$ (351 mg, 4.18 mmol). The reaction mixture was stirred at 100°C overnight, then concentrated under reduced pressure. The crude was purified by reverse-phase flash chromatography (C18 aq) using $H_2O$/MeCN (25/75) as eluent to afford rac-tert-butyl 2-{ 6-bromoimidazo[1,2-a]pyrazin-2-yl}-4,4-dimethylpyrrolidine-1-carboxylate **(Intermediate 148:** 280 mg, 20%) as a yellow oil. Mass spec: m/z 395 $[M+H]^+$.

**Step 4**

**Intermediate 149: rac-tert-butyl 2-[6-(1,3-dimethylindazole-6-amido)imidazo[1,2-a]pyrazin-2-yl]-4,4-dimethylpyrrolidine-1-carboxylate**

To a solution of rac-tert-butyl 2-{6-bromoimidazo[1,2-a]pyrazin-2-yl}-4,4-dimethylpyrrolidine-1-carboxylate **(Intermediate 148:** 280 mg, 0.567 mmol) in dioxane (5 mL) were added 1,3-dimethylindazole-6-carboxamide **(Intermediate 52:** 124 mg, 0.624 mmol), $Cs_2CO_3$ (554 mg, 1.70 mmol), Gphos (30.4 mg, 0.057 mmol) and Gphos Pd G6 TES (26.8 mg, 0.028 mmol) under a nitrogen atmosphere. The mixture reaction was stirred at 100°C for 3h, then concentrated under reduced pressure. The crude was purified by reverse-phase flash chromatography (C18 aq) using $H_2O$/MeCN (25/75) as eluent to afford rac-tert-butyl 2-[6-(1,3-dimethylindazole-6-amido)imidazo[1,2-a]pyrazin-2-yl]-4,4-dimethylpyrrolidine-1-carboxylate **(Intermediate 149:** 170 mg, 54%) as a yellow oil. Mass spec: m/z 504 $[M+H]^+$.

**Step 5**

**Intermediate 150: rac- N-[2-(4,4-dimethylpyrrolidin-2-yl)imidazo[1,2-a]pyrazin-6-yl]-1,3-dimethylindazole-6-carboxamide trifluoroacetate**

To a solution of rac-tert-butyl 2-[6-(1,3-dimethylindazole-6-amido)imidazo[1,2-a]pyrazin-2-yl]-4,4-dimethylpyrrolidine-1-carboxylate **(Intermediate 149:** 170 mg, 0.304 mmol) in DCM (3 mL) was added TFA (1 mL). The reaction mixture was stirred at rt for 2h and concentrated under reduced pressure to provide N-[2-(4,4-dimethylpyrrolidin-2-yl)imidazo[1,2-a]pyrazin-6-yl]-1,3-dimethylindazole-6-carboxamide trifluoroacetate **(Intermediate 150:** 130 mg, 95%) as a yellow solid which was used in Step 6 without further purification. Mass spec: m/z 404 $[M+H]^+$.

**Step 6**

**Example 81: rel-1,3-dimethyl-N-12-[(2S)-1,4,4-trimethylpyrrolidin-2-yl]imidazo[1,2-a]pyrazin-6-yl}-1H-indazole-6-carboxamide and Example 82: rel-1,3-dimethyl-N-{2-[(2R)-1,4,4-trimethylpyrrolidin-2-yl]imidazo[1,2-a]pyrazin-6-yl}-1H-indazole-6-carboxamide**

To a solution of rac-N-[2-(4,4-dimethylpyrrolidin-2-yl)imidazo[1,2-a]pyrazin-6-yl]-1,3-dimethylindazole-6-carboxamide trifluoroacetate **(Intermediate 150:** 130 mg, 0.290 mmol) in MeOH (2 mL) was added paraformaldehyde (26.1 mg, 0.870 mmol) and $Et_3N$ (147 mg, 1.45 mmol). The mixture was stirred at rt for 30 min and $NaBH_3CN$ (91.1 mg, 1.45 mmol) was added. The reaction mixture was stirred at 60°C for 1h and then quenched with water (1 mL). The solution was concentrated under reduced pressure and purified by preparative HPLC (column: Chiralpak IF, 2x25 cm, 5 $\mu$m; Eluent: (Hexane/DCM 3:1 (0.5% 2M $NH_3$ in MeOH))/EtOH 70/30; flow rate: 20 mL/min) to afford:

**Example 81: rel-1,3-dimethyl-N-{2-[(2S)-1,4,4-trimethylpyrrolidin-2-yl]imidazo[1,2-a]pyrazin-6-yl}-1H-indazole-6-carboxamide**

First eluting compound (retention time = 6.89 min):
Mass spec: m/z: 418 $[M+H]^+$; $^1H$ NMR (400 MHz, MeOH-$d_4$) $\delta$ ppm 9.41 (s, 1H), 8.82 (s, 1H), 8.18 (s, 1H), 8.07 (s, 1H), 7.82 - 7.83 (m, 1H), 7.70 - 7.71 (m, 1H), 4.11 (s, 3H), 3.67 - 3.72 (m, 1H), 3.02 - 3.05 (m, 1H), 2.57 (s, 3H), 2.33 - 2.35 (m, 4H), 2.14 - 2.16 (m, 1H), 1.94 - 1.97 (m, 1H), 1.30 (s, 3H), 1.18 (s, 3H).

**Example 82: rel-1,3-dimethyl-N-{2-[(2R)-1,4,4-trimethylpyrrolidin-2-yl]imidazo[1,2-a]pyrazin-6-yl}-1H-indazole-6-carboxamide**

Second eluting compound (retention time = 8.74 min):
Mass spec: m/z: 418 $[M+H]^+$; $^1H$ NMR (400 MHz, MeOH-$d_4$) $\delta$ ppm 9.41 (s, 1H), 8.81 (s, 1H), 8.15 - 8.18 (m, 1H), 8.05 - 8.08 (m, 1H), 7.80 - 7.85 (m, 1H), 7.70 - 7.75 (m, 1H), 4.11 (s, 3H), 3.66 - 3.70 (m, 1H), 3.01 - 3.05 (m, 1H), 2.56 - 2.57 (m, 3H), 2.38 - 2.40 (m, 4H), 2.10 - 2.15 (m, 1H), 1.94 - 1.97 (m, 1H), 1.28 (s, 3H), 1.18 (s, 3H).

**Example 83 was synthesized following Scheme 36**

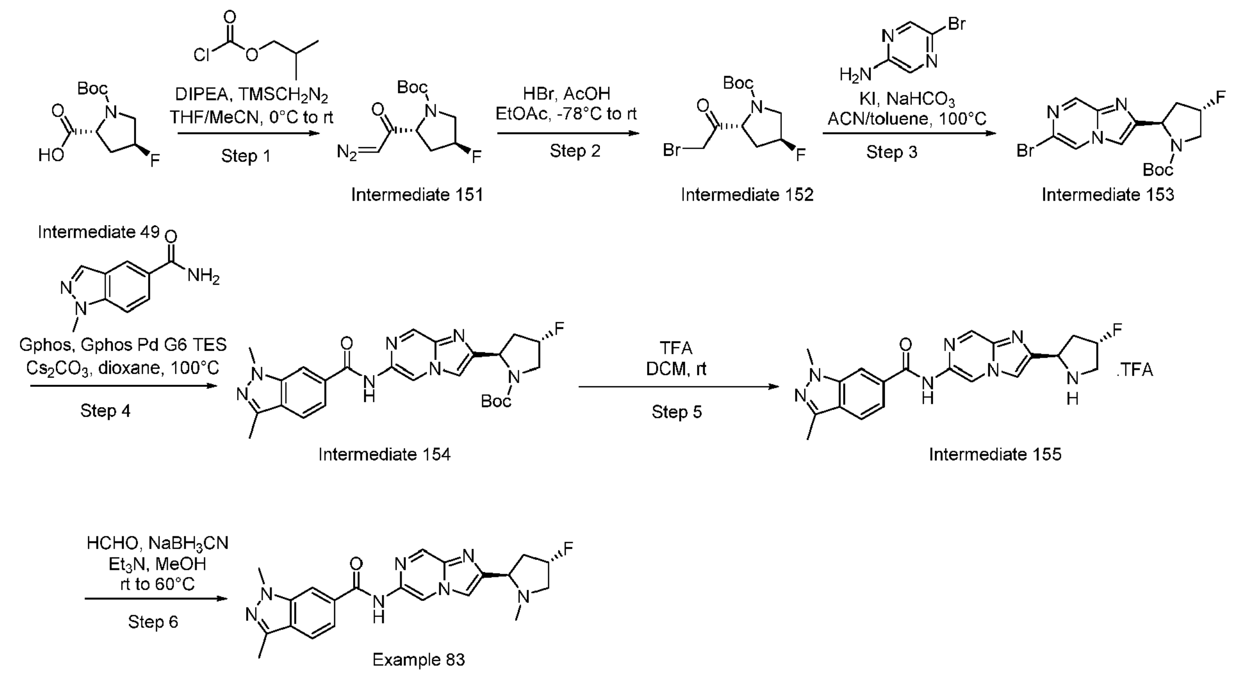


**Scheme 36**

**Step 1**

**Intermediate 151: tert-butyl (2R,4S)-2-(2-diazoacetyl)-4-fluoropyrrolidine-1-carboxylate**

A solution of (2R,4S)-1-(tert-butoxycarbonyl)-4-fluoropyrrolidine-2-carboxylic acid (CAS: 681128-50-7, 5.00 g, 21.4 mmol), DIPEA (4.71 g, 36.4 mmol) and 2-methylpropyl carbonochloridate (4.39 g, 32.1 mmol) in THF (40 mL) at 0°C was stirred for 4h. $M_eCN$ was added until a clear solution was observed and then a solution of trimethylsilyldiazomethane (21.4 mL, 42.9 mmol, 2M in THF) in THF (10 mL) and MeCN (10 mL) was added slowly added. The mixture was stirred at 0°C for 4h and at rt overnight. The reaction mixture was concentrated under reduced pressure and purified by flash chromatography on silica gel using PE/EtOAc (75/25) as eluent to afford tert-butyl (2R,4S)-2-(2-diazoacetyl)-4-fluoropyrrolidine-1-carboxylate **(Intermediate 151:** 3.04 g, 55%) as a yellow oil. Mass spec: m/z 258 $[M+H]^+$.

**Step 2**

**Intermediate 152: tert-butyl (2R,4S)-2-(2-bromoacetyl)-4-fluoropyrrolidine-1-carboxylate**

To a solution of tert-butyl (2R,4S)-2-(2-diazoacetyl)-4-fluoropyrrolidine-1-carboxylate **(Intermediate 151:** 2.6 g, 10.1 mmol) in EtOAc (30 mL) at -78°C was added a solution of HBr (1.3 mL, 1.01 mmol, 33% in AcOH). The reaction mixture was stirred at -78°C for 3h and quenched with a saturated aqueous solution of $NaHCO_3$. The resulting solution was extracted three times with EtOAc and the combined organic layers were washed with brine, dried over anhydrous $Na_2SO_4$, filtered, and concentrated under reduced pressure to provide tert-butyl (2R,4S)-2-(2-bromoacetyl)-4-fluoropyrrolidine-1-carboxylate **(Intermediate 152:** 2.6 g, 83%) as a yellow oil. Mass spec: m/z 310 $[M+H]^+$.

**Step 3**

**Intermediate 153: tert-butyl (2R,4S)-2-{6-bromoimidazo[1,2-a] pyrazin-2-yl}-4-fluoropyrrolidine-1-carboxylate**

To a mixture of tert-butyl (2R,4S)-2-(2-bromoacetyl)-4-fluoropyrrolidine-1-carboxylate **(Intermediate 152:** 2.3 g, 7.41 mmol) in $CH_3CN$ (69 mL) and toluene (23 mL) were added 5-bromopyridin-2-amine (1.29 g, 7.41 mmol), KI (1.85

g, 11.1 mmol) and $NaHCO_3$ (0.750 g, 8.90 mmol). The reaction mixture was stirred at 100°C for 18h and concentrated under reduced pressure. The crude was purified by reverse-phase flash chromatography (C18 aq) using $H_2O$/MeCN (56/44) as eluent to afford tert-butyl (2R,4S)-2-{6-bromoimidazo[1,2-a] pyrazin-2-yl}-4-fluoropyrrolidine-1-carboxylate **(Intermediate 153:** 680 mg, 52%) as a brown solid. Mass spec: m/z 385 $[M+H]^+$.

**Step 4**

**Intermediate 154: (2R,4S)-tert-butyl 2-(6-(1-methyl-1H-indazole-5-carboxamido)imidazo[1,2-a]pyrazin-2-yl)-4-fluoropyrrolidine-1-carboxylate**

A mixture of tert-butyl (2R,4S)-2-{6-bromoimidazo[1,2-a]pyrazin-2-yl}-4-fluoropyrrolidine-1-carboxylate **(Intermediate 153:** 300 mg, 0.779 mmol), 1-methylindazole-5-carboxamide **(Intermediate 49:** 164 mg, 0.935 mmol), $Cs_2CO_3$ (761 mg, 2.34 mmol), Gphos (41.8 mg, 0.078 mmol) and Gphos Pd G6 TES (73.5 mg, 0.078 mmol) in dioxane (10 mL) was stirred at 100°C overnight under a nitrogen atmosphere. The reaction mixture was quenched with water and concentrated under reduced pressure. The crude was purified by reverse-phase flash chromatography (C18 aq) using $H_2O$/MeCN (56/44) as eluent to afford (2R,4S)-tert-butyl 2-(6-(1-methyl-1H-indazole-5-carboxamido)imidazo[1,2-a]pyrazin-2-yl)-4-fluoropyrrolidine-1-carboxylate **(Intermediate 154:** 200 mg, 54%) as a yellow oil. Mass spec: m/z 480 $[M+H]^+$.

**Step 5**

**Intermediate 155: N-{2-[(2R,4S)-4-fluoropyrrolidin-2-yl] imidazo[1,2-a] pyrazin-6-yl}-1-methylindazole-5-carboxamide trifluoroacetate**

To a solution of (2R,4S)-tert-butyl 2-(6-(1-methyl-1H-indazole-5-carboxamido)imidazo[1,2-a]pyrazin-2-yl)-4-fluoropyrrolidine-1-carboxylate **(Intermediate 154:** 200 mg, 0.417 mmol) in DCM (9 mL) was added TFA (3 mL). The mixture was stirred at rt for 2h and concentrated under reduced pressure to provide N-{2-[(2R,4S)-4-fluoropyrrolidin-2-yl] imidazo[1,2-a] pyrazin-6-yl }-1-methylindazole-5-carboxamide trifluoroacetate **(Intermediate 155:** 160 mg, 98%) as a yellow solid. Mass spec: m/z 380 $[M+H]^+$.

**Step 6**

**Example 83: N-(2-((2R,4S)-4-fluoro-1-methylpyrrolidin-2-yl)imidazo[1,2-a]pyrazin-6-yl)-1,3-dimethyl-1H-indazole-6-carboxamide**

A solution of N-{2-[(2R,4S)-4-fluoropyrrolidin-2-yl] imidazo[1,2-a] pyrazin-6-yl}-1-methylindazole-5-carboxamide trifluoroacetate **(Intermediate 155:** 160 mg, 0.422 mmol) in MeOH (3 mL) were added $Et_3N$ (213 mg, 2.11 mmol) and paraformaldehyde (63.3 mg, 2.11 mmol). The reaction mixture was stirred at rt for 1h and $NaBH_3CN$ (79.5 mg, 1.27 mmol) was added. The reaction mixture was stirred at 60°C for 2h, quenched with water, and concentrated under reduced pressure. The mixture was purified by preparative HPLC (Column: XBridge Prep OBD C18 30x150 mm, 5μm; ($H_2O$ (10 mmol/L $NH_4HCO_3$ + 0.05% $NH_4OH$)/MeCN from 30 to 60% from MeCN; Flow rate: 60 mL/min) to afford N-{2-[(2R,4S)-4-fluoro-1-methylpyrrolidin-2-yl]imidazo[1,2-a]pyrazin-6-yl}-1-methylindazole-5-carboxamide **(Example 83**: 53.6 mg, 32%) as a white solid. Mass spec: m/z 394 $[M+H]^+$; $^1H$ NMR (400 MHz, DMSO-$d_6$) δ ppm 10.84 (s, 1H), 9.40 (s, 1H), 8.92 (s, 1H), 8.59 (s, 1H), 8.19 - 8.24 (m, 2H), 8.09 (d, J = 8.8 Hz, 1H), 7.75 (d, J = 8.8 Hz, 1H), 5.34 (d, J = 56 Hz,1H), 4.10 (s, 3H), 3.76 - 3.80 (m, 1H), 3.55 - 3.66 (m, 1H), 2.61 (d, J = 12 Hz,1H), 2.21 - 2.41 (m, 5H).

**Example 84 was synthesized following Scheme 37**

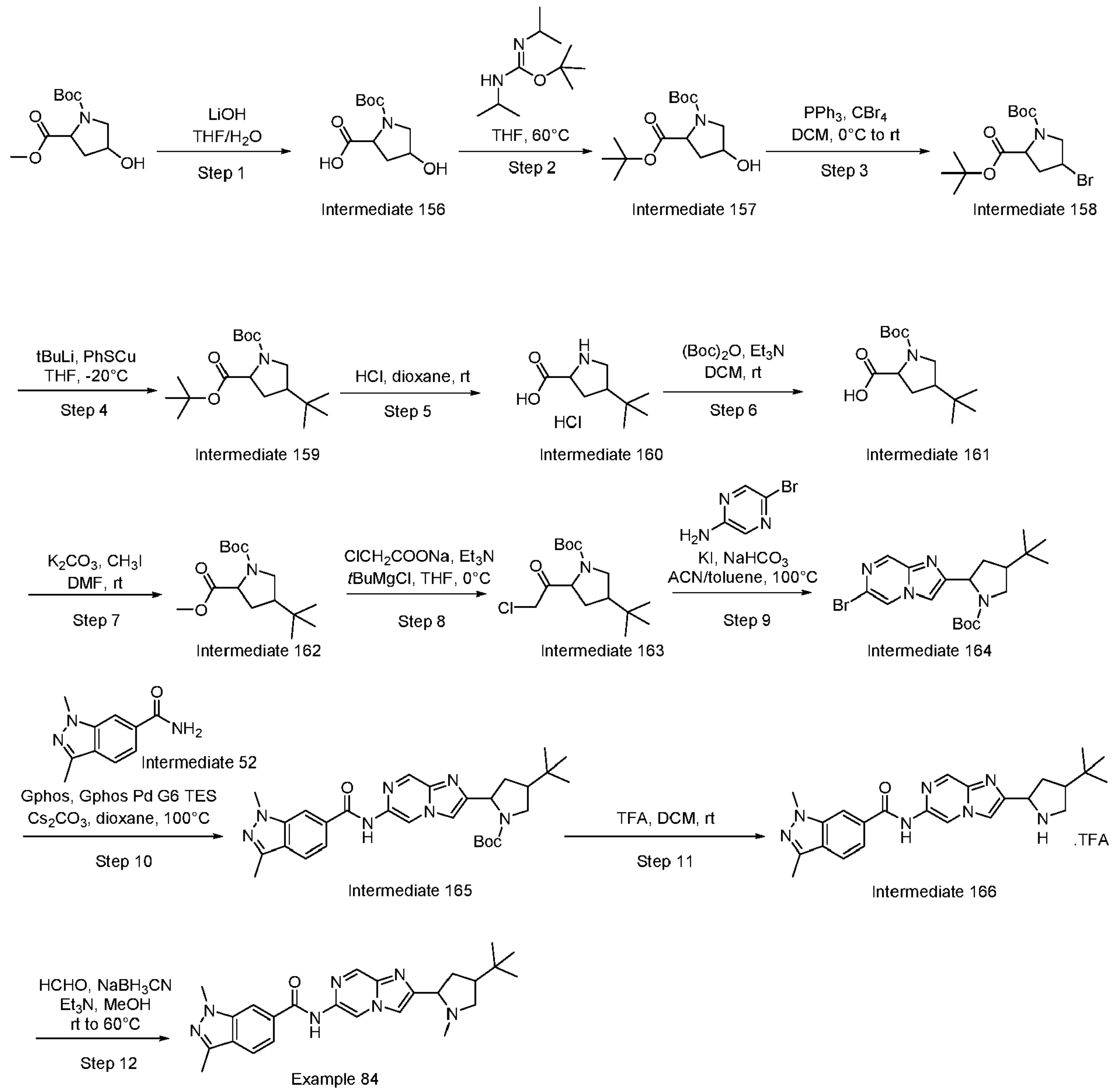


## Scheme 37

### Step 1

### Intermediate 156: rac-1-(tert-butoxycarbonyl)-4-hydroxypyrrolidine-2-carboxylic acid

To a solution of rac-1-tert-butyl 2-methyl 4-hydroxypyrrolidine-1,2-dicarboxylate (CAS: 897046-42-3, 6.0 g, 23.2 mmol) in THF (100 mL) and water (20 mL) was added lithium hydroxide (2.78 g, 116 mmol). The reaction mixture was stirred overnight at rt. The pH value of the solution was adjusted to 4 with an aqueous solution of hydrogen chloride (1 mol/L) and the resulting mixture was extracted three times with EtOAc, washed with brine, dried over anhydrous $Na_2SO_4$, filtered, and concentrated under vacuum to afford 1-(tert-butoxycarbonyl)-4-hydroxypyrrolidine-2-carboxylic acid **(Intermediate 156:** 5.5 g, 95%) as a colorless oil which was used in Step 2 without further purification. Mass spec: m/z 232 $[M+H]^+$.

**Step 2**

**Intermediate 157: rac-di-tert-butyl 4-hydroxypyrrolidine-1,2-dicarboxylate**

To a solution of rac-1-(tert-butoxycarbonyl)-4-hydroxypyrrolidine-2-carboxylic acid **(Intermediate 156:** 5.5 g, 23.8 mmol) in THF (60 mL) was added (Z)-N,N'-diisopropyltert-butoxymethanimidamide (CAS: 71432-55-8, 7.52 g, 35.7 mmol). The reaction mixture was stirred for 2.5h at 60°C. Additional amount of (Z)-N,N'-diisopropyltert-butoxymethanimidamide (7.52 g, 35.7 mmol) was added and the mixture was stirred overnight at 60°C. Precipitated urea was filtered off through celite, washed with $Et_2O$, and the filtrate was evaporated under vacum. The crude material was purified by flash chromatography on silica gel using PE/EtOAc (1/1) as eluent to afford rac-di-tert-butyl 4-hydroxypyrrolidine-1,2-dicarboxylate **(Intermediate 157:** 5.4 g, 71%) as a colorless oil. Mass spec: m/z 288 $[M+H]^+$.

**Step 3**

**Intermediate 158: rac-di-tert-butyl 4-bromopyrrolidine-1,2-dicarboxylate**

To a solution of rac-di-tert-butyl 4-hydroxypyrrolidine-1,2-dicarboxylate **(Intermediate 157:** 5.0 g, 15.7 mmol) and $CBr_4$ (15.4 g, 47.0 mmol) in DCM (45 mL) at 0°C was added carefully $PPh_3$ (12.83 g, 49.0 mmol). The reaction was stirred overnight at rt. EtOH (4 mL) was added, and the solution was stirred for 2h at rt. Precipitation of the phosphine oxide was observed after addition of $Et_2O$ (40 mL). The solids were filtered off, the filter cake was washed with DCM, and the filtrate was evaporated in vacuo. The crude material was purified by flash chromatography on silica gel using EtOAc/PE (3/7) as eluent to afford rac-di-tert-butyl 4-bromopyrrolidine-1,2-dicarboxylate **(Intermediate 158:** 2.7 g, 42%) as a colorless oil. Mass spec: m/z 350 $[M+H]^+$.

**Step 4**

**Intermediate 159: rac-di-tert-butyl 4-(tert-butyl)pyrrolidine-1,2-dicarboxylate**

A suspension of copper(I) thiophenolate PhSCu (1.96 g, 11.4 mmol) in THF (30 mL) was cooled to -20 °C and treated with careful addition of a solution of t-BuLi (8.77 mL, 11.4 mmol, 1.3 M in pentane). The black solution was stirred for 20 min, and a precooled (-20 °C) solution of rac-di-tert-butyl 4-bromopyrrolidine-1,2-dicarboxylate **(Intermediate 158:** 700 mg, 1.91 mmol) in dry THF (5 mL) was added. The reaction was stirred at -20 °C for 16 h and quenched by pouring into a solution of saturated aqueous $NH_4Cl$. The aqueous mixture was stirred vigorously until the black color disappeared. Solids were filtered off, and the phases were separated. The aqueous phase was extracted three times with $Et_2O$, and the combined organic phases were washed with a saturated aqueous $NaHCO_3$ solution and brine. The organic layer was dried over anhydrous $Na_2SO_4$, filtered, and concentrated under reduced pressure. The crude material was purified by flash chromatography on silica gel using EtOAc/PE (595) as eluent to afford rac-di-tert-butyl 4-(tert-butyl)pyrrolidine-1,2-dicarboxylate **(Intermediate 159:** 400 mg, 52%) as a colorless oil. Mass spec: m/z 328 $[M+H]^+$.

**Step 5**

**Intermediate 160: rac-4-(tert-butyl)pyrrolidine-2-carboxylic acid hydrochloride**

To a solution of di-tert-butyl 4-(tert-butyl)pyrrolidine-1,2-dicarboxylate **(Intermediate 159:** 800 mg, 2.08 mmol) in dioxane (20 mL) was added an aqueous solution of HCl (5 mL, 12N). The reaction mixture was stirred for 3h at rt. The mixture was concentrated under reduced pressure to provide rac-4-(tert-butyl)pyrrolidine-2-carboxylic acid hydrochloride **(Intermediate 160:** 450 mg, 86%) as a white solid which was used in Step 6 without further purification. Mass spec: m/z 172 $[M+H]^+$.

**Step 6**

**Intermediate 161: rac-1-(tert-butoxycarbonyl)-4-tert-butylpyrrolidine-2-carboxylic acid**

A solution of rac-4-tert-butylpyrrolidine-2-carboxylic acid hydrochloride **(Intermediate 160:** 450 mg, 2.11 mmol), di-tert-butyl decarbonate $(Boc)_2O$ (1.38 g, 6.32 mmol) and $Et_3N$ (1.07 g, 10.6 mmol) in DCM (15 mL) was stirred for 3h at rt and quenched with water. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using PE/EtOAc (4/96) as eluent to afford rac-1-(tert-butoxycarbonyl)-4-tert-butylpyrrolidine-2-carboxylic acid **(Intermediate 161:** 500 mg, 63%) as a colorless oil. Mass spec: m/z 272 $[M+H]^+$.

**Step 7**

**Intermediate 162: rac-1-tert-butyl 2-methyl 4-tert-butylpyrrolidine-1,2-dicarboxylate**

To a solution of rac-1-(tert-butoxycarbonyl)-4-tert-butylpyrrolidine-2-carboxylic acid **(Intermediate 161:** 500 mg, 1.66 mmol) in DMF (10 mL) was added $K_2CO_3$ (687 mg, 4.98 mmol). The reaction mixture was stirred for 15 min at rt and then $CH_3I$ (707 mg, 4.98 mmol) was added. The reaction mixture was stirred for 2h at rt. The reaction mixture was quenched with water and extracted three times with EtOAc. The combined organic layers were washed with brine, dried over anhydrous $Na_2SO_4$, filtered, and concentrated under reduced pressure to provide 1-tert-butyl 2-methyl 4-tert-butylpyrrolidine-1,2-dicarboxylate **(Intermediate 162:** 500 mg, 76%) as a yellow oil which was used in Step 8 without further purification. Mass spec: m/z 286 $[M+H]^+$.

**Step 8**

**Intermediate 163: rac-tert-butyl 4-tert-butyl-2-(2-chloroacetyl)pyrrolidine-1-carboxylate**

To a solution of rac-1-tert-butyl 2-methyl 4-tert-butylpyrrolidine-1,2-dicarboxylate **(Intermediate 162:** 500 mg, 1.40 mmol), sodium 2-chloroacetate (814 mg, 7.01 mmol) and $Et_3N$ (566 mg, 5.60 mmol) in THF (20 mL) at 0°C was added dropwise a solution of tert-butylmagnesium chloride (4.12 mL, 7.01 mmol, 1.7M in THF). The reaction mixture was stirred overnight at rt. The reaction was quenched with an aqueous saturated $NH_4Cl$ solution and extracted three times with EtOAc. The combined organic layers were washed with brine, dried over anhydrous $Na_2SO_4$, filtered, and concentrated under reduced pressure to provide rac-tert-butyl 4-tert-butyl-2-(2-chloroacetyl)pyrrolidine-1-carboxylate **(Intermediate 163:** 500 mg, 75%) as a yellow oil which was used in Step 9 without further purification. Mass spec: m/z 304 $[M+H]^+$.

**Step 9**

**Intermediate 164: rac-tert-butyl 2-{6-bromoimidazo[1,2-a]pyrazin-2-yl}-4-tert-butylpyrrolidine-1-carboxylate**

A solution of rac-tert-butyl 4-tert-butyl-2-(2-chloroacetyl)pyrrolidine-1-carboxylate **(Intermediate 163:** 500 mg, 1.32 mmol), 5-bromopyrazin-2-amine (264 mg, 1.45 mmol), KI (329 mg, 1.98 mmol) and $NaHCO_3$ (133 mg, 1.58 mmol) in MeCN (10 mL) and toluene (5 mL) was stirred for 3h at 100°C. The reaction mixture was concentrated under reduced pressure and the residue was purified by reverse-phase flash chromatography (C18 aq) using $H_2O$/MeCN (7/3) as eluent to afford tert-butyl 2-{6-bromoimidazo[1,2-a]pyrazin-2-yl}-4-tert-butylpyrrolidine-1-carboxylate **(Intermediate 164:** 65 mg, 7%) as a yellow oil. Mass spec: m/z 423 $[M+H]^+$.

**Step 10**

**Intermediate 165: rac-tert-butyl 4-tert-butyl-2-[6-(1,3-dimethylindazole-6-amido)imidazo[1,2-a]pyrazin-2-yl]pyrrolidine-1-carboxylate**

A solution of rac-tert-butyl 2-{6-bromoimidazo[1,2-a]pyrazin-2-yl}-4-tert-butylpyrrolidine-1-carboxylate **(Intermediate 164:** 60 mg, 0.113 mmol), 1,3-dimethylindazole-6-carboxamide **(Intermediate 52:** 26.2 mg, 0.124 mmol), $Cs_2CO_3$ (111 mg, 0.339 mmol), Gphos (12.17 mg, 0.023 mmol) and Gphos Pd G6 TES (10.70 mg, 0.011 mmol) in dioxane (8 mL) was stirred overnight at 100°C under nitrogen. The reaction mixture was concentrated under vacuo and the residue was purified by reverse-phase flash chromatography (C18 aq) using $H_2O$/MeCN (45/55) as eluent to afford rac-tert-butyl 4-tert-butyl-2-[6-(1,3-dimethylindazole-6-amido)imidazo[1,2-a]pyrazin-2-yl]pyrrolidine-1-carboxylate **(Intermediate 165:** 50 mg, 63%) as a yellow solid. Mass spec: m/z 532 $[M+H]^+$.

**Step 11**

**Intermediate 166: rac-N-[2-(4-tert-butylpyrrolidin-2-yl)imidazo[1,2-a]pyrazin-6-yl]-1,3-dimethylindazole-6-carboxamide trifluoroacetate**

To a solution of rac-tert-butyl 4-tert-butyl-2-[6-(1,3-dimethylindazole-6-amido)imidazo[1,2-a]pyrazin-2-yl]pyrrolidine-1-carboxylate **(Intermediate 165:** 50 mg, 0.071 mmol) in DCM (3 mL) was added TFA (1 mL). The reaction was stirred for 2h at rt and concentrated under reduced pressure to provide rac-N-[2-(4-tert-butylpyrrolidin-2-yl)imidazo[1,2-a]pyrazin-6-yl]-1,3-dimethylindazole-6-carboxamide trifluoroacetate **(Intermediate 166:** 40 mg, 82%) as a yellow oil which was used in Step 12 without further purification. Mass spec: m/z 432 $[M+H]^+$.

**Step 12**

**Example 84: rac-N-[2-(4-tert-butyl-1-methylpyrrolidin-2-yl)imidazo[1,2-a]pyrazin-6-yl]-1,3-dimethylindazole-6-carboxamide**

A solution of rac-N-[2-(4-tert-butylpyrrolidin-2-yl)imidazo[1,2-a]pyrazin-6-yl]-1,3-dimethylindazole-6-carboxamide trifluoroacetate **(Intermediate 166:** 40 mg, 0.058 mmol), $Et_3N$ (29.55 mg, 0.290 mmol) and paraformaldehyde (3.51 mg, 0.116 mmol) in MeOH (2 mL) was stirred for 1h at rt. $NaBH_3CN$ (5.50 mg, 0.087 mmol) was added and the reaction mixture was stirred for 1h at 60°C and quenched with water. The mixture was concentrated under reduced pressure and the residue was purified by preparative HPLC (Column: XBridge Prep OBD C18 Column, 30x150 mm, 5μm; Eluent: MeCN in Water (10mmol/L $NH_4HCO_3$) from 28 to 58%; flow rate: 60 mL/min) to afford rac-N-[2-(4-tert-butyl-1-methylpyrrolidin-2-yl)imidazo[1,2-a]pyrazin-6-yl]-1,3-dimethylindazole-6-carboxamide **(Example 84:** 5.2 mg, 20%) as a white solid. Mass spec: m/z 446 $[M+H]^+$; $^1H$ NMR (400 MHz, MeOH-$d_4$) δ ppm 9.43 (s, 1H), 8.84 (s, 1H), 8.20 (s, 1H), 8.06 (s, 1H), 7.85 (d, J = 8.8 Hz, 1H), 7.73 - 7.75 (m, 1H), 4.10 (s, 3H), 3.48 (t, J = 8.4 Hz, 1H), 3.16 - 3.17 (m, 1H), 2.59 (m, 3H), 2.31 - 2.34 (m, 5H), 2.20 - 2.28 (m, 1H), 2.10 - 2.18 (m, 1H), 0.97 (s, 9H).

**Examples 85 and 86 were synthesized following Scheme 37**

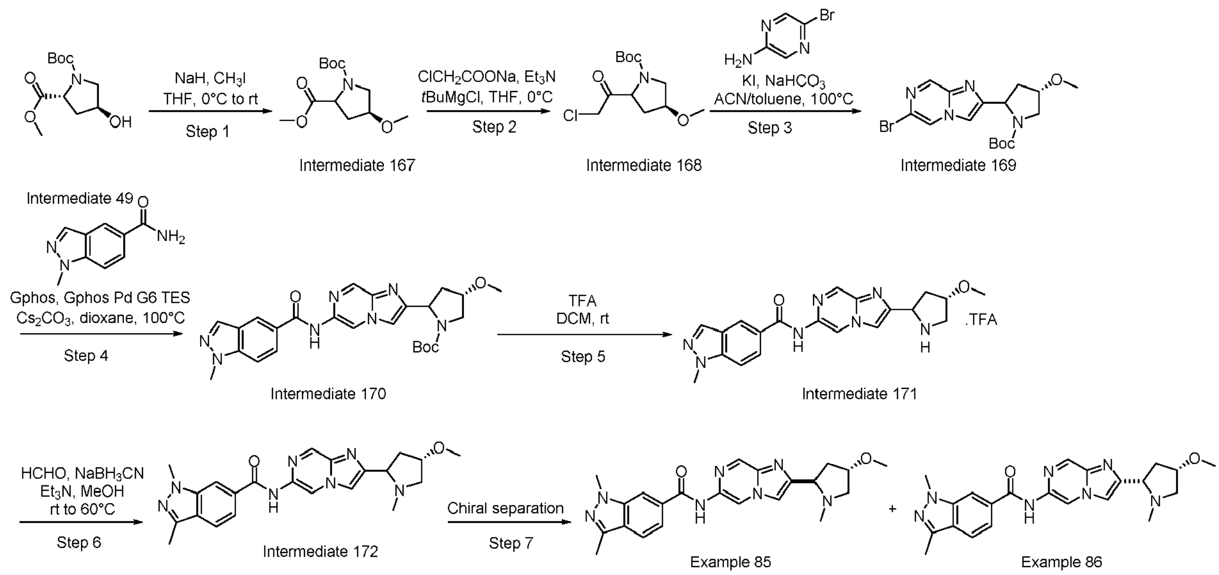


**Scheme 37**

**Step 1**

**Intermediate 167: 1-tert-butyl 2-methyl (4S)-4-methoxypyrrolidine-1,2-dicarboxylate**

To a solution of 1-tert-butyl 2-methyl (2R,4S)-4-hydroxypyrrolidine-1,2-dicarboxylate (CAS: 135042-17-0, 4.95g, 20.2 mmol) in THF (50.0 mL) was added NaH (60% in mineral oil, 0.980 g, 24.5 mmol) at 0°C. The reaction mixture was stirred for 30 min. Then MeI (4.34 g, 30.6 mmol) was added and the reaction mixture was stirred 2h at rt. The reaction mixture was quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (6/4) as eluent to afford 1-tert-butyl 2-methyl (4S)-4-methoxypyrrolidine-1,2-dicarboxylate **(Intermediate 167:** 4.52 g, 78%) as an off-white oil. Mass spec: m/z 260 $[M+H]^+$.

**Step 2**

**Intermediate 168: tert-butyl (4S)-2-(2-chloroacetyl)-4-methoxypyrrolidine-1-carboxylate**

To a solution of 1-tert-butyl 2-methyl (4S)-4-methoxypyrrolidine-1,2-dicarboxylate **(Intermediate 167:** 4.04 g, 15.6 mmol) and sodium 2-chloroacetate (10.1 g, 86.8 mmol) in THF (40 mL) was added a solution of tert-butylmagnesium chloride (51.0 mL, 86.8mmol 1.7 M in THF) at 0°C under nitrogen atmosphere. The reaction mixture was stirred overnight at rt. The reaction mixture was quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (8/2) as eluent to afford tert-butyl (4S)-2-(2-chloroacetyl)-4-methoxypyrrolidine-1-carboxylate **(Intermediate 168:** 3.12 g, 39%) as a yellow oil. Mass spec: m/z 278 $[M+H]^+$.

**Step 3**

**Intermediate 169: tert-butyl (4S)-2-{6-bromoimidazo[1,2-a]pyrazin-2-yl}-4-methoxypyrrolidine-1-carboxylate**

To a solution of tert-butyl (4S)-2-(2-chloroacetyl)-4-methoxypyrrolidine-1-carboxylate **(Intermediate 168:** 1.86 g, 6.72 mmol) in MeCN (22.5 mL) and toluene (7.50 mL) were added KI (3.71 g, 22.3 mmol), 5-bromopyrazin-2-amine (2.33 g, 13.4 mmol) and $NaHCO_3$ (4.69 g, 55.8 mmol). The reaction mixture was stirred for 3h at 100°C and quenched with water. The resulting solution was extracted three times with EtOAc. The combined organic layers were washed with brine, dried over anhydrous $Na_2SO_4$, filtered, and concentrated under reduced pressure. The crude material was purified by flash chromatography on silica gel using EtOAc/PE (3/1) as eluent to afford tert-butyl (4S)-2-{6-bromoimidazo[1,2-a]pyrazin-2-yl}-4-methoxypyrrolidine-1-carboxylate **(Intermediate 169:** 485 mg, 11%) as a yellow oil. Mass spec: m/z 397 $[M+H]^+$.

**Step 4**

**Intermediate 170: tert-butyl(4S)-4-methoxy-2-[6-(1-methylindazole-5-amido) imidazo[1,2-a] pyrazin-2-yl]pyrrolidine-1-carboxylate**

A solution of tert-butyl (4S)-2-{6-bromoimidazo[1,2-a] pyrazin-2-yl}-4-methoxypyrrolidine-1-carboxylate **(Intermediate 169:** 295 mg, 0.742 mmol) , 1-methylindazole-5-carboxamide **(Intermediate 49:** 159 mg, 0.906 mmol), $Cs_2CO_3$ (1.23 g, 3.78 mmol), Gphos (80.9 mg, 0.151 mmol) and Gphos Pd G6 TES (142mg, 0.151 mmol) in 1,4-dioxane (2 mL) was stirred 2h at 100°C under a nitrogen atmosphere. The mixture was concentrated in vacuo and the residue was purified by reverse-phase flash chromatography (C18 aq) using $H_2O$/MeCN (5/2) as eluent to afford tert-butyl(4S)-4-methoxy-2-[6-(1-methylindazole-5-amido) imidazo[1,2-a] pyrazin-2-yl]pyrrolidine-1-carboxylate **(Intermediate 170:** 290 mg, 78%) as a brown solid. Mass spec: m/z 492 $[M+H]^+$.

**Step 5**

**Intermediate 171: N-{2-[(4S)-4-methoxypyrrolidin-2-yl]imidazo[1,2-a]pyrazin-6-yl}-1-methylindazole-5-carboxamide trifluoroacetate**

To a solution of tert-butyl (4S)-4-methoxy-2-[6-(1-methylindazole-5-amido)imidazo[1,2-a]pyrazin-2-yl]pyrrolidine-1-carboxylate **(Intermediate 170:** 258 mg, 0.525 mmol) in DCM (3 mL) was added TFA (1 mL). The reaction mixture was stirred for 2h at rt and concentrated under reduced pressure to provide N-{2-[(4S)-4-methoxypyrrolidin-2-yl]imidazo[1,2-a]pyrazin-6-yl}-1-methylindazole-5-carboxamide trifluoroacetate **(Intermediate 171:** 220 mg, 65%) as a brown oil which was used in Step 6 without further purification. Mass spec: m/z 392 $[M+H]^+$.

**Step 6**

**Intermediate 172: N-{2-[(4S)-4-methoxy-1-methylpyrrolidin-2-yl]imidazo[1,2-a]pyrazin-6-yl}-1-methylindazole-5-carboxamide**

To a solution of N-{2-[(4S)-4-methoxypyrrolidin-2-yl]imidazo[1,2-a]pyrazin-6-yl}-1-methylindazole-5-carboxamide trifluoroacetate **(Intermediate 171:** 34.3 mg, 0.088 mmol) in MeOH was added $Et_3N$ (26.6 mg,0.263 mmol) and paraformaldehyde (23.0 mg, 0.768 mmol). The reaction mixture was stirred 1h at rt, then $NaBH_3CN$ (24.1 mg, 0.384

mmol) was added. The reaction mixture was stirred 2h at 60°C and then concentrated under reduced pressure. The residue was purified by preparative HPLC (Column: XBridge Prep OBD C18 Column, 30x150 mm, 5μm; Eluent: MeCN in Water (10mmol/L $NH_4HCO_3$ + 0.05% $NH_4OH$) from 27 to 57%; flow rate: 60 mL/min) to afford N-{2-[(4S)-4-methoxy-1-methylpyrrolidin-2-yl]imidazo[1,2-a]pyrazin-6-yl}-1-methylindazole-5-carboxamide **(Intermediate 172:** 33 mg, 62%) as a white solid as a mixture of diastereoisomers. Mass spec: m/z 406 $[M+H]^+$.

**Step 7**

**Example 85: N-{2-[(rel-2R,4S)-4-methoxy-1-methylpyrrolidin-2-yl]imidazo[1,2-a]pyrazin-6-yl}-1-methylindazole-5-carboxamide and Example 86: N-{2-[(rel-2S,4S)-4-methoxy-1-methylpyrrolidin-2-yl]imidazo[1,2-a]pyrazin-6-yl}-1-methylindazole-5-carboxamide**

N-{2-[(4S)-4-methoxy-1-methylpyrrolidin-2-yl]imidazo[1,2-a]pyrazin-6-yl}-1-methylindazole-5-carboxamide **(Intermediate 172)** was subjected to chiral preparative HPLC (Column: ChiralPak IF, 2x25 cm, 5 μm; Eluent: EtOH/(Hexane/DCM 3/1 (0.5% 2M $NH_3$ in MeOH)) 30/70; flow rate: 20 mL/min) to isolate the following examples:

**Example 85: N-{2-[(rel-2R,4S)-4-methoxy-1-methylpyrrolidin-2-yl]imidazo[1,2-a]pyrazin-6-yl}-1-methylindazole-5-carboxamide**

First eluting compound (retention time = 14.3 min):
Mass spec: m/z: 406 $[M+H]^+$; $^1H$ NMR (400 MHz, DMSO-$d_6$) δ ppm 10.84 (s, 1H), 9.40 (s, 1H), 8.90 (s, 1H), 8.58 (s, 1H), 8.23 (s, 1H), 8.16 - 8.17 (m, 1H), 8.07 - 8.10 (m, 1H), 7.74 - 7.76 (m, 1H), 4.10 (s, 3H), 4.00 - 4.02 (m, 1H), 3.46 - 3.50 (m, 1H), 3.32 - 3.46 (m, 1H), 3.20 (s, 3H), 2.50 - 2.52 (m, 1H), 2.30 - 2.32 (m, 1H), 2.17 - 2.21 (m, 2H), 2.15 - 2.16 (m, 2H).

**Example 86: N-{2-[(rel-2S,4S)-4-methoxy-1-methylpyrrolidin-2-yl]imidazo[1,2-a]pyrazin-6-yl}-1-methylindazole-5-carboxamide**

White solid (26.6 mg, 21%)
Second eluting compound (retention time = 8.74 min):
Mass spec: m/z: 406 $[M+H]^+$; $^1H$ NMR (400 MHz, DMSO-$d_6$) δ ppm 10.82 (s, 1H), 9.40 (s, 1H), 8.90 (s, 1H), 8.59 (s, 1H), 8.23 s, 1H), 8.17 (s, 1H), 8.09 - 8.10 (m, 1H), 7.74 - 7.76 (m, 1H), 4.10 (s, 3H), 3.90 - 3.95 (m, 1H), 3.33 - 3.37 (m, 1H), 3.16 - 3.20 (m, 4H), 2.50 - 2.60 (m, 1H), 2.31 - 2.36 (m, 1H), 2.18 (s, 3H), 1.86 - 1.90 (m, 1H).

**Intermediates 173-175 were synthesized following an analogous procedure to Scheme 8.**

**Step 2.**

**Intermediate 173: tert-butyl (2R)-2-(6-bromo-8-cyclopropyl-imidazo[1,2-a]pyrazin-2-yl)pyrrolidine-1-carboxylate**

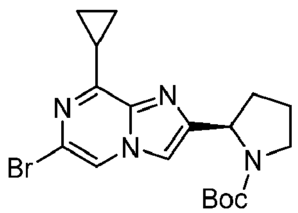

Following an analogous procedure to **Scheme 8, Step 2,** using 5-bromo-3-cyclopropylpyrazin-2-amine (CAS: 1845754-43-9, 1.00 g, 4.67 mmol) as the amine and tert-butyl (2R)-2-(2-chloroacetyl)pyrrolidine-1-carboxylate **(Intermediate 40:** 1.83 g, 7.00 mmol) as the ketone, afforded tert-butyl (2R)-2-(6-bromo-8-cyclopropyl-imidazo[1,2-a]pyrazin-2-yl)pyrrolidine-1-carboxylate **(Intermediate 173:** 639 mg, 34%) as a white solid. Mass spec: m/z: 407 $[M+H]^+$, 409 $[M+H+2]^+$; $^1H$ NMR (400 MHz, DMSO-$d_6$) δ ppm 8.64 (d, J = 13.8 Hz, 1H), 7.83 (d, J = 12.0 Hz, 1H), 4.98 (s, 1H), 3.46 (s, 1H), 3.40 (s, 1H), 2.79 (d, J = 7.0 Hz, 1H), 2.23 (s, 1H), 2.06 (d, J = 6.9 Hz, 1H), 1.96 - 1.83 (m, 2H), 1.41 (s, 4H),

1.22 - 1.14 (m, 9H).

Step3.

**Intermediate 174: 6-bromo-8-cyclopropyl-2-[(2R)-pyrrolidin-2-yl]imidazo[1,2-a]pyrazine trifluoroacetate**

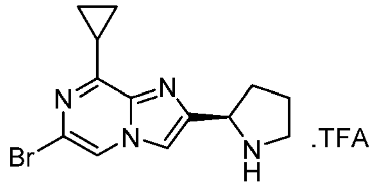

Following an analogous procedure to **Scheme 8, Step 3,** starting with tert-butyl (2R)-2-(6-bromo-8-cyclopropylimidazo[1,2-a]pyrazin-2-yl)pyrrolidine-1-carboxylate **(Intermediate 173:** 639 mg, 1.57 mmol), afforded 6-bromo-8-cyclopropyl-2-[(2R)-pyrrolidin-2-yl]imidazo[1,2-a]pyrazine trifluoroacetate **(Intermediate 174:** 1.13 g, quant.) as a colorless oil. Mass spec: m/z: 307 $[M+H]^+$, 309 [M+H+2]+; $^1$H NMR (400 MHz, DMSO-$d_6$) δ ppm 9.65 (s, 1H), 8.96 (s, 1H), 8.77 (s, 1H), 8.19 (s, 1H), 4.89 (p, J = 7.0 Hz, 1H), 3.43 - 3.23 (m, 2H), 2.84 (tt, J = 7.7, 5.0 Hz, 1H), 2.43 (qd, J = 7.9, 4.4 Hz, 1H), 2.27 - 2.10 (m, 2H), 2.10 - 2.01 (m, 1H), 1.25 - 1.19 (m, 4H).

Step 4

**Intermediate 175: 6-bromo-8-cyclopropyl-2-[(2R)-1-methylpyrrolidin-2-yl]imidazo[1,2-a]pyrazine**

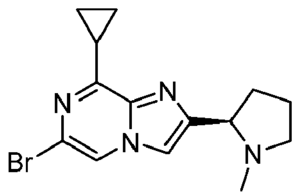

Following an analogous procedure to Scheme 8, Step 4, starting with 6-bromo-8-cyclopropyl-2-[(2R)-pyrrolidin-2-yl]imidazo[1,2-a]pyrazine trifluoroacetate **(Intermediate 174:** 1134 mg, 1.64 mmol) as the amine, afforded 6-bromo-8-cyclopropyl-2-[(2R)-1-methylpyrrolidin-2-yl]imidazo[1,2-a]pyrazine **(Intermediate 175:** 520 mg, 99%) as a yellow oil. Mass spec: m/z: 321 $[M+H]^+$, 323 $[M+H+2]^+$; $^1$H NMR (400 MHz, DMSO-$d_6$) δ ppm 10.02 (s, 1H), 8.78 (s, 1H), 8.25 (s, 1H), 4.69 (s, 1H), 3.70 (s, 1H), 3.26 (s, 1H), 2.86 (ddd, J = 14.8, 6.8, 4.2 Hz, 4H), 2.35 (d, J = 10.6 Hz, 1H), 2.11 (d, J = 29.9 Hz, 2H), 1.28 - 1.15 (m, 4H).

**Intermediates 176-180 were synthesized following** Scheme 38.

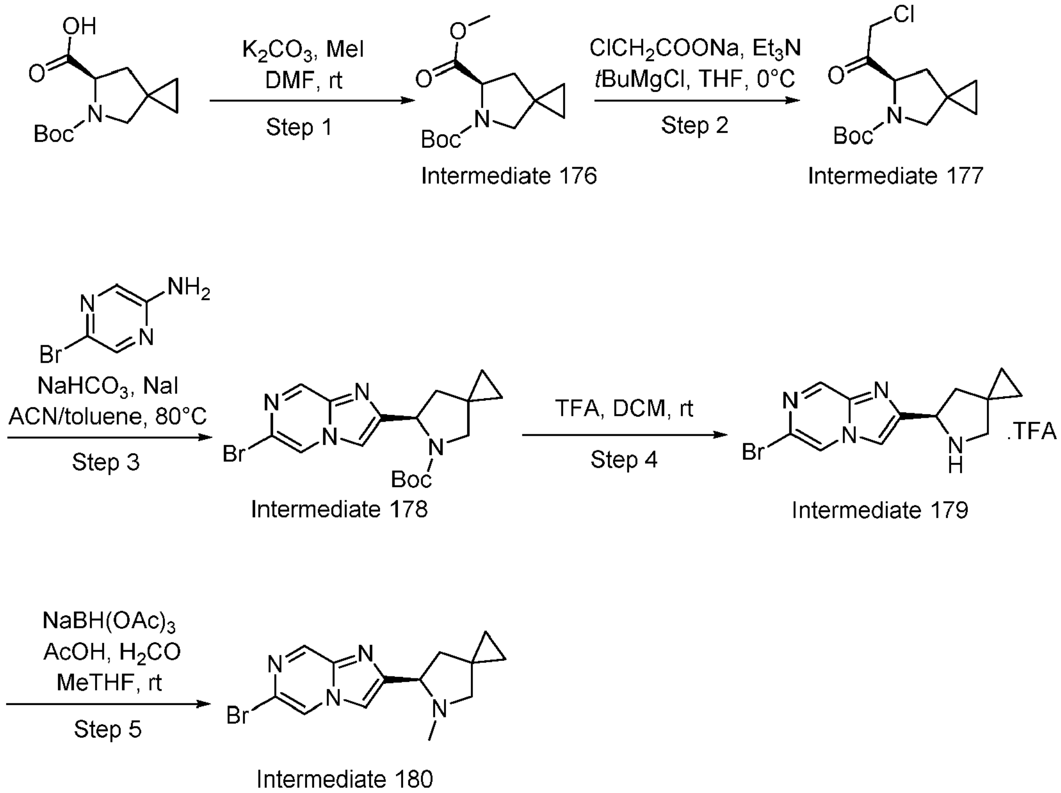


Intermediate 176

Intermediate 177

Intermediate 178

Intermediate 179

Intermediate 180

**Scheme 38**

**Step 1**

**Intermediate 176: 5-(tert-butyl) 6-methyl (R)-5-azaspiro[2.4]heptane-5,6-dicarboxylate**

To a stirred solution of (R)-5-(tert-butoxycarbonyl)-5-azaspiro[2.4]heptane-6-carboxylic acid (CAS: 1454843-78-7, 930 mg, 3.85 mmol) in DMF (13 mL) was added $K_2CO_3$ (1116 mg, 8.07 mmol). The mixture was stirred at rt for 5 min, followed by addition of iodomethane (576 uL, 9.25 mmol). The reaction mixture was stirred at rt overnight. After addition of water and EtOAc to the mixture, the phases were separated and the aqueous phase was extracted twice with EtOAc. The combined organic layers were washed with brine, dried over anhydrous $Na_2SO_4$, filtered, and concentrated under vacuum to afford 5-(tert-butyl) 6-methyl (R)-5-azaspiro[2.4]heptane-5,6-dicarboxylate **(Intermediate 176:** 930 mg, 95%) as a colorless oil which was used in Step 2 without further purification. $^1H$ NMR (400 MHz, DMSO-$d_6$) $\delta$ ppm 4.30 (ddd, J = 11.5, 8.6, 4.2 Hz, 1H), 3.65 (d, J = 10.7 Hz, 3H), 3.32 - 3.15 (m, 2H), 2.26 (ddd, J = 12.7, 11.2, 8.6 Hz, 1H), 1.70 (ddd, J = 34.3, 12.7, 4.2 Hz, 1H), 1.36 (d, J = 21.2 Hz, 9H), 0.62 - 0.40 (m, 4H).

**Step 2**

**Intermediate 177: tert-butyl (6R)-6-(2-chloroacetyl)-5-azaspiro[2.4]heptane-5-carboxylate**

To a solution of 5-(tert-butyl) 6-methyl (R)-5-azaspiro[2.4]heptane-5,6-dicarboxylate **(Intermediate 176:** 0.93 g, 3.64 mmol), sodium chloroacetate (1.11 g, 9.05 mmol) and $Et_3N$ (1.0 mL, 7.17 mmol) in anhydrous THF (8.2 mL) was added dropwise a solution of tert-butylmagnesium chloride (9.0 mL, 9.00 mmol, 1M in THF) at 2-3 °C over 20 min. The reaction mixture was stirred at 0-5 °C for 1.5h. A solution of citric acid (1.62 g, 8.39 mmol) in water (4.5 mL) was slowly added to the reaction mixture while maintaining the temperature between 0-5 °C. EtOAc was added to the reaction mixture and the phases were separated. The organic layer was washed twice with a solution of $NaHCO_3$/NaCl (2.35 g of $NaHCO_3$, 8 g of NaCl in 50 mL) and once with brine. The combined organic layers were dried over hydrophobic paper and concentrated under reduced pressure to afford tert-butyl (6R)-6-(2-chloroacetyl)-5-azaspiro[2.4]heptane-5-carboxylate **(Intermediate 177:** 0.965 g, 92%) as a colorless oil which was used in Step 3 without further purification. Mass spec: m/z: 174 $[M+H-Boc]^+$, 218 $[M+H-tBu]^+$; $^1H$ NMR (400 MHz, DMSO-$d_6$) $\delta$ ppm 4.74 - 4.58 (m, 2H), 4.54 (ddd, J = 11.1, 8.9, 4.8 Hz, 1H), 3.32 - 3.20 (m, 2H), 2.21 (ddd, J = 12.8, 10.6, 8.8 Hz, 1H), 1.77 (ddd, J = 12.9, 11.4, 4.6 Hz, 1H),

1.36 (d, J = 27.3 Hz, 9H), 0.62 - 0.42 (m, 4H).

**Step 3**

**Intermediate 178: tert-butyl (6R)-6-(6-bromoimidazo[1,2-a]pyrazin-2-yl)-5-azaspiro[2.4]heptane-5-carboxylate**

To a solution of 5-bromopyrazin-2-amine (CAS: 59489-71-3, 530 mg, 3.05 mmol) and tert-butyl (6R)-6-(2-chloroacetyl)-5-azaspiro[2.4]heptane-5-carboxylate **(Intermediate 177:** 965 mg, 3.35 mmol) in toluene (3 mL)/MeCN (3 mL) were added $NaHCO_3$ (307 mg, 3.65 mmol) and sodium iodide (686 mg, 4.55 mmol). The mixture was stirred overnight at 80 °C. Solvents were removed under vacuum and the residue was taken up in EtOAc. The mixture was filtered through a pad of decalite, then the filtrate was washed with a saturated aqueous solution of $Na_2S_2O_3$. The organic layer was dried over anhydrous $Na_2SO_4$, filtered, and concentrated in vacuo. The crude material was purified by flash chromatography on silica gel using a gradient of EtOAc in heptane from 30% to 100% to afford tert-butyl (6R)-6-(6-bromoimidazo[1,2-a]pyrazin-2-yl)-5-azaspiro[2.4]heptane-5-carboxylate **(Intermediate 178:** 175 mg, 15%) as an orange solid. Mass spec: m/z: 393 $[M+H]^+$, 395 $[M+H+2]^+$; $^1H$ NMR (400 MHz, DMSO-$d_6$) δ ppm 8.93 (d, J = 16.2 Hz, 1H), 8.86 (s, 1H), 7.93 (d, J = 20.7 Hz, 1H), 5.09 (s, 1H), 3.52 (d, J = 10.3 Hz, 1H), 3.25 (dd, J = 26.0, 11.0 Hz, 1H), 2.44 (d, J = 10.3 Hz, 1H), 1.86 (dd, J = 25.2,12.4 Hz, 1H), 1.30 (d, J = 87.1 Hz, 9H), 0.53 (dd, J = 19.6, 8.8 Hz, 3H), 0.25 (s, 1H).

**Step 4**

**Intermediate 179: 2-[(6R)-5-azaspiro[2.4]heptan-6-yl]-6-bromo-imidazo[1,2-a]pyrazine trifluoroacetate**

To a solution of tert-butyl (6R)-6-(6-bromoimidazo[1,2-a]pyrazin-2-yl)-5-azaspiro[2.4]heptane-5-carboxylate **(Intermediate 178:** 175 mg, 0.445 mmol) in DCM (4 mL) was added TFA (2.0 mL, 26.1 mmol). The reaction mixture was stirred at rt for 1.5h, then concentrated under vacuum to afford 2-[(6R)-5-azaspiro[2.4]heptan-6-yl]-6-bromo-imidazo[1,2-a]pyrazine trifluoroacetate **(Intermediate 179:** 180 mg, 99%) as a yellow oil which was used in Step 5 without further purification. Mass spec: m/z: 293 $[M+H]^+$, 295 $[M+H+2]^+$, $^1H$ NMR (400 MHz, DMSO-$d_6$) δ ppm 9.88 (s, 1H), 9.28 (s, 1H), 9.06 (d, J = 1.3 Hz, 1H), 9.03 (d, J = 1.3 Hz, 1H), 8.25 (s, 1H), 5.19 - 5.08 (m, 1H), 3.32 (ddd, J = 10.8, 6.4, 4.1 Hz, 1H), 3.20 (dt, J = 11.3, 5.6 Hz, 1H), 2.37 (dd, J = 12.9, 9.0 Hz, 1H), 2.26 (dd, J = 12.9, 7.5 Hz, 1H), 0.75 (tdd, J = 7.5, 3.6, 1.7 Hz, 4H)

**Step 5**

**Intermediate 180: 6-bromo-2-[(6R)-5-methyl-5-azaspiro[2.4]heptan-6-yl]imidazo[1,2-a]pyrazine**

To a solution of 2-[(6R)-5-azaspiro[2.4]heptan-6-yl]-6-bromo-imidazo[1,2-a]pyrazine trifluoroacetate **(Intermediate 179:** 130 mg, 0.319 mmol) in 2-MeTHF (1.6 mL) was added acetic acid (37 uL, 0.646 mmol) followed by formaldehyde (37% aq, 49 uL, 0.653 mmol). The reaction mixture was stirred at rt for 20 min prior addition of $NaBH(OAc)_3$ (100 mg, 0.472 mmol). The reaction mixture was then stirred at rt for 2h. The reaction mixture was quenched with a saturated aqueous solution of $NaHCO_3$ and the aqueous layer was extracted with EtOAc twice. The organic layers were combined, washed with brine, dried over $Na_2SO_4$, filtered, and concentrated under vacuo to afford 6-bromo-2-[(6R)-5-methyl-5-azaspiro[2.4]heptan-6-yl]imidazo[1,2-a]pyrazine **(Intermediate 180:** 79 mg, 81%) as an orange oil which was used in the next step without further purification. Mass spec: m/z: 307 $[M+H]^+$, 309 $[M+H+2]^+$; $^1H$ NMR (400 MHz, DMSO-$d_6$) δ ppm 8.89 (d, J = 1.4 Hz, 1H), 8.84 (d, J = 1.3 Hz, 1H), 8.03 (s, 1H), 3.70 (t, J = 8.2 Hz, 1H), 2.85 (d, J = 8.9 Hz, 1H), 2.54 (d, J = 8.9 Hz, 1H), 2.21 (s, 3H), 2.19 - 2.12 (m, 1H), 1.96 (dd, J = 12.3, 8.9 Hz, 1H), 0.66 - 0.46 (m, 4H).

**Intermediates 181, 182 and 183 were synthesized following Scheme 39**

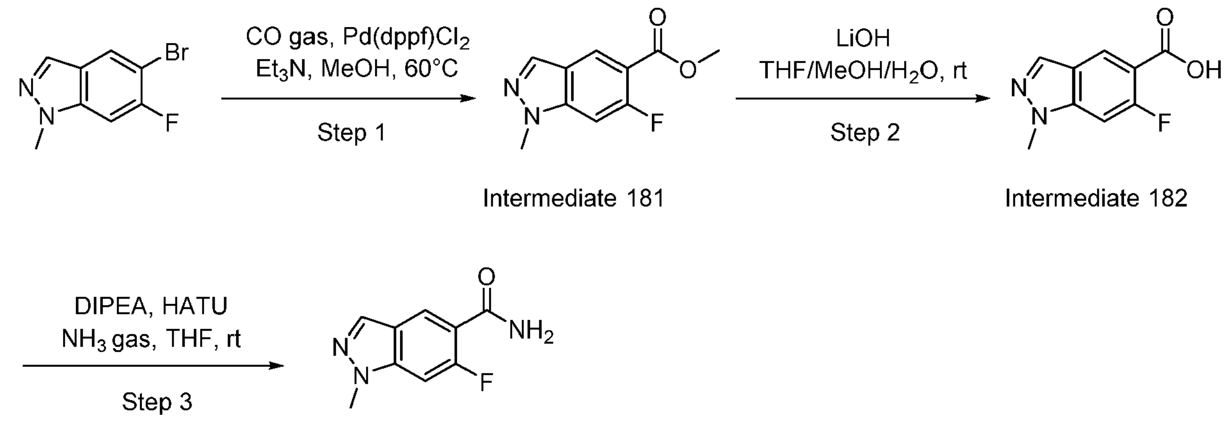


Intermediate 181

Intermediate 182

Intermediate 183

## Scheme 39

**Step 1**

**Intermediate 181: methyl 6-fluoro-1-methyl-indazole-5-carboxylate**

To a solution of 5-bromo-6-fluoro-1-methyl-1H-indazole (CAS: 1185767-06-9, 200 mg, 0.847 mmol) and $Et_3N$ (590 uL, 4.23 mmol) in MeOH (2.5 mL) was added Pd(dppf)$Cl_2$ (63 mg, 0.0846 mmol). The mixture was stirred in a sealed vial and the solvent was saturated with CO gas. The reaction mixture was stirred at 60°C for 17h. The reaction mixture was cooled to rt and additional amount of Pd(dppf)$Cl_2$ (63 mg, 0.0846 mmol) and $Et_3N$ (590 uL, 4.23 mmol) were added at rt and the solvent was saturated with CO gas. The reaction mixture was stirred at 60°C for 22 h. The reaction mixture was filtered through a pad of celite and the filtrate was diluted with EtOAc and water. The layers were separated, and the aqueous layer was extracted with EtOAc twice. The combined organic layers were dried over anhydrous $Na_2SO_4$, filtered, and concentrated under vacuum. The crude material was purified by flash chromatography on silica gel using a gradient of (DCM/MeOH, 9/1) in DCM from 0% to 50% to afford methyl 6-fluoro-1-methyl-indazole-5-carboxylate as a brown solid **(Intermediate 181:** 166 mg, 93%). Mass spec: m/z: 209 $[M+H]^+$, $^1H$ NMR (400 MHz, DMSO-$d_6$) $\delta$ ppm 8.42 (d, J = 7.0 Hz, 1H), 8.24 (d, J = 1.0 Hz, 1H), 7.66 (d, J = 11.9 Hz, 1H), 4.04 (s, 3H), 3.86 (s, 3H).

**Step 2**

**Intermediate 182: 6-fluoro-1-methyl-indazole-5-carboxylic acid**

To a solution of methyl 6-fluoro-1-methyl-indazole-5-carboxylate **(Intermediate 181:** 166 mg, 0.789 mmol) in a mixture of MeOH (1.75 mL), THF (1.75 mL) and $H_2O$ (1.75 mL) was added lithium hydroxide hydrate (66 mg, 1.57 mmol). The reaction mixture was stirred at rt for 3h. The reaction mixture was concentrated under reduced pressure and the residue was diluted with water, acidified to pH 4 by the addition of a saturated aqueous solution of citric acid. A precipitate was formed, filtered, and dried under vacuum overnight to afford 6-fluoro-1-methyl-indazole-5-carboxylic acid **(Intermediate 182:** 135 mg, 76% Yield) as light brown solid. Mass spec: m/z: 195 $[M+H]^+$; $^1H$ NMR (400 MHz, DMSO-$d_6$) $\delta$ ppm 12.99 (s, 1H), 8.38 (d, J = 7.1 Hz, 1H), 8.21 (s, 1H), 7.61 (d, J = 11.7 Hz, 1H), 2.50 (s, 3H).

**Step 3**

**Intermediate 183: 6-fluoro-1-methyl-indazole-5-carboxamide**

A solution of 6-fluoro-1-methyl-indazole-5-carboxylic acid **(Intermediate 182:** 135 mg, 0.598 mmol), DIPEA (0.52 mL, 2.98 mmol) and HATU (279 mg, 0.719 mmol) in DMF (6 mL) was stirred at rt for 30 min in a sealed vial. After this time, $NH_3$ gas was injected and the reaction mixture was stirred at rt for 18h. Then the mixture was diluted with DCM and water. The phases were separated and the aqueous phase was extracted three times with DCM. The combined organic layers were dried over anhydrous $Na_2SO_4$, filtered, and concentrated under vacuum. The residue was triturated in DCM, filtered, and washed with DCM and finally dried under vacuum overnight to afford 6-fluoro-1-methyl-indazole-5-carboxamide **(Intermediate 183:** 94 mg, 77%) as a beige solid. Mass spec: m/z: 194 $[M+H]^+$, $^1H$ NMR (400 MHz, DMSO-$d_6$) δ ppm 8.16 (d, J = 1.0 Hz, 1H), 8.12 (d, J = 6.9 Hz, 1H), 7.67 (s, 1H), 7.60 (d, J = 11.5 Hz, 1H), 7.56 (s, 1H), 4.03 (s, 3H).

**Examples 87-90** shown in **Table 4** below, were prepared as shown **Scheme 32** and following **General Procedure 8.**

**Table 4**

| Example Number | Example Name & Structure | Inter-mediates | NMR and Mass Spec |
|---|---|---|---|
| 87 | 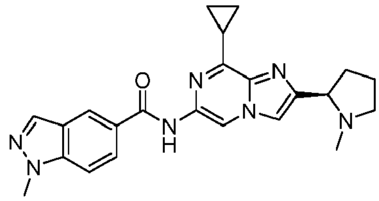 N-[8-cyclopropyl-2-[(2R)-1-methylpyrrolidin-2- yl]imidazo[1,2-a] pyrazin-6- yl]-1-methyl-indazole-5-carboxamide | 175 & 49 | Mass spec: m/z: 416 $[M+H]^+$, $^1H$ NMR (600 MHz, DMSO-$d_6$) δ ppm 10.40 (s, 1 H) 9.10 (s, 1 H) 8.55 (dd, J = 1.5, 0.8 Hz, 1 H) 8.23 (d, J = 1.0 Hz, 1 H) 8.07 (s, 1 H) 8.04 (dd, J = 8.8, 1.6 Hz, 1 H) 7.73 (d, J = 8.9 Hz, 1 H) 4.09 (s, 3 H) 3.41 (br s, 1 H) 3.14 (br s, 1 H) 2.81 - 2.86 (m, 1 H) 2.28 - 2.42 (m, 1 H) 2.20 - 2.28 (m, 4 H) 1.84 - 1.93 (m, 2 H) 1.80 (br s, 1 H) 1.22 - 1.30 (m, 2 H) 1.11 - 1.18 (m, 2 H) |
| 88 | 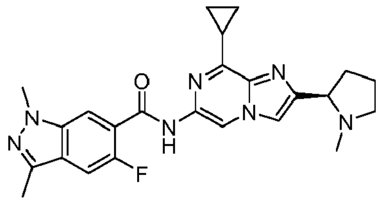 N-[8-cyclopropyl-2-[(2R)-1-methylpyrrolidin-2-yl]imidazo[1,2-a] pyrazin-6-yl]-5-fluoro-1,3-dimethyl-indazole-6-carboxamide | 175 & 55 | Mass spec: m/z: 448 $[M+H]^+$, $^1H$ NMR (600 MHz, DMSO-$d_6$) δ ppm 10.66 (s, 1 H) 9.14 (s, 1 H) 8.10 (br s, 1 H) 7.94 (d, J = 5.4 Hz, 1 H) 7.63 (d, J = 10.3 Hz, 1 H) 4.02 (s, 3 H) 3.35 - 3.50 (m, 1 H) 3.06 - 3.21 (m, 1 H) 2.73 - 2.89 (m, 1 H) 2.47 - 2.48 (m, 3 H) 2.11 - 2.44 (m, 5 H) 1.71 - 1.97 (m, 3 H) 1.22 (br dd, J = 12.2, 5.1 Hz, 2 H) 1.13 (br dd, J = 7.9, 3.2 Hz, 2 H) |
| 89 | 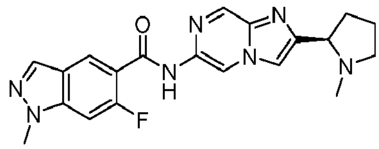 6-fluoro-1 -methyl-N- [2-[(2R)-1-methylpyrrolidin-2-yl]imidazo[1,2-a] pyrazin-6-yl] indazole-5-carboxamide | 183 & 43 | Mass spec: m/z: 394 $[M+H]^+$, $^1H$ NMR (500 MHz, DMSO-$d_6$) δ ppm 10.77 (d, J = 1.25 Hz, 1 H) 9.38 (d, J = 0.75 Hz, 1 H) 8.86 (d, J = 0.75 Hz, 1 H) 8.21 (d, J = 0.88 Hz, 1 H) 8.19 (d, J = 6.88 Hz, 1 H) 8.16 (s, 1 H) 7.68 (d, J = 11.13 Hz, 1 H) 4.06 (s, 3 H) 3.38 - 3.46 (m, 1 H) 3.12 (br t, J = 7.07 Hz, 1 H) 2.31 (br d, J = 8.50 Hz, 1 H) 2.24 (s, 4H) 1.76 - 1.91 (m, 3 H) |
| 90 | 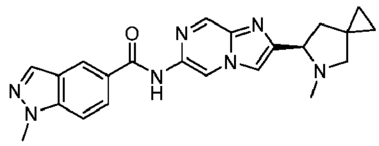 1-methyl-N-[2-[(6R)-5-methyl-5-azaspiro[2.4]heptan-6-yl]imidazo[1,2-a]pyrazin-6-yl] indazole-5-carboxamide | 180 & 49 | Mass spec: m/z: 402 $[M+H]^+$, $^1H$ NMR (600 MHz, DMSO-$d_6$) δ ppm 10.81 (s, 1 H) 9.38 (d, J=1.5 Hz, 1 H) 8.89 (d, J=1.3 Hz, 1 H) 8.58 (d, J=0.9 Hz, 1 H) 8.23 (d, J=0.9 Hz, 1 H) 8.19 (s, 1 H) 8.08 (dd, J=8.8, 1.6 Hz, 1 H) 7.74 (d, J=8.8 Hz, 1 H) 4.09 (s, 3 H) 3.65 (br t, J=8.3 Hz, 1 H) 2.85 (d, J=8.9 Hz, 1 H) 2.52 (d, J=8.9 Hz, 1 H) 2.22 (s, 3 H) 2.14 (dd, J=12.3, 7.5 Hz, 1 H) 2.02 (dd, J=12.3, 9.2 Hz, 1 H) 0.56 - 0.70 (m, 2 H) 0.34 - 0.56 (m, 2 H) |

**Examples 91A and 91B were synthesized following Scheme 40.**

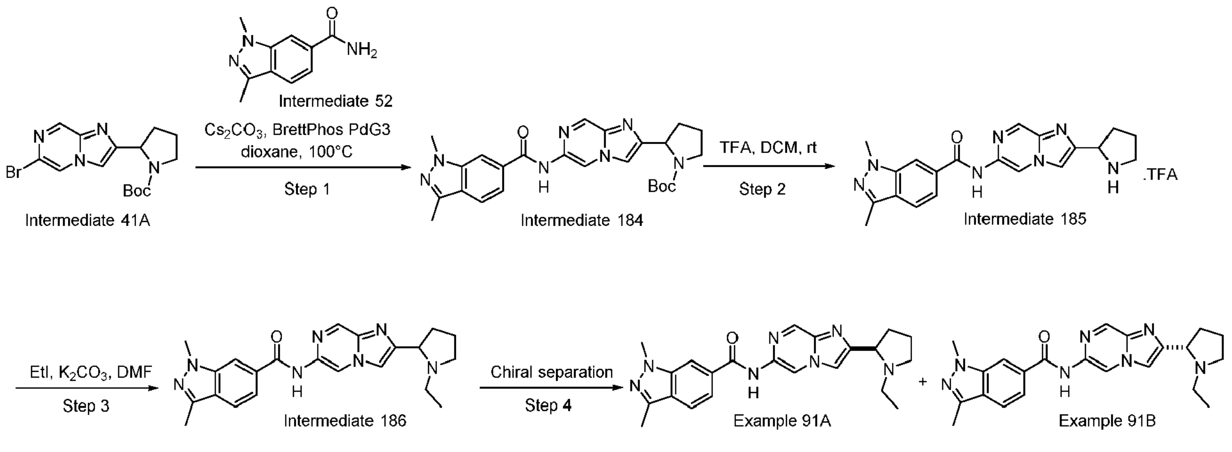


## Scheme 40

### Step 1

**Intermediate 184: rac-tert-butyl 2-[6-(1,3-dimethylindazole-6-amido)imidazo[1,2-a]pyrazin-2-yl]pyrrolidine-1-carboxylate**

A solution of rac-tert-butyl 2-{6-bromoimidazo[1,2-a]pyrazin-2-yl}pyrrolidine-1-carboxylate **(Intermediate 41A:** 500 mg, 1.29 mmol), 1,3-dimethylindazole-6-carboxamide **(Intermediate 52:** 326 mg, 1.55 mmol), $Cs_2CO_3$ (2.11 g, 6.47 mmol), Gphos (69.3 mg, 0.129 mmol) and Gphos Pd G6 TES (122 mg, 0.129 mmol) in dioxane (10 mL) was stirred overnight at 100°C under nitrogen atmosphere. The reaction mixture was diluted with water and extracted three times with EtOAc. The combined organic layers were dried over anhydrous $Na_2SO_4$, filtered, and concentrated under vacuum. The crude material was purified by flash chromatography on silica gel using DCM/MeOH (9/1) as eluent to afford rac-tert-butyl 2-[6-(1,3-dimethylindazole-6-amido)imidazo[1,2-a]pyrazin-2-yl]pyrrolidine-1-carboxylate **(Intermediate 184:** 340 mg, 50%) as a yellow oil. Mass spec: m/z: 476 $[M+H]^+$.

### Step 2

**Intermediate 185: rac-1,3-dimethyl-N-[2-(pyrrolidin-2-yl)imidazo[1,2-a]pyrazin-6-yl]indazole-6-carboxamide trifluoroacetate**

To a solution of rac-tert-butyl 2-[6-(1,3-dimethylindazole-6-amido)imidazo[1,2-a]pyrazin-2-yl]pyrrolidine-1-carboxylate **(Intermediate 184:** 340 mg, 0.643 mmol) in DCM (10 mL) was added TFA (2 mL). The reaction mixture was stirred for 2h at rt, then concentrated under reduced pressure to provide 1,3-dimethyl-N-[2-(pyrrolidin-2-yl)imidazo[1,2-a]pyrazin-6-yl]indazole-6-carboxamide trifluoroacetate **(Intermediate 185:** 270 mg, 80%) as a yellow oil which was used in Step 3 without further purification. Mass spec: m/z: 376 $[M+H]^+$.

### Step 3

**Intermediate 186: rac-N-[2-(1-ethylpyrrolidin-2-yl)imidazo[1,2-a]pyrazin-6-yl]-1,3-dimethylindazole-6-carboxamide**

A suspension of rac-1,3-dimethyl-N-[2-(pyrrolidin-2-yl)imidazo[1,2-a]pyrazin-6-yl]indazole-6-carboxamide trifluoroacetate **(Intermediate 185:** 180 mg, 0.403 mmol) and $K_2CO_3$ (167 mg, 1.21 mmol) in DMF (4 mL) was stirred for 1h at rt. Ethyl iodide (69.1 mg, 0.443 mmol) was added and the reaction was stirred for 3h at rt. The reaction mixture was concentrated under reduced pressure and the residue was purified by reverse-phase flash chromatography (C18) using $MeCN/H_2O$ (1/1) (0.5% $NH_3HCO_3$) to afford rac-N-[2-(1-ethylpyrrolidin-2-yl)imidazo[1,2-a]pyrazin-6-yl]-1,3-dimethylindazole-6-carboxamide **(Intermediate 186:** 60 mg, 26%) as a yellow oil. Mass spec: m/z: 404 $[M+H]^+$.

**Step 4**

**Example 91A: N-{2-[(rel-2R)-1-ethylpyrrolidin-2-yl]imidazo[1,2-a]pyrazin-6-yl}-1,3-dimethylindazole-6-carboxamide and Example 91B: N-{2-[(rel-2S)-1-ethylpyrrolidin-2-yl]imidazo[1,2-a]pyrazin-6-yl}-1,3-dimethylindazole-6-carboxamide**

Rac-N-[2-(1-ethylpyrrolidin-2-yl)imidazo[1,2-a]pyrazin-6-yl]-1,3-dimethylindazole-6-carboxamide **(Intermediate 186,** 150 mg, 0.335 mmol) was separated by chiral HPLC to afford Examples **91A** and **91B** using the following conditions:
Column: Chiral Art IF 20 x 250 mm, 5.0 um; Eluent: (Hexane/DCM, 3/1 (0.5% 2M $NH_3$ in MeOH))/EtOH 7/3; Flow rate: 20 mL/min to provide:

**Example 91A: N-{2-[(rel-2R)-1-ethylpyrrolidin-2-yl]imidazo[1,2-a]pyrazin-6-yl}-1,3-dimethylindazole-6-carboxamide**

First eluting compound (retention time = 7.9 min)
Mass spec: m/z: 404 $[M+H]^+$; $^1H$ NMR (400 MHz, MeOH-$d_4$) $\delta$ ppm 9.42 (s, 1H), 8.83 (s, 1H), 8.19 (s, 1H), 8.06 (s, 1H), 8.84 (d, J = 8.4 Hz, 1H), 7.75 - 7.72 (m, 1H), 4.10 (s, 3H), 3.70 - 3.73 (m, 1H), 3.32 - 3.38 (m, 1H), 2.82 - 2.87 (m, 1H), 2.59 (s, 3H), 2.32 - 2.45 (m, 3H), 2.02 - 2.08 (m, 3H), 1.12 (t, J = 8.0 Hz, 3H).

**Example 91B: N-{2-[(rel-2S)-1-ethylpyrrolidin-2-yl]imidazo[1,2-a]pyrazin-6-yl}-1,3-dimethylindazole-6-carboxamide**

Second eluting compound (retention time = 9.7 min)
Mass spec: m/z: 404 $[M+H]^+$; $^1H$ NMR (400 MHz, MeOH-$d_4$) $\delta$ ppm 9.43 (s, 1H), 8.84 (s, 1H), 8.20 (s, 1H), 8.07 (s, 1H), 7.85 (d, J = 8.4 Hz, 1H), 7.73 - 7.76 (m, 1H), 4.10 (s, 3H), 3.71 - 7.80 (m, 1H), 3.32 - 3.33 (m, 1H), 2.84 - 2.85 (m, 1H), 2.60 (s, 3H), 2.33 - 2.44 (m, 3H), 2.01 - 2.07 (m, 3H), 1.12 (t, J = 8.0 Hz, 3H).

**Examples 92A and 92B were synthesized following Scheme 41**

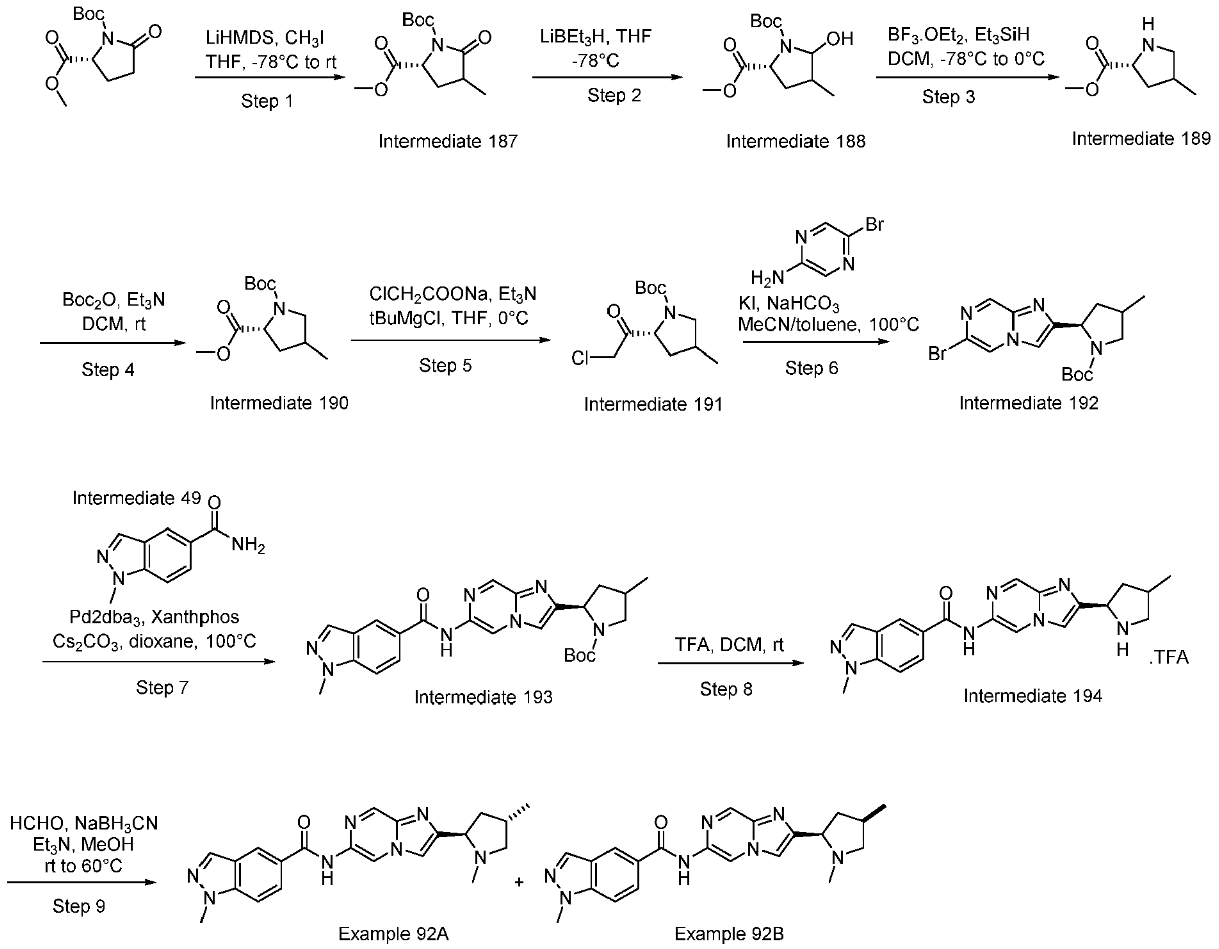


## Scheme 41

### Step 1

**Intermediate 187: rac-1-tert-butyl 2-methyl (2R)-4-methyl-5-oxopyrrolidine-1,2-dicarboxylate**

To a solution of 1-tert-butyl 2-methyl (2R)-5-oxopyrrolidine-1,2-dicarboxylate (CAS: 128811-48-3, 5 g, 20.6 mmol) in THF (15 mL) was added a solution of LiHMDS (24.7 mL, 24.7 mmol, 1M in THF) dropwise at -78°C under nitrogen atmosphere. The reaction mixture was stirred for 1h at -78°C. $CH_3I$ (3.50 g, 24.7 mmol) was added, and the reaction mixture was stirred for 30min at -78°C. The mixture was quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (1/4) as eluent to afford 1-tert-butyl 2-methyl (2R)-4-methyl-5-oxopyrrolidine-1,2-dicarboxylate **(Intermediate 187:** 2 g, 19%) as a colorless oil which was used in the next step without further purification. Mass spec: m/z: 258 $[M+H]^+$.

### Step 2

**Intermediate 188: 1-tert-butyl 2-methyl (2R)-5-hydroxy-4-methylpyrrolidine-1,2-dicarboxylate**

To a solution of 1-tert-butyl 2-methyl (2R)-4-methyl-5-oxopyrrolidine-1,2-dicarboxylate **(Intermediate 187:** 2 g, 7.77 mmol) in THF (16 mL) was added a solution of $LiBEt_3H$ (7.77 mL, 7.77 mmol 1M in THF) dropwise at -78°C under nitrogen atmosphere. The reaction mixture was stirred for 30 min at -78°C. The mixture was quenched with a saturated aqueous solution of $NaHCO_3$ and extracted three times with EtOAc. The combined organic layers were washed with brine, dried over anhydrous $Na_2SO_4$, filtered, and concentrated under reduced pressure to provide 1-tert-butyl 2-methyl (2R)-5-hydroxy-4-methylpyrrolidine-1,2-dicarboxylate **(Intermediate 188**: 2 g, 96%) as a brown oil which was used in

step 3 without further purification. Mass spec: m/z: 260 $[M+H]^+$.

**Step 3**

**Intermediate 189: methyl (2R)-4-methylpyrrolidine-2-carboxylate**

To a solution of 1-tert-butyl 2-methyl (2R)-5-hydroxy-4-methylpyrrolidine-1,2-dicarboxylate **(Intermediate 188:** 1.2 g, 4.63 mmol) in DCM (12 mL) was added $Et_3SiH$ (1.13 g, 9.72 mmol) dropwise at rt under nitrogen atmosphere. The mixture was cooled to -78°C and $BF_3.Et_2O$ (1.58 g, 11.1 mmol) was added. The reaction mixture was stirred for 1h at 0°C under nitrogen atmosphere. The reaction mixture was concentrated under vacuum to provide methyl (2R)-4-methylpyrrolidine-2-carboxylate **(Intermediate 189:** 1 g crude) as a colorless oil which was used in Step 4 without further purification. Mass spec: m/z: 144 $[M+H]^+$.

**Step 4**

**Intermediate 190: 1-tert-butyl 2-methyl (2R)-4-methylpyrrolidine-1,2-dicarboxylate**

A solution of methyl (2R)-4-methylpyrrolidine-2-carboxylate **(Intermediate 189:** 1 g, 6.98 mmol), $Boc_2O$ (2.29 g, 10.5 mmol) and $Et_3N$ (2.12 g, 20.9 mmol) in DCM (15 mL) was stirred for 2h at rt. The reaction mixture was concentrated under reduced pressure. The crude material was purified by flash chromatography on silica gel using PE/EtOAc (1/4) as eluent to afford 1-tert-butyl 2-methyl (2R)-4-methylpyrrolidine-1,2-dicarboxylate **(Intermediate 190:** 1 g, 59%) as a colorless oil. Mass spec: m/z: 244 $[M+H]^+$.

**Step 5**

**Intermediate 191: tert-butyl (2R)-2-(6-bromoimidazo[1,2-a]pyrazin-2-yl)-4-methylpyrrolidine-1-carboxylate**

To a solution of 1-tert-butyl 2-methyl (2R)-4-methylpyrrolidine-1,2-dicarboxylate **(Intermediate 190:** 51% purity, 1 g, 4.11 mmol), sodium 2-chloroacetate (2.39 g, 20.6 mmol) and $Et_3N$ (1.66 g, 16.4 mmol) in THF (15 mL) was added a solution of tert-butylmagnesium chloride (20.6 mL, 20.6 mmol, 1.7M in THF) dropwise at 0°C under nitrogen atmosphere. The reaction mixture was stirred overnight at rt. The reaction mixture was quenched with a saturated aqueous solution of $NH_4Cl$ (200 mL), and the aqueous layer extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (4/1) as eluent to afford tert-butyl (2R)-2-(2-chloroacetyl)-4-methylpyrrolidine-1-carboxylate **(Intermediate 191:** 930 mg, 87%) as a colorless oil which was used Step 6 without further purification. Mass spec: m/z: 262 $[M+H]^+$.

**Step 6**

**Intermediate 192: tert-butyl (2R)-2-(6-bromoimidazo[1,2-a]pyrazin-2-yl)-4-methylpyrrolidine-1-carboxylate**

A solution of tert-butyl (2R)-2-(2-chloroacetyl)-4-methylpyrrolidine-1-carboxylate **(Intermediate 191:** 930 mg, 3.55 mmol), 5-bromopyrazin-2-amine (CAS: 59489-71-3, 618 mg, 3.55 mmol), KI (884 mg, 5.33 mmol) and $NaHCO_3$ (358 mg, 4.26 mmol) in toluene (6 mL) and MeCN (2 mL) was stirred for 3h at 100°C The reaction mixture was concentrated under reduced pressure. The crude material was purified by flash chromatography on silica gel using DCM/MeOH (95/5) as eluent, then by reverse-phase flash chromatography (C18) using $MeCN/H_2O$ (47/53) as eluent to afford tert-butyl (2R)-2-(6-bromoimidazo[1,2-a]pyrazin-2-yl)-4-methylpyrrolidine-1-carboxylate **(Intermediate 192:** 417 mg, 49%) as a brown solid. Mass spec: m/z: 381 $[M+H]^+$.

**Step 7**

**Intermediate 193: tert-butyl (2R)-4-methyl-2-(6-(1-methyl-1H-indazole-5-carboxamido)imidazo[1,2-a]pyrazin-2-yl)pyrrolidine-1-carboxylate**

A solution of tert-butyl (2R)-2-(6-bromoimidazo[1,2-a]pyrazin-2-yl)-4-methylpyrrolidine-1-carboxylate **(Intermediate 192:** 400 mg, 1.05 mmol), 1-methylindazole-5-carboxamide **(Intermediate 49:** 183 mg, 1.05 mmol), XantPhos (121 mg, 0.210 mmol), $Pd_2dba_3$ (96.1 mg, 0.105 mmol) and $Cs_2CO_3$ (1.03 g, 3.15 mmol) in dioxane (8 mL) was stirred overnight at 100°C under nitrogen atmosphere. The reaction mixture was concentrated under reduced pressure and the

residue was purified by preparative TLC using DCM/MeOH (10/1) as eluent to provide tert-butyl (2R)-4-methyl-2-(6-(1-methyl-1H-indazole-5-carboxamido)imidazo[1,2-a]pyrazin-2-yl)pyrrolidine-1-carboxylate **(Intermediate 193:** 170 mg, 41%) as a brown solid. Mass spec: m/z: 476 $[M+H]^+$.

**Step 8**

**Intermediate 194: 1-methyl-N-(2-((2R)-4-methylpyrrolidin-2-yl)imidazo[1,2-a]pyrazin-6-yl)-1H-indazole-5-carboxamide trifluoroacetate**

To a solution of tert-butyl (2R)-4-methyl-2-(6-(1-methyl-1H-indazole-5-carboxamido)imidazo[1,2-a]pyrazin-2-yl)pyrrolidine-1-carboxylate **(Intermediate 193:** 170 mg, 0.357 mmol) in DCM (3 mL) was added TFA (0.6 mL). The reaction mixture was stirred for 2h at rt, then concentrated under reduced pressure to provide 1-methyl-N-(2-((2R)-4-methylpyrrolidin-2-yl)imidazo[1,2-a]pyrazin-6-yl)-1H-indazole-5-carboxamide trifluoroacetate **(Intermediate 194:** 130 mg, 74%) as a brown oil which was used in Step 9 without further purification. Mass spec: m/z: 376 $[M+H]^+$.

**Step 9**

**Example 92A: N-(2-((2R, rel-4S)-1,4-dimethylpyrrolidin-2-yl)imidazo[1,2-a]pyrazin-6-yl)-1-methyl-1H-indazole-5-carboxamide and Example 92B: N-(2-((2R, rel-4R)-1,4-dimethylpyrrolidin-2-yl)imidazo[1,2-a]pyrazin-6-yl)-1-methyl-1H-indazole-5-carboxamide**

A solution of 1-methyl-N-(2-((2R)-4-methylpyrrolidin-2-yl)imidazo[1,2-a]pyrazin-6-yl)-1H-indazole-5-carboxamide (130 mg, 0.346 mmol), paraformaldehyde (51.2 mg, 1.73 mmol) and $Et_3N$ (105 mg, 1.04 mmol) in MeOH (4 mL) was stirred for 1h at room temperature. $NaBH_3CN$ (95%, 65.3 mg, 1.04 mmol) was added and the resulting mixture was stirred for 1h at 60°C. The volatiles were removed under reduced pressure and the crude material was purified by chiral preparative HPLC using the following conditions:
Column: XBridge OBD, 30 x 150 mm, 5μm; Eluent: Water (10 mmol $NH_4HCO_3$ + 0.05% $NH_4OH$)/MeCN from 76/24 to 46/54; Flow rate: 60 mL/min to afford:

**Example 92A: N-(2-((2R, rel-4S)-1,4-dimethylpyrrolidin-2-yl)imidazo[1,2-a]pyrazin-6-yl)-1-methyl-1H-indazole-5-carboxamide**

First eluting compound (retention time = 7.6 min)

Mass spec: m/z: 390 $[M+H]^+$; $^1H$ NMR (400 MHz, MeOH-$d_4$) δ ppm 9.39 (s, 1H), 8.83 (s, 1H), 8.50 (s, 1H), 8.19 (s, 1H), 8.05 - 8.08 (m, 2H), 7.68 - 7.70 (m, 1H), 4.13 (s, 3H), 3.63 - 3.65 (m, 1H), 3.37 (s, 1H), 2.50 - 2.52 (m, 1H), 2.34 - 2.36 (m, 3H), 2.32 - 2.33 (m, 1H), 2.29 - 2.31 (m, 1H), 1.96 - 1.98 (m, 1H), 1.13 - 1.16 (m, 3H).

**Example 92B: N-(2-((2R,rel-4R)-1,4-dimethylpyrrolidin-2-yl)imidazo[1,2-a]pyrazin-6-yl)-1-methyl-1H-indazole-5-carboxamide**

Second eluting compound (retention time = 7.8 min)
Mass spec: m/z: 390 $[M+H]^+$; $^1H$ NMR (400 MHz, MeOH-$d_4$) δ ppm 9.41 (s, 1H), 8.83 (s, 1H), 8.51 (s, 1H), 8.20 (s, 1H), 8.07 - 8.08 (m, 2H), 7.69 - 7.71 (m, 1H), 4.14 (s, 3H), 3.60 - 3.62 (m, 1H), 2.90 - 2.93 (m, 1H),2.68 - 2.70 (m, 1H), 2.55 - 2.57 (m, 1H), 2.50 - 2.52 (m, 1H), 2.36 (s, 3H), 1.73 - 1.75 (m, 1H), 1.20 - 1.22 (m, 3H).

**Examples 93A and 93B were synthesized following Scheme 42**

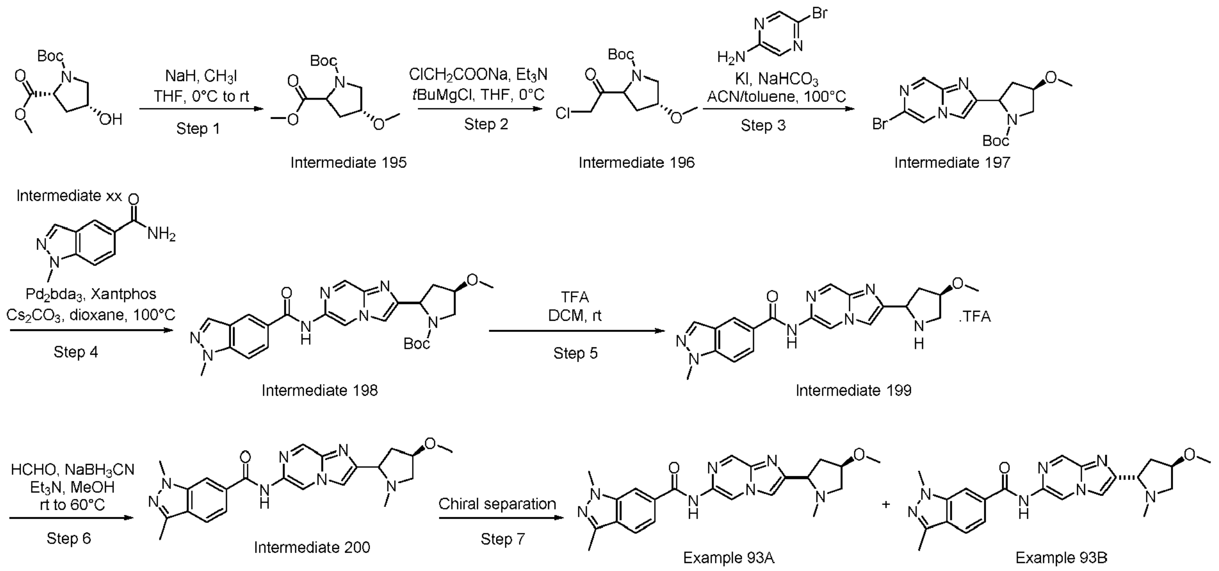


## Scheme 42

### Step 1

**Intermediate 195: 1-(tert-butyl) 2-methyl (4R)-4-methoxypyrrolidine-1,2-dicarboxylate**

To a solution of 1-tert-butyl 2-methyl (2R,4R)-4-hydroxypyrrolidine-1,2-dicarboxylate (CAS: 114676-69-6, 7 g, 28.5 mmol) in DMF (80 mL) was added NaH (60% in mineral oil, 1.37 g, 34.2 mmol) at 0°C. The reaction mixture was stirred for 30 min at rt. Then, MeI (6.08 g, 42.8 mmol) was added and the reaction mixture was stirred for 2.5h at rt. The reaction mixture was quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (75/25) as eluent to afford 1-(tert-butyl) 2-methyl (4R)-4-methoxypyrrolidine-1,2-dicarboxylate **(Intermediate 195:** 6.37 g, 68%) as a yellow oil. Mass spec: m/z 260 $[M+H]^+$.

### Step 2

**Intermediate 196: tert-butyl (4R)-2-(2-chloroacetyl)-4-methoxypyrrolidine-1-carboxylate**

To a solution of 1-(tert-butyl) 2-methyl (4R)-4-methoxypyrrolidine-1,2-dicarboxylate **(Intermediate 195:** 5.35 g, 20.6 mmol) and sodium 2-chloroacetate (12.02 g, 103 mmol) in THF (50 mL) was a solution of tert-butylmagnesium chloride (60.8 mL, 103 mmol, 1.7M in THF) at 0°C under nitrogen atmosphere. The reaction mixture was stirred at rt overnight, then quenched with water and extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (8/2) as eluent to afford tert-butyl (4R)-2-(2-chloroacetyl)-4-methoxypyrrolidine-1-carboxylate **(Intermediate 196:** 3.12 g, 27%, 39% purity) as a purple oil. Mass spec: m/z 278 $[M+H]^+$.

### Step 3

**Intermediate 197: tert-butyl (4R)-2-(6-bromoimidazo[1,2-a]pyrazin-2-yl)-4-methoxypyrrolidine-1-carboxylate**

To a solution of tert-butyl (4R)-2-(2-chloroacetyl)-4-methoxypyrrolidine-1-carboxylate **(Intermediate 196:** 39% purity, 3 g, 10.8 mmol) in MeCN (15 mL) and toluene (5mL) were added KI (2.69 g, 16.2 mmol), $NaHCO_3$ (1.09 g, 12.9 mmol) and 5-bromopyrazin-2-amine (1.88 g, 10.8 mmol). The reaction mixture was stirred for 3h at 100°C, then concentrated under reduced pressure. The crude material was purified by flash chromatography on silica gel using DCM/MeOH (9/1) as eluent, then by reverse-phase flash chromatography (C18) using MeCN/$H_2O$ (35/65) as eluent to afford tert-butyl (4R)-2-(6-bromoimidazo[1,2-a]pyrazin-2-yl)-4-methoxypyrrolidine-1-carboxylate **(Intermediate 197:** 760 mg,

31%, 67% purity) as a brown oil. Mass spec: m/z 397 $[M+H]^+$.

**Step 4**

**Intermediate 198: tert-butyl (4R)-4-methoxy-2-(6-(1-methyl-1H-indazole-5-carboxamido)imidazo[1,2-a]pyrazin-2-yl)pyrrolidine-1-carboxylate**

A solution of tert-butyl (4R)-2-(6-bromoimidazo[1,2-a]pyrazin-2-yl)-4-methoxypyrrolidine-1-carboxylate **(Intermediate 197:** 67% purity, 710 mg, 1.79 mmol), 1-methylindazole-5-carboxamide **(Intermediate 49:** 313 mg, 1.79 mmol), $Pd_2dba_3$ (164 mg, 0.179 mmol), XantPhos (207 mg, 0.357 mmol) and $Cs_2CO_3$ (1.75 g, 5.36 mmol) in dioxane (10 mL) was stirred for 2h at 100°C under nitrogen atmosphere. The reaction mixture was concentrated under reduced pressure and the residue was purified by flash chromatography on silica gel using DCM/MeOH (86/14) as eluent to afford tert-butyl (4R)-4-methoxy-2-(6-(1-methyl-1H-indazole-5-carboxamido)imidazo[1,2-a]pyrazin-2-yl)pyrrolidine-1-carboxylate **(Intermediate 198:** 390 mg, 50%) as a yellow oil. Mass spec: m/z 492 $[M+H]^+$.

**Step 5**

**Intermediate 199: N-(2-((4R)-4-methoxypyrrolidin-2-yl)imidazo[1,2-a]pyrazin-6-yl)-1-methyl-1H-indazole-5-carboxamide trifluoroacetate**

To a solution of tert-butyl (4R)-4-methoxy-2-(6-(1-methyl-1H-indazole-5-carboxamido)imidazo[1,2-a]pyrazin-2-yl)pyrrolidine-1-carboxylate **(Intermediate 198:** 390 mg, 0.581 mmol) in DCM (5 mL) was added TFA (1 mL, 13.5 mmol) at rt. The reaction mixture was stirred for 2h at rt, then concentrated under reduced pressure to provide N-(2-((4R)-4-methoxypyrrolidin-2-yl)imidazo[1,2-a]pyrazin-6-yl)-1-methyl-1H-indazole-5-carboxamide trifluoroacetate **(Intermediate 199:** 300 mg crude, 66% purity) as a colorless oil which was used in Step 6 without further purification. Mass spec: m/z 392 $[M+H]^+$.

**Step 6**

**Intermediate 200: N-{2-[(4R)-4-methoxy-1-methylpyrrolidin-2-yl]imidazo[1,2-a]pyrazin-6-yl}-1-methylindazole-5-carboxamide**

To a solution of N-(2-((4R)-4-methoxypyrrolidin-2-yl)imidazo[1,2-a]pyrazin-6-yl)-1-methyl-1H-indazole-5-carboxamide trifluoroacetate **(Intermediate 199:** 66% purity, 300 mg, 0.766 mmol) in MeOH (5 mL) were added $Et_3N$ (388 mg, 3.83 mmol) and paraformaldehyde (115 mg, 3.83 mmol) at rt. The reaction mixture was stirred for 1h at rt, prior addition of $NaBH_3CN$ (144mg, 2.30 mmol). The reaction mixture was stirred 2h at 60°C and then concentrated under reduced pressure. The mixture was purified by Preparative HPLC with the following conditions (Column: XBridge OBD, 30 × 150 mm, 5μm; Eluent: MeCN in Water (10mmol/L $NH_4HCO_3$ + 0.05% $NH_4OH$) from 24 to 54%; Flow rate: 60 mL/min mL/min) to afford N-{2-[(4R)-4-methoxy-1-methylpyrrolidin-2-yl]imidazo[1,2-a]pyrazin-6-yl}-1-methylindazole-5-carboxamide **(Intermediate 200:** 80 mg, 38%) as a white solid as a mixture of diastereoisomeres. Mass spec: m/z 406 $[M+H]^+$.

**Step 7**

**Example 93A: N-{2-[(rel-2R,4R)-4-methoxy-1-methylpyrrolidin-2-yl]imidazo[1,2-a]pyrazin-6-yl}-1-methylindazole-5-carboxamide and Example 93B: N-{2-[(rel-2S,4R)-4-methoxy-1-methylpyrrolidin-2-yl]imidazo[1,2-a]pyrazin-6-yl}-1-methylindazole-5-carboxamide**

N-{2-[(4R)-4-methoxy-1-methylpyrrolidin-2-yl]imidazo[1,2-a]pyrazin-6-yl}-1 - methylindazole-5-carboxamide **(Intermediate 200)** was subjected to chiral preparative HPLC (Column: ChiralPak IH, 3 × 25 cm, 5 μm; Eluent: $CO_2$/(IPA/MeCN 1/1 (1% 2M solution of $NH_3$ in MeOH)) 1/1; flow rate: 85 mL/min) to isolate the following examples:

**Example 93A: N-{2-[(rel-2R,4R)-4-methoxy-1-methylpyrrolidin-2-yl]imidazo[1,2-a]pyrazin-6-yl}-1-methylindazole-5-carboxamide**

White solid (24.2 mg, 30%)

First eluting compound (retention time = 5.79 min):
Mass spec: m/z: 406 [M+H]$^+$; $^1$H NMR (400 MHz, DMSO-$d_6$) δ ppm 10.82 (s, 1H), 9.40 (s, 1H), 8.90 (s, 1H), 8.59 (s, 1H), 8.24 (s, 1H), 8.17 (s, 1H), 8.08 - 8.10 (m, 1H), 7.74 - 7.77 (m, 1H), 4.10 (s, 3H), 3.91 - 3.95 (m, 1H), 3.32 - 3.34 (m, 1H), 3.17 - 3.20 (m, 4H), 2.60 - 2.67 (m, 1H), 2.32 - 2.36 (m, 1H), 2.18 (s, 3H), 1.83 - 1.87 (m, 1H).

**Example 93B: N-{2-[(rel-2S,4R)-4-methoxy-1-methylpyrrolidin-2-yl]imidazo[1,2-a]pyrazin-6-yl}-1-methylindazole-5-carboxamide**

White solid (29.4 mg, 37%)
Second eluting compound (retention time = 9.74 min):
Mass spec: m/z: 406 [M+H]$^+$; $^1$H NMR (400 MHz, DMSO-$d_6$) δ ppm 10.83 (s, 1H), 9.40 (s, 1H), 8.90 (s, 1H), 8.59 (s, 1H), 8.24 (s, 1H), 8.15 (s, 1H), 8.07 - 8.10 (m, 1H), 7.74 - 7.77 (m, 1H), 4.10 (s, 3H), 4.00 - 4.02 (m, 1H), 3.57 - 3.61 (m, 1H), 3.46 - 3.50 (m, 1H), 3.23 (s, 3H), 2.26 - 2.29 (m, 1H), 2.21 (s, 3H), 2.06 - 2.19 (m, 2H).

**Example 94 was synthesized following Scheme 43**

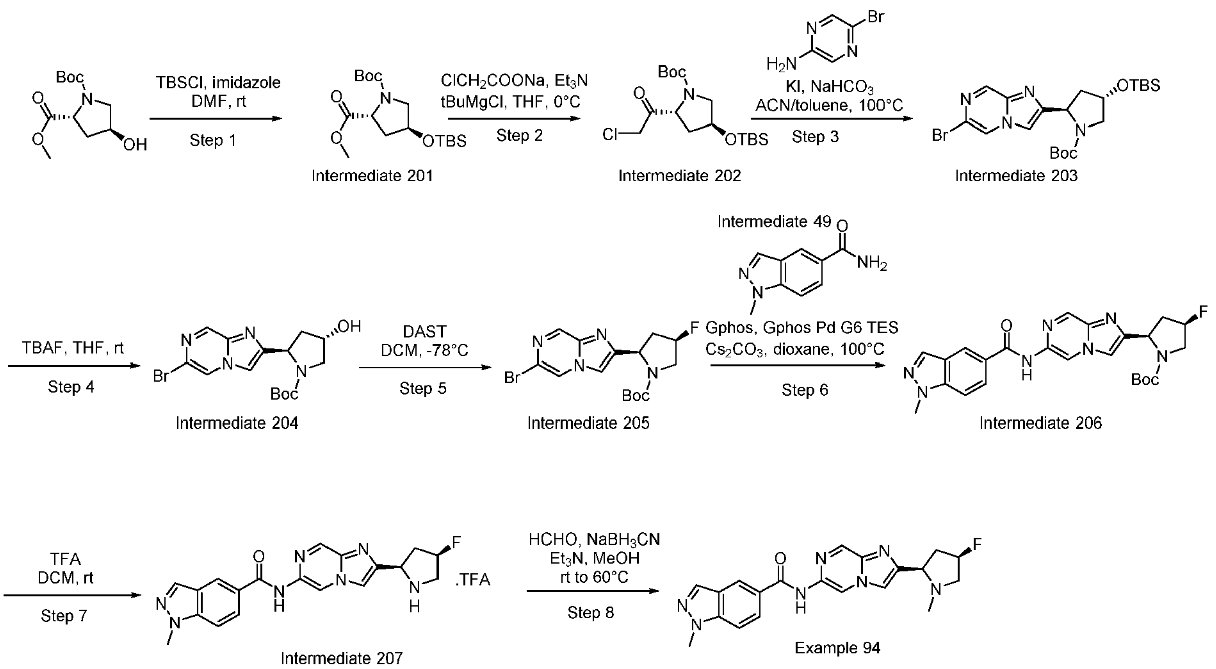


## Scheme 43

**Step 1**

**Intermediate 201: 1-tert-butyl 2-methyl (2R,4S)-4-[(tert-butyldimethylsilyl)oxy]pyrrolidine-1,2-dicarboxylate**

To a solution of 1-tert-butyl 2-methyl (2R,4S)-4-hydroxypyrrolidine-1,2-dicarboxylate (CAS: 135042-17-0, 6.00 g, 24.5 mmol) in DMF (60 mL) were added imidazole (5.00 g, 73.4 mmol) and TBSCl (4.79 g, 31.8 mmol). The reaction mixture was stirred at rt overnight and then quenched with water. The aqueous layer was extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (7/3) as eluent to afford 1-tert-butyl 2-methyl (2R,4S)-4-[(tertbutyldimethylsilyl)oxy]pyrrolidine-1,2-dicarboxylate **(Intermediate 201:** 9 g, 82%) as a yellow oil. Mass spec: m/z 360 [M+H]$^+$.

**Step 2**

**Intermediate 202: tert-butyl (2R,4S)-4-[(tert-butyldimethylsilyl)oxy]-2-(2-chloroacetyl)pyrrolidine-1-carboxylate**

To a solution of 1-tert-butyl 2-methyl (2R,4S)-4-[(tertbutyldimethylsilyl)oxy]pyrrolidine-1,2-dicarboxylate **(Intermediate 201:** 9.00 g, 20.0 mmol) in THF (400 mL) were added $Et_3N$ (8.11 g, 80.1 mmol) and sodium 2-chloroacetate (11.7 g, 100 mmol), followed by a solution of tert-butylmagnesium chloride (200 mL, 200 mmol, 1.7 M in THF) at 0°C under nitrogen atmosphere. The reaction mixture was stirred at rt overnight and then quenched with water. The resulting solution was extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (97/3) as eluent to afford tert-butyl (2R,4S)-4-[(tertbutyldimethylsilyl)oxy]-2-(2-chloroacetyl)pyrrolidine-1-carboxylate **(Intermediate 202:** 4.5 g, 54%) as a yellow oil. Mass spec: m/z 378 $[M+H]^+$.

**Step 3**

**Intermediate 203: tert-butyl (2R,4S)-2-{6-bromoimidazo[1,2-a]pyrazin-2-yl}-4-[(tert-butyldimethylsilyl)oxy]pyrrolidine-1-carboxylate**

To a solution of tert-butyl (2R,4S)-4-[(tert-butyldimethylsilyl)oxy]-2-(2-chloroacetyl)pyrrolidine-1-carboxylate **(Intermediate 202:** 4.50 g, 10.1 mmol) in MeCN (200 mL)/toluene (70 mL) were added 5-bromopyrazin-2-amine (2.11 g, 12.1 mmol), KI (2.52 g, 15.2 mmol) and $NaHCO_3$ (1.02 g, 12.1 mmol). The reaction mixture was stirred at 100°C overnight and then quenched with water. The resulting solution was extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (6/4) as eluent to afford tert-butyl (2R,4S)-2-{6-bromoimidazo[1,2-a]pyrazin-2-yl}-4-[(tert-butyldimethylsilyl)oxy]pyrrolidine-1-carboxylate **(Intermediate 203:** 1.5 g, 27%) as a yellow oil. Mass spec: m/z 497 $[M+H]^+$.

**Step 4**

**Intermediate 204: tert-butyl (2R,4S)-2-{6-bromoimidazo[1,2-a]pyrazin-2-yl}-4-hydroxypyrrolidine-1 -carboxylate**

To a solution of tert-butyl (2R,4S)-2-{6-bromoimidazo[1,2-a]pyrazin-2-yl}-4-[(tertbutyldimethylsilyl)oxy]pyrrolidine-1-carboxylate **(Intermediate 203:** 1.50 g, 2.71 mmol) in THF (18 mL) was added a solution of TBAF (4.07 mL, 1M in THF). The reaction mixture was stirred at rt overnight and then quenched with water. The resulting solution was extracted three times with EtOAc. The combined organic layers were washed with brine, dried over anhydrous $Na_2SO_4$, filtered, and concentrated under reduced pressure. The crude material was purified by flash chromatography on silica gel using DCM/MeOH (93/7) as eluent to afford tert-butyl (2R,4S)-2-{6-bromoimidazo[1,2-a]pyrazin-2-yl}-4-hydroxypyrrolidine-1-carboxylate **(Intermediate 204:** 930 mg, 76%) as a yellow oil. Mass spec: m/z 383 $[M+H]^+$.

**Step 5**

**Intermediate 205: tert-butyl (2R,4R)-2-{6-bromoimidazo[1,2-a]pyrazin-2-yl}-4-fluoropyrrolidine-1-carboxylate**

To a solution of tert-butyl (2R,4S)-2-{6-bromoimidazo[1,2-a]pyrazin-2-yl}-4-hydroxypyrrolidine-1-carboxylate **(Intermediate 204:** 900 mg, 2.00 mmol) in DCM (30 mL) was added DAST (644 mg, 3.99 mmol) at -78°C under nitrogen atmosphere. The reaction mixture was stirred at -78°C for 3h and then quenched with water. The resulting solution was extracted three times with EtOAc. The combined organic layers were washed with brine, dried over anhydrous $Na_2SO_4$, filtered, and concentrated under reduced pressure. The crude material was purified by flash chromatography on silica gel using PE/EtOAc (4/6) as eluent to afford tert-butyl (2R,4R)-2-{6-bromoimidazo[1,2-a]pyrazin-2-yl}-4-fluoropyrrolidine-1-carboxylate **(Intermediate 205:** 240 mg, 28%) as a yellow oil. Mass spec: m/z 385 $[M+H]^+$.

**Step 6**

**Intermediate 206: tert-butyl (2R,4R)-4-fluoro-2-(6-(1-methyl-1H-indazole-5-carboxamido)imidazo[1,2-a]pyrazin-2-yl)pyrrolidine-1-carboxylate**

A solution of tert-butyl (2R,4R)-2-{6-bromoimidazo[1,2-a]pyrazin-2-yl}-4-fluoropyrrolidine-1-carboxylate **(Intermediate 205:** 220 mg, 0.514 mmol), 1-methylindazole-5-carboxamide **(Intermediate 49:** 108 mg, 0.617 mmol), $Cs_2CO_3$

(502 mg, 1.54 mmol), GPhos (27.6 mg, 0.051 mmol) and GPhos Pd G6 TES (48.7 mg, 0.051 mmol) in dioxane (4 mL) was heated at 100°C overnight under nitrogen atmosphere. The reaction mixture was quenched with water, then concentrated under reduced pressure. The residue was purified by reverse-phase flash chromatography (C18) using MeCN/$H_2O$ (62/38) as eluent to afford tert-butyl (2R,4R)-4-fluoro-2-(6-(1-methyl-1H-indazole-5-carboxamido)imidazo[1,2-a]pyrazin-2-yl)pyrrolidine-1-carboxylate **(Intermediate 206:** 110 mg, 40%) as a yellow oil. Mass spec: m/z 480 $[M+H]^+$.

**Step 7**

**Intermediate 207: N-(2-((2R,4R)-4-fluoropyrrolidin-2-yl)imidazo[1,2-a]pyrazin-6-yl)-1-methyl-1H-indazole-5-carboxamide trifluoroacetate**

To a solution of tert-butyl (2R,4R)-4-fluoro-2-(6-(1-methyl-1H-indazole-5-carboxamido)imidazo[1,2-a]pyrazin-2-yl)pyrrolidine-1-carboxylate **(Intermediate 206:** 110 mg, 0.206 mmol) in DCM (3 mL) was added TFA (1 mL). The reaction mixture was stirred at rt for 2h, then concentrated under reduced pressure to provide N-(2-((2R,4R)-4-fluoropyrrolidin-2-yl)imidazo[1,2-a]pyrazin-6-yl)-1-methyl-1H-indazole-5-carboxamide trifluoroacetate **(Intermediate 207:** 85 mg, 92%) as a yellow solid. Mass spec: m/z 380 $[M+H]^+$.

**Step 8**

**Example 94: N-(2-((2R,4R)-4-fluoro-1-methylpyrrolidin-2-yl)imidazo[1,2-a]pyrazin-6-yl)-1-methyl-1H-indazole-5-carboxamide**

To a solution of N-(2-((2R,4R)-4-fluoropyrrolidin-2-yl)imidazo[1,2-a]pyrazin-6-yl)-1-methyl-1H-indazole-5-carboxamide trifluoroacetate **(Intermediate 207:** 85.0 mg, 0.190 mmol) in MeOH (2 mL) were added paraformaldehyde (28.6 mg, 0.950 mmol) and $Et_3N$ (96.4 mg, 0.950 mmol). The mixture was stirred at rt for 30 min and $NaBH_3CN$ was added (35.9 mg, 0.570 mmol). The reaction mixture was stirred at 60°C overnight, quenched with water and concentrated under reduced pressure. The residue was purified by reverse-phase flash chromatography (C18) using MeCN/$H_2O$ (75/25) as eluent, followed by preparative HPLC (Column: XBridge OBD, 30x150 mm, 5μm; Eluent: Water (10mmol/L $NH_4HCO_3$)/MeCN from 84/16 to 54/46; flow rate: 60 mL/min) to afford N-(2-((2R,4R)-4-fluoro-1-methylpyrrolidin-2-yl)imidazo[1,2-a]pyrazin-6-yl)-1-methyl-1H-indazole-5-carboxamide **(Example 94:** 43.1 mg, 57%) as a white solid. Mass spec: m/z: 394 $[M+H]^+$; $^1H$ NMR (400 MHz, DMSO-$d_6$) δ ppm 10.83 (s, 1H), 9.40 (s, 1H), 8.92 (s, 1H), 8.59 (s, 1H), 8.22 (d, J = 12 Hz, 2H), 8.08 (d, J = 1.6 Hz, 1H), 7.75 (d, J = 8.8 Hz, 1H), 5.15 - 5.32 (m, 1H), 4.10 (s, 3H), 3.37 - 3.39 (m, 1H), 3.26 - 3.34 (m, 1H), 2.70 - 2.83 (m, 1H), 2.40 - 2.41 (m, 1H), 2.22 (s, 3H), 2.00 - 2.22 (m, 1H).

**Intermediates 208A and 208B: (rel-R)-6-bromo-2-(1-methylpyrrolidin-2-yl)imidazo[1,2-a]pyrazine and (rel-S)-6-bromo-2-(1-methylpyrrolidin-2-yl)imidazo[1,2-a]pyrazine**

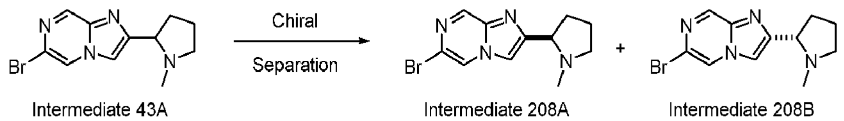


rac-2-{6-Bromoimidazo[1,2-a]pyrazin-2-yl}-1-methylpyrrolidine **(Intermediate 43A)** was separated by preparative chiral SFC using the following conditions :
Column: CHIRALPAK IH 3*25 cm, 5μm; Mobile Phase A: $CO_2$, Mobile Phase B: IPA (1% 2M $NH_3$ in MeOH); Flow rate: 85 mL/min; Gradient: isocratic 30% B to afford:

**Intermediate 208A: (rel-R)-6-bromo-2-(1-methylpyrrolidin-2-yl)imidazo[1,2-a]pyrazine**

First eluting isomer (retention time: 5.87 min)
Mass spec: m/z: 281 $[M+H]^+$, 283 $[M+H+2]^+$; 1H NMR (400 MHz, DMSO-d6) δ ppm 8.91 (d, J = 1.4 Hz, 1H), 8.86 (d, J = 1.2 Hz, 1H), 8.02 (s, 1H), 3.64 (s, 1H), 3.18 (d, J = 9.0 Hz, 1H), 2.46 (d, J = 8.6 Hz, 1H), 2.30 (s, 4H), 1.95 - 1.80 (m, 3H).

**Intermediate 208B: (rel-S)-6-bromo-2-(1-methylpyrrolidin-2-yl)imidazo[1,2-a]pyrazine**

Second eluting compound (retention time: 8.43 mins)
Mass spec: m/z: 281 $[M+H]^+$, 283 $[M+H+2]^+$; 1H NMR (400 MHz, DMSO-d6) δ ppm 8.91 (d, J = 1.4 Hz, 1H), 8.86 (d, J = 1.2 Hz, 1H), 8.02 (s, 1H), 3.64 (s, 1H), 3.18 (d, J = 9.0 Hz, 1H), 2.46 (d, J = 8.6 Hz, 1H), 2.30 (s, 4H), 1.95 - 1.80 (m, 3H).

**Example 95 was synthesized following Scheme 44**

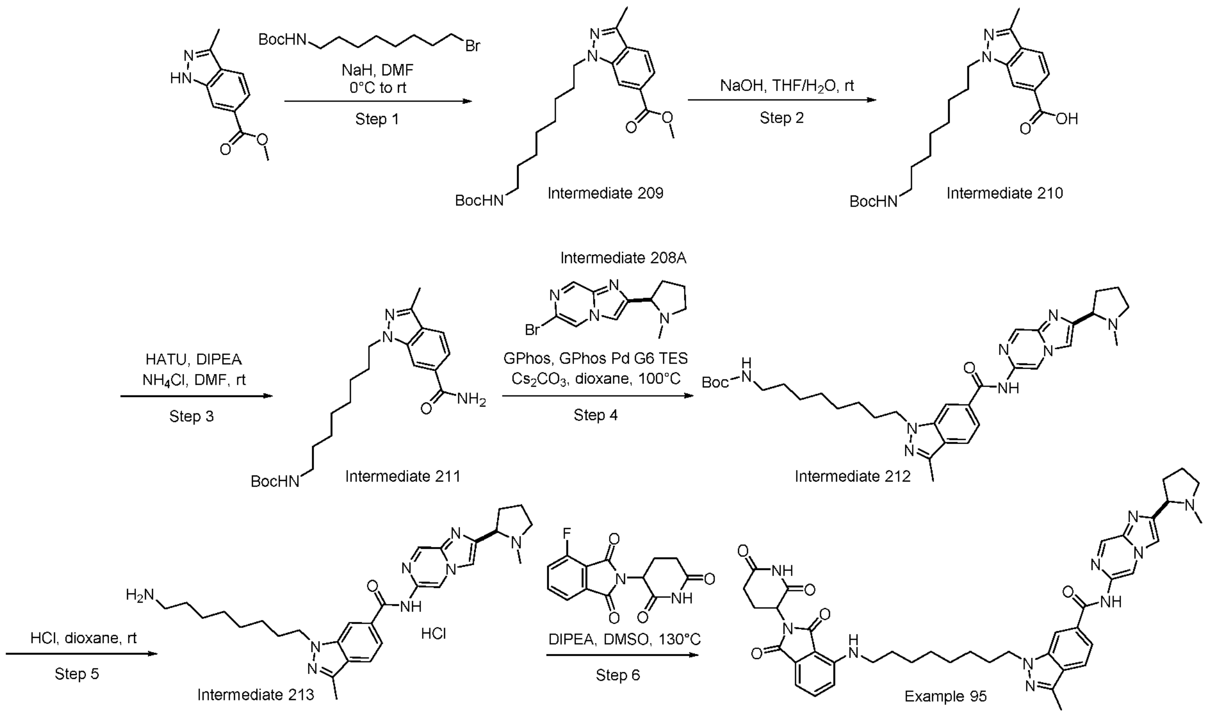


## Scheme 44

**Step 1**

**Intermediate 209: methyl 1-{8-[(tert-butoxycarbonyl)amino]octyl}-3-methylindazole-6-carboxylate**

To a solution of methyl 3-methyl-1H-indazole-6-carboxylate (CAS: 201286-95-5, 500 mg, 2.63 mmol) in DMF (20 mL) was added NaH (158 mg, 3.94 mmol, 60% dispersion in mineral oil) at 0°C. The reaction mixture was stirred at rt for 0.5h, followed by the dropwise addition of tert-butyl N-(8-bromooctyl)carbamate (CAS: 142356-35-2, 1.05 g, 3.42 mmol) at rt. The reaction mixture was stirred at rt for 2h and then quenched with water. The solution was adjusted to pH = 6 with a solution of HCl (12N in $H_2O$). The mixture was extracted three times with EtOAc, washed with brine, dried over anhydrous $Na_2SO_4$, filtered, and concentrated under reduced pressure to afford methyl 1-{ 8-[(tert-butoxycarbonyl)amino]octyl}-3-methylindazole-6-carboxylate **(Intermediate 209:** 800 mg, 66%) as an off yellow solid which was used in Step 2 without further purification. Mass spec: m/z: 418 $[M+H]^+$.

**Step 2**

**Intermediate 210: 1-{8-[(tert-butoxycarbonyl)amino]octyl}-3-methylindazole-6-carboxylic acid**

To a solution of methyl 1-{8-[(tert-butoxycarbonyl)amino]octyl}-3-methylindazole-6-carboxylate **(Intermediate 209:** 500 mg, 1.08 mmol) in $H_2O$ (30 mL)/THF (30 mL) was added NaOH (3.00 g, 75.0 mmol). The reaction mixture was stirred at rt for 2h, followed by addition of water. The solution was adjusted to pH = 6 with a solution of HCl (12N in $H_2O$).

The mixture was extracted three times with EtOAc, washed with brine, dried over anhydrous $Na_2SO_4$, filtered, and concentrated under reduced pressure to afford 1-{8-[(tert-butoxycarbonyl)amino]octyl}-3-methylindazole-6-carboxylic acid **(Intermediate 210:** 451 mg, 91%) which was used in Step 3 without further purification. Mass spec: m/z: 404 $[M+H]^+$.

**Step 3**

**Intermediate 211: tert-butyl N-[8-(6-carbamoyl-3-methylindazol-1-yl)octyl]carbamate**

To a solution of 1-{8-[(tert-butoxycarbonyl)amino]octyl}-3-methylindazole-6-carboxylic acid **(Intermediate 210:** 451 mg, 1.13 mmol) in DMF (15 mL) were added $NH_4Cl$ (265 mg, 5.00 mmol), DIPEA (961 mg, 7.43 mmol) and HATU (612 mg, 1.61mmol). The reaction mixture was stirred at rt overnight and quenched with water. The mixture was extracted three times with EtOAc, washed with brine, dried over anhydrous $Na_2SO_4$, filtered, and concentrated under reduced pressure to afford tert-butyl N-[8-(6-carbamoyl-3-methylindazol-1-yl)octyl]carbamate **(Intermediate 211:** 265 mg, 65%) as a yellow solid which was used in Step 4 without further purification. Mass spec: m/z: 403 $[M+H]^+$.

**Step 4**

**Intermediate 212: tert-butyl N-{8-[3-methyl-6-({2-[(rel-2R)-1-methylpyrrolidin-2-yl]imidazo[1,2-a]pyrazin-6-yl}carbamoyl)indazol-1-yl]octyl}carbamate**

To a solution of tert-butyl N-[8-(6-carbamoyl-3-methylindazol-1-yl)octyl]carbamate **(Intermediate 211:** 110 mg, 0.273 mmol) in dioxane (5 mL) were added (rel-2R)-2-{ 6-bromoimidazo[1,2-a]pyrazin-2-yl}-1-methylpyrrolidine **(Intermediate 208A:** 69.1 mg, 0.246 mmol), $Cs_2CO_3$ (445 mg, 1.37 mmol), Gphos (17.5 mg, 0.583 mmol) and Gphos Pd G6 TES (23.8 mg, 0.085 mmol). The reaction mixture was stirred at 100°C for 3h under nitrogen atmosphere and then quenched with water. The mixture was extracted three times with EtOAc, washed with brine, dried over anhydrous $Na_2SO_4$, filtered, and concentrated under reduced pressure. The crude material was purified by flash chromatography on silica gel using DCM/MeOH (8/2) as eluent to afford tert-butyl N-{8-[3-methyl-6-({2-[(rel-2R)-1-methylpyrrolidin-2-yl]imidazo[1,2-a]pyrazin-6-yl}carbamoyl)indazol-1-yl]octyl}carbamate **(Intermediate 212:** 100 mg, 66%) as an off-white solid. Mass spec: m/z: 603 $[M+H]^+$.

**Step 5**

**Intermediate 213: 1-(8-aminooctyl)-3-methyl-N-{2-[(rel-2R)-1-methylpyrrolidin-2-yl]imidazo[1,2-a]pyrazin-6-yl}indazole-6-carboxamide hydrochloride**

To a solution of tert-butyl N-{8-[3-methyl-6-({2-[(rel-2R)-1-methylpyrrolidin-2-yl]imidazo[1,2-a]pyrazin-6-yl}carbamoyl)indazol-1-yl]octyl}carbamate **(Intermediate 212:** 100 mg, 0.166 mmol) in dioxane (4 mL) was added a solution of HCl (2 mL, 12N in $H_2O$). The reaction mixture was stirred at rt for 2h and concentrated under reduced pressure to provide 1-(8-aminooctyl)-3-methyl-N-{2-[(rel-2R)-1-methylpyrrolidin-2-yl]imidazo[1,2-a]pyrazin-6-yl}indazole-6-carboxamide hydrochloride **(Intermediate 213:** 80.0 mg, 95%) as a yellow oil which was used in Step 7 without further purification. Mass spec: m/z: 503 $[M+H]^+$.

**Step 6**

**Example 95: 1-(8-{[2-(2,6-dioxopiperidin-3-yl)-1,3-dioxoisoindol-4-yl]amino}octyl)-3-methyl-N-{2-[(rel-2R)-1-methylpyrrolidin-2-yl]imidazo[1,2-a]pyrazin-6-yl}indazole-6-carboxamide**

A solution of 1-(8-aminooctyl)-3-methyl-N-{2-[(rel-2R)-1-methylpyrrolidin-2-yl]imidazo[1,2-a]pyrazin-6-yl}indazole-6-carboxamide hydrochloride **(Intermediate 213:** 80.0 mg, 0.199 mmol), 2-(2,6-dioxopiperidin-3-yl)-4-fluoroisoindole-1,3-dione (CAS: 835616-60-9, 54.9 mg, 0.199 mmol) and DIPEA (257 mg, 1.99 mmol) in DMSO (2 mL) was stirred at 130°C for 3h and then concentrated under reduced pressure. The crude material was purified by Preparative HPLC (Column: Xselect CSH, 30 × 150 mm, 5μm; Eluent: Water (0.1% Formic acid)/(20 mM NaOH + 10%MeCN) from 81/19 to 51/49; Flow rate: 60 mL/min) to afford 1-(8-{ [2-(2,6-dioxopiperidin-3-yl)-1,3-dioxoisoindol-4-yl]amino}octyl)-3-methyl-N-{2-[(rel-2R)-1-methylpyrrolidin-2-yl]imidazo[1,2-a]pyrazin-6-yl}indazole-6-carboxamide **(Example 95:** 35.4 mg, 30%) as a yellow solid. Mass spec: m/z: 759 $[M+H]^+$; $^1H$ NMR (400 MHz, DMSO-$d_6$) δ ppm 11.15 (s, 1H), 10.84 (s, 1H), 9.47 (s, 1H), 8.96 (s, 1H), 8.51 (s, 1H), 8.16 (s, 1H), 7.72 - 7.86 (m, 2H), 7.63 (s, 1H), 7.01 - 7.11 (m, 1H), 6.51 (s, 1H), 5.06 - 5.11 (m, 1H), 4.48 - 4.52 (m, 2H), 3.41 - 3.47 (m, 1H), 3.36 -3 .39 (m, 3H), 3.21 - 3.26 (m, 1H), 2.81 - 2.94 (m, 1H), 2.67 - 2.72 (m, 1H), 2.50 (s, 3H), 2.26 - 2.33 (m, 1H), 2.03 - 2.08 (m, 4H), 1.96 - 2.01 (m, 1H), 1.73 - 1.92(m, 5H), 1.67

- 1.74(m, 2H), 1.26 - 1.37 (m, 8H).

**Example 96: was synthesized following Scheme 45**

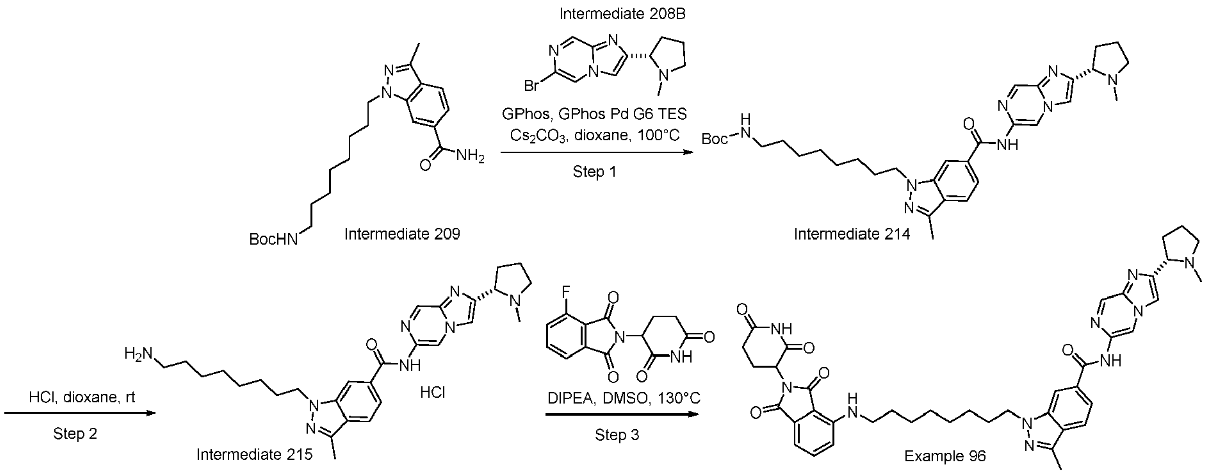


**Step 1**

**Intermediate 214: tert-butyl N-{8-[3-methyl-6-({2-[(rel-2S)-1-methylpyrrolidin-2-yl]imidazo[1,2-alpyrazin-6-yllcarbamoyl)indazol-1-ylloctyllcarbamate**

Following an analogous procedure to **Scheme 44, Step 4,** using tert-butyl N-[8-(6-carbamoyl-3-methylindazol-1-yl)octyl]carbamate **(Intermediate 209:** 150 mg, 0.373 mmol) and (rel-2S)-2-{6-bromoimidazo[1,2-a]pyrazin-2-yl}-1-methylpyrrolidine **(Intermediate 208B:** 94.5 mg, 0.336 mol), afforded tert-butyl N-{8-[3-methyl-6-({2-[(rel-2S)-1-methylpyrrolidin-2-yl]imidazo[1,2-a]pyrazin-6-yl}carbamoyl)indazol-1-yl]octyl}carbamate **(Intermediate 214:** 160 mg, 73%) as an off-white solid. Mass spec: m/z: 603 $[M+H]^+$.

**Intermediate 215: 1-(8-aminooctyl)-3-methyl-N-{2-[(rel-2S)-1-methylpyrrolidin-2-yl]imidazo[1,2-a]pyrazin-6-yl}indazole-6-carboxamide hydrochloride**

Following an analogous procedure to **Scheme 44, Step 5,** using tert-butyl N-{8-[3-methyl-6-({2-[(rel-2S)-1-methylpyrrolidin-2-yl]imidazo[1,2-a]pyrazin-6-yl}carbamoyl)indazol-1-yl]octyl}carbamate **(Intermediate 214:** 100 mg, 0.166 mmol), afforded 1-(8-aminooctyl)-3-methyl-N-{2-[(rel-2S)-1-methylpyrrolidin-2-yl]imidazo[1,2-a]pyrazin-6-yl}indazole-6-carboxamide hydrochloride **(Intermediate 215:** 80 mg, 97%) as a yellow oil which was used in Step 7 without further purification. Mass spec: m/z: 503 $[M+H]^+$.

**Example 96: 1-(8-{[2-(2,6-dioxopiperidin-3-yl)-1,3-dioxoisoindol-4-yl]amino}octyl)-3-methyl-N-{2-[(rel-2S)-1-methylpyrrolidin-2-yl]imidazo[1,2-a]pyrazin-6-yl}indazole-6-carboxamide**

Following an analogous procedure to **Scheme 44, Step 6,** using 1-(8-aminooctyl)-3-methyl-N-{2-[(rel-2S)-1-methylpyrrolidin-2-yl]imidazo[1,2-a]pyrazin-6-yl}indazole-6-carboxamide hydrochloride **(Intermediate 215:** 80 mg, 0.239 mmol), after purification by preparative HPLC (Column: Xselect CSH, 30 × 150 mm, 5μm; Eluent: Water (0.1% Formic acid)/(20 mM NaOH + 10%MeCN) from 78/22 to 48/52; Flow rate: 60 mL/min), afforded 1-(8-{ [2-(2,6-dioxopiperidin-3-yl)-1,3-dioxoisoindol-4-yl]amino}octyl)-3-methyl-N-{2-[(rel-2S)-1-methylpyrrolidin-2-yl]imidazo[1,2-a]pyrazin-6-yl}indazole-6-carboxamide **(Example 96:** 44.5 mg, 37%) as a yellow oil. Mass spec: m/z: 759 $[M+H]^+$; $^1H$ NMR (400 MHz, DMSO-$d_6$) δ ppm 11.11 (s, 1H), 10.91 (s, 1H), 9.41 (s, 1H), 8.90 (s, 1H), 8.44 (s, 1H), 8.15 (s, 1H), 7.71 - 7.82 (m, 2H), 7.59 (s, 1H), 6.98 - 7.05 (m, 1H), 6.48 - 6.53 (m, 1H), 5.02 - 5.07 (m, 1H), 4.38 - 4.41 (m, 2H), 3.37 - 3.39 (m, 1H), 3.27 - 3.32(m, 3H), 3.21 - 3.26 (m, 1H), 2.76 - 2.84 (m, 1H), 2.69 - 2.72 (m, 1H), 2.54 - 2.57 (m, 3H), 2.31 - 2.33 (m, 1H), 2.01 -2.09 (m, 4H), 1.96 - 1.98 (m, 1H), 1.87 - 1.92(m, 4H), 1.81 - 1.86 (m, 1H), 1.67 - 1.74 (m, 2H), 1.26 - 1.32 (m, 8H).

**Example 97 was synthesized following Scheme 46**

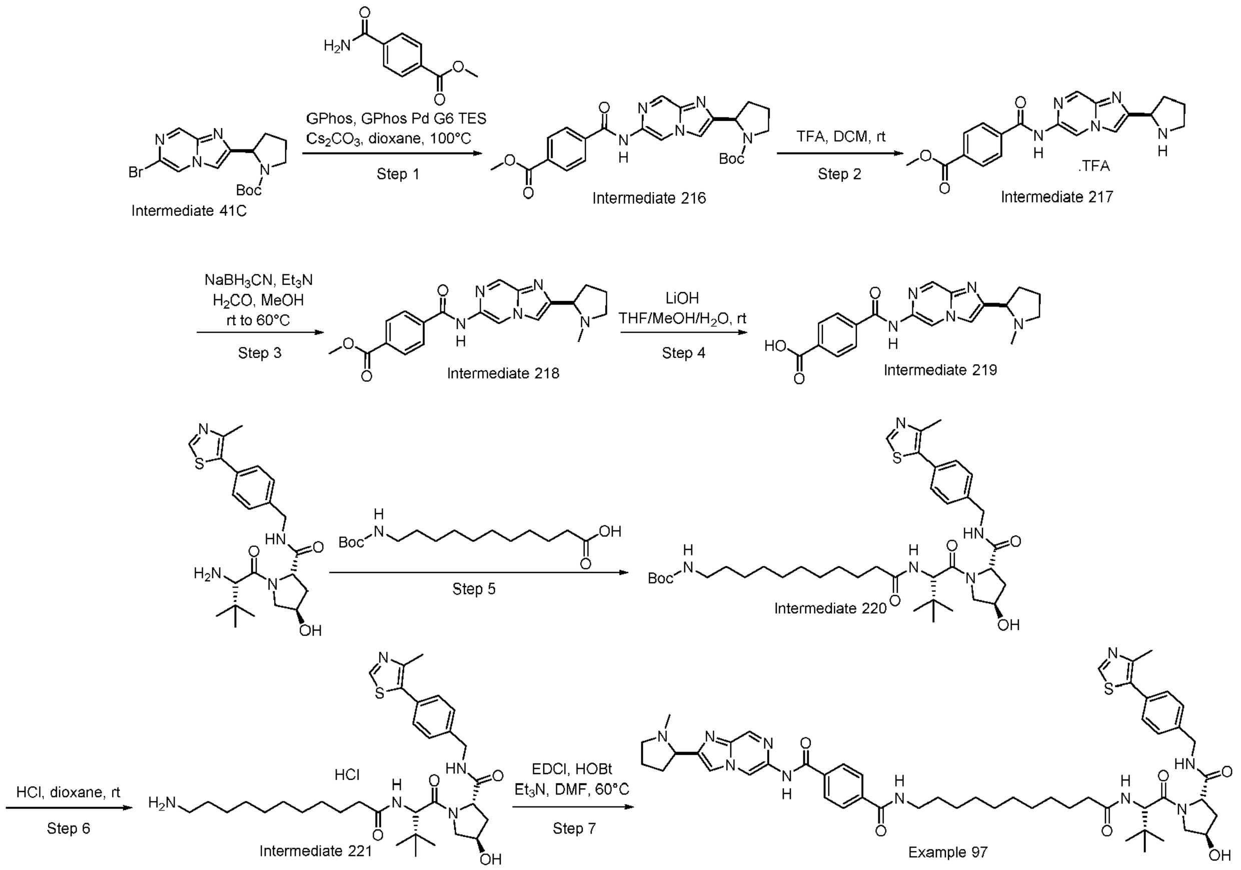


## Scheme 46

**Step 1**

**Intermediate 216: tert-butyl (rel-2R)-2-{6-[4-(methoxycarbonyl)benzamido]imidazo[1,2-a]pyrazin-2-yl}pyrrolidine-1-carboxylate**

To a solution of tert-butyl (rel-2R)-2-{6-bromoimidazo[1,2-a]pyrazin-2-yl}pyrrolidine-1-carboxylate **(Intermediate 41C:** 200 mg, 0.539 mmol), methyl 4-(aminocarbonyl)benzoate (CAS: 6757-31-9, 145 mg, 0.808 mmol) and $Cs_2CO_3$ (527 mg, 1.62 mmol) in dioxane (4 mL) were added GPhos Pd G6 TES (57.9 mg, 0.108 mmol) and GPhos (50.9 mg, 0.0540 mmol). The reaction reaction was stirred at 100°C overnight under nitrogen atmosphere, then concentrated under reduced pressure. The residue was purified by reverse-phase flash chromatography (C18) using MeCN/$H_2O$ (45/55) as eluent to afford tert-butyl (rel-2R)-2-{6-[4-(methoxycarbonyl)benzamido]imidazo[1,2-a]pyrazin-2-yl}pyrrolidine-1-carboxylate **(Intermediate 216:** 190 mg, 70%) as a yellow solid. Mass spec: m/z: 466 $[M+H]^+$.

**Step 2**

**Intermediate 217: methyl 4-(12-[(rel-2R)-pyrrolidin-2-yl]imidazo[1,2-alpyrazin-6-yl}carbamoyl)benzoate trfluoroacetate**

To a solution of tert-butyl (rel-2R)-2-{6-[4-(methoxycarbonyl)benzamido]imidazo[1,2-a]pyrazin-2-yl}pyrrolidine-1-carboxylate **(Intermediate 216:** 190 mg, 0.376 mmol) in DCM (10 mL) was added TFA (2 mL). The reaction mixture was stirred at rt for 2h, then concentrated under reduced pressure to afford methyl 4-({2-[(rel-2R)-pyrrolidin-2-yl]imidazo[1,2-a]pyrazin-6-yl}carbamoyl)benzoate trifluoroacetate **(Intermediate 217:** 148 mg, 99%) as yellow oil which was used in Step 4 without further purification. Mass spec: m/z: 366 $[M+H]^+$.

**Step 3**

**Intermediate 218: methyl 4-({2-[(rel-2R)-1-methylpyrrolidin-2-yl]imidazo[1,2-a]pyrazin-6-yl}carbamoyl)benzoate**

A solution of methyl 4-({2-[(rel-2R)-pyrrolidin-2-yl]imidazo[1,2-a]pyrazin-6-yl}carbamoyl)benzoate trifluoroacetate **(Intermediate 217:** 148 mg, 0.373 mmol), $Et_3N$ (188 mg, 1.86 mmol) and paraformaldehyde (33.6 mg, 1.12 mmol) in MeOH (4 mL) was stirred at rt for 1h. After addition of $NaBH_3CN$ (70.2mg, 1.12 mmol), the reaction mixture was heated at 60°C for 1h, then quenched with water and concentrated under reduced pressure. The residue was purified by reverse-phase flash chromatography (C18) using MeCN/$H_2O$ (65/35) as eluent to afford methyl 4-({2-[(rel-2R)-1-methylpyrrolidin-2-yl]imidazo[1,2-a]pyrazin-6-yl}carbamoyl)benzoate **(Intermediate 218:** 120 mg, 83%) as a yellow solid. Mass spec: m/z: 380 $[M+H]^+$.

**Step 4**

**Intermediate 219: 4-({2-[(rel-2R)-1-methylpyrrolidin-2-yl]imidazo[1,2-a]pyrazin-6-yl}carbamoyl)benzoic acid**

To a solution of methyl 4-({2-[(rel-2R)-1-methylpyrrolidin-2-yl]imidazo[1,2-a]pyrazin-6-yl}carbamoyl)benzoate **(Intermediate 218:** 120 mg, 0.310 mmol) in THF (6 mL)/MeOH (1 mL)/$H_2O$ (1 mL) was added LiOH (37.1 mg, 1.55 mmol). The reaction mixture was stirred at rt for 3h. The solution was adjusted to pH = 5 with a solution of HCl (4M in $H_2O$), then concentrated under reduced pressure. The residue was purified by reverse-phase flash chromatography (C18) using MeCN/$H_2O$ (18/82) as eluent to afford 4-({2-[(rel-2R)-1-methylpyrrolidin-2-yl]imidazo[1,2-a]pyrazin-6-yl}carbamoyl)benzoic acid **(Intermediate 219:** 85 mg, 74%) as a yellow solid. Mass spec: m/z: 366 $[M+H]^+$.

**Step 5**

**Intermediate 220: tert-butyl N-(10-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}decyl)carbamate**

To a solution of 11-[(tert-butoxycarbonyl)amino]undecanoic acid (CAS no: 10436-25-6: 5.00 g, 16.5 mmol) in DMF (100 mL) was added (2S,4R)-1-[(2S)-2-amino-3,3-dimethylbutanoyl]-4-hydroxy-N-{ [4-(4-methyl-1,3-thiazol-5-yl)phenyl]methyl}pyrrolidine-2-carboxamide (CAS: 1448189-80-7, 7.14 g, 16.5 mmol), DIEA (11.6 mL, 66.3 mmol) and HATU (8.20 g, 21.5 mmol) under nitrogen protection. The reaction mixture was stirred at rt for 4h and quenched with water (200 mL). The resulting solution was extracted with EtOAc (3 $\times$ 500 mL) and the organic layers were combined, washed with brine (200 mL), dried over anhydrous sodium sulfate, filtered and concentrated under reduced pressure. The residue was chromatographed on a silica gel column using DCM/MeOH (20/1) as eluent to afford tert-butyl N-(10-{ [(2S)-1-[(2S,4R)-4-hydroxy-2-({ [4-(4-methyl-1,3-thiazol-5-yl)phenyl]methyl}carbamoyl)pyrrolidin-1-yl]-3,3-dimethyl-1-oxobutan-2-yl]carbamoyl}decyl)carbamate **(Intermediate 220:** 8 g, 68% yield) as a yellow solid. Mass spec m/z: 714.7 [M+H]+.

**Step 6**

**Intermediate 221: (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide**

A solution of tert-butyl N-(10-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}decyl)carbamate **(Intermediate 220:** 800 mg, 1.052 mmol,) in dioxane (3 mL) was added HCl (3 mL, 4M in 1,4-dioxane) and the reaction mixture was stirred at rt for 2h. The reaction mixture was then concentrated under reduced pressure to provide (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide **(Intermediate 221:** 700 mg, 97.5%) as a brown solid, which was used in Step 7 without further purification. Mass spectrum: m/z: 614.4 [M+H]+.

**Step 7**

**Example 97 : (2S,4R)-1-[(2S)-3,3-dimethyl-2-(11-{[4-({2-[(rel-2R)-1-methylpyrrolidin-2-yl]imidazo[1,2-a]pyrazin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide**

To a solution of 4-({2-[(2R)-1-methylpyrrolidin-2-yl]imidazo[1,2-a]pyrazin-6-yl}carbamoyl)benzoic acid **(Intermediate 219:** 70 mg, 0.182 mmol), (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide **(Intermediate 221:** 134 mg, 0.218 mmol), in DMF (3 mL) was added HOBT (24.6 mg, 0.182 mmol), $Et_3N$ (127 µL, 0.910 mmol), EDCI (45.7 mg, 0.237 mmol). The mixture was stirred at 60°C overnight and quenched with water. The solution was purified by preparative HPLC (Column: XselectCSH Prep OBD C18 Column, 30x150 mm, 5µm; Mobile Phase A: Water (0.1% FA), Mobile Phase B: MeCN; Flow rate: 60 mL/min; Gradient: 20% B to 40% B) to afford (2S,4R)-1-[(2S)-3,3-dimethyl-2-(11-{[4-({2-[(2R)-1-methylpyrrolidin-2-yl]imidazo[1,2-a]pyrazin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide (36.5 mg, 21% yield) as a white solid. **(Example 97:** 7.60 mg, 10%) as a white solid. Mass spec: m/z 961.5 $[M+H]^+$; $^1H$ NMR (400 MHz, DMSO-$d_6$) δ ppm 9.43 (s, 1H), 8.85 - 8.88 (m, 2H), 8.07 - 8.11 (m, 3H), 7.95 - 7.97 (m, 2H), 7.40 - 7.48 (m, 4H), 4.65 (s, 1H), 4.55 - 4.60 (m, 2H), 4.51 - 4.52 (m, 1H), 4.34 - 4.38 (m, 1H), 3.83 - 3.90 (m, 1H), 3.79 - 3.82 (m, 2H),3.43 - 3.41(m, 2H), 2.64 - 2.70 (m, 1H), 2.31- 2.47 (m, 7H), 2.22 - 2.29 (m, 4H), 2.04 - 2.20 (m, 4H), 1.60 - 1.68 (m, 4H), 1.34 - 1.39 (m, 12H), 1.04 (s, 9H).

**Example 98 was synthesised following the Scheme 47**

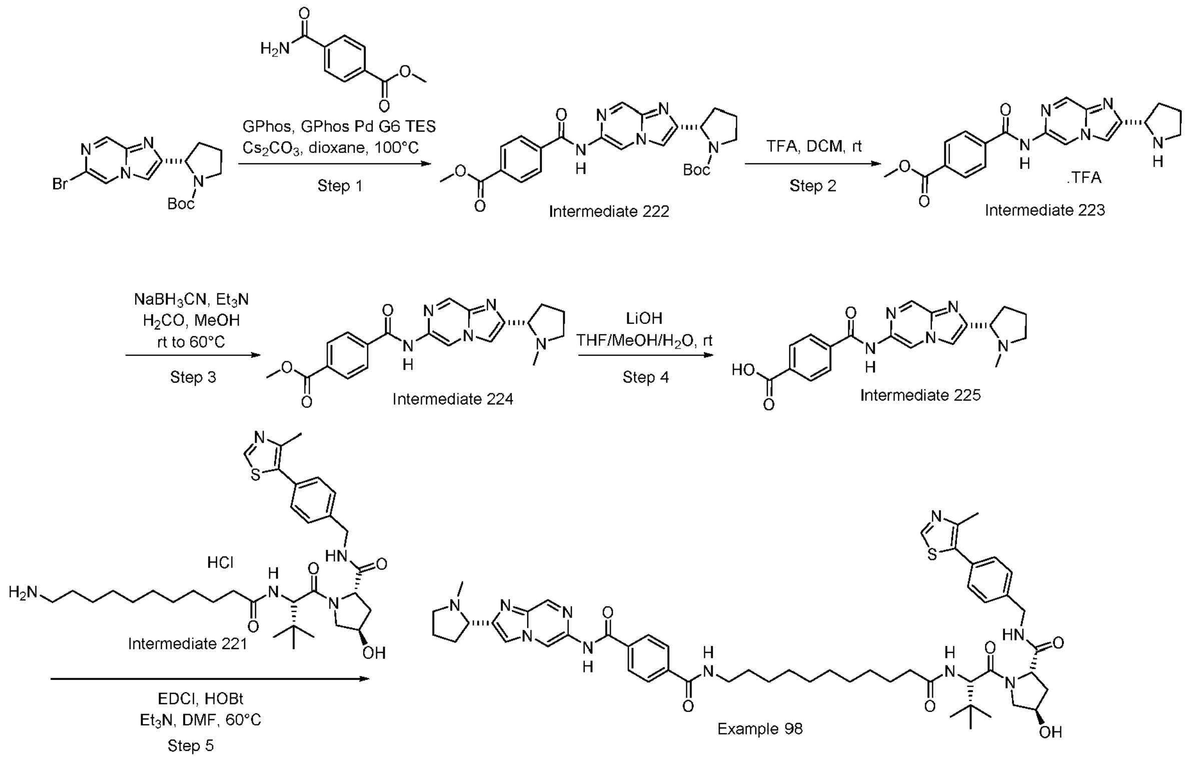


**Scheme 47**

**Step 1**

**Intermediate 222: tert-butyl (rel-2S)-2-{6-[4-(methoxycarbonyl)benzamido]imidazo[1,2-a]pyrazin-2-yl}pyrrolidine-1-carboxylate**

Following an analogous procedure to **Scheme 46, Step 1,** using tert-butyl (rel-2S)-2-{6-bromoimidazo[1,2-a]pyrazin-2-yl}pyrrolidine-1-carboxylate **(Intermediate 41D:** 190 mg, 0.512 mmol), afforded tert-butyl (rel-2S)-2-{6-[4-(methoxycarbonyl)benzamido]imidazo[1,2-a]pyrazin-2-yl}pyrrolidine-1-carboxylate **(Intermediate 222:** 180 mg, 71%) as a yellow solid. Mass spec: m/z: 466 $[M+H]^+$.

**Step 2**

**Intermediate 223: methyl 4-({2-[(rel-2S)-pyrrolidin-2-yl]imidazo[1,2-a]pyrazin-6-yl}carbamoyl)benzoate trifluoroacetate**

Following an analogous procedure to **Scheme 46, Step 2,** using tert-butyl (rel-2S)-2-{6-[4-(methoxycarbonyl)benzamido]imidazo[1,2-a]pyrazin-2-yl}pyrrolidine-1-carboxylate **(Intermediate 222:** 180 mg, 0.363 mmol), afforded methyl 4-({2-[(rel-2S)-pyrrolidin-2-yl]imidazo[1,2-a]pyrazin-6-yl}carbamoyl)benzoate trifluoroacetate **(Intermediate 223:** 140 mg, 99%) as a yellow oil. Mass spec: m/z: 366 $[M+H]^+$.

**Step 3**

**Intermediate 224: methyl 4-({2-[(rel-2S)-1-methylpyrrolidin-2-yl]imidazo[1,2-a]pyrazin-6-yl}carbamoyl)benzoate**

Following an analogous procedure to **Scheme 46, Step 3,** using methyl 4-({2-[(rel-2S)-pyrrolidin-2-yl]imidazo[1,2-a]pyrazin-6-yl}carbamoyl)benzoate trifluoroacetate **(Intermediate 223:** 140 mg, 0.360 mmol), afforded methyl 4-({2-[(rel-2S)-1-methylpyrrolidin-2-yl]imidazo[1,2-a]pyrazin-6-yl}carbamoyl)benzoate **(Intermediate 224:** 105 mg, 76%) as a yellow solid. Mass spec: m/z: 380 $[M+H]^+$.

**Step 4**

**Intermediate 225: 4-({2-[(rel-2S)-1-methylpyrrolidin-2-yl]imidazo[1,2-a]pyrazin-6-yl}carbamoyl)benzoic acid**

Following an analogous procedure to **Scheme 46, Step 4,** using methyl 4-({2-[(rel-2S)-1-methylpyrrolidin-2-yl]imidazo[1,2-a]pyrazin-6-yl}carbamoyl)benzoate **(Intermediate 224:** 105 mg, 0.274 mmol), afforded 4-({2-[(rel-2S)-1-methylpyrrolidin-2-yl]imidazo[1,2-a]pyrazin-6-yl}carbamoyl)benzoic acid **(Intermediate 225:** 70 mg, 69%) as a white solid. Mass spec: m/z: 366 $[M+H]^+$.

**Example 98: (2S,4R)-1-[(2S)-3,3-dimethyl-2-(11-{[4-({2-[(rel-2S)-1-methylpyrrolidin-2-yl]imidazo[1,2-a]pyrazin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide**

Following an analogous procedure to **Scheme 46, Step 7,** using 4-({2-[(rel-2S)-1-methylpyrrolidin-2-yl]imidazo[1,2-a]pyrazin-6-yl}carbamoyl)benzoic acid **(Intermediate 225:** 80 mg, 0.0.21 mmol) and (2S,4R)-1-[(2R)-2-(11-aminoundecanamido)-3,3-dimethylbutanoyl]-4-hydroxy-N-{ [4-(4-methyl-1,3-thiazol-5-yl)phenyl]methyl}pyrrolidine-2-carboxamide **(Intermediate 221:** 153 mg, 0.25 mmol), being purified by preparative HPLC (Column: Xselect CSH Prep OBD, 30 x 150 mm, 5μm; Eluent: Water (10mmol/L $NH_4HCO_3$ + 0.05% $NH_3H_2O$)/MeCN from 85/15 to 55/45; Flow rate: 60 mL/min), afforded (2S,4R)-1-[(2S)-3,3-dimethyl-2-(11-{[4-({2-[(rel-2S)-1-methylpyrrolidin-2-yl]imidazo[1,2-a]pyrazin-6-yl } carbamoyl)phenyl] formamido } undecanamido)butanoyl]-4-hydroxy-N- { [4-(4-methyl-1,3-thiazol-5-yl)phenyl]methyl}pyrrolidine-2-carboxamide **(Example 98:** 36.3 mg, 18%) as a white solid. Mass spec: m/z: 961.6 $[M+H]^+$; $^1H$ NMR (400 MHz, Methanol-$d_4$) δ ppm 9.42 (s, 1H), 8.84 - 8.87 (m, 2H), 8.07 - 8.08 (m, 3H), 7.95 - 7.97 (m, 2H), 7.40 - 7.48 (m, 4H), 4.65 (s, 1H), 4.51 - 4.58 (m, 3H), 4.34 - 4.38 (m, 1H), 3.83 - 3.90 (m, 1H), 3.79 - 3.82 (m, 1H), 3.60 - 3.70 (m, 1H), 3.39 - 3.43 (m, 2H), 2.42 - 2.61 (m, 4H), 2.32 - 2.41 (m, 4H), 2.20 - 2.29 (m, 8H), 1.54 - 1.69 (m, 4H), 1.34 - 1.39 (m, 12H), 1.04 (s, 9H).

**Example 99 was synthesised following Scheme 47**

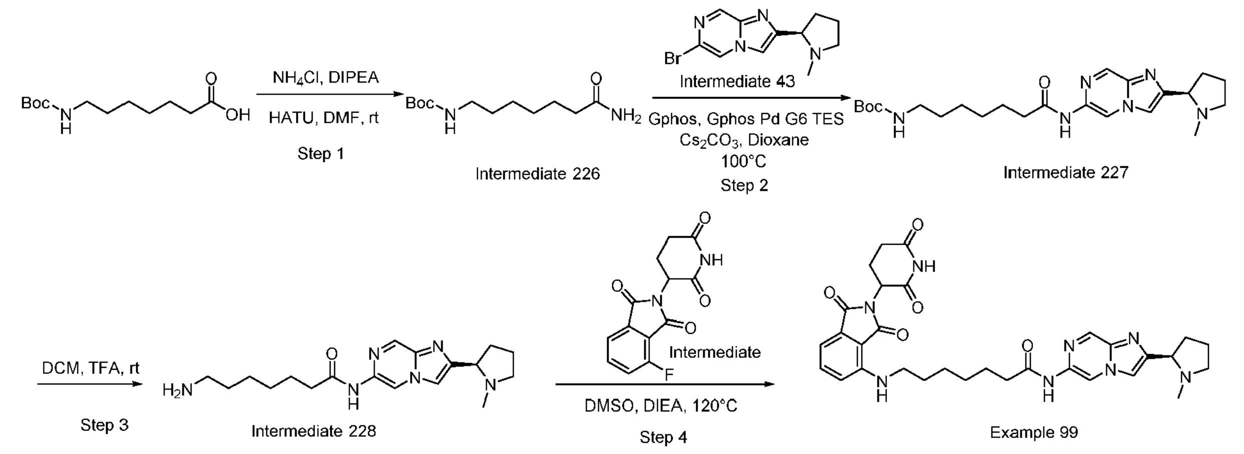


## Scheme 47

### Step 1

**Intermediate 226: tert-Butyl N-(6-carbamoylhexyl)carbamate**

To a solution of 7-[(tert-butoxycarbonyl)amino]heptanoic acid (CAS No: 60142-89-4: 2.00 g, 7.74 mmol) in DMF (20 mL) was added HATU (3.53 g, 9.29 mmol) and DIPEA (4.26 mL, 23.2 mmol). The reaction mixture was stirred at room temperature for 10 minutes and $NH_4Cl$ (1.24 g, 23.2 mmol) in DMF (10 mL) was added. The resulting mixture was stirred at room temperature overnight. The mixture was quenched with water (300 mL) and extracted with EtOAc (3 × 500 mL). The combined organic layers were washed with brine, dried over anhydrous $Na_2SO_4$ and filtered. The filtrate was concentrated under reduced pressure and the residue was purified by flash column chromatography, on a C18 column, with MeCN:$H_2O$ (43:57, with 0.05% TFA) as eluent afford tert-butyl N-(6-carbamoylhexyl)carbamate **(Intermediate 226:** 1.80 g, 94%) as a white solid. Mass spec: m/z: 245.2 $[M+H]^+$.

### Step 2

**Intermediate 227: tert-Butyl N-[6-({2-[(2R)-1-methylpyrrolidin-2-yl]imidazo[1,2-a]pyrazin-6-yl}carbamoyl)hexyl]carbamate**

To a solution of tert-butyl N-(6-carbamoylhexyl)carbamate **(Intermediate 226:** 150 mg, 0.608 mmol) in dioxane (5 mL) was added (2R)-2-{6-bromoimidazo[1,2-a]pyrazin-2-yl}-1-methylpyrrolidine **(Intermediate 43:** 256 mg, 0.912 mmol), $Cs_2CO_3$ (594 mg, 1.82 mmol), Gphos (32.6 mg, 0.061mmol) and Gphos Pd G6 TES (57.4 mg, 0.061 mmol) under $N_2$. The resulting mixture was stirred at 100 °C overnight, under $N_2$. The volatiles were removed under reduced pressure and the residue was purified by flash column chromatography, on a C18 column, with MeCN:$H_2O$ (35:65, with 0.05% TFA) to afford tert-butyl N-[6-({2-[(2R)-1-methylpyrrolidin-2-yl]imidazo[1,2-a]pyrazin-6-yl}carbamoyl)hexyl]carbamate **(Intermediate 227:** 230 mg, 84%) as a yellow oil. Mass spec: m/z:445.4 $[M+H]^+$.

### Step 3

**Intermediate 228: 7-Amino-N-{2-[(2R)-1-methylpyrrolidin-2-yl]imidazo[1,2-a]pyrazin-6-yl}heptanamide trifluoroacetate**

To a solution of tert-butyl N-[6-({2-[(2R)-1-methylpyrrolidin-2-yl]imidazo[1,2-a]pyrazin-6-yl}carbamoyl)hexyl]carbamate **(Intermediate 227:** 250 mg, 0.556 mmol) in DCM (4 mL) was added TFA (2 mL). The reaction mixture was stirred at room temperature for 1 h. The reaction mixture was then concentrated under reduced pressure to afford get 7-amino-N-{2-[(2R)-1-methylpyrrolidin-2-yl]imidazo[1,2-a]pyrazin-6-yl}heptanamide trifluoroacetate **(Intermediate 228:** 252 mg, 98%) as a yellow solid, which was used in the next steps without further purification. Mass spec: m/z: 345.2 $[M+H]^+$.

**Step 4**

**Example 99: 7-{[2-(2,6-dioxopiperidin-3-yl)-1,3-dioxoisoindol-4-yl]amino}-N-{2-[(2R)-1-methylpyrrolidin-2-yl]imidazo[1,2-a]pyrazin-6-yl}heptanamide**

To a solution of 7-amino-N-{2-[(2R)-1-methylpyrrolidin-2-yl]imidazo[1,2-a]pyrazin-6-yl}heptanamide trifluoroacetate **(Intermediate 228:** 206 mg, 0.450 mmol) in DMSO (5 mL) was added 2-(2,6-dioxopiperidin-3-yl)-4-fluoroisoindole-1,3-dione (CAS: 835616-60-9: 124 mg, 0.450 mmol) and DIPEA (705 μL, 4.05 mmol). The reaction was stirred for 5 h at 120 °C. The reaction mixture was subjected to flash column chromatography, on a C18 column, using MeCN:$H_2O$ (35:65, with 0.05% TFA) as eluent. Further purification by preparative HPLC (Column: XselectCSH Prep OBD C18 Column, 30x150 mm, 5μm; Mobile Phase A: Water (0.1% FA), Mobile Phase B: MeCN; Flow rate: 60 mL/min; Gradient: 5% B to 30% B) afforded 7-{[2-(2,6-dioxopiperidin-3-yl)-1,3-dioxoisoindol-4-yl]amino}-N-{2-[(2R)-1-methylpyrrolidin-2-yl]imidazo[1,2-a]pyrazin-6-yl}heptanamide **(Example 99:** 31.5 mg, 11%) as a yellow solid. $^1$H NMR (400 MHz, $CD_3OD$) δ ppm 9.23 (s, 1H), 8.81 (s, 1H), 8.10 (s, 1H), 7.52 - 7.55 (m, 1H), 7.00 - 7.06 (m, 2H), 5.05 - 5.09 (m, 1H), 4.09 - 4.15 (m, 1H), 3.50 (s, 1H), 3.32 - 3.33 (m, 3H), 2.89 - 2.92 (m, 2H), 2.87 - 2.88 (m, 1H), 2.83 - 2.84 (m, 1H), 2.74 - 2.78 (m, 3H), 2.46 - 2.50 (m, 3H), 2.29 - 2.31(m, 1H), 2.25 - 2.26 (m, 3H), 1.71 - 1.79 (m, 4H), 1.45 - 1.55 (m, 4H). Mass spec: m/z: 601.4 $[M+H]^+$.

**Example 100: Efficacy *in vitro***

This procedure is intended to measure the interaction between YEATS proteins and histone peptides modified by an appropriate epigenetic mark and can be used to assay compounds which bind to the YEATS proteins and inhibit this interaction. The assay uses 6His tagged MLLT1 or MLLT3 with an anti-6His antibody conjugated with $Eu^{3+}$ chelate (donor) and a biotinylated peptide with Streptavidin conjugated XL-665 (acceptor). When the donor and acceptor labels are in close proximity, by binding of the peptide by MLLT1 or MLLT3, the excitation of the donor (337 nm) triggers a Fluorescence Resonance Energy Transfer (FRET) to the acceptor which then fluoresces at specific wavelength (665 nm).

**HTRF Protein-Peptide Titration**

This step optimises the protein/peptide ratio that will be used in HTRF screening assay in order to allow the best signal-to-noise ratio. Each new purified protein should be titrate this way as the signal-to-noise ratio may differ from different protein batches. Displacement assay for MLLT1 and MLLT3 was performed by using H3K9cro peptide (ARTKQTAR(Kcrot)STGGKAPRKQLYK(biotin)).

A serial dilution of 4X solution of biotinylated peptide was prepared in assay buffer (25 mM HEPES, 20 mM NaCl, 0.05 % BSA, 0.05 % Tween-20, pH 7.0) covering concentrations from 3.2 μM to 0.2 nM. and 5 μL of each dilution were transferred horizontally to fill 15 columns of a white ProxiPlate-384 Plus shallow well Microplate (Perkin Elmer, USA). A sixteenth column of buffer was also included for a background reference. In parallel, a serial dilution of the 6His-tag protein of interest was prepared in the same way and 5 μL of each dilution were vertically added to the wells containing the peptide dilutions. Buffer has been transferred to the last line of the plate in order to make a no-protein reference. The microplate has been covered and incubated 1 hour at room temperature. A 2X detection mix was prepared in assay buffer by diluting streptavidin conjugated with XL-665 (610SAXLB, Cisbio Bioassays, France) to 1/2000 and anti-6His(Eu) chelate (LANCE Eu-W1024 Anti-6xHis, AD0111, Perkinelmer LAS (UK)LTD) to 1/20000, 10 μL were added to each well and the plate has been incubated for 1 to 24 hours at room temperature in the dark. The plate was read in the Pherastar using the HTRF module with dual emission protocol (A = ex. 320nm em. 665nm, B = ex. 320nm em. 620nm).

The HTRF ratio was calculated as channel A/B * 10000 and the specific signal as follow: specific signal = HTRF Ratio Positive signal - HTRF Ratio Negative signal (without protein). The concentrations of protein and peptide that allow the maximum specific signal without saturation were selected to perform HRTF screening assay described below.

**HTRF $IC_{50}$ Determination Assay**

The compounds were dissolved in DMSO at a stock concentration of 10 mM and dispensed into microplate using the Echo acoustic liquid handler (Labcyte, Beckman Coulter, USA). The assay covers a window of compound concentrations from 20 μM to 20 nM spread in 11 data points in duplicate. Solvent and compound controls were added to each plate in order to calculate the background signal.

Ten microliters of 2x mix of 6His-protein and biotinylated peptide, the concentrations were determined above, were added to the dispensed compounds and incubated for one hour at room temperature in the dark. Ten microliters of the 2X detection mix was added to each well and the plate was incubated for another hour at room temperature in the dark and the plate was read in order to calculate the HTRF specific signal. The % inhibition values as follows:

$$\% I = \frac{positive\ control - signal}{positive\ control} \times 100$$

The $IC_{50}$ values were determined by plotting % inhibition vs compound concentration and fitting the data to a non-linear sigmoidal dose response equation (4 parameter fit):

$$I_i = I_o + \frac{I_{complete} - I_o}{1 + \left(\frac{IC_{50}}{c_i}\right)^S}$$

$I_i$: Inhibition at given concentration $c_i$
$I_{complete}$: Complete absence of binding, bottom of curve
$I_0$: No inhibition of binding, top of curve
$IC_{50}$: Concentration at which inhibition is 50%
$c_i$: concentration i in range tested
$s$: Hill coefficient (slope at $IC_{50}$)

Calculations were carried out in Excel and GraphPad Prism templates.

***Results***

**Table 4** exhibits the ranges of $IC_{50}$ (nM) of the compounds when tested in the MLLT1 and MLLT3 assays.

**Category A:** $IC_{50} \leq 150$ nM; **Category B:** 150 nM < $IC_{50} \leq 1500$ nM; **Category C:** 1500 nM < $IC_{50} \leq 20000$ nM; **Category D:** $IC_{50} > 20000$ nM.

**Table 4**

| Example | MLLT1 assay $IC_{50}$ (nM) | MLLT3 assay $IC_{50}$ (nM) |
|---|---|---|
| **1** | B | B |
| **2** | C | C |
| **3** | B | B |
| **4** | B | C |
| **5** | B | B |
| **6** | C | C |
| **7** | C | C |
| **8** | B | B |
| **9** | C | C |
| **10** | C | D |
| **11** | B | B |
| **12** | B | C |
| **13** | C | C |
| **14** | B | B |
| **15** | C | C |
| **16** | B | B |
| **17** | C | C |
| **18** | C | C |

(continued)

| Example | MLLT1 assay $IC_{50}$ (nM) | MLLT3 assay $IC_{50}$ (nM) |
|---|---|---|
| **19** | B | C |
| **20** | C | C |
| **21** | B | B |
| **22** | B | C |
| **23** | B | B |
| **24** | B | C |
| **25** | B | B |
| **26** | B | B |
| **27** | C | D |
| **28** | B | B |
| **29** | C | C |
| **30** | B | B |
| **31** | C | C |
| **32** | B | C |
| **33** | B | C |
| **34** | C | C |
| **35** | C | C |
| **36** | C | D |
| **37** | B | C |
| **38** | C | C |
| **39** | C | D |
| **40** | B | C |
| **41** | B | B |
| **42** | B | C |
| **43** | B | C |
| **44** | C | C |
| **45** | B | B |
| **46** | C | C |
| **47** | B | C |
| **48** | B | B |
| **49** | B | B |
| **50** | C | C |
| **51** | B | C |
| **52** | C | D |
| **53** | B | B |
| **54** | B | C |
| **56** | B | B |
| **57** | B | C |

(continued)

| Example | MLLT1 assay $IC_{50}$ (nM) | MLLT3 assay $IC_{50}$ (nM) |
|---|---|---|
| **58** | B | B |
| **59** | C | C |
| **60** | A | B |
| **61** | B | B |
| **62** | C | C |
| **63** | C | C |
| **64** | B | B |
| **65** | B | C |
| **67** | B | B |
| **68** | B | B |
| **69** | C | C |
| **70** | B | B |
| **71** | B | B |
| **72** | B | B |
| **73** | B | B |
| **76** | B | C |
| **77** | B | C |
| **78** | B | B |
| **79** | A | A |
| **80** | A | A |
| **81** | C | C |
| **82** | A | A |
| **83** | B | B |
| **84** | A | B |
| **87** | B | C |
| **88** | B | B |
| **89** | A | B |
| **90** | A | B |
| **91A** | B | B |
| **91B** | C | C |
| **92A** | B | B |
| **92B** | A | B |
| **93A** | B | B |
| **93B** | B | C |
| **94** | B | B |
| **95** | B | B |
| **96** | B | B |
| **97** | B | C |

(continued)

| Example | MLLT1 assay $IC_{50}$ (nM) | MLLT3 assay $IC_{50}$ (nM) |
|---|---|---|
| **98** | C | Not determined |

**MV4-11 Cellular Proliferative Assay**

An MV4-11 proliferation assay was used to test the ability of compounds to reduce cell viability. MV4-11 cells (ATCC #CRL9591) were grown in IMDM medium (11510596, Gibco) supplemented with 10% fetal bovine serum (F7524, Gibco) and 1% Penicillin/Streptomycin (15140-122, Gibco) at 37°C with 5% $CO_2$. Cells were seeded in 384-well Elplasia plates (4447, Corning) at a density of 50 cells/well and treated with vehicle (DMSO) or compounds (10 serial semi-log dilutions, 30 μM as top concentration).

After 120 hours of treatment, cells were incubated for 10 min at RT with the CellTiter-Glo (CTG) reagent (G7571, Promega). Measurement of the luminescence signal was performed on a microplate reader (Ensight, PerkinElmer). CTG reagent measures the amount of ATP, which is directly proportional to the number of cells, to determine the number of viable cells in culture.

The data were analysed as follows:

$$\text{Data normalization, } N(x) = \frac{x - \langle cr \rangle}{\langle sr \rangle - \langle cr \rangle} \times 100$$

where x is the measured raw signal value of a well, (cr) is the median of the measured signal values for the Central Reference (DMSO) wells on a plate, (sr) is the median of the measured signal values for the Scale Reference (reference compound) wells on a plate.

$$\text{Curve fitting, } S_0 + \frac{S_{\text{inf}} - S_0}{1 + \left(\frac{AC_{50}}{10^C}\right)^n}$$

where the experimental set-up assumes the Hill parameters in certain ranges: $S_0$ ~ Central Reference (CR) from normalization (= Bottom), $S_{inf}$ ~ Scale Reference (SR) from normalization (= Top), nHill ~ 1, $AC_{50}$ ~ within the measured concentration range.

**Category A:** $IC_{50} \leq 150$ nM; **Category B:** 150 nM < $IC_{50} \leq 200$ nM; **Category C:** 500 nM < $IC_{50} \leq 1000$ nM; **Category D:** $IC_{50} > 1000$ nM.

**Table 5**

| Example | MV4-11 cell antiproliferative activity $IC_{50}$ (nM) |
|---|---|
| **95** | A |
| **96** | B |
| **97** | B |
| **98** | C |

**Western blot to measure degradation of MLLT1 in MV4-11 cells**

MV4-11 cells were seeded in 12-well plates at a density of 500,000 cells/well and treated for 24 hours with vehicle (DMSO) or compound of Example 95 (3, 1 or 0.3 μM). Cells were then collected, washed with PBS and lysed in RIPA buffer (R0278, Sigma-Aldrich) supplemented with protease inhibitors (Halt™ Protease and Phosphatase Inhibitor Cocktail, EDTA-free, 78445, ThermoFisher). Protein samples (10 μg) were resolved on 4-20% Criterion TGX Precast gels (5671095, Biorad) for 30 minutes at 250V. The antibodies used were anti-MLLT1/ENL (Cell Signaling Technology #14893, diluted 1:1000), anti-GAPH-HRP (Santa Cruz Biotechnology sc47724, diluted 1:2000), Goat anti-Rabbit-HRP (Cell Signaling Technology #7074, diluted 1:2000). HRP activity was revealed using the SuperSignal™ West Pico Plus kit (34580, ThermoScientific) and acquired with the Pxi6 Syngene imager. The resulting Western blot is provided in

**Figure 1.**

In one embodiment, the invention provides compounds according to the following aspects.

1. A compound which is a Proteolysis Targeting Chimera (PROTAC) or a pharmaceutically acceptable salt thereof, wherein the PROTAC has the structure:

M - LINK - U

wherein U is an E3 ubiquitin ligase binding moiety, LINK is a moiety that covalently links M and U, and M is an MLLT1 and/or MLLT3 binder of formula (I):

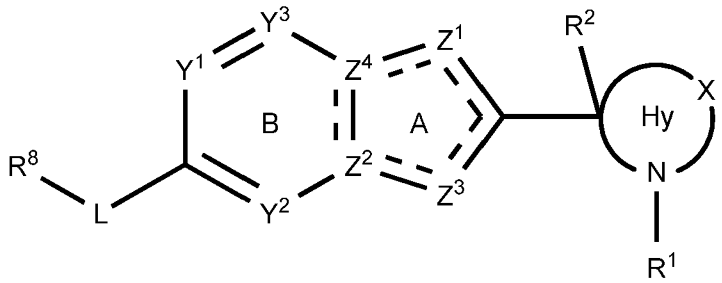

(I)

wherein:

one of $Z^2$ and $Z^4$ is N and the other is C, $Z^1$ is N or -C($R^4$)-, $Z^3$ is -C($R^4$)-, $Y^1$ is N or - C($R^7$)-, $Y^2$ is N or -C($R^6$)-, and $Y^3$ is N or -C($R^5$)-;

Hy is a 4- to 7-membered heterocyclyl ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;

L is -C(O)N(H)-, wherein either (i) the C atom of L is bonded to $R^8$, and the N atom of L is bonded to ring B; or (ii) the C atom of L is bonded to ring B, and the N atom of L is bonded to $R^8$;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;

$R^4$, $R^6$, and $R^7$ are independently selected from H, halo, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

$R^8$ is (i) a bond to LINK or (ii) a group selected from a 6- to 10-membered aryl ring, a 5- to 10-membered heteroaryl ring, a 5- to 10-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

each $R^{11}$ is independently selected from H, $C_{1-4}$ alkyl, and $C_{1-4}$ cycloalkyl;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

2. A compound according to aspect 1, wherein LINK is (a) a single bond, or (b) a chemical linker group represented by formula (L):

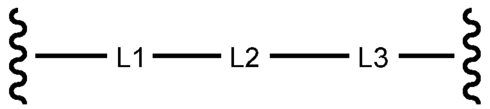

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-, $-S(O_2)N(R')-$, $-N(R')S(O_2)-$, -($C_{1-6}$ alkylene)-, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

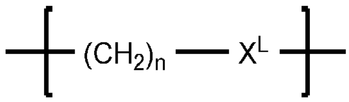

;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30; and
each R' is independently selected from H and $C_{1-4}$ alkyl.

3. A compound according to aspect 2, wherein each L4 is independently selected from a divalent 4- to 7-membered heterocyclyl ring and a unit of formula

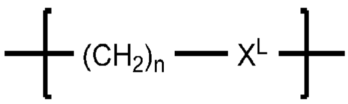

;

wherein each $X^L$ is independently selected from a single bond, -O-, or -S-; and
n is selected from 1 or 2.

4. A compound according to aspect 2 or 3, wherein each divalent 4- to 7-membered heterocyclyl ring of L1, L2 and L3 when present contains at least one N atom.

5. A compound according to aspect 4, wherein each divalent 4- to 7-membered heterocyclyl ring of L1, L2 and L3 when present contains one or two N atoms.

6. A compound according to aspect 5, wherein each divalent 4- to 7-membered heterocyclyl ring of L1, L2 and L3 when present is independently selected from piperidinyl and diazinanyl.

7. A compound according to any one of aspects 2 to 6, wherein L2 is selected from $-(CH_2)_m-$, $-(CH_2CH_2O)_m-$, $-(OCH_2CH_2)_m-$, $-(CH_2CH_2S)_m-$, $-(SCH_2CH_2)_m-$, and a divalent 4- to 7-membered heterocyclyl ring.

8. A compound according to any one of aspects 2 to 7, wherein m is selected from 1 to 10.

9. A compound according to any one of aspects 2 to 8, wherein L1 and L3 are each independently selected from a

single bond, -N(R')-, -C(O)N(R')-, -O-, - N(R')C(O)-, -C(O)-, $-S(O_2)N(R')-$, $-N(R')S(O_2)-$, and a divalent 4- to 7-membered heterocyclyl ring.

10. A compound according to any one of aspects 2 to 9, wherein L1 and L3 are each independently selected from a single bond, -N(R')-, -C(O)N(R')-, -N(R')C(O)-, -O-, and a divalent 4- to 7-membered heterocyclyl ring.

11. A compound according to any one of aspects 2 to 10, wherein L1 and L3 are each independently selected from a single bond, -N(R')-, -C(O)N(R')-, -N(R')C(O)-, -O-, piperidinyl and diazinanyl.

12. A compound according to any one of aspects 1 to 11, wherein U is a CRBN or VHL E3 ubiquitin ligase binding moiety.

13. A compound according to aspect 12, wherein U is a CRBN E3 ubiquitin ligase binding moiety.

14. A compound according to aspect 12, wherein U is a VHL E3 ubiquitin ligase binding moiety.

15. A compound according to any one of aspects 1 to 12, wherein U is:

(a) a CRBN E3 ubiquitin ligase binding moiety of formula (U1):

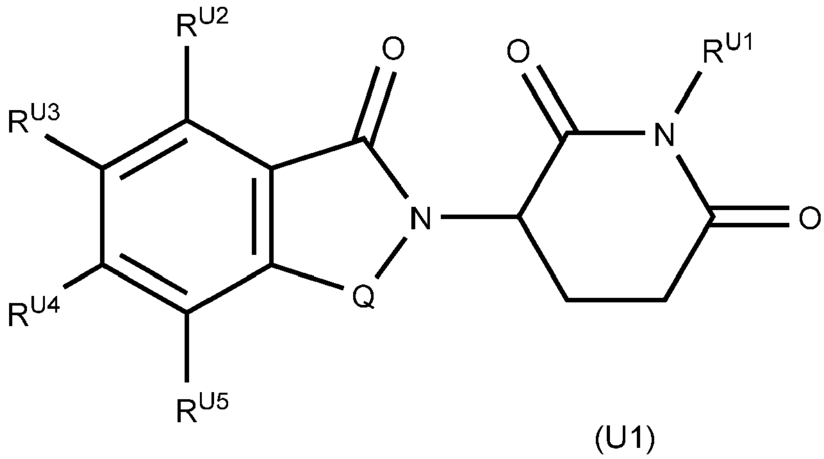

(U1)

wherein:

$R^{U1}$ is H;
Q is selected from $-CH(R^{U6})-$, $-N(R^{U6})-$, -O-, -C(O)-, $-NH-CH(R^{U6})-$, $-N=C(R^{U6})-$, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$ , $R^{U4}$ and $R^{U5}$ is a single bond to LINK;

(b) a CRBN E3 ubiquitin ligase binding moiety of formula (U4):

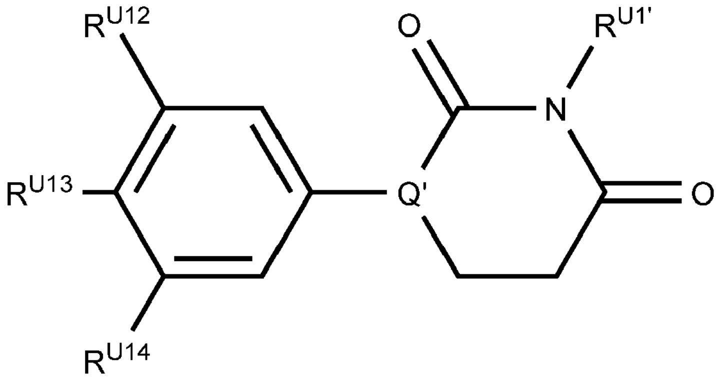

(U4)

wherein:

$R^{U1'}$ is H;
Q' is selected from CH and N;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

or (c) a VHL E3 ubiquitin ligase binding moiety of formula (U2):

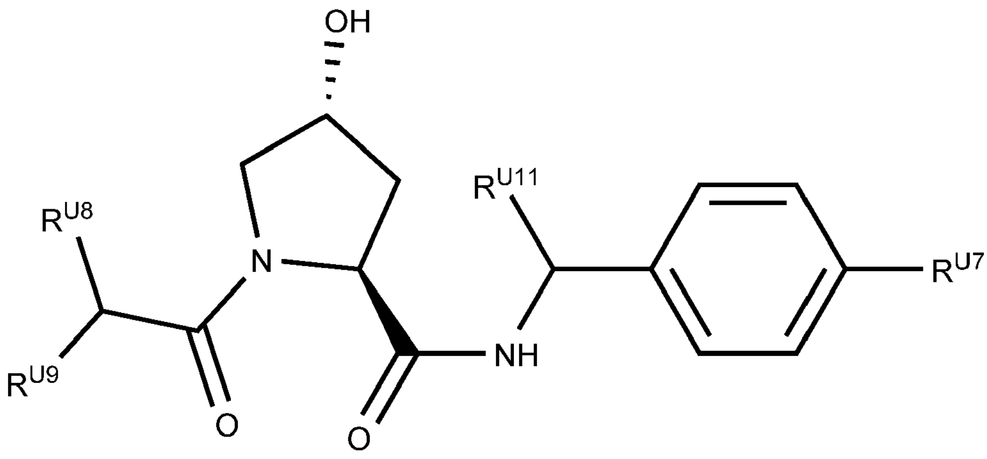

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

16. A compound according to aspect 15, wherein U is a VHL E3 ubiquitin ligase binding moiety of formula (U3):

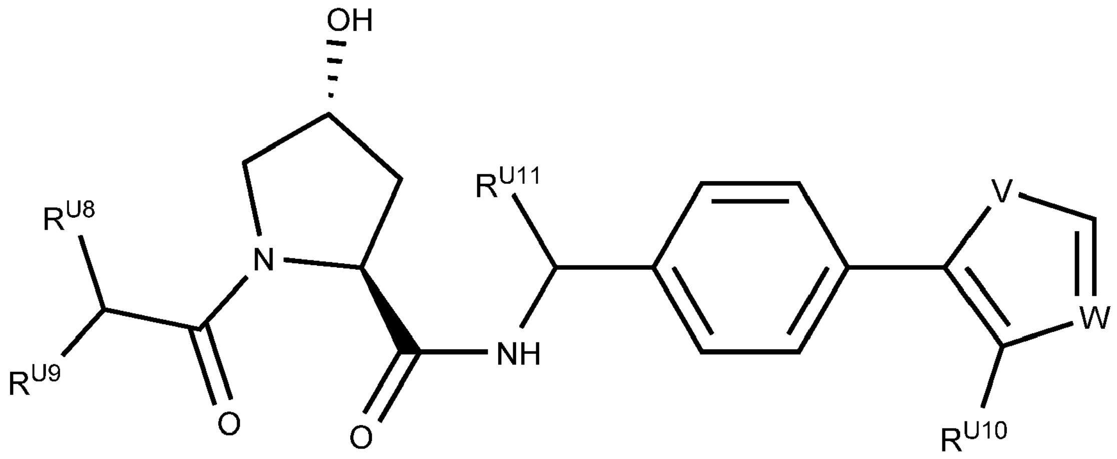

(U3)

wherein:

V is S or O;
W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

17. A compound according to aspect 16, wherein $R^{U10}$ is H or methyl.

18. A compound according to any one of aspects 15 to 17, wherein $R^{U8}$ is t-butyl.

19. A compound according to aspect 15, wherein U is a CRBN ubiquitin ligase binding moiety of formula (U1), wherein $R^{U6}$ is H or methyl.

20. A compound according to aspect 15 or 19, wherein U is a CRBN ubiquitin ligase binding moiety of formula (U1), wherein Q is selected from -C(O)- and $-CH_2-$.

21. A compound according to any one of aspects 15, 19 and 20, wherein U is a CRBN ubiquitin ligase binding moiety of formula (U1), wherein three of $R^{U2}$, $R^{U3}$, $R^{U4}$ and $R^{U5}$ are H.

22. A compound according to aspect 15, wherein U is a CRBN ubiquitin ligase binding moiety of formula (U4), wherein Q' is N.

23. A compound according to aspect 15 or 22, wherein U is a CRBN ubiquitin ligase binding moiety of formula (U4), wherein Q' is CH.

24. A compound according to any one of aspects 15, 22 and 23, wherein U is a CRBN ubiquitin ligase binding moiety of formula (U4), wherein two of $R^{U12}$, $R^{U13}$ and $R^{U14}$ are H.

25. A compound according to any one of the preceding aspects, wherein the N atom of ring Hy which is substituted by $R^1$ is adjacent to the C atom of ring Hy that is bonded to ring A.

26. A compound according to any one of the preceding aspects, wherein no more than two of $Y^1$, $Y^2$ and $Y^3$ are N.

27. A compound according to any one of the preceding aspects, wherein $Z^1$ and $Z^2$ are N, $Z^3$ is $-C(R^4)-$, $Z^4$ is C, $Y^1$ is $-C(R^7)-$ or N, $Y^2$ is $-C(R^6)-$, and $Y^3$ is $-C(R^5)-$.

28. A compound according to any one of the preceding aspects, wherein the C atom of L is bonded to $R^8$, and the

N atom of L is bonded to ring B.

29. A compound according to any one of the preceding aspects, wherein M is of formula (II):

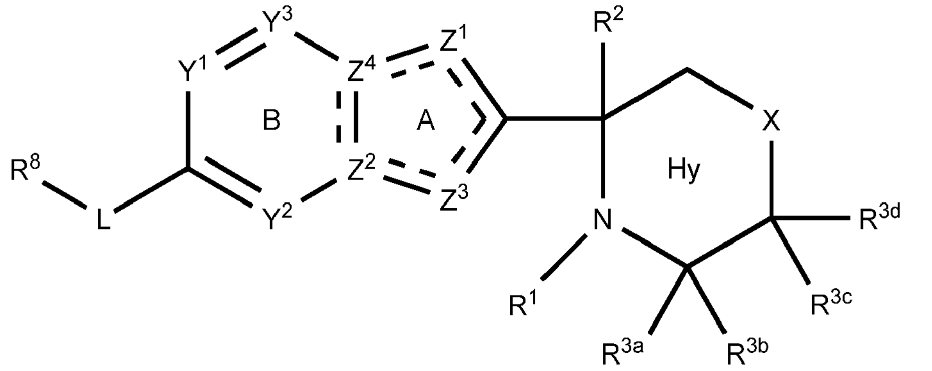

(II)

wherein:

$Z^1$, $Z^2$, $Z^3$, $Z^4$, $Y^1$, $Y^2$, $Y^3$, $R^1$, $R^2$, $R^8$, X and L are as defined in any one of the preceding aspects;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring,
with the proviso that when X is $NR^{11}$, O, or S, then neither $R^{3c}$ nor $R^{3d}$ are halo, or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

30. A compound according to any one of the preceding aspects, wherein $Y^1$ is N.

31. A compound according to any one of aspects 1 to 29, wherein $Y^1$ is $-C(R^7)-$.

32. A compound according to any one of aspects 1 to 31, wherein $R^4$, $R^5$, $R^6$, and $R^7$ are H.

33. A compound according to any one of aspects 1 to 31, wherein one of $R^4$, $R^5$, $R^6$, and $R^7$ is selected from halo, $C_{1-4}$ alkoxy, and $C_{1-4}$ alkyl which is itself unsubstituted or substituted by one, two or three halo, and the rest are H.

34. A compound according to any one of aspects 1 to 33, wherein $R^4$ is H.

35. A compound according to any one of aspects 1 to 29, wherein M is of formula (III):

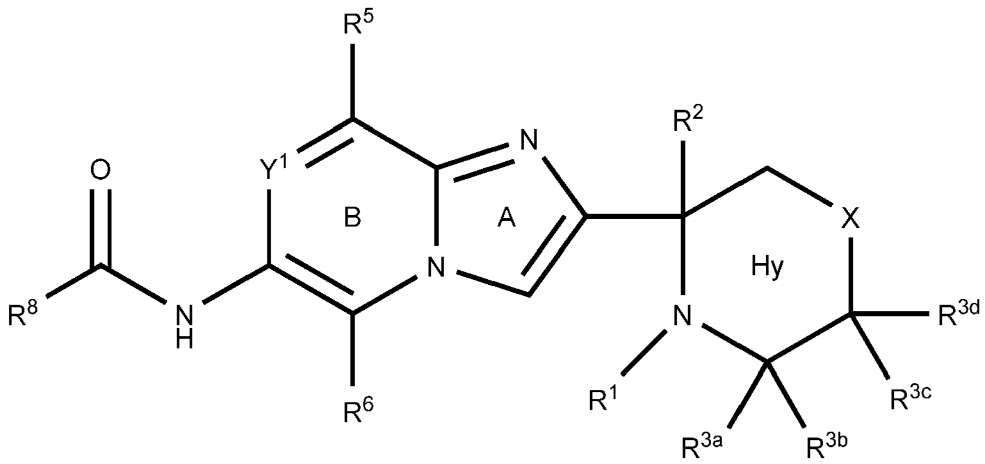

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or $R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
X is a bond, -N(Me)-, O or $-CH_2-$;
$Y^1$ is N or $-C(R^7)-$;
$R^5$, $R^6$ and $R^7$ are independently selected from H, halo $C_{1-4}$ alkoxy, and $C_{1-4}$ alkyl;
$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

36. A compound according to aspect 35, wherein M is of formula (IIIa):

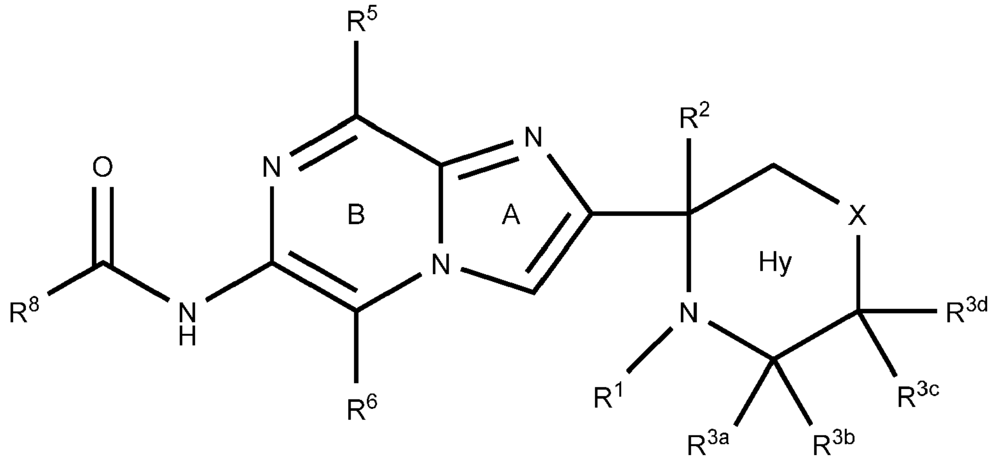

(IIIa)

wherein $R^1$, $R^2$, $R^{3a}$, $R^{3b}$, $R^{3c}$, $R^{3d}$, $R^5$, $R^6$, $R^8$ and X are as defined in aspect 35.

37. A compound according to aspect 35, wherein M is of formula (IIIb):

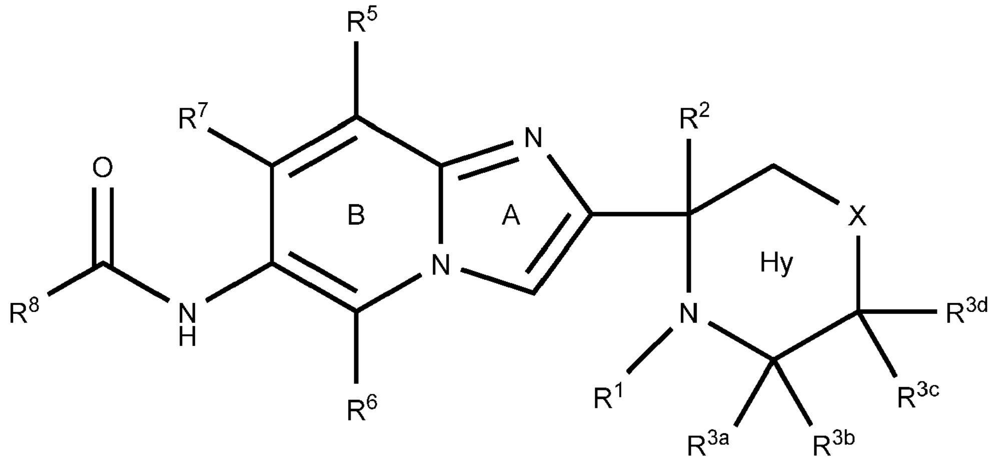

(IIIb)

wherein $R^1$, $R^2$, $R^{3a}$, $R^{3b}$, $R^{3c}$, $R^{3d}$, $R^5$, $R^6$, $R^7$, $R^8$ and X are as defined in aspect 35.

38. A compound according to any one of the preceding aspects, wherein $R^8$ is a group (ii) and M is bonded to LINK via a C or N atom within group $R^8$.

39. A compound according to any one of aspects 1 to 37, wherein $R^8$ is a group (ii) and M is bonded to LINK via a C or N atom within ring Hy.

40. A compound according to any one of the preceding aspects, wherein when M is bonded to LINK via a C or N atom within group $R^8$ or ring Hy, the C or N atom within group $R^8$ or ring Hy that is bonded to LINK is a C or N atom that forms part of the ring of group $R^8$ or ring Hy.

41. A compound according to any one of aspects 1 to 40, wherein $R^8$ is selected from a 6- to 10-membered aryl ring, a 5- to 10-membered heteroaryl ring, and a 5- to 6-membered heterocyclyl ring, the group $R^8$ being unsubstituted or substituted by one, two or three substituents independently selected from halo, $C_{1-4}$ alkoxy, $R^{10}$, $-(CH_2)-R^{10}$, =O, -CN, and $C_{1-4}$ alkyl which is itself unsubstituted or substituted by one $R^9$.

42. A compound according to aspect 41, wherein $R^8$ is a 5- to 10-membered heteroaryl ring which is unsubstituted or substituted by one or two $C_{1-4}$ alkyl groups.

43. A compound according to any one of aspects 1 to 37, wherein $R^8$ is a bond to LINK.

44. A compound according to any one of aspects 1 to 43, wherein Hy is an unsubstituted pyrrolidine ring, $R^1$ is methyl, and $R^2$ is H.

45. A compound according to any one of aspects 1 to 24, wherein M is a monovalent moiety of:

1-methyl-N-{ 8-methyl-2-[(2R)-1-methylpyrrolidin-2-yl]imidazo[1,2-a]pyrazin-6-yl}-1H-indazole-5-carboxamide;
1,3-dimethyl-N- { 8-methyl-2-[(2R)-1-methylpyrrolidin-2-yl]imidazo[1,2-a]pyrazin-6-yl}-1H-indazole-6-carboxamide;
rel-7-fluoro-N-{2-[(2R)-1-methylpyrrolidin-2-yl]imidazo[1,2-a]pyridin-6-yl}quinoline-6-carboxamide;
N-{2-[(2R,4S)-4-fluoro-1-methylpyrrolidin-2-yl]imidazo[1,2-a]pyrazin-6-yl}-1-methyl-1H-indazole-5-carboxamide;
1,3-dimethyl-N-{2-[(2R)-pyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
N-{2-[4-ethyl-1-methylpyrrolidin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-7-fluoro-N-{2-[(2R)-1-methylpyrrolidin-2-yl]imidazo[1,2-a]pyridin-6-yl}isoquinoline-6-carboxamide;
rel-5-fluoro-1,3-dimethyl-N-{2-[(2R)-1-methylpyrrolidin-2-yl]imidazo[1,2-a]pyrazin-6-yl}-1H-indazole-6-carboxamide;
1-methyl-N-(2-{2-methyl-2-azaspiro[4.4]nonan-3-yl}imidazo[1,2-a]pyridin-6-yl)-1H-indazole-5-carboxamide;
rel-3-ethyl-1-methyl-N-{2-[(2R)-1-methylpyrrolidin-2-yl]imidazo[1,2-a]pyrazin-6-yl}-1H-indazole-6-carboxamide;
rel-3-cyclopropyl-1-methyl-N-{2-[(2R)-1-methylpyrrolidin-2-yl]imidazo[1,2-a]pyrazin-6-yl}-1H-indazole-6-carboxamide;
N-[2-(1,2-dimethylpyrrolidin-2-yl)imidazo[1,2-a]pyridin-6-yl]-1-methyl-1H-indazole-5-carboxamide;
rel-2-fluoro-4-(2-methyl-1,3-thiazol-5-yl)-N-{ 2-[(2R)-1-methylpyrrolidin-2-yl]imidazo[1,2-a]pyridin-6-yl}benzamide;
rel-1-methyl-N-{2-[(2R)-1-methylpiperidin-2-yl]imidazo[1,2-a]pyridin-6-yl1,2-a]pyridin-6-yl}-1H-indazole-5-carboxamide;
rel-1-methyl-N-{2-[(2R)-1-methylpiperidin-2-yl]imidazo[1,2-a]pyridin-6-yl1,2-a]pyridin-6-yl}-1H-indazole-5-carboxamide;
1-methyl-N-{2-[(2S)-1-methyl-octahydro-1H-indol-2-yl]imidazo[1,2-a]pyridin-6-yl}-1H-indazole-5-carboxamide;
1-methyl-N-{2-[(2R)-1-methyl-octahydro-1H-indol-2-yl]imidazo[1,2-a]pyridin-6-yl}-1H-indazole-5-carboxamide;
rel-2-fluoro-N-{2-[(2R)-1-methylpyrrolidin-2-yl]imidazo[1,2-a]pyridin-6-yl}-5-(pyrazin-2-yl)benzamide;
rel-1,3-dimethyl-N-{2-[(2R)-1-methylpyrrolidin-2-yl]imidazo[1,2-a]pyrazin-6-yl}-1H-indazole-6-carboxamide;
rel-1,3 -dimethyl-N- { 2-[(2R)-1-methylpyrrolidin-2-yl]imidazo[1,2-a]pyrazin-6-yl}-1H-indazole-6-carboxamide;
rel-1,3 -dimethyl-N- { 2-[(2R)-1-methylpyrrolidin-2-yl]imidazo[1,2-a]pyrazin-6-yl}-1H-indazole-6-carboxamide;
rel-1,3 -dimethyl-N- { 2-[(2R)-1-methylpyrrolidin-2-yl]imidazo[1,2-a]pyrazin-6-yl}-1H-indazole-6-carboxamide;
N-[2-(4-methoxy-1-methylpyrrolidin-2-yl)imidazo[1,2-a]pyridin-6-yl]-1-methyl-1H-indazole-5-carboxamide;
rel-2-fluoro-5-(2-methyl-1,3-thiazol-5-yl)-N-{2-[(2R)-1-methylpyrrolidin-2-yl]imidazo[1,2-a]pyridin-6-yl}benzamide;
N-[2-(1,4-dimethylpyrrolidin-2-yl)imidazo[1,2-a]pyridin-6-yl]-1-methyl-1H-indazole-5-carboxamide;
rel-2-fluoro-4-(4-methyl-1,3-thiazol-5-yl)-N-{2-[(2R)-1-methylpyrrolidin-2-yl]imidazo[1,2-a]pyridin-6-yl}benzamide;
N-[2-(1,5-dimethylpyrrolidin-2-yl)imidazo[1,2-a]pyridin-6-yl]-1-methyl-1H-indazole-5-carboxamide;
1-methyl-N-[2-(1-methylpyrrolidin-2-yl)imidazo[1,2-a]pyrazin-6-yl]-1H-indazole-5-carboxamide;
rel-2-fluoro-4-(1-methyl-1H-pyrazol-4-yl)-N-{2-[(2R)-1-methylpyrrolidin-2-yl]imidazo[1,2-a]pyridin-6-yl}benzamide;
rel-2-fluoro-N-{2-[(2R)-1-methylpyrrolidin-2-yl]imidazo[1,2-a]pyridin-6-yl}-4-(pyrimidin-2-yl)benzamide;
rel-2-fluoro-N-{2-[(2R)-1-methylpyrrolidin-2-yl]imidazo[1,2-a]pyridin-6-yl}-4-(pyrazin-2-yl)benzamide;
1,3-dimethyl-N-[2-(1-methylpyrrolidin-2-yl)imidazo[1,2-a]pyridin-6-yl]-1H-thieno[2,3-c]pyrazole-5-carboxamide;
6-methoxy-N-[2-(1-methylpyrrolidin-2-yl)imidazo[1,2-a]pyridin-6-yl]-1,3-benzothiazole-2-carboxamide;
4-(2-methyl-1,3-thiazol-4-yl)-N-[2-(1-methylpyrrolidin-2-yl)imidazo[1,2-a]pyridin-6-yl]benzamide;
4-chloro-1,3-dimethyl-N-[2-(1-methylpyrrolidin-2-yl)imidazo[1,2-a]pyridin-6-yl]-1H-pyrazolo[3,4-b]pyridine-5-carboxamide;
1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-6-(1H-pyrazol-1-yl)pyridine-3-carboxamide;
N-[2-(1-methylpyrrolidin-2-yl)imidazo[1,2-a]pyridin-6-yl]-4-(1H-1,2,4-triazol-1-yl)benzamide;
2,3-dimethyl-N-[2-(1-methylpyrrolidin-2-yl)imidazo[1,2-a]pyridin-6-yl]quinoxaline-6-carboxamide;
N-[2-(1-methylpyrrolidin-2-yl)imidazo[1,2-a]pyridin-6-yl]-1,5-naphthyridine-2-carboxamide;
7-chloro-N-[2-(1-methylpyrrolidin-2-yl)imidazo[1,2-a]pyridin-6-yl]quinoline-3-carboxamide;
N-[8-methoxy-2-(1-methylpyrrolidin-2-yl)imidazo[1,2-a]pyridin-6-yl]-1,3-dimethyl-1H-indazole-6-carboxamide;
N-{2-[1-(2-methoxyethyl)pyrrolidin-2-yl]imidazo[1,2-a]pyridin-6-yl}-1,3-dimethyl-1H-indazole-6-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;
5-fluoro-1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-[1,2,4]triazolo[4,3-a]pyridine-7-carboxamide;
4-chloro-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-3H-imidazo[4,5-b]pyridine-5-carboxamide;
N-[2-(1-methylpyrrolidin-2-yl)imidazo[1,2-a]pyridin-6-yl]imidazo[1,5-a]pyridine-7-carboxamide;
N-[2-(1-methylpyrrolidin-2-yl)imidazo[1,2-a]pyridin-6-yl]-4-(1,2,4-oxadiazol-3-yl)benzamide;
3-ethyl-N-[2-(1-methylpyrrolidin-2-yl)imidazo[1,2-a]pyridin-6-yl]-[1,2,4]triazolo[4,3-alpyridine-7-carboxamide;
1,3-dimethyl-N-[2-(1-methylpyrrolidin-2-yl)imidazo[1,2-a]pyridin-6-yl]-1H-pyrazolo[3,4-b]pyridine-6-carboxamide;
N-[2-(1-methylpyrrolidin-2-yl)imidazo[1,2-a]pyridin-6-yl]imidazo[1,2-a]pyrazine-2-carboxamide;
N-[2-(1-methylpyrrolidin-2-yl)imidazo[1,2-a]pyridin-6-yl]furo[2,3-c]pyridine-2-carboxamide;
1-methyl-N-[2-(4-methylmorpholin-3-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-[1,2]oxazolo[5,4-b]pyridine-6-carboxamide;
N-[7-fluoro-2-(1-methylpyrrolidin-2-yl)imidazo[1,2-a]pyridin-6-yl]-1,3-dimethyl-1H-indazole-6-carboxamide;
4-fluoro-N-[2-(1-methylpyrrolidin-2-yl)imidazo[1,2-a]pyridin-6-yl]-1H-indole-5-carboxamide;
3-(2-methoxyethyl)-1-methyl-N-[2-(1-methylpyrrolidin-2-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-1,2-benzoxazole-6-carboxamide;
N-[2-(1-methylpyrrolidin-2-yl)imidazo[1,2-a]pyridin-6-yl]quinazoline-7-carboxamide; 2-(4-methoxyphenyl)-N-[2-(1-methylpyrrolidin-2-yl)imidazo[1,2-a]pyridin-6-yl]-1,3-thiazole-4-carboxamide;
N-[2-(1-methylpyrrolidin-2-yl)imidazo[1,2-a]pyridin-6-yl]-1,3-benzoxazole-6-carboxamide;
N-[2-(1-methylpyrrolidin-2-yl)imidazo[1,2-a]pyridin-6-yl]-2-phenyl-1,3-oxazole-5-carboxamide;
1-cyclohexyl-N-[2-(1-methylpyrrolidin-2-yl)imidazo[1,2-a]pyridin-6-yl]-1H-1,2,3-benzotriazole-5-carboxamide;
5-methyl-N-[2-(1-methylpyrrolidin-2-yl)imidazo[1,2-a]pyridin-6-yl]-4-oxo-1H,4H-thieno[2,3-d]pyrimidine-6-carboxamide;
1-methyl-N-[2-(1-methylpyrrolidin-2-yl)imidazo[1,2-a]pyridin-6-yl]-3-(pyrrolidin-3-yl)-1H-indazole-5-carboxamide;
N-[2-(1-methylpyrrolidin-2-yl)imidazo[1,2-a]pyridin-6-yl]-3-(1H-pyrazol-1-yl)benzamide;
3-cyclopentyl-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-4-[(1H-pyrazol-1-yl)methyl]benzamide;
N-[2-(1-methylpyrrolidin-2-yl)imidazo[1,2-a]pyridin-6-yl]-1,3-benzothiazole-5-carboxamide;
N-[2-(1-methylpyrrolidin-2-yl)imidazo[1,2-a]pyridin-6-yl]-4-(1H-pyrazol-1-yl)benzamide;
6-methoxy-N-[2-(1-methylpyrrolidin-2-yl)imidazo[1,2-a]pyridin-6-yl]-1H-indole-3-carboxamide;
3-ethyl-1-methyl-N-[2-(1-methylpyrrolidin-2-yl)imidazo[1,2-a]pyridin-6-yl]-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
N-{2-[(rel-2R,4S)-4-methoxy-1-methylpyrrolidin-2-yl]imidazo[1,2-a]pyrazin-6-yl}-1-methylindazole-5-carboxamide;
N-{2-[(rel-2S,4S)-4-methoxy-1-methylpyrrolidin-2-yl]imidazo[1,2-a]pyrazin-6-yl}-1-methylindazole-5-carboxamide;
rac-N-[2-(4-tert-butyl-1-methylpyrrolidin-2-yl)imidazo[1,2-a]pyrazin-6-yl]-1,3-dimethylindazole-6-carboxamide;
N-[8-cyclopropyl-2-[(2R)-1-methylpyrrolidin-2-yl]imidazo[1,2-a]pyrazin-6-yl]-1-methyl-indazole-5-carboxamide;
N-[8-cyclopropyl-2-[(2R)-1-methylpyrrolidin-2-yl]imidazo[1,2-a]pyrazin-6-yl]-5-fluoro-1,3-dimethyl-indazole-6-carboxamide;
6-fluoro-1-methyl-N-[2-[(2R)-1-methylpyrrolidin-2-yl]imidazo[1,2-a]pyrazin-6-yl]indazole-5-carboxamide;
1-methyl-N-[2-[(6R)-5-methyl-5-azaspiro[2.4]heptan-6-yl]imidazo[1,2-a]pyrazin-6-yl]indazole-5-carboxamide;
N-{2-[(rel-2R)-1-ethylpyrrolidin-2-yl]imidazo[1,2-a]pyrazin-6-yl}-1,3-dimethylindazole-6-carboxamide;
N-{2-[(rel-2S)-1-ethylpyrrolidin-2-yl]imidazo[1,2-a]pyrazin-6-yl}-1,3-dimethylindazole-6-carboxamide;
N-(2-((2R, rel-4S)-1,4-dimethylpyrrolidin-2-yl)imidazo[1,2-a]pyrazin-6-yl)-1-methyl-1H-indazole-5-carboxamide;
N-(2-((2R,rel-4R)-1,4-dimethylpyrrolidin-2-yl)imidazo[1,2-a]pyrazin-6-yl)-1-methyl-1H-indazole-5-carboxamide;
N-{2-[(rel-2R,4R)-4-methoxy-1-methylpyrrolidin-2-yl]imidazo[1,2-a]pyrazin-6-yl}-1-methylindazole-5-carboxamide;
N-{2-[(rel-2S,4R)-4-methoxy-1-methylpyrrolidin-2-yl]imidazo[1,2-a]pyrazin-6-yl}-1-methylindazole-5-carboxamide; and
N-(2-((2R,4R)-4-fluoro-1-methylpyrrolidin-2-yl)imidazo[1,2-a]pyrazin-6-yl)-1-methyl-1H-indazole-5-carboxamide.

46. A compound according to any one of aspects 1 to 24, wherein M is of formula (IV):

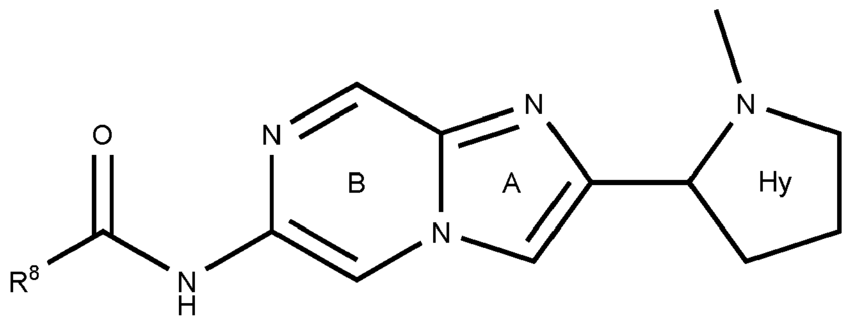

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;
wherein M is bonded to LINK via a C or N atom within group $R^8$ such that a hydrogen atom on the C or N atom within group $R^8$ is replaced with LINK.

47. A compound according to aspect 46, wherein $R^8$ is a group selected from phenyl and indazolyl.

48. A compound according to aspect 46 or 47, wherein the group $R^8$ is unsubstituted or substituted by one, two or three substituents independently selected from fluoro and methyl.

49. A compound according to any one of aspects 46 to 48, wherein $R^8$ is selected from one of the following structures:

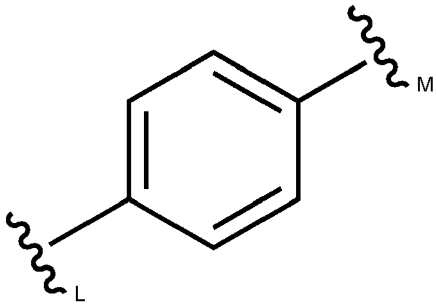

A

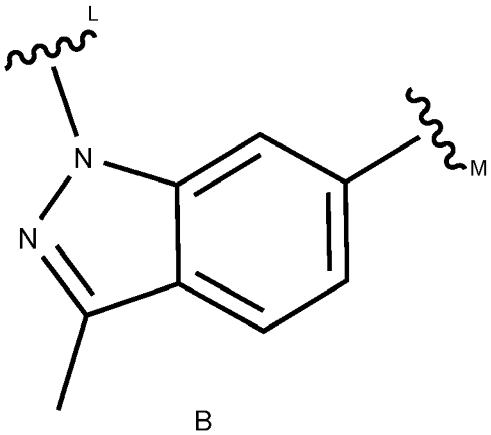

B

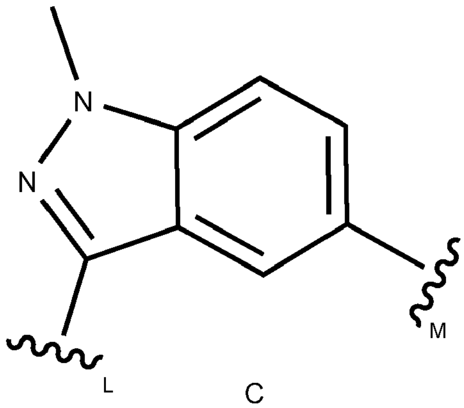

C

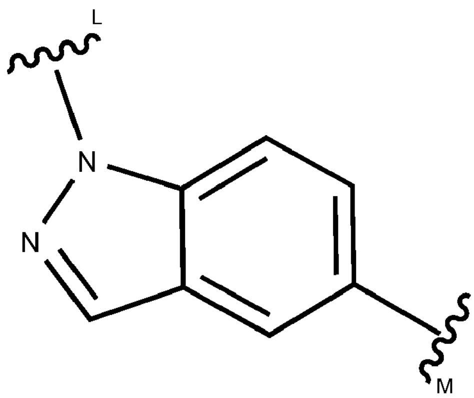

D

wherein:

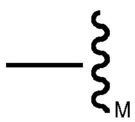

represents the point of attachment of $R^8$ to the rest of moiety M;

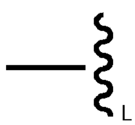

represents the point of attachment of $R^8$ to LINK; and
the group $R^8$ is further unsubstituted or substituted by one or two substituents independently selected from fluorine and methyl.

50. A compound according to any one of aspects 1 to 24, wherein M is of formula (IVA):

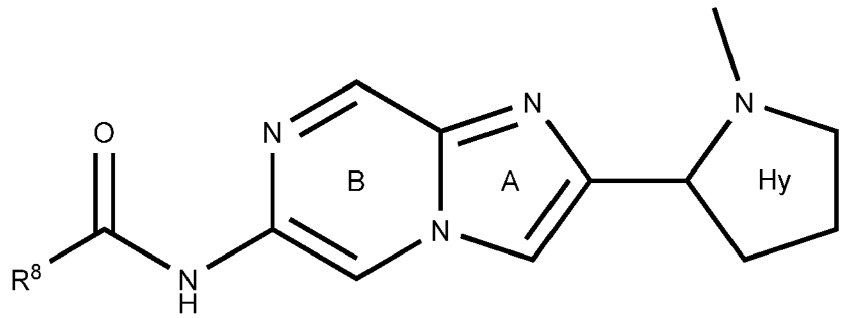

(IVA)

wherein $R^8$ is a bond to LINK.

51. A compound according to aspect 1, wherein (i) M is of formula (III):

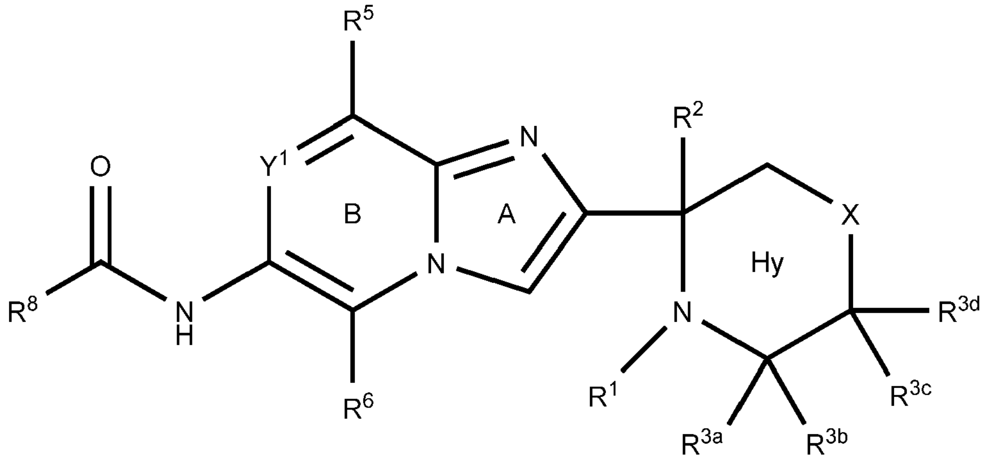

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
X is a bond, -N(Me)-, O or $-CH_2-$;
$Y^1$ is N or $-C(R^7)-$;
$R^5$, $R^6$ and $R^7$ are independently selected from H, halo $C_{1-4}$ alkoxy, and $C_{1-4}$ alkyl;
$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK; and (ii) LINK is (a) a single bond, or (b) a chemical linker group represented by formula (L):

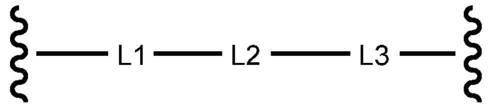

(L)

wherein:

LINK is attached to M via L1;
LINK is attached to U via L3;
when $R^8$ is (i) a bond to LINK, then L1 is a single bond and L3 is selected from a single bond, -N(R')-, -C(O)N(R')-, -O-, -N(R')C(O)-, -C(O)-, $-S(O_2)N(R')-$, $-N(R')S(O_2)-$, -($C_{1-6}$ alkylene)-, -($C_{2-6}$ alkenylene)-, a divalent 3- to 6-membered cycloalkyl ring and a divalent 4- to 7-membered heterocyclyl ring; and when $R^8$ is a group (ii), then L1 and
L3 are each independently selected from a single bond, -N(R')-, -C(O)N(R')-, -O-, -N(R')C(O)-, -C(O)-, $-S(O_2)N(R')-$, $-N(R')S(O_2)-$, -($C_{1-6}$ alkylene)-, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

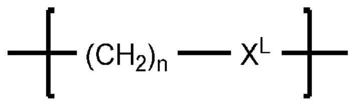

;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30;
each R' is independently selected from H and $C_{1-4}$ alkyl; and
(iii) U is:

(a) a CRBN E3 ubiquitin ligase binding moiety of formula (U1):

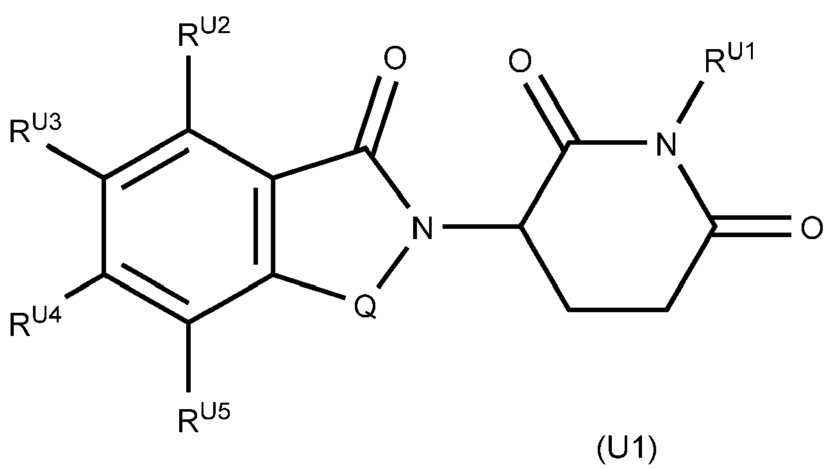

(U1)

wherein:

$R^{U1}$ is H;
Q is selected from $-CH(R^{U6})-$, $-N(R^{U6})-$, -O-, -C(O)-, $-NH-CH(R^{U6})-$, $-N=C(R^{U6})-$, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

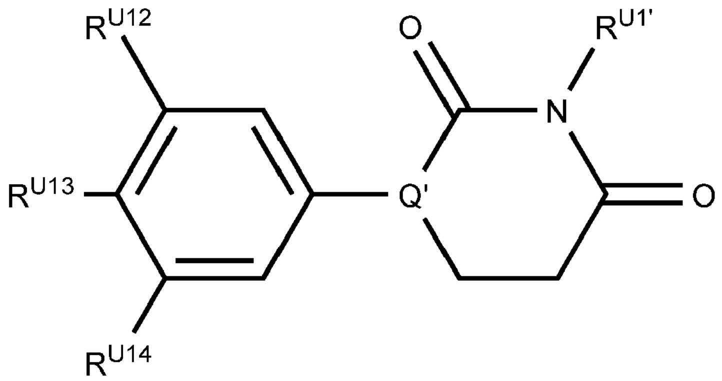

(U4)

wherein:

$R^{U1'}$ is H;
Q' is selected from CH and N;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

or (c) a VHL E3 ubiquitin ligase binding moiety of formula (U2):

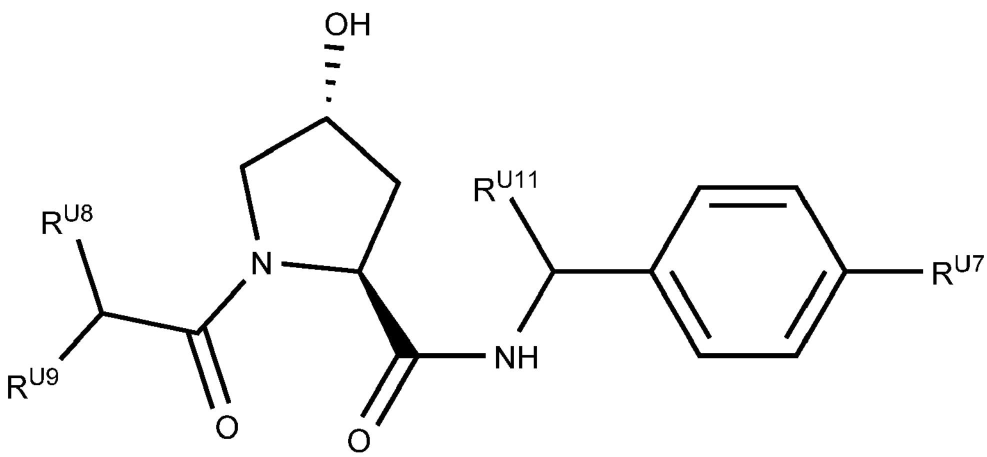

(U2)

wherein:

$R^{U7}$ is a group selected from phenyl, a 5- to 6-membered heteroaryl ring, a 5- to 6-membered

heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

52. A compound according to aspect 51, wherein M is of formula (IV):

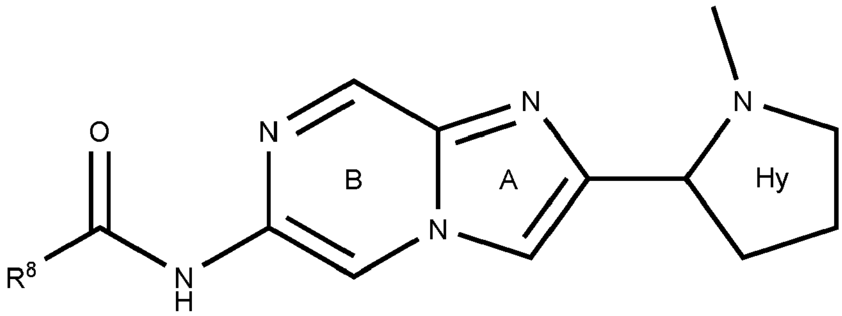

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;
wherein M is bonded to LINK via a C or N atom within group $R^8$ such that a hydrogen atom on the C or N atom within group $R^8$ is replaced with LINK.

53. A compound according to aspect 51 or 52, wherein $R^8$ is selected from one of the following structures:

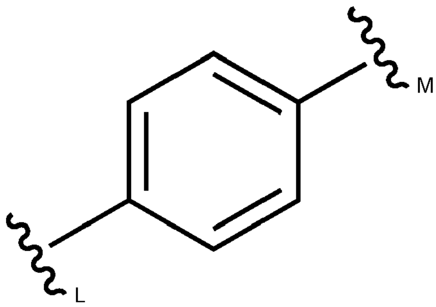

A

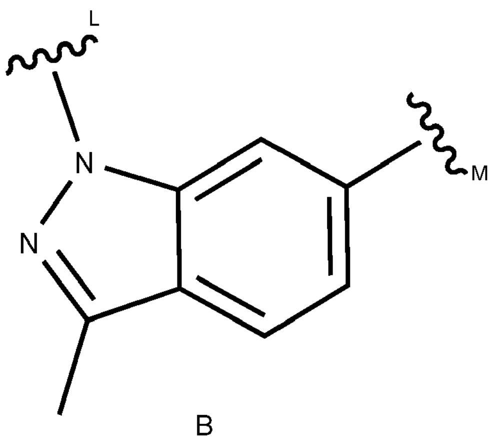

B

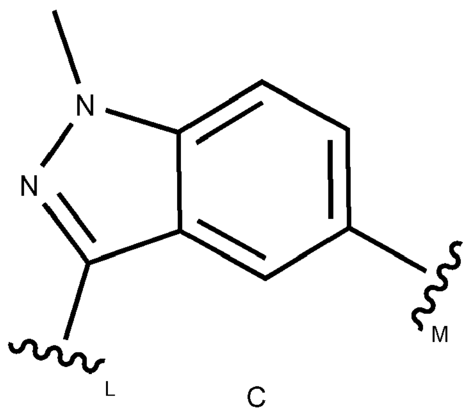

C

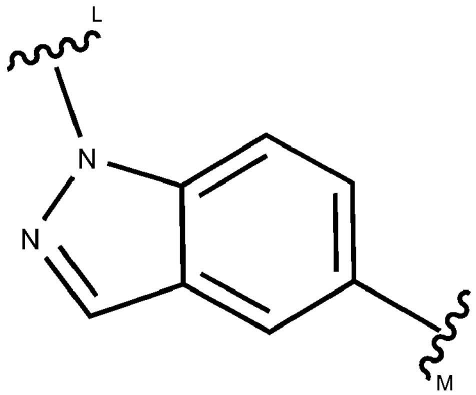

D

wherein:

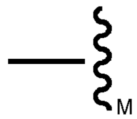

represents the point of attachment of $R^8$ to the rest of moiety M;

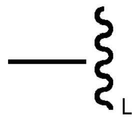

represents the point of attachment of $R^8$ to LINK; and
the group $R^8$ is further unsubstituted or substituted by one or two substituents independently selected from fluorine and methyl.

54. A compound according to aspect 51, wherein M is of formula (IV):

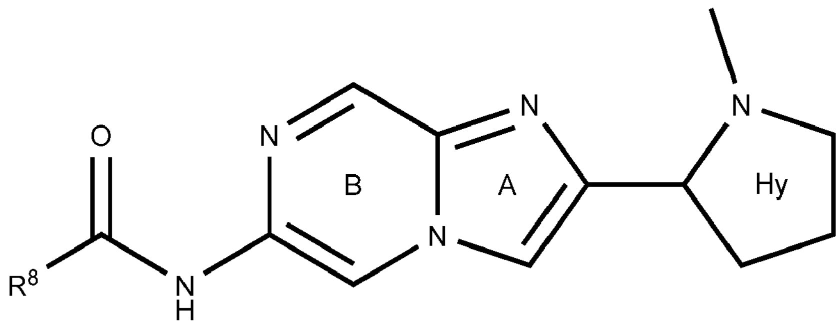

(IVA)

wherein $R^8$ is a bond to LINK.

55. A compound according to any one of aspects 51 to 54, wherein L2 is selected from $-(CH_2)_m-$, $-(CH_2CH_2O)_m-$, $-(OCH_2CH_2)_m-$, $-(CH_2CH_2S)_m-$, $-(SCH_2CH_2)_m-$, and a divalent 4- to 7-membered heterocyclyl ring.

56. A compound according to any one of aspects 51 to 55, wherein when $R^8$ is a bond to LINK, then L1 is a single bond and L3 is selected from a single bond, -N(R')-, -C(O)N(R')-, -N(R')C(O)-, -O-, and a divalent 4- to 7-membered heterocyclyl ring; and when $R^8$ is an aryl or heteroaryl ring, then L1 and L3 are each independently selected from a single bond, -N(R')-, -C(O)N(R')-, -N(R')C(O)-, -O-, and a divalent 4- to 7-membered heterocyclyl ring.

57. A compound according to aspect 1 which is selected from:

1-(8- { [2-(2,6-dioxopiperidin-3-yl)-1,3-dioxoisoindol-4-yl] amino }octyl)-3-methyl-N- { 2-[(rel-2R)-1-methylpyrrolidin-2-yl]imidazo[1,2-a]pyrazin-6-yl}indazole-6-carboxamide;
1-(8- { [2-(2,6-dioxopiperidin-3-yl)-1,3-dioxoisoindol-4-yl] amino }octyl)-3-methyl-N- { 2-[(rel-2S)-1-methylpyrrolidin-2-yl]imidazo[1,2-a]pyrazin-6-yl}indazole-6-carboxamide;(2S,4R)-1-[(2S)-3,3-dimethyl-2-(11-{[4-({2-[(rel-2R)-1-methylpyrrolidin-2-yl]imidazo[1,2-a]pyrazin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide;
(2S,4R)-1-[(2S)-3,3-dimethyl-2-(11-{[4-({2-[(rel-2S)-1-methylpyrrolidin-2-yl]imidazo[1,2-a]pyrazin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide; 7-{ [2-(2,6-dioxopiperidin-3-yl)-1,3-dioxoisoindol-4-yl] amino }-N-{ 2-[(2R)-1-methylpyrrolidin-2-yl]imidazo[1,2-a]pyrazin-6-yl}heptanamide;
and the pharmaceutically acceptable salts thereof.

58. A compound which is selected from:

N-[8-cyclopropyl-2-[(2R)-1-methylpyrrolidin-2-yl]imidazo[1,2-a]pyrazin-6-yl]-1-methyl-indazole-5-carboxamide;
N-[8-cyclopropyl-2-[(2R)-1-methylpyrrolidin-2-yl]imidazo[1,2-a]pyrazin-6-yl]-5-fluoro-1,3-dimethyl-indazole-6-carboxamide;
6-fluoro-1-methyl-N-[2-[(2R)-1-methylpyrrolidin-2-yl]imidazo[1,2-a]pyrazin-6-yl]indazole-5-carboxamide;
1-methyl-N-[2-[(6R)-5-methyl-5-azaspiro[2.4]heptan-6-yl]imidazo[1,2-a]pyrazin-6-yl]indazole-5-carboxamide;
N-{2-[(rel-2R)-1-ethylpyrrolidin-2-yl]imidazo[1,2-a]pyrazin-6-yl}-1,3-dimethylindazole-6-carboxamide;
N-{2-[(rel-2S)-1-ethylpyrrolidin-2-yl]imidazo[1,2-a]pyrazin-6-yl}-1,3-dimethylindazole-6-carboxamide;
N-(2-((2R, rel-4S)-1,4-dimethylpyrrolidin-2-yl)imidazo[1,2-a]pyrazin-6-yl)-1-methyl-1H-indazole-5-carboxamide;
N-(2-((2R,rel-4R)-1,4-dimethylpyrrolidin-2-yl)imidazo[1,2-a]pyrazin-6-yl)-1-methyl-1H-indazole-5-carboxamide;
N-{2-[(rel-2R,4R)-4-methoxy-1-methylpyrrolidin-2-yl]imidazo[1,2-a]pyrazin-6-yl}-1-methylindazole-5-carboxamide;
N-{2-[(rel-2S,4R)-4-methoxy-1-methylpyrrolidin-2-yl]imidazo[1,2-a]pyrazin-6-yl}-1-methylindazole-5-carboxamide;
N-(2-((2R,4R)-4-fluoro-1-methylpyrrolidin-2-yl)imidazo[1,2-a]pyrazin-6-yl)-1-methyl-1H-indazole-5-carboxamide;
and the pharmaceutically acceptable salts thereof.

59. A pharmaceutical composition which comprises a compound as defined in any one of aspects 1 to 58 and a pharmaceutically acceptable carrier or diluent.

60. A compound as defined in any one of aspects 1 to 58 for use in the treatment of the human or animal body by therapy.

61. A compound as defined in any one of aspects 1 to 58 for use in the treatment of cancer.

62. The compound for use according to aspect 61, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma or sarcoma.

63. The compound for use according to aspect 61, wherein the cancer is acute myeloid leukaemia (AML) or acute lymphoblastic leukaemia (ALL).

64. The compound for use according to aspect 63, wherein the cancer is mixed-lineage leukaemia (MLL) rearranged acute leukemia (AML and ALL).

65. The compound for use according to aspect 63, wherein the cancer is NPM1 mutant leukemia.

## Claims

1. A compound which is a Proteolysis Targeting Chimera (PROTAC) or a pharmaceutically acceptable salt thereof, wherein the PROTAC has the structure:

M-LINK-U

wherein U is an E3 ubiquitin ligase binding moiety, LINK is a moiety that covalently links M and U, and M is an MLLT1 and/or MLLT3 binder of formula (I):

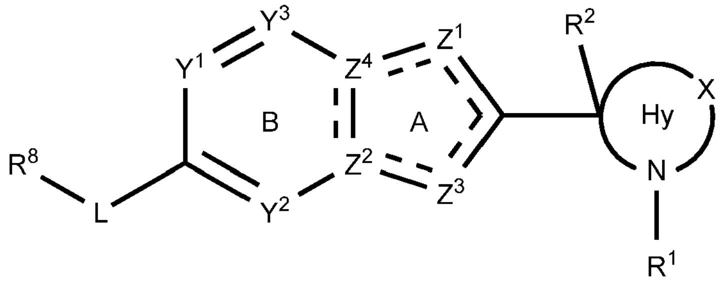

(I)

wherein:

one of $Z^2$ and $Z^4$ is N and the other is C, $Z^1$ is N or $-C(R^4)-$, $Z^3$ is $-C(R^4)-$, $Y^1$ is N or - $C(R^7)-$, $Y^2$ is N or $-C(R^6)-$, and $Y^3$ is N or $-C(R^5)-$;
Hy is a 4- to 7-membered heterocyclyl ring containing X and at least one N atom, wherein: ring Hy is linked to ring A via a C atom within ring Hy, said C atom also being linked to $R^2$; a N atom within ring Hy is substituted by $R^1$; X is a bond, $-N(R^{11})-$, O, S, $-S(O)_2-$, $-S(O)(NR^{11})-$, or $-C(R^{11})_2-$; and the rest of ring Hy is unsubstituted or substituted by one or two $R^3$;
L is -C(O)N(H)-, wherein either (i) the C atom of L is bonded to $R^8$, and the N atom of L is bonded to ring B; or (ii) the C atom of L is bonded to ring B, and the N atom of L is bonded to $R^8$;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$, $R^6$, and $R^7$ are independently selected from H, halo, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
$R^8$ is (i) a bond to LINK or (ii) a group selected from a 6- to 10-membered aryl ring, a 5- to 10-membered heteroaryl ring, a 5- to 10-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, $-(C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
each $R^{11}$ is independently selected from H, $C_{1-4}$ alkyl, and $C_{1-4}$ cycloalkyl;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

**2.** A compound according to claim 1, wherein LINK is (a) a single bond, or (b) a chemical linker group represented by formula (L):

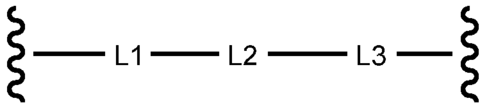

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-, -S($O_2$)N(R')-, -N(R')S($O_2$)-, -($C_{1-6}$ alkylene)-, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

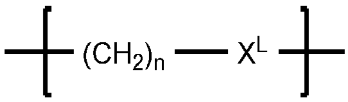
;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30; and
each R' is independently selected from H and $C_{1-4}$ alkyl.

**3.** A compound according to claim 1 or 2, wherein U is:

(a) a CRBN E3 ubiquitin ligase binding moiety of formula (U1):

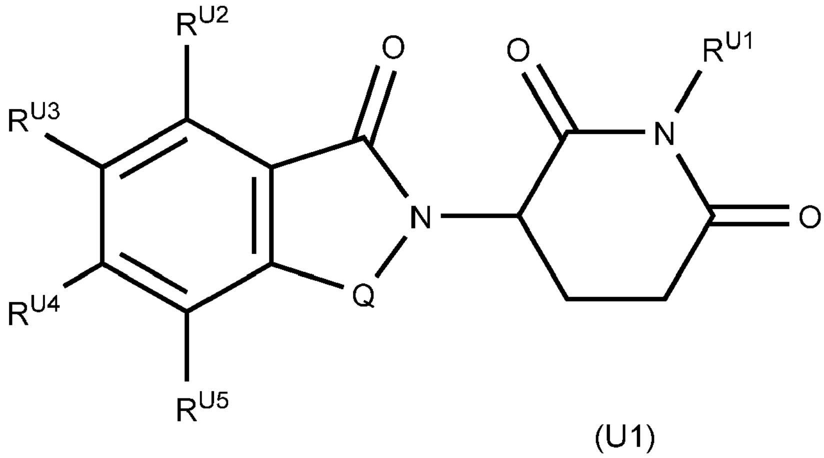

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN,

$CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

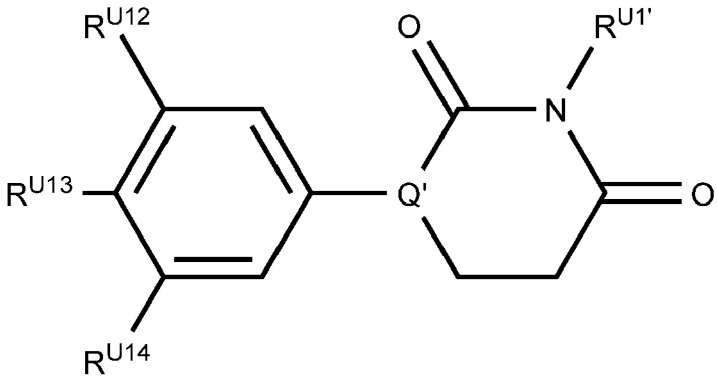

(U4)

wherein:

$R^{U1'}$ is H;
Q' is selected from CH and N;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

or (c) a VHL E3 ubiquitin ligase binding moiety of formula (U2):

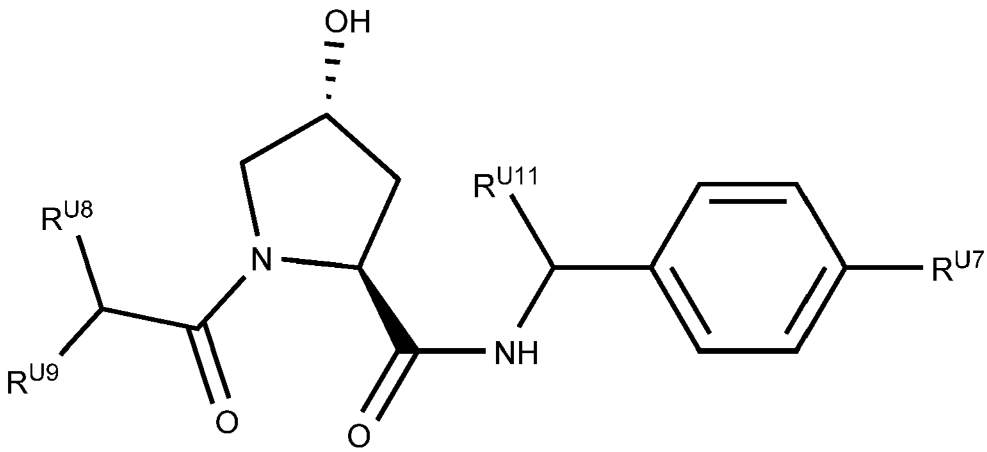

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

**4.** A compound according to any one of the preceding claims, wherein M is of formula (II):

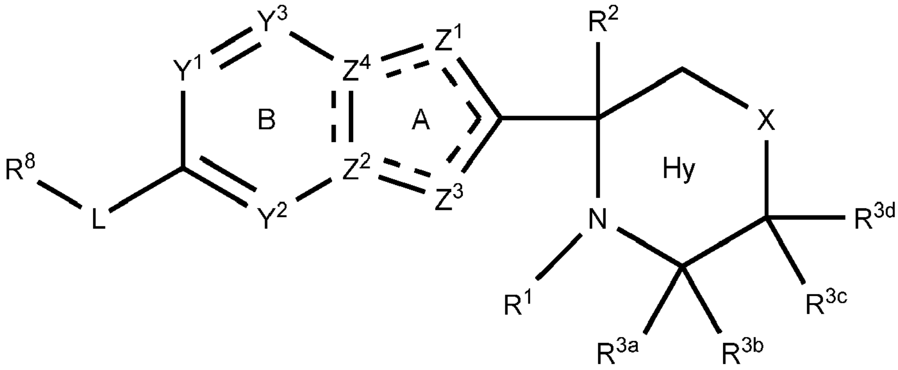

(II)

wherein:

$Z^1$, $Z^2$, $Z^3$, $Z^4$, $Y^1$, $Y^2$, $Y^3$, $R^1$, $R^2$, $R^8$, X and L are as defined in any one of the preceding claims;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, with the proviso that when X is $NR^{11}$, O, or S, then neither $R^{3c}$ nor $R^{3d}$ are halo, or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

**5.** A compound according to any one of claims 1 to 4, wherein M is of formula (III):

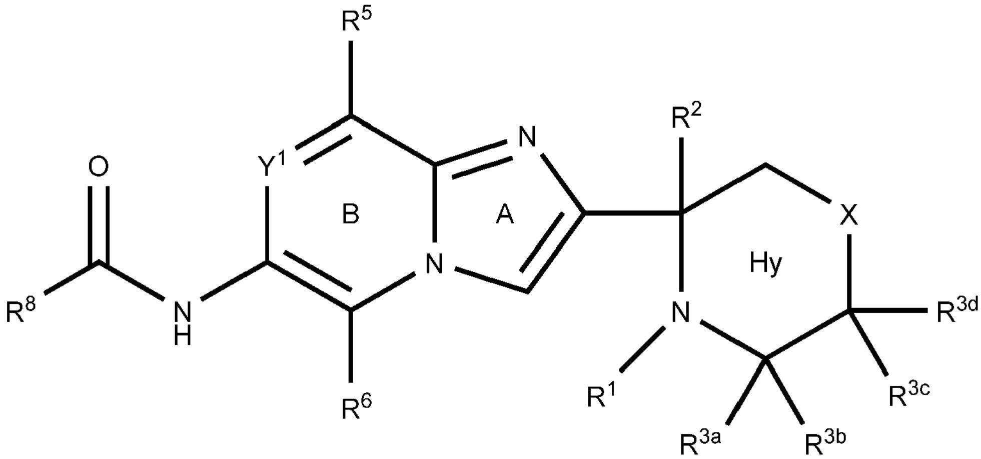

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and

$R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is O, then neither $R^{3c}$ nor $R^{3d}$ are halo, or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;
X is a bond, -N(Me)-, O or $-CH_2-$;
$Y^1$ is N or $-C(R^7)-$;
$R^5$, $R^6$ and $R^7$ are independently selected from H, halo $C_{1-4}$ alkoxy, and $C_{1-4}$ alkyl;
$R^8$ is (i) a bond to LINK or (ii) a group selected from a 6- to 10-membered aryl ring, a 5- to 10-membered heteroaryl ring, and a 5- to 10-membered heterocyclyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

**6.** A compound according to any one of claims 1 to 3, wherein M is a monovalent moiety of:

1-methyl-N-{ 8-methyl-2-[(2R)-1-methylpyrrolidin-2-yl]imidazo[1,2-a]pyrazin-6-yl}-1H-indazole-5-carboxamide;
1,3-dimethyl-N- { 8-methyl-2-[(2R)-1-methylpyrrolidin-2-yl]imidazo[1,2-a]pyrazin-6-yl}-1H-indazole-6-carboxamide;
rel-7-fluoro-N-{2-[(2R)-1-methylpyrrolidin-2-yl]imidazo[1,2-a]pyridin-6-yl}quinoline-6-carboxamide;
N-{2-[(2R,4S)-4-fluoro-1-methylpyrrolidin-2-yl]imidazo[1,2-a]pyrazin-6-yl}-1-methyl-1H-indazole-5-carboxamide;
1,3-dimethyl-N-{2-[(2R)-pyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
rel-1,3-dimethyl-N-{2-[(2R)-1,4,4-trimethylpyrrolidin-2-yl]imidazo[1,2-a]pyrazin-6-yl}-1H-indazole-6-carboxamide;
N-{2-[4-ethyl-1-methylpyrrolidin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-N-{2-[(2R)-1,4-dimethylpiperazin-2-yl]imidazo[1,2-a]pyrazin-6-yl}-1,3-dimethyl-1H-indazole-6-carboxamide;
rel-7-fluoro-N-{2-[(2R)-1-methylpyrrolidin-2-yl]imidazo[1,2-a]pyridin-6-yl}isoquinoline-6-carboxamide;
rel-5-fluoro-1,3-dimethyl-N- {2-[(2R)-1-methylpyrrolidin-2-yl]imidazo[1,2-a]pyrazin-6-yl}-1H-indazole-6-carboxamide;
1-methyl-N-(2-{2-methyl-2-azaspiro[4.4]nonan-3-yl}imidazo[1,2-a]pyridin-6-yl)-1H-indazole-5-carboxamide;
rel-3-ethyl-1-methyl-N-{2-[(2R)-1-methylpyrrolidin-2-yl]imidazo[1,2-a]pyrazin-6-yl}-1H-indazole-6-carboxamide;
rel-3-cyclopropyl-1-methyl-N-{2-[(2R)-1-methylpyrrolidin-2-yl]imidazo[1,2-a]pyrazin-6-yl}-1H-indazole-6-carboxamide;
N-[2-(1,2-dimethylpyrrolidin-2-yl)imidazo[1,2-a]pyridin-6-yl]-1-methyl-1H-indazole-5-carboxamide;
rel-2-fluoro-4-(2-methyl-1,3-thiazol-5-yl)-N-{ 2-[(2R)-1-methylpyrrolidin-2-yl]imidazo[1,2-a]pyridin-6-yl}benzamide;
rel-1-methyl-N-{2-[(2R)-1-methylpiperidin-2-yl]imidazo[1,2-a]pyridin-6-yl}-1H-indazole-5-carboxamide;
rel-1-methyl-N-{2-[(2R)-1-methylpiperidin-2-yl]imidazo[1,2-a]pyridin-6-yl}-1H-indazole-5-carboxamide;
1-methyl-N-{2-[(2S)-1-methyl-octahydro-1H-indol-2-yl]imidazo[1,2-a]pyridin-6-yl}-1H-indazole-5-carboxamide;
1-methyl-N-{2-[(2R)-1-methyl-octahydro-1H-indol-2-yl]imidazo[1,2-a]pyridin-6-yl} - 1H-indazole-5-carboxamide;
rel-2-fluoro-N-{2-[(2R)-1-methylpyrrolidin-2-yl]imidazo[1,2-a]pyridin-6-yl}-5-(pyrazin-2-yl)benzamide;
rel-1,3-dimethyl-N-{2-[(2R)-1-methylpyrrolidin-2-yl]imidazo[1,2-a]pyrazin-6-yl}-1H-indazole-6-carboxamide;

rel-1,3-dimethyl-N-{2-[(2R)-1-methylpyrrolidin-2-yl]imidazo[1,2-a]pyrazin-6-yl}-1H-indazole-6-carboxamide;
rel-1,3-dimethyl-N-{2-[(2R)-1-methylpyrrolidin-2-yl]imidazo[1,2-a]pyrazin-6-yl}-1H-indazole-6-carboxamide;
rel-1,3-dimethyl-N-{2-[(2R)-1-methylpyrrolidin-2-yl]imidazo[1,2-a]pyrazin-6-yl}-1H-indazole-6-carboxamide;
N-[2-(4-methoxy-1-methylpyrrolidin-2-yl)imidazo[1,2-a]pyridin-6-yl]-1-methyl-1H-indazole-5-carboxamide;
rel-2-fluoro-5-(2-methyl-1,3-thiazol-5-yl)-N-{2-[(2R)-1-methylpyrrolidin-2-yl]imidazo[1,2-a]pyridin-6-yl}benzamide;
N-[2-(1,4-dimethylpyrrolidin-2-yl)imidazo[1,2-a]pyridin-6-yl]-1-methyl-1H-indazole-5-carboxamide;
rel-2-fluoro-4-(4-methyl-1,3-thiazol-5-yl)-N-{2-[(2R)-1-methylpyrrolidin-2-yl]imidazo[1,2-a]pyridin-6-yl}benzamide;
N-[2-(1,5-dimethylpyrrolidin-2-yl)imidazo[1,2-a]pyridin-6-yl]-1-methyl-1H-indazole-5-carboxamide;
1-methyl-N-[2-(1-methylpyrrolidin-2-yl)imidazo[1,2-a]pyrazin-6-yl]-1H-indazole-5-carboxamide;
rel-2-fluoro-4-(1-methyl-1H-pyrazol-4-yl)-N-{2-[(2R)-1-methylpyrrolidin-2-yl]imidazo[1,2-a]pyridin-6-yl}benzamide;
rel-2-fluoro-N-{2-[(2R)-1-methylpyrrolidin-2-yl]imidazo[1,2-a]pyridin-6-yl}-4-(pyrimidin-2-yl)benzamide;
rel-2-fluoro-N-{2-[(2R)-1-methylpyrrolidin-2-yl]imidazo[1,2-a]pyridin-6-yl}-4-(pyrazin-2-yl)benzamide;
1,3-dimethyl-N-[2-(1-methylpyrrolidin-2-yl)imidazo[1,2-a]pyridin-6-yl]-1H-thieno[2,3-c]pyrazole-5-carboxamide;
6-methoxy-N-[2-(1-methylpyrrolidin-2-yl)imidazo[1,2-a]pyridin-6-yl]-1,3-benzothiazole-2-carboxamide;
4-(2-methyl-1,3-thiazol-4-yl)-N-[2-(1-methylpyrrolidin-2-yl)imidazo[1,2-a]pyridin-6-yl]benzamide;
4-chloro-1,3-dimethyl-N-[2-(1-methylpyrrolidin-2-yl)imidazo[1,2-a]pyridin-6-yl]-1H-pyrazolo[3,4-b]pyridine-5-carboxamide;
1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-6-(1H-pyrazol-1-yl)pyridine-3-carboxamide;
N-[2-(1-methylpyrrolidin-2-yl)imidazo[1,2-a]pyridin-6-yl]-4-(1H-1,2,4-triazol-1-yl)benzamide;
2,3-dimethyl-N-[2-(1-methylpyrrolidin-2-yl)imidazo[1,2-a]pyridin-6-yl]quinoxaline-6-carboxamide;
N-[2-(1-methylpyrrolidin-2-yl)imidazo[1,2-a]pyridin-6-yl]-1,5-naphthyridine-2-carboxamide;
7-chloro-N-[2-(1-methylpyrrolidin-2-yl)imidazo[1,2-a]pyridin-6-yl]quinoline-3-carboxamide;
N-[8-methoxy-2-(1-methylpyrrolidin-2-yl)imidazo[1,2-a]pyridin-6-yl]-1,3-dimethyl-1H-indazole-6-carboxamide;
N-{2-[1-(2-methoxyethyl)pyrrolidin-2-yl]imidazo[1,2-a]pyridin-6-yl}-1,3-dimethyl-1H-indazole-6-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-6-fluoro-N-{2-[(2R)-1-methylpyrrolidin-2-yl]imidazo[1,2-a]pyridin-6-yl}naphthalene-2-carboxamide;
rel-2-fluoro-N-{2-[(2R)-1-methylpyrrolidin-2-yl]imidazo[1,2-a]pyridin-6-yl}-4-(1,3-thiazol-5-yl)benzamide;
5-fluoro-1,3-dimethyl-N-[2-(1-methylpyrrolidin-2-yl)imidazo[1,2-a]pyridin-6-yl]-1H-indazole-6-carboxamide;
N-[2-(1-methylpyrrolidin-2-yl)imidazo[1,2-a]pyridin-6-yl]-[1,2,4]triazolo[4,3-a]pyridine-7-carboxamide;
4-chloro-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-3H-imidazo[4,5-b]pyridine-5-carboxamide;
N-[2-(1-methylpyrrolidin-2-yl)imidazo[1,2-a]pyridin-6-yl]imidazo[1,5-a]pyridine-7-carboxamide;
N-[2-(1-methylpyrrolidin-2-yl)imidazo[1,2-a]pyridin-6-yl]-4-(1,2,4-oxadiazol-3-yl)benzamide;
3-ethyl-N-[2-(1-methylpyrrolidin-2-yl)imidazo[1,2-a]pyridin-6-yl]-[1,2,4]triazolo[4,3-a]pyridine-7-carboxamide;
1,3-dimethyl-N-[2-(1-methylpyrrolidin-2-yl)imidazo[1,2-a]pyridin-6-yl]-1H-pyrazolo[3,4-b]pyridine-6-carboxamide;
N-[2-(1-methylpyrrolidin-2-yl)imidazo[1,2-a]pyridin-6-yl]imidazo[1,2-a]pyrazine-2-carboxamide;
N-[2-(1-methylpyrrolidin-2-yl)imidazo[1,2-a]pyridin-6-yl]furo[2,3-c]pyridine-2-carboxamide;
1-methyl-N-[2-(4-methylmorpholin-3-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-[1,2]oxazolo[5,4-b] pyridine-6-carboxamide;
N-[7-fluoro-2-(1-methylpyrrolidin-2-yl)imidazo[1,2-a]pyridin-6-yl]-1,3-dimethyl-1H-indazole-6-carboxamide;
4-fluoro-N-[2-(1-methylpyrrolidin-2-yl)imidazo[1,2-a]pyridin-6-yl]-1H-indole-5-carboxamide;
3-(2-methoxyethyl)-1-methyl-N-[2-(1-methylpyrrolidin-2-yl)imidazo[1,2-a]pyridin-6-yl]-1H-indazole-5-carboxamide;
3-methyl-N-[2-(1-methylpyrrolidin-2-yl)imidazo[1,2-a]pyridin-6-yl]-1,2-benzoxazole-6-carboxamide;
N-[2-(1-methylpyrrolidin-2-yl)imidazo[1,2-a]pyridin-6-yl]quinazoline-7-carboxamide; 2-(4-methoxyphenyl)-N-[2-(1-methylpyrrolidin-2-yl)imidazo[1,2-a]pyridin-6-yl]-1,3-thiazole-4-carboxamide;
N-[2-(1-methylpyrrolidin-2-yl)imidazo[1,2-a]pyriin-6-yl]-1,3-benzoxazole-6-carboxamide;
N-[2-(1-methylpyrrolidin-2-yl)imidazo[1,2-a]pyridin-6-yl]-2-phenyl-1,3-oxazole-5-carboxamide;
1-cyclohexyl-N-[2-(1-methylpyrrolidin-2-yl)imidazo[1,2-a]pyridin-6-yl]-1H-1,2,3-benzotriazole-5-carboxamide;

5-methyl-N-[2-(1-methylpyrrolidin-2-yl)imidazo[1,2-a]pyridin-6-yl]-4-oxo-1H,4H-thieno[2,3-d]pyrimidine-6-carboxamide;
1-methyl-N-[2-(1-methylpyrrolidin-2-yl)imidazo[1,2-a]pyridin-6-yl]-3-(pyrrolidin-3-yl)-1H-indazole-5-carboxamide;
N-[2-(1-methylpyrrolidin-2-yl)imidazo[1,2-a]pyridin-6-yl]-3-(1H-pyrazol-1-yl)benzamide;
3-cyclopentyl-1-methyl-N-[2-(1-methylpyrrolidin-2-yl)imidazo[1,2-a]pyridin-6-yl]-1H-indazole-5-carboxamide;
N-[2-(1-methylpyrrolidin-2-yl)imidazo[1,2-a]pyridin-6-yl]-4-[(1H-pyrazol-1-yl)methyl] benzamide;
N-[2-(1-methylpyrrolidin-2-yl)imidazo[1,2-a]pyridin-6-yl]-1,3-benzothiazole-5-carboxamide;
N-[2-(1-methylpyrrolidin-2-yl)imidazo[1,2-a]pyridin-6-yl]-4-(1H-pyrazol-1-yl)benzamide;
6-methoxy-N-[2-(1-methylpyrrolidin-2-yl)imidazo[1,2-a]pyridin-6-yl]-1H-indole-3-carboxamide;
3-ethyl-1-methyl-N-[2-(1-methylpyrrolidin-2-yl)imidazo[1,2-a]pyridin-6-yl]-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
rel-1-methyl-N-{2-[(2R)-1-methylpyrrolidin-2-yl]imidazo[1,2-a]pyridin-6-yl1,2-a]pyridin-6-yl}-1H-indazole-5-carboxamide;
N-{2-[(rel-2R,4S)-4-methoxy-1-methylpyrrolidin-2-yl]imidazo[1,2-a]pyrazin-6-yl}-1-methylindazole-5-carboxamide;
N-{2-[(rel-2S,4S)-4-methoxy-1-methylpyrrolidin-2-yl]imidazo[1,2-a]pyrazin-6-yl}-1-methylindazole-5-carboxamide;
rac-N-[2-(4-tert-butyl-1-methylpyrrolidin-2-yl)imidazo[1,2-a]pyrazin-6-yl]-1,3-dimethylindazole-6-carboxamide;
N-[8-cyclopropyl-2-[(2R)-1-methylpyrrolidin-2-yl]imidazo[1,2-a]pyrazin-6-yl]-1-methyl-indazole-5-carboxamide;
N-[8-cyclopropyl-2-[(2R)-1-methylpyrrolidin-2-yl]imidazo[1,2-a]pyrazin-6-yl]-5-fluoro-1,3-dimethyl-indazole-6-carboxamide;
6-fluoro-1-methyl-N-[2-[(2R)-1-methylpyrrolidin-2-yl]imidazo[1,2-a]pyrazin-6-yl]indazole-5-carboxamide;
1-methyl-N-[2-[(6R)-5-methyl-5-azaspiro[2.4]heptan-6-yl]imidazo[1,2-a]pyrazin-6-yl]indazole-5-carboxamide;
N-{2-[(rel-2R)-1-ethylpyrrolidin-2-yl]imidazo[1,2-a]pyrazin-6-yl}-1,3-dimethylindazole-6-carboxamide;
N-{2-[(rel-2S)-1-ethylpyrrolidin-2-yl]imidazo[1,2-a]pyrazin-6-yl}-1,3-dimethylindazole-6-carboxamide;
N-(2-((2R, rel-4S)-1,4-dimethylpyrrolidin-2-yl)imidazo[1,2-a]pyrazin-6-yl)-1-methyl-1H-indazole-5-carboxamide;
N-(2-((2R,rel-4R)-1,4-dimethylpyrrolidin-2-yl)imidazo[1,2-a]pyrazin-6-yl)-1-methyl-1H-indazole-5-carboxamide;
N-{2-[(rel-2R,4R)-4-methoxy-1-methylpyrrolidin-2-yl]imidazo[1,2-a]pyrazin-6-yl}-1-methylindazole-5-carboxamide;
N-{2-[(rel-2S,4R)-4-methoxy-1-methylpyrrolidin-2-yl]imidazo[1,2-a]pyrazin-6-yl}-1-methylindazole-5-carboxamide; and
N-(2-((2R,4R)-4-fluoro-1-methylpyrrolidin-2-yl)imidazo[1,2-a]pyrazin-6-yl)-1-methyl-1H-indazole-5-carboxamide.

**7.** A compound according to any one of claims 1 to 3, wherein M is of formula (IV):

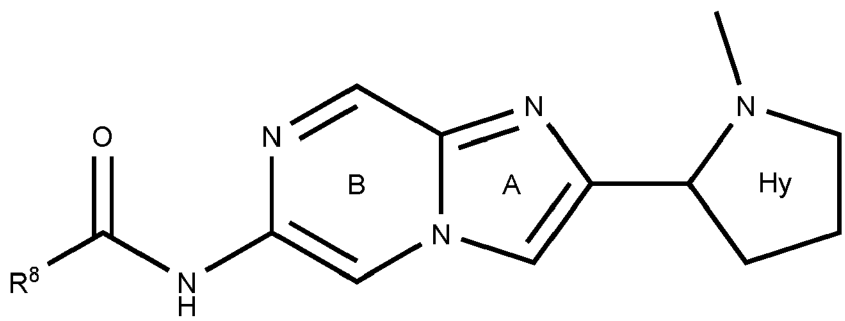

(IV)

wherein $R^8$ is a group selected from a 6- to 10-membered aryl ring and a 5- to 10-membered heteroaryl ring, the group $R^8$ being unsubstituted or substituted by one, two or three substituents independently selected from halo and $C_{1-4}$ alkyl;
wherein M is bonded to LINK via a C or N atom within group $R^8$ such that a hydrogen atom on the C or N atom within group $R^8$ is replaced with LINK.

**8.** A compound according to claim 7, wherein $R^8$ is selected from one of the following structures:

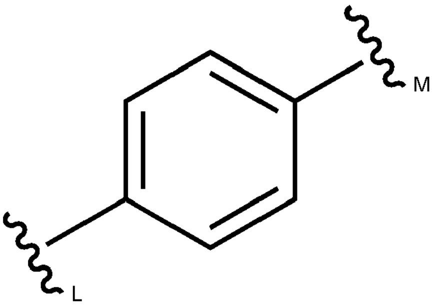

A

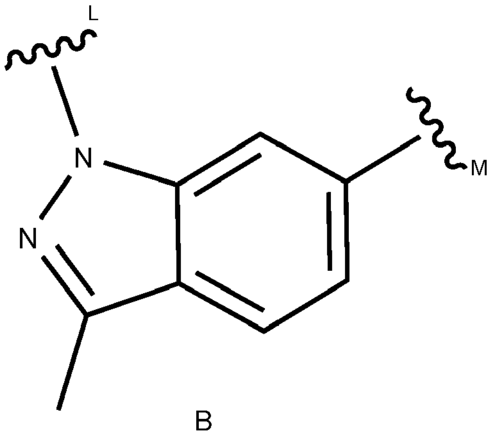

B

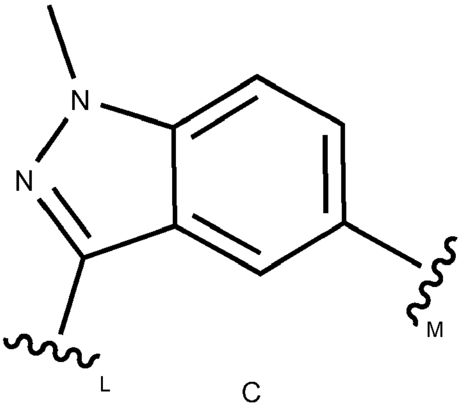

C

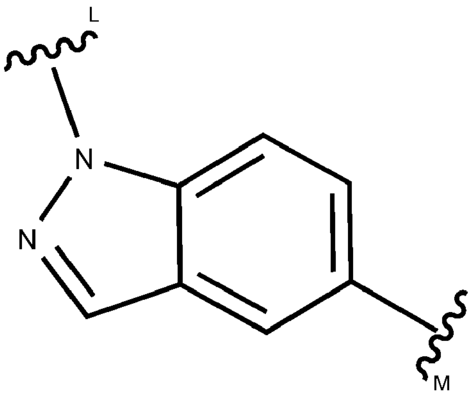

D

wherein:

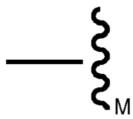

represents the point of attachment of $R^8$ to the rest of moiety M;

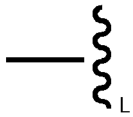

represents the point of attachment of $R^8$ to LINK; and

the group $R^8$ is further unsubstituted or substituted by one or two substituents independently selected from fluorine and methyl.

**9.** A compound according to any one of claims 1 to 3, wherein M is of formula (IVA):

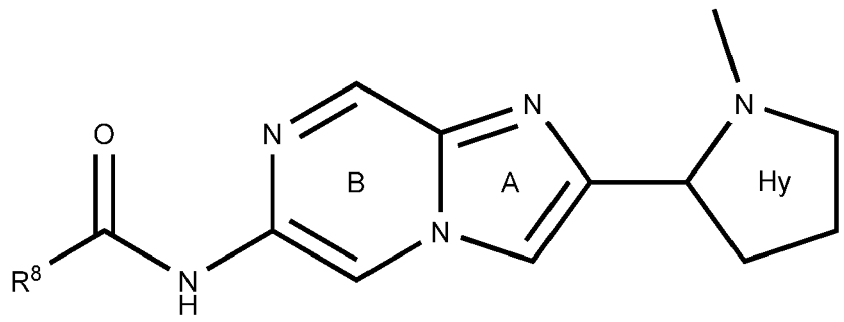

(IVA)

wherein $R^8$ is a bond to LINK.

**10.** A compound according to claim 1 which is selected from:

1-(8-{[2-(2,6-dioxopiperidin-3-yl)-1,3-dioxoisoindol-4-yl]amino}octyl)-3-methyl-N-{2-[(rel-2R)-1-methylpyrrolidin-2-yl]imidazo[1,2-a]pyrazin-6-yl}indazole-6-carboxamide;
1-(8-{[2-(2,6-dioxopiperidin-3-yl)-1,3-dioxoisoindol-4-yl]amino}octyl)-3-methyl-N-{2-[(rel-2S)-1-methylpyrrolidin-2-yl]imidazo[1,2-a]pyrazin-6-yl}indazole-6-carboxamide;
(2S,4R)-1-[(2S)-3,3-dimethyl-2-(11-{[4-({2-[(rel-2R)-1-methylpyrrolidin-2-yl]imidazo[1,2-a]pyrazin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide;
(2S,4R)-1-[(2S)-3,3-dimethyl-2-(11-{[4-({2-[(rel-2S)-1-methylpyrrolidin-2-yl]imidazo[1,2-a]pyrazin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
7-{[2-(2,6-dioxopiperidin-3-yl)-1,3-dioxoisoindol-4-yl]amino}-N-{2-[(2R)-1-methylpyrrolidin-2-yl]imidazo[1,2-a]pyrazin-6-yl}heptanamide;
and the pharmaceutically acceptable salts thereof.

**11.** A pharmaceutical composition which comprises a compound as defined in any one of claims 1 to 10 and a pharmaceutically acceptable carrier or diluent.

**12.** A compound as defined in any one of claims 1 to 10 for use in the treatment of the human or animal body by therapy.

**13.** A compound as defined in any one of claims 1 to 10 for use in the treatment of cancer.

**14.** The compound for use according to claim 13, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma or sarcoma.

**15.** The compound for use according to claim 13, wherein the cancer is acute myeloid leukaemia (AML) or acute lymphoblastic leukaemia (ALL), for instance wherein the cancer is mixed-lineage leukaemia (MLL) rearranged acute leukemia (AML and ALL) or NPM1 mutant leukemia.

FIGURE 1

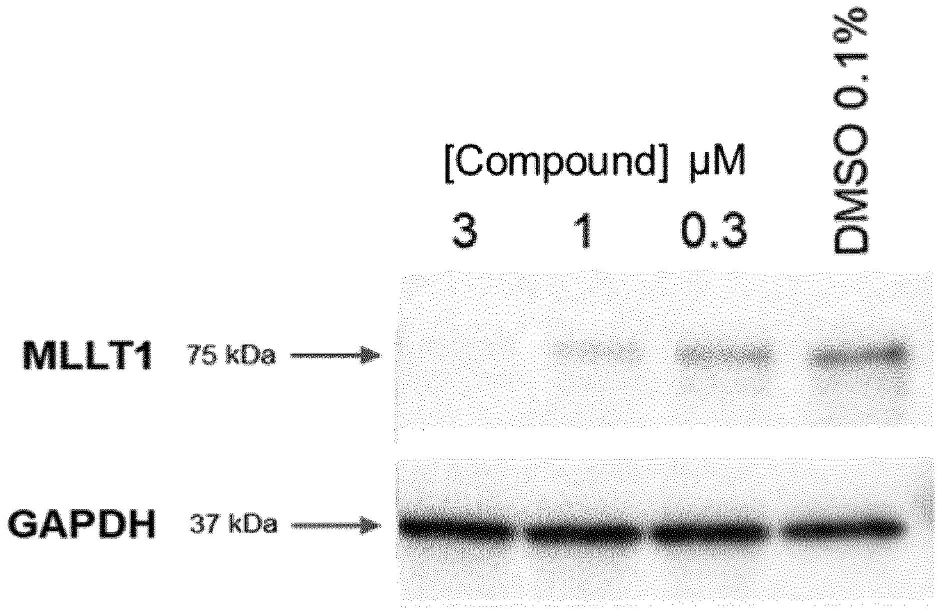

Europäisches Patentamt
European Patent Office
Office européen des brevets

# EUROPEAN SEARCH REPORT

Application Number
EP 23 16 1182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LI XIN ET AL: "A proteolysis-targeting chimera molecule selectively degrades ENL and inhibits malignant gene expression and tumor growth", JOURNAL OF HEMATOLOGY & ONCOLOGY, vol. 15, no. 1, 8 April 2022 (2022-04-08), XP93046397, DOI: 10.1186/s13045-022-01258-8 Retrieved from the Internet: URL:https://link.springer.com/article/10.1186/s13045-022-01258-8/fulltext.html> * abstract * * page 2; figure 1 * * page 3; figure 2 * * pages 4-9; figures 3-7 * ----- | 1-15 | INV. C07D487/04 A61P35/00 A61P35/02 A61K31/498 |
| Y | GARNAR-WORTZEL LEOPOLD ET AL: "Chemical Inhibition of ENL/AF9 YEATS Domains in Acute Leukemia", ACS CENTRAL SCIENCE, vol. 7, no. 5, 30 April 2021 (2021-04-30), pages 815-830, XP055935704, ISSN: 2374-7943, DOI: 10.1021/acscentsci.0c01550 Retrieved from the Internet: URL:https://pubs.acs.org/doi/pdf/10.1021/acscentsci.0c01550> * abstract * * page 816; figure 1 * * page 818; figure 2 * ----- | 1-15 | TECHNICAL FIELDS SEARCHED (IPC) C07D A61P |
| Y | WO 2022/235698 A1 (NURIX THERAPEUTICS INC [US]; WEGRZYNIAK ERIC [US]) 10 November 2022 (2022-11-10) * abstract * * paragraph [0184] * * examples 1-102 * * claims 1-315 * ----- -/-- | 1-15 | |

The present search report has been drawn up for all claims

1

| Place of search | Date of completion of the search | Examiner |
|---|---|---|
| Munich | 13 July 2023 | Dunet, Guillaume |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 23 16 1182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2022/086937 A1 (ICAHN SCHOOL MED MOUNT SINAI [US]; VAN ANDEL RES INSTITUTE [US]) 28 April 2022 (2022-04-28)<br>* abstract *<br>* examples 1-351 *<br>* compounds *;<br>pages 294-368<br>* claims 1-18 *<br>----- | 1-15 | |
| Y | WO 2022/240830 A1 (BRIDGE MEDICINES [US]) 17 November 2022 (2022-11-17)<br>* abstract *<br>* examples 1-107 *<br>* claims 1-33 *<br>----- | 1-15 | |
| Y | WO 2021/021904 A1 (SCRIPPS RESEARCH INST [US]) 4 February 2021 (2021-02-04)<br>* abstract *<br>* examples 1-9 *<br>* claims 1-20 *<br>----- | 1-15 | |
| X | WO 2023/283372 A1 (BIOGEN MA INC [US]; C4 THERAPEUTICS INC [US])<br>12 January 2023 (2023-01-12)<br>* abstract *<br>* examples 1-87 *<br>* claims 1-58 *<br>----- | 1-3,<br>11-15 | TECHNICAL FIELDS SEARCHED (IPC) |
| X | WO 2023/283610 A1 (BIOGEN MA INC [US]; C4 THERAPEUTICS INC [US])<br>12 January 2023 (2023-01-12)<br>* abstract *<br>* examples 1-199 *<br>* claims 1-62 *<br>----- | 1-3,<br>11-15 | |

The present search report has been drawn up for all claims

1

| Place of search | Date of completion of the search | Examiner |
|---|---|---|
| Munich | 13 July 2023 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 16 1182

This annex lists the patent family members relating to the patent documents cited in the above-mentioned European search report. The members are as contained in the European Patent Office EDP file on
The European Patent Office is in no way liable for these particulars which are merely given for the purpose of information.

13-07-2023

| Patent document cited in search report | Publication date | Patent family member(s) | Publication date |
|---|---|---|---|
| WO 2022235698 A1 | 10-11-2022 | US 2023158151 A1 | 25-05-2023 |
| | | WO 2022235698 A1 | 10-11-2022 |
| WO 2022086937 A1 | 28-04-2022 | NONE | |
| WO 2022240830 A1 | 17-11-2022 | NONE | |
| WO 2021021904 A1 | 04-02-2021 | US 2022274980 A1 | 01-09-2022 |
| | | WO 2021021904 A1 | 04-02-2021 |
| WO 2023283372 A1 | 12-01-2023 | NONE | |
| WO 2023283610 A1 | 12-01-2023 | UY 39844 A | 31-01-2023 |
| | | WO 2023283610 A1 | 12-01-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020041331 A
- WO 2019140003 A
- WO 2013106643 A
- US 20160045607 A
- WO 2014187777 A
- US 20140356322 A
- US 9249153 A
- US 20160058872 A
- US 20150291562 A


### Non-patent literature cited in the description


- *Cell,* 2013, vol. 153, 17
- *Cell,* 2017, vol. 168, 629
- *Cell Mol Life Sci,* 2018, vol. 75, 3931
- *Nat Rev Mol Cell Biol,* 2012, vol. 13, 543
- *Nature,* 2020, vol. 577, 121
- *Nature,* 2017, vol. 543, 270
- *New Engl J Med,* 2016, vol. 374, 2209
- *Cancer Disc,* 2022, vol. 12, 2684
- *Cell Rep.,* 16 February 2021, vol. 34 (7), 108749
- *ACS Cent Sci,* 2021, vol. 7, 815
- *Angew. Chem. Int. Ed.,* 2018, vol. 57, 16302
- **GREG T. HERMANSON.** Bioconjugate Techniques. Academic Press Inc, 1996
- **GU et al.** *BioEssays,* 2018, vol. 40, 1700247
- **SUN et al.** *Signal Transduction and Targeted Therapy,* 2019, vol. 4, 64
- **WINTER et al.** *Science,* 19 June 2015, 1376
- *Cell Rep,* 2021, vol. 16, 1087
- **EISENHAUER et al.** *European Journal of Cancer,* 2009, vol. 45, 228-247
- *Blood,* 2007, vol. 109, 1815
- *Leukemia Res,* 2018, vol. 68, 32
- *CHEMICAL ABSTRACTS,* 145681-01-2
- *CHEMICAL ABSTRACTS,* 1072-97-5
- *CHEMICAL ABSTRACTS,* 944401-69-8
- *CHEMICAL ABSTRACTS,* 167423-93-0
- *CHEMICAL ABSTRACTS,* 1217525-04-6
- *CHEMICAL ABSTRACTS,* 1248546-01-1
- *CHEMICAL ABSTRACTS,* 362706-26-1
- *CHEMICAL ABSTRACTS,* 897046-42-3
- *CHEMICAL ABSTRACTS,* 73323-65-6
- *CHEMICAL ABSTRACTS,* 59489-71-3
- *CHEMICAL ABSTRACTS,* 74290-67-8
- *CHEMICAL ABSTRACTS,* 1041205-25-7
- *CHEMICAL ABSTRACTS,* 4433-63-0
- *CHEMICAL ABSTRACTS,* 411235-57-9
- *CHEMICAL ABSTRACTS,* 1176754-31-6
- *CHEMICAL ABSTRACTS,* 1059549-72-2
- *CHEMICAL ABSTRACTS,* 123942-11-0
- *CHEMICAL ABSTRACTS,* 57268-16-3
- *CHEMICAL ABSTRACTS,* 505083-04-5
- *CHEMICAL ABSTRACTS,* 14508-49-7
- *CHEMICAL ABSTRACTS,* 3034-55-7
- *CHEMICAL ABSTRACTS,* 874219-35-9
- *CHEMICAL ABSTRACTS,* 111600-83-0
- *CHEMICAL ABSTRACTS,* 1722-12-9
- *CHEMICAL ABSTRACTS,* 1019918-39-8
- *CHEMICAL ABSTRACTS,* 1118-61-2
- *CHEMICAL ABSTRACTS,* 1092351-82-0
- *CHEMICAL ABSTRACTS,* 14678-02-5
- *CHEMICAL ABSTRACTS,* 65260-58-4
- *CHEMICAL ABSTRACTS,* 1129-35-7
- *CHEMICAL ABSTRACTS,* 1785427-70-4
- *CHEMICAL ABSTRACTS,* 90924-43-9
- *CHEMICAL ABSTRACTS,* 16209-00-0
- *CHEMICAL ABSTRACTS,* 68867-17-4
- *CHEMICAL ABSTRACTS,* 160388-53-4
- *CHEMICAL ABSTRACTS,* 264264-33-7
- *CHEMICAL ABSTRACTS,* 101667-97-4
- *CHEMICAL ABSTRACTS,* 691363-11-8
- *CHEMICAL ABSTRACTS,* 106833-79-8
- *CHEMICAL ABSTRACTS,* 1234616-41-1
- *CHEMICAL ABSTRACTS,* 42409-58-5
- *CHEMICAL ABSTRACTS,* 158980-21-3
- *CHEMICAL ABSTRACTS,* 861657-91-2
- *CHEMICAL ABSTRACTS,* 203869-80-1
- *CHEMICAL ABSTRACTS,* 181955-79-3
- *CHEMICAL ABSTRACTS,* 1530-32-1
- *CHEMICAL ABSTRACTS,* 194924-96-4
- *CHEMICAL ABSTRACTS,* 1613115-21-1
- *CHEMICAL ABSTRACTS,* 681128-50-7
- *CHEMICAL ABSTRACTS,* 71432-55-8

- *CHEMICAL ABSTRACTS,* 135042-17-0
- *CHEMICAL ABSTRACTS,* 1845754-43-9
- *CHEMICAL ABSTRACTS,* 1454843-78-7
- *CHEMICAL ABSTRACTS,* 1185767-06-9
- *CHEMICAL ABSTRACTS,* 128811-48-3
- *CHEMICAL ABSTRACTS,* 114676-69-6
- *CHEMICAL ABSTRACTS,* 201286-95-5
- *CHEMICAL ABSTRACTS,* 142356-35-2
- *CHEMICAL ABSTRACTS,* 835616-60-9
- *CHEMICAL ABSTRACTS,* 6757-31-9
- *CHEMICAL ABSTRACTS,* 10436-25-6
- *CHEMICAL ABSTRACTS,* 1448189-80-7
- *CHEMICAL ABSTRACTS,* 60142-89-4